(19) Europäisches Patentamt
European Patent Office
Office européen des brevets

(11) EP 1 312 682 A1

(12) **EUROPEAN PATENT APPLICATION**
published in accordance with Art. 158(3) EPC

(43) Date of publication:
**21.05.2003 Bulletin 2003/21**

(21) Application number: **01956988.8**

(22) Date of filing: **21.08.2001**

(51) Int Cl.[7]: **C12Q 1/68**, C12N 15/09

(86) International application number:
**PCT/JP01/07139**

(87) International publication number:
**WO 02/016639 (28.02.2002 Gazette 2002/09)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **23.08.2000 JP 2000251981**
**19.09.2000 JP 2000284419**
**22.09.2000 JP 2000288750**
**03.04.2001 JP 2001104191**

(83) **Declaration under Rule 28(4) EPC (expert solution)**

(71) Applicant: **TAKARA BIO INC.**
**Otsu-shi, Shiga 520-2193 (JP)**

(72) Inventors:
- **SAGAWA, Hiroaki**
**Kusatsu-shi, Shiga 525-0025 (JP)**
- **UEMORI, Takashi**
**Otsu-shi, Shiga 520-2141 (JP)**
- **MUKAI, Hiroyuki**
**Moriyama-shi, Shiga 524-0102 (JP)**
- **YAMAMOTO, Junko**
**Moriyama-shi, Shiga 524-0044 (JP)**
- **TOMONO, Jun;**
**Kusatsu-shi, Shiga 525-0025 (JP)**
- **KOBAYASHI, Eiji**
**Otsu-shi, Shiga 520-2153 (JP)**
- **ENOKI, Tatsuji**
**Otsu-shi, Shiga 520-0865 (JP)**
- **ASADA, Kiyozo**
**Koka-gun, Shiga 520-3333 (JP)**
- **KATO, Ikunoshin**
**Uji-shi, Kyoto 611-0028 (JP)**

(74) Representative: **Grund, Martin, Dr. et al**
**Dr. Volker Vossius**
**Patentanwaltskanzlei**
**Geibelstrasse 6**
**81679 München (DE)**

# (54) METHOD OF AMPLIFYING NUCLEIC ACID

(57) A method of highly sensitively and specifically amplifying a target nucleic acid in a sample by using a chimeric oligonucleotide primer having a ribonucleotide provided at the 3'-terminus or in the 3'-terminal side, an endoribonuclease and a DNA polymerase having a chain transfer activity, i.e., an isothermal and chimeric primer-initiated amplification of nucleic acids (ICAN) method; a method of detecting an amplified fragment obtained by using the above method; a process for producing a target nucleic acid by using the above amplification method; and chimeric oligonucleotide primers to be used in these methods.

Fig.35

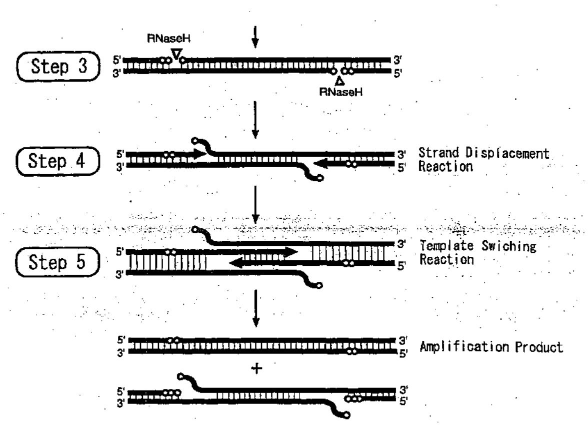


EP 1 312 682 A1

## Description

Technical Field

The present invention relates to a method for detecting a target nucleic acid which is useful in a field of clinical medicine and a method for synthesizing a DNA which is useful in a field of genetic engineering. It relates to a method for amplifying a nucleic acid as a template and a method for detecting a target nucleic acid amplified by said method.

Background Art

DNA synthesis is used for various purposes in studies in a field of genetic engineering. Most of the DNA synthesis with the exception of that of a short-chain DNA such as an oligonucleotide is. carried out by enzymatic methods in which a DNA polymerase is utilized. An example of the methods is the polymerase chain reaction (PCR) method as described in United States Patent Nos. 4,683,195, 4,683,202 and 4,800,159 in detail. Another example is the reverse transcription-PCR (RT-PCR) method, which is a combination of the PCR and a reverse transcriptase reaction, as described in Trends in Biotechnology, 10:146-152 (1992). The development of the above-mentioned methods has enabled the amplification of a region of interest from a DNA or an RNA.

The above-mentioned DNA synthesis methods are conducted, for example, using a reaction that consists of three steps. The three steps are a step of dissociating (denaturing) a double-stranded DNA into single-stranded DNAs, a step of annealing a primer to the single-stranded DNA and a step of synthesizing (extending) a complementary strand from the primer in order to amplify a region of a DNA of interest. Alternatively, they.are conducted using a reaction designated as "the shuttle PCR" ("PCR hou saizensen" (Recent advances in PCR methodology), Tanpakushitsu Kakusan Kouso, Bessatsu, (Protein, Nucleic Acid and Enzyme, Supplement), 41(5) : 425-428 (1996)) in which two of the three steps, that is, the step of annealing the primer and the step of extending are carried out at the same temperature.

Alternatively, the ligase chain reaction (LCR) method as described in EP 320,308 published on June 14, 1989 or the transcription-based amplification system (TAS) method as described in PCR Protocols, Academic Press Inc., 1990, pp. 245-252 may be used. The four methods as mentioned above require repeating a reaction at a high temperature and that at a low temperature several times in order to regenerate a single-stranded target molecule for the next amplification cycle. The reaction system should be conducted using discontinuous phases or cycles because the reaction is restricted by temperature as described above.

Thus, the methods require the use of an expensive thermal cycler that can strictly adjust a wide range of temperature over time. Furthermore, the reaction requires time for adjusting the temperature to the two or three predetermined ones. The loss of time increases in proportion to the cycle number.

Nucleic acid amplification methods that can be carried out isothermally have been developed in order to solve the problems. Examples thereof include the strand displacement amplification (SDA) method as described in JP-B 7-114718, the self-sustained sequence replication (3SR) method, the nucleic acid sequence based amplification (NASBA) method as described in Japanese Patent No. 2650159, the transcription-mediated amplification (TMA) method, the Qβ replicase method as described in Japanese Patent No. 2710159 and the various modified SDA methods as described in United States Patent No. 5,824,517, WO 99/09211, WO 95/25180 and WO 99/49081. A method of isothermal enzymatic synthesis of an oligonucleotide is described in United States Patent No. 5,916,777. Extension from a primer and/or annealing of a primer to a single-stranded extension product (or to an original target sequence) followed by extension from the primer take place in parallel in a reaction mixture incubated at a constant temperature in the reaction of these methods of isothermal nucleic acid amplification or synthesis of an oligonucleotide.

Among the isothermal nucleic acid amplification methods, the SDA method is an example of systems in which a DNA is finally amplified. The SDA method is a method for amplifying a target nucleic acid sequence (and a complementary strand thereof) in a sample by.displacement of double strands using a DNA polymerase and a restriction endonuclease. The method requires four primers used for the amplification, two of which should be designed to contain a recognition site for the restriction endonuclease. The method requires the use of a modified deoxyribonucleotide triphosphate as a substrate for DNA synthesis in large quantities., An example of the modified deoxyribonucleotide triphosphates is an ($\alpha$-S) deoxyribonucleotide triphosphate in which the oxygen atom of the phosphate group at the $\alpha$-position is replaced by a sulfur atom (S). The problem of running cost associated with the use of the modified 'deoxyribonucleotide triphosphate becomes serious if the reaction is routinely conducted, for example, for genetic test. Furthermore, the incorporation of the modified nucleotide such as the ($\alpha$-S) deoxyribonucleotide into the amplified DNA fragment in the method may abolish the cleavability of the amplified DNA fragment with a restriction enzyme, for example, when it is subjected to a restriction enzyme fragment length polymorphism (RFLP) analysis.

The modified SDA method as described in United States Patent No. 5,824,517 is a DNA amplification method that uses a chimeric primer that is composed of an RNA and a DNA and has as an essential element a structure in which DNA is positioned at least at the 3'-terminus. The modified SDA method as described in WO 99/09211 requires the use of a restriction enzyme that generates a 3'-protruding end. The modified SDA method as described in WO 95/25180 requires the use of at least two pairs of primers. The modified SDA method as described in WO 99/49081 requires the use of at least two pairs of primers and at least one modified deoxyribonucleotide triphosphate. On the other hand, the method for synthesizing an oligonucleotide as described in United States Patent No. 5,916,777 comprises synthesizing a DNA using a primer having a ribonucleotide at the 3'-terminus, completing a reaction using the primer, introducing a nick between the primer and an extended strand in an primer-extended strand with an endonuclease to separate them, digesting a template and recovering the primer to reuse it. It is required to isolate the primer from the reaction system and then anneal it to the template again in order to reuse the primer in the method. Additionally, the Loop-mediated Isothermal Amplification (LAMP) method as described in WO 00/28082 requires four primers for amplification and the products amplified using the method are DNAs having varying size in which the target regions for the amplification are repeated.

As described above, the conventional isothermal nucleic acid amplification methods still have various problems. Thus, a method for amplifying a nucleic acid at low running cost by which a DNA fragment that can be further genetically engineered is obtained has been desired.

Objects of Invention

The main object of the present invention is to provide a method for amplifying a target nucleic acid which specifically amplifies with high sensitivity a target nucleic acid in a sample by DNA synthesis reaction using a chimeric oligonucleotide primer, a method for detecting a amplified fragment obtained by said method, a method for producing a target nucleic acid using said amplification method and a chimeric oligonucleotide primer used for these methods.

Summary of Invention

As a result of intensive studies, the present inventors have constructed an excellent system for gene amplification reaction. The construction was accomplished by developing a method in which a region of a DNA of interest is amplified in the presence of a chimeric oligonucleotide primer having a ribonucleotide positioned at the 3'-terminus or on the 3'-terminal side, an endonuclease and a DNA polymerase. Thus, the present invention has been completed. The method is a method for amplifying a nucleic acid in which a chimeric oligonucleotide primer is used and herein referred to as ICAN (Isothermal and Chimeric primer-initiated Amplification of Nucleic acids) method.

The first aspect of the present, invention relates to a method for amplifying a target nucleic acid, the method comprising:

(a) preparing a reaction mixture by mixing a nucleic acid as a template, a deoxyribonucleotide triphosphate, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer; and

(b) incubating the reaction mixture for a sufficient time to generate a reaction product.

In the first aspect of the present invention, a reaction mixture that further contains a chimeric oligonucleotide primer having a sequence that is substantially homologous to the nucleotide sequence of the nucleic acid as the template can be used.

The second aspect of the present invention relates to a method for amplifying a nucleic acid, the method comprising:

(a) treating a nucleic acid as a template with at least one primer that is substantially complementary to the nucleotide sequence of the nucleic acid and a DNA polymerase to synthesize a primer-extended strand that is complementary to the template and synthesize a double-stranded nucleic acid, wherein the primer is a chimeric oligonucleotide primer containing a ribonucleotide as well as at least one selected from the group consisting of a deoxyribonucleotide and a nucleotide analog, the ribonucleotide being positioned at the 3'-terminus or on the 3'-terminal side of the primer;

(b) extending a nucleotide sequence that is complementary to the double-stranded nucleic acid as a template obtained in the previous step using a DNA polymerase having a strand displacement activity in the presence of an RNase H to effect a strand displacement and synthesize a displaced strand and a double-stranded nucleic acid; and

(c) reusing in step (b) the double-stranded nucleic acid obtained in step (b) as a template.

The third aspect of the present invention relates to a method for amplifying a nucleic acid using at least two primers, the method comprising:

(a) treating a nucleic acid as a template with at least one primer that is substantially complementary to the nucleotide sequence of the nucleic acid and a DNA polymerase to synthesize a primer-extended strand that is complementary to the template, wherein the primer is a chimeric oligonucleotide primer containing a ribonucleotide as well as at least one selected from the group consisting of a deoxyribonucleotide and a nucleotide analog, the ribonucleotide being positioned at the 3'-terminus or on the 3'-terminal side of the primer;
(b) extending a nucleotide sequence that is complementary to the double-stranded nucleic acid as a template obtained in the previous step using a DNA polymerase having a strand displacement activity in the presence of an RNase H to effect a strand displacement and synthesize a displaced strand and a double-stranded nucleic acid;
(c) reusing in step (b) the double-stranded nucleic acid obtained in step (b) as a template;
(d) treating a displaced strand obtained in step (b) as a template with at least one primer that is different from that used in step (a) and a DNA polymerase to synthesize a primer-extended strand that is complementary to the displaced strand, wherein the primer that is different from that used in step (a) is a chimeric oligonucleotide primer that is substantially complementary to the nucleotide sequence of the displaced strand and contains a ribonucleotide as well as at least one selected from the group consisting of a deoxyribonucleotide and a a nucleotide analog, the ribonucleotide being positioned at the 3'-terminus or on the 3'-terminal side of the primer;
(e) extending a nucleotide sequence that is complementary to the double-stranded nucleic acid as a template obtained in the previous step using a DNA polymerase having a strand displacement activity in the presence of an RNase H to effect a strand displacement and synthesize a displaced strand and a double-stranded nucleic acid; and
(f) reusing in step (e) the double-stranded nucleic acid obtained in step (e) as a template.

In the second or third aspect of the present invention, a DNA polymerase having a strand displacement activity can be used as the DNA polymerase.
The fourth aspect of the present invention relates to method for amplifying a nucleic acid, the method comprising:

(a) treating a double-stranded nucleic acid as a template with two primers that are substantially complementary to the nucleotide sequences of the respective strands of the double-stranded nucleic acid and a DNA polymerase having a strand displacement activity to synthesize primer-extended strands that are complementary to the template and obtain a double-stranded nucleic acid consisting of the synthesized primer-extended strands being annealed each other, wherein each primer is a chimeric oligonucleotide primer containing a ribonucleotide as well as at least one selected from the group consisting of a deoxyribonucleotide and a nucleotide analog, the ribonucleotide being positioned at the 3'-terminus or on the 3'-terminal side of the primer;
(b) cleaving the double-stranded nucleic acid consisting of the primer-extended strands obtained in step (a) with the endonuclease at sites that contain the ribonucleotides; and
(c) extending nucleotide sequences that are complementary to the template using a DNA polymerase having a strand displacement activity from the 3'-termini of the respective primer portions of the double-stranded nucleic acid in which the primer-extended strands are cleaved obtained in step (b) to effect strand displacements and obtain a double-stranded nucleic acid consisting of the template and the primer-extended strand.

The fifth aspect of the present invention relates to a method for amplifying a nucleic acid, the method comprising:

(a) treating a double-stranded nucleic acid as a template with two primers that are substantially complementary to the nucleotide sequences of the respective strands of the double-stranded nucleic acid and a DNA polymerase having a strand displacement activity to synthesize primer-extended strands that are complementary to the template and obtain a double-stranded nucleic acid consisting of the synthesized primer-extended strands being annealed each other, wherein each primer is a chimeric oligonucleotide primer containing a ribonucleotide as well as at least one selected from the group consisting of a deoxyribonucleotide and a nucleotide analog, the ribonucleotide being positioned at the 3'-terminus or on the 3'-terminal side of the primer;
(b) cleaving the double-stranded nucleic acid consisting of the primer-extended strands obtained in step (a) with the endonuclease at sites that contain the ribonucleotides; and
(c) extending nucleotide sequences that are complementary to the template using a DNA polymerase having a strand displacement activity from the 3'-termini of the respective primer portions of the double-stranded nucleic acid in which the primer-extended strands are cleaved obtained in step (b) to effect strand displacements and obtain a double-stranded nucleic acid consisting of the primer-extended strands being annealed each other.

The sixth aspect of the present invention relates to a method for amplifying a nucleic acid, the method comprising:

(a) treating a double-stranded nucleic acid as a template with two primers that are substantially complementary to the nucleotide sequences of the respective strands of the double-stranded nucleic acid and a DNA polymerase having a strand displacement activity to synthesize primer-extended strands that are complementary to the template and obtain a double-stranded nucleic acid consisting of the synthesized primer-extended strands being annealed each other, wherein each primer is a chimeric oligonucleotide primer containing a ribonucleotide as well as at least one selected from the group consisting of a deoxyribonucleotide and a nucleotide analog, the ribonucleotide being positioned at the 3'-terminus or on the 3'-terminal side of the primer;
(b) cleaving the double-stranded nucleic acid consisting of the primer-extended strands obtained in step (a) with the endonuclease at sites that contain the ribonucleotides;
(c) extending nucleotide sequences that are complementary to the template using a DNA polymerase having a strand displacement activity from the 3'-termini of the respective primer portions of the double-stranded nucleic acid in which the primer-extended strands are cleaved obtained in step (b) to effect strand displacements and obtain a double-stranded nucleic acid consisting of the primer-extended strands being annealed each other and a double-stranded nucleic acid consisting of the templates being annealed each other to which the two primers in step (a) are annealed;
(d) extending nucleotide sequences that are complementary to the template using a DNA polymerase having a strand displacement activity from the 3'-termini of the respective primer portions of the double-stranded nucleic acid to which the two primers are annealed obtained in step (c) to effect strand displacements and obtain a double-stranded nucleic acid consisting of the primer-extended strands being annealed each other and a double-stranded nucleic acid consisting of the templates being annealed each other to which the two primers in step (a) are annealed; and
(e) reusing in step (d) the double-stranded nucleic acid to which the two primers are annealed obtained in step (d).

The seventh aspect of the present invention relates to a method for amplifying a nucleic acid, the method comprising:

(a) treating a double-stranded nucleic acid as a template with two primers that are substantially complementary to the nucleotide sequences of the respective strands of the double-stranded nucleic acid and a DNA polymerase having a strand displacement activity to synthesize primer-extended strands that are complementary to the template and obtain a double-stranded nucleic acid consisting of the synthesized primer-extended strands being annealed each other, wherein each primer is a chimeric oligonucleotide primer containing a ribonucleotide as well as at least one selected from the group consisting of a deoxyribonucleotide and a nucleotide analog, the ribonucleotide being positioned at the 3'-terminus or on the 3'-terminal side of the primer;
(b) cleaving the double-stranded nucleic acid consisting of the primer-extended strands obtained in step (a) with the endonuclease at sites that contain the ribonucleotides;
(c) extending nucleotide sequences that are complementary to the template using a DNA polymerase having a strand displacement activity from the 3'-termini of the respective primer portions of the double-stranded nucleic acid in which the primer-extended strands are cleaved obtained in step (b) to effect strand displacements and obtain a double-stranded nucleic acid consisting of the primer-extended strands being annealed each other and a double-stranded nucleic acid consisting of the templates being annealed each other to which the two primers in step (a) are annealed;
(d) extending nucleotide sequences that are complementary to the template using a DNA polymerase having a strand displacement activity from the 3'-termini of the respective primer portions of the double-stranded nucleic acid to which the two primers are annealed obtained in step (c) to effect a strand displacement and obtain a double-stranded nucleic acid consisting of the template and the primer-extended strand;
(e) cleaving the double-stranded nucleic acid consisting of the template and the primer-extended strand obtained in step (d) with the endonuclease at a site that contains the ribonucleotide; and
(f) extending a nucleotide sequence that is complementary to the template using a DNA polymerase having a strand displacement activity from the 3'-terminus of the primer portion of the double-stranded nucleic acid in which the primer-extended strand is cleaved obtained in step (e) to synthesize a displaced strand.

In the fourth to seventh aspect, an endoribonuclease such as an RNase H can be used as the endonuclease.
In the first to seventh aspect wherein an RNase H is used, an RNase H from Escherichia coli, a bacterium of genus Thermotoga, a bacterium of genus Thermus, a bacterium of genus Pyrococcus, a bacterium of genus Archaeoglobus, a bacterium of genus Bacillus or the like can be used.

In the first to seventh aspect, an example of the suitable length of the region to be specifically amplified in the nucleotide sequence of the target nucleic acid is 200 bp or shorter.

For the first to seventh aspect of the present invention, a chimeric oligonucleotide primer represented by general formula below can be used:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns, and the nucleotide at the 3'-terminus may be modified such that extension from the 3'-terminus by the action of the DNA polymerase does not take place).

Such chimeric oligonucleotide primers are exemplified by a primer in which c is 0 and a primer in which the nucleotide analog is deoxyriboinosine nucleotide or deoxyribouracil nucleotide, and the modified ribonucleotide is ($\alpha$-S) ribonucleotide. Furthermore, when such a chimeric oligonucleotide primer is used, the DNA extension reaction is conducted at a DNA extension reaction temperature suitable for the primer.

The amplification method of the first to seventh aspect can comprise annealing the nucleic acid as the template to the chimeric oligonucleotide primer that is substantially complementary to the nucleotide sequence of the nucleic acid in an annealing solution containing a substance that enhances the annealing of the nucleic acid to the primer. For example, the annealing solution may contain spermidine and/or propylenediamine. The annealing can be conducted by incubating the annealing solution containing the nucleic acid as the template and the chimeric oligonucleotide primer that is substantially complementary to the nucleotide sequence of the nucleic acid at 90°C or above and then cooling the solution to a temperature at which the amplification reaction is conducted or below.

The amplification reaction of the first to seventh aspect can be conducted in a buffer containing a buffering component selected from the group consisting of Bicine and HEPES.

In the first to seventh aspect, for example, a DNA polymerase selected from the group consisting of Klenow fragment of DNA polymerase I from Escherichia coli, Bst DNA polymerase lacking 5'→3' exonuclease from Bacillus stearothermophilus and Bca DNA polymerase lacking 5'→3' exonuclease from Bacillus caldotenax can be used as the DNA polymerase having a strand displacement activity.

In one embodiment of the first to seventh aspect, Bca DNA polymerase lacking 5'→3' exonuclease from Bacillus caldotenax is used as the DNA polymerase having a strand displacement activity and an RNase H from Escherichia coli, a bacterium of genus Pyrococcus or a bacterium of genus Archaeoglobus is used as the endonuclease. The RNase H is exemplified by type I RNase H from Escherichia coli, or type II RNase H from a bacterium of genus Pyrococcus or a bacterium of genus Archaeoglobus.

In the first to seventh aspect, a DNA polymerase having an endonuclease activity can be used. Bca DNA polymerase lacking 5'→3' exonuclease from Bacillus caldotenax can be used as such a DNA polymerase and the amplification reaction can be conducted in the presence of a substance that allows the endonuclease activity of the DNA polymerase to express. The substance that allows the endonuclease activity of the Bca DNA polymerase to express is exemplified by a manganese ion.

The method for amplifying a target nucleic acid of the first to seventh aspect can be conducted in the presence of a substance that inhibits the reverse transcription activity of the DNA polymerase. The substance that inhibits the reverse transcription activity of the DNA polymerase is exemplified by phosphonoformic acid.

The first to seventh aspect of the present invention can be conducted using a single-stranded DNA or a double-stranded DNA as the template. If the nucleic acid as the template is a double-stranded DNA, the amplification reaction can be conducted after converting it into single-stranded DNAs.

The nucleic acid as the template may be a cDNA obtained by a reverse transcription reaction using an RNA as a template. In one aspect, the amplification reaction is conducted after synthesizing a cDNA by a reverse transcription reaction using an RNA as a template. A DNA polymerase having a reverse transcriptase activity can be used for the reverse transcription reaction. For example, the reverse transcription reaction and the synthesis of the extended strand that is complementary to the template can be conducted using one DNA polymerase having a reverse transcriptase activity and a strand displacement activity. Such a DNA polymerase is exemplified by Bst DNA polymerase lacking 5'→3' exonuclease from Bacillus stearothermophilus or Bca DNA polymerase lacking 5'→3' exonuclease from Bacillus caldotenax.

In the first to seventh aspect, the amplification reaction can be conducted under isothermal conditions. It can be conducted in the presence of a deoxynucleotide triphosphate analog such as deoxyuridine triphosphate or a derivative thereof.

The eighth aspect of the present invention relates to a composition for amplifying a nucleic acid which contains:

(a) at least one primer that is substantially complementary to a nucleotide sequence of a nucleic acid as a template, wherein the primer is a chimeric oligonucleotide primer that contains a ribonucleotide as well as at least one selected from the group consisting of a deoxyribonucleotide and a nucleotide analog, the ribonucleotide being positioned at the 3'-terminus or on the 3'-terminal side of the primer;
(b) an endonuclease; and
(c) a DNA polymerase having a strand displacement activity.

The ninth aspect of the present invention relates to a composition for amplifying a nucleic acid which contains:

(a) at least two primers that are substantially complementary to nucleotide sequences of respective strands of a double-stranded nucleic acid as a template, wherein each primer is a chimeric oligonucleotide primer that contains a ribonucleotide as well as at least one selected from the group consisting of a deoxyribonucleotide and a nucleotide analog, the ribonucleotide being positioned at the 3'-terminus or on the 3'-terminal side of the primer;
(b) an endonuclease; and
(c) a DNA polymerase having a strand displacement activity.

The tenth aspect of the present invention relates to a composition for amplifying a nucleic acid obtained by mixing a nucleic acid as a template, a deoxyribonucleotide triphosphate, a DNA polymerase having a strand displacement activity, at least one primer and an endonuclease, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer.
The eleventh aspect of the present invention relates to a composition for amplifying a nucleic acid obtained by mixing a nucleic acid as a template, a deoxyribonucleotide triphosphate, a DNA polymerase having a strand displacement activity, at least two primers and an endonuclease, wherein each primer is a chimeric oligonucleotide primer that is substantially complementary to the nucleotide sequence of each strand of the double-stranded nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer.
The primer contained in the composition of the eighth to eleventh aspect of the present invention is exemplified by a chimeric oligonucleotide primer represented by general formula below:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns, and the nucleotide at the 3'-terminus may be modified such that extension from the 3'-terminus by the action of the DNA polymerase does not take place).
Such chimeric oligonucleotide primers are exemplified by a primer in which c is 0 and a primer in which the nucleotide analog is deoxyriboinosine nucleotide or deoxyribouracil nucleotide, and the modified ribonucleotide is ($\alpha$-S) ribonucleotide.
The composition of the eighth to eleventh aspect can contain a buffering component suitable for a nucleic acid amplification reaction. For example, it can contain a buffering component selected from the group consisting of Bicine and HEPES.
The eighth to eleventh aspect is exemplified by a composition containing a DNA polymerase selected from the group consisting of Klenow fragment of DNA polymerase I from Escherichia coli, Bst DNA polymerase lacking 5'$\rightarrow$3' exonuclease from Bacillus stearothermophilus and Bca DNA polymerase lacking 5'$\rightarrow$3' exonuclease from Bacillus caldotenax as the DNA polymerase having a strand displacement activity. An endoribonuclease such as an RNase H can be used as the endonuclease. The RNase H is exemplified by an RNase H from Escherichia coli, a bacterium of genus Thermotoga, a bacterium of genus Thermus, a bacterium of genus Pyrococcus, a bacterium of genus Archaeoglobus or a bacterium of genus Bacillus.
In one embodiment, the composition of the eighth to eleventh aspect contains Bca DNA polymerase lacking 5'$\rightarrow$3' exonuclease from Bacillus caldotenax as the DNA polymerase having a strand displacement activity and an RNase H from Escherichia coli, a bacterium of genus Pyrococcus or a bacterium of genus Archaeoglobus as the endonuclease. The RNase H is exemplified by type I RNase H from Escherichia coli, or type II RNase H from a bacterium of genus Pyrococcus or a bacterium of genus Archaeoglobus.

The composition of the eighth to eleventh aspect may contain a DNA polymerase having an endonuclease activity. Bca DNA polymerase lacking 5'→3' exonuclease from Bacillus caldotenax can be used as such a DNA polymerase, which can be used in the presence of a substance that allows the endonuclease activity of the DNA polymerase to express. The substance that allows the endonuclease activity of the Bca DNA polymerase to express is exemplified by a manganese ion.

The composition of the eighth to eleventh aspect can contain a substance that inhibits the reverse transcription activity of the DNA polymerase. The substance that inhibits the reverse transcription activity of the DNA polymerase is exemplified by phosphonoformic acid. Furthermore, the composition may contain a deoxynucleotide triphosphate analog such as deoxyuridine triphosphate or a derivative thereof.

The twelfth aspect of the present invention relates to a composition for amplifying a nucleic acid used for the method for amplifying a nucleic acid of the first to third aspect, which contains:

(a) an RNase H; and
(b) a DNA polymerase having a strand displacement activity.

The thirteenth aspect of the present invention relates to a composition for amplifying a nucleic acid used for the method for amplifying a nucleic acid of the fourth to seventh aspect, which contains:

(a) an endonuclease; and
(b) a DNA polymerase having a strand displacement activity.

An endoribonuclease such as an RNase H can be used as the endonuclease for the composition of the thirteenth aspect.

An RNase H selected from those from Escherichia coli, a bacterium of genus Thermotoga, a bacterium of genus Thermus, a bacterium of genus Pyrococcus, a bacterium of genus Archaeoglobus and a bacterium of genus Bacillus can be used as the RNase H for the composition of the twelfth or thirteenth aspect which contains an RNase H.

The composition of the twelfth or thirteenth aspect can further contain a buffering -component suitable for a nucleic acid amplification reaction. For example, the composition may contain a buffering component selected from the group consisting of Bicine and HEPES.

The twelfth or thirteenth aspect is exemplified by a composition that contains a DNA polymerase selected from the group consisting of Klenow fragment of DNA polymerase I from Escherichia coli, Bst DNA polymerase lacking 5'→3' exonuclease from Bacillus stearothermophilus and Bca DNA polymerase lacking 5'→3' exonuclease from Bacillus caldotenax as the DNA polymerase having a strand displacement activity. An endoribonuclease such as an RNase H can be used as the endonuclease. The RNase H is exemplified by an RNase H from Escherichia coli, a bacterium of genus Thermotoga, a bacterium of genus Thermus, a bacterium of genus Pyrococcus, a bacterium of genus Archaeoglobus or a bacterium of genus Bacillus.

In one embodiment, the composition of the twelfth or thirteenth aspect contains Bca DNA polymerase lacking 5'→3' exohuclease from Bacillus caldotenax as the DNA polymerase having a strand displacement activity and an RNase H from Escherichia coli, a bacterium of genus Pyrococcus or a bacterium of genus Archaeoglobus as the endonuclease. The RNase H is exemplified by type I RNase H from Escherichia coli, or type II RNase H from a bacterium of genus Pyrococcus or a bacterium of genus Archaeoglobus.

The composition of the twelfth or thirteenth aspect may contain a DNA polymerase having an endonuclease activity. Bca DNA polymerase lacking 5'→3' exonuclease from Bacillus caldotenax can be used as such a DNA polymerase, which can be used in the presence of a substance that allows the endonuclease activity of the DNA polymerase to express. The substance that allows the endonuclease activity of the Bca DNA polymerase to express is exemplified by a manganese ion.

The composition of the twelfth or thirteenth aspect can contain a substance that inhibits the reverse transcription activity of the DNA polymerase. The substance that inhibits the reverse transcription activity of the DNA polymerase is exemplified by phosphonoformic acid. Furthermore, the composition may contain a deoxynucleotide triphosphate analog such as deoxyuridine triphosphate or a derivative thereof.

The fourteenth aspect of the present invention relates to a kit for amplifying a nucleic acid used for the method for amplifying a nucleic acid of the first to third aspect of the present invention, which contains:

(a) an RNase H; and
(b) a DNA polymerase having a strand displacement activity.

The fifteenth aspect of the present invention relates to a kit for amplifying a nucleic acid used for the method for amplifying a nucleic acid of the fourth to seventh aspect of the present invention, which contains:

(a) an endonuclease; and
(b) a DNA polymerase having a strand displacement activity.

An endoribonuclease such as an RNase H can be used as the endonuclease for the kit of the fifteenth aspect.
The kit of the fourteenth or fifteenth aspect which contains an RNase H is exemplified by a kit containing an RNase H selected from the group consisting of an RNase H from Escherichia coli, an RNase H from a bacterium of genus Thermotoga, an RNase H from a bacterium of genus Thermus, an RNase H from a bacterium of genus Pyrococcus, an RNase H from a bacterium of genus Archaeoglobus and an RNase H from a bacterium of genus Bacillus.
The kit of the fourteenth or fifteenth aspect may further contain a buffering component suitable for a nucleic acid amplification reaction. For example, the composition can contain a buffering component selected from the group consisting of Bicine and HEPES. The kit may contain an annealing solution containing a substance that enhances the annealing of the nucleic acid as the template to the primer that is substantially complementary to the nucleotide sequence of the nucleic acid. For example, the annealing solution may contain spermidine and/or propylenediamine.
The DNA polymerase having a strand displacement activity contained in the kit of the fourteenth or fifteenth aspect of the present invention is exemplified by a DNA polymerase selected from the group consisting of Klenow fragment of DNA polymerase I from Escherichia coli, Bst DNA polymerase lacking 5'→3' exonuclease from Bacillus stearothermophilus and Bca DNA polymerase lacking 5'→3' exonuclease from Bacillus caldotenax.
In one embodiment, the kit of the fourteenth or fifteenth aspect contains Bca DNA polymerase lacking 5'→3' exonuclease from Bacillus caldotenax and an RNase H from Escherichia coli, a bacterium of genus Pyrococcus or a bacterium of genus Archaeoglobus. The RNase H is exemplified by type I RNase H from Escherichia coli, or type II RNase H from a bacterium of genus Pyrococcus or a bacterium of genus Archaeoglobus.
The kit of the fourteenth or fifteenth aspect of the present invention may contain a DNA polymerase having an endonuclease activity. Bca DNA polymerase lacking 5'→3' exonuclease from Bacillus caldotenax can be used as such a DNA polymerase. The kit can contain a substance that allows the endonuclease activity of the DNA polymerase to express. The substance that allows the endonuclease activity of the Bca DNA polymerase to express is exemplified by a manganese ion.
The kit of the fourteenth or fifteenth aspect can contain a substance that inhibits the reverse transcription activity of the DNA polymerase. The substance that inhibits the reverse transcription activity of the DNA polymerase is exemplified by phosphonoformic acid. Furthermore, the kit may contain a deoxynucleotide triphosphate analog such as deoxyuridine triphosphate or a derivative thereof.
The sixteenth aspect of the present invention relates to a kit for amplifying a nucleic acid used for the method for amplifying a nucleic acid of the first to third aspect of the present invention, which is in a packaged form and contains instructions that direct the use of a DNA polymerase having a strand displacement activity and an RNase H.
The seventeenth aspect of the present invention relates to a kit for amplifying a nucleic acid used for the method for amplifying a nucleic acid of the fourth to seventh aspect of the present invention, which is in a packaged form and contains instructions that direct the use of a DNA polymerase having a strand displacement activity and an endonuclease.
The eighteenth aspect of the present invention relates to a product of a reagent for amplifying a nucleic acid consisting of a packing material and a reagent for amplifying a nucleic acid enclosed in the packing material, wherein the reagent for amplifying a nucleic acid contains a DNA polymerase having a strand displacement activity and/or an RNase H, and description that the reagent for amplifying a nucleic acid can be used for nucleic acid amplification under isothermal conditions is indicated in a label stuck to the packaging material or instructions attached to the packaging material.
The nineteenth aspect of the present invention relates to a product of a reagent for amplifying a nucleic acid consisting of a packing material and a reagent for amplifying a nucleic acid enclosed in the packing material, wherein the reagent for amplifying a nucleic acid contains a DNA polymerase having a strand displacement activity and/or an endonuclease, and description that the reagent for amplifying a nucleic acid can be used for nucleic acid amplification under isothermal conditions is indicated in a label stuck to the packaging material or instructions attached to the packaging material.
The twentieth aspect of the present invention relates to a method for detecting a target nucleic acid in a sample, the method comprising:

(a) amplifying a nucleic acid by the method for amplifying a nucleic acid of the first to seventh aspect of the present invention; and
(b) detecting a target nucleic acid amplified in step (a).

The detection method of the twentieth aspect of the present invention can comprise detecting the amplified nucleic acid using a probe for detection. The probe may be a probe that has been labeled with a labeling substance. For example, an RNA probe labeled with two or more fluorescent substances positioned at a distance that results in a quenching state can be used.
The twenty-first aspect of the present invention relates to a chimeric oligonucleotide primer used for the twentieth aspect. The chimeric oligonucleotide primer is exemplified by a chimeric oligonucleotide primer represented by general formula below:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns, and the nucleotide at the 3'-terminus may be modified such that extension from the 3'-terminus by the action of the DNA polymerase does not take place).
Such chimeric oligonucleotide primers are exemplified by a primer in which c is 0 and a primer in which the nucleotide analog is deoxyriboinosine nucleotide or deoxyribouracil nucleotide, and the modified ribonucleotide is ($\alpha$-S) ribonucleotide.
The primer of the twenty-first aspect of the present invention is exemplified by a primer for detecting a pathogenic microorganism or a disease-related gene. A chimeric oligonucleotide primer for detecting a pathogenic microorganism such as enterohemorrhagic Escherichia coli, Clostridium botulinum, Staphylococcus aureus, Mycobacterium tuberculosis, Chlamydia, papilloma virus, hepatitis C virus or a viroid is encompassed by the present invention.
The twenty-second aspect of the present invention relates to a chimeric oligonucleotide primer for detecting enterohemorrhagic Escherichia coli having a nucleotide sequence selected from the group consisting of SEQ ID NOS: 31-34, 47, 48, 51-53, 64-72, 84, 85, 113, 114, 130 and 131.
The twenty-third aspect of the present invention relates to a chimeric oligonucleotide primer for detecting a viroid having a nucleotide sequence selected from the group consisting of SEQ ID NOS: 59, 60, 119, 120, 122 and 123.
The twenty-fourth aspect of the present invention relates to a chimeric oligonucleotide primer for detecting Clostridium botulinum having a nucleotide sequence represented by SEQ ID NO: 116 or 117.
The twenty-fifth aspect of the present invention relates to chimeric oligonucleotide primer for detecting papilloma virus having a nucleotide sequence represented.by SEQ ID NO: 96 or 97.
The twenty-sixth aspect of the present invention relates to a chimeric oligonucleotide primer for detecting hepatitis C virus having a nucleotide sequence selected from the group consisting of SEQ ID NOS: 101, 102, 138, 139, 200, 201, 205 and 206.
The twenty-seventh aspect of the present invention relates to a chimeric oligonucleotide primer for detecting Staphylococcus aureus having a nucleotide sequence represented by SEQ ID NO: 136 or 137.
The twenty-eighth aspect of the present invention relates to a chimeric oligonucleotide primer for detecting Mycobacterium tuberculosis having a nucleotide sequence selected from the group consisting of SEQ ID NOS: 155, 156, 159 to 162, 194 and 195.
The twenty-ninth aspect of the present invention relates to a chimeric oligonucleotide primer for detecting Chlamydia having a nucleotide sequence selected from the group consisting of SEQ ID NOS: 157, 158, 203 and 204.
The thirtieth aspect of the present invention relates to a kit for amplifying a nucleic acid used for the method for amplifying a nucleic acid of the first to fifth aspect of the present invention, which contains the chimeric oligonucleotide primer of the twenty-first to twenty-ninth aspect.
The thirty-first aspect of the present invention relates to a kit for detecting a target nucleic acid used for the method for detecting a target nucleic acid of the twentieth aspect of the present invention, which contains the chimeric oligonucleotide primer of the twenty-first to twenty-ninth aspect.
The thirty-second aspect of the present invention relates to a probe used in the method of the twentieth aspect.
The thirty-third aspect of the present invention relates to a probe which hybridizes to the nucleic acid amplified by the method of the first to seventh aspect.
The thirty-fourth aspect of the present invention relates to a probe which hybridizes to a region amplified using the chimeric oligonucleotide primer of the twenty-first to twenty-ninth aspect.
The probe of the thirty-second to thirty-fourth aspect may be a probe which has been labeled with a labeling substance, for example, an RNA probe labeled with two or more fluorescent substances positioned at a distance that results in a quenching state.
The thirty-fifth aspect of the present invention relates to a kit used for the detection of the target nucleic acid in the method of the twentieth aspect, which contains the probe of the thirty-second to thirty-fourth aspect.
The thirty-sixth aspect of the present invention relates to a method for amplifying a nucleic acid, which comprises using a DNA polymerase having a strand displacement activity to effect a template switching reaction.

The DNA polymerase having a strand displacement activity used in the thirty-sixth aspect is exemplified by Klenow fragment of DNA polymerase I from Escherichia coli, Bst DNA polymerase lacking 5'→3' exonuclease from Bacillus stearothermophilus and Bca DNA polymerase lacking 5'→3' exonuclease from Bacillus caldotenax.

The thirty-seventh aspect of the present invention relates to a method for producing a material having an immobilized nucleic acid in which the nucleic acid is arrayed in a predefined region, the method comprising:

(a) amplifying a nucleic acid to be immobilized by the method for amplifying a nucleic acid of the first to seventh aspect of the present invention; and
(b) arraying and immobilizing the nucleic acid amplified in step (a) in a predefined region.

The thirty-eighth aspect of the present invention relates to a material having an immobilized nucleic acid in which the nucleic acid is arrayed in a predefined region produced by the method of the thirty-seventh aspect.

The thirty-ninth aspect of the present invention relates to a method for producing a nucleic acid in large quantities, the method comprising:

(a) amplifying a nucleic acid by the method for amplifying a nucleic acid of the first to seventh aspect of the present invention; and
(b) collecting the nucleic acid amplified in step (a).

The fortieth aspect of the present invention relates to a method for amplifying a nucleic acid, the method comprising:

(a) duplicating a DNA or an RNA containing a sequence to be amplified to prepare a nucleic acid as a template; and
(b) amplifying the nucleic acid as the template obtained in step (a) by the method for amplifying a nucleic acid of the first-to seventh aspect.

The forty-first aspect of the present invention relates to a method for determining a nucleotide sequence of a nucleic acid, the method comprising amplifying a nucleic acid according to the method of the first to seventh, thirty-ninth or fortieth aspect.

Brief Description of Drawings

Figure 1 illustrates agarose gel electrophoresis pattern of amplified DNA fragments amplified according to the method of the present invention.
Figure 2: illustrates agarose gel electrophoresis pattern of amplified DNA fragments amplified according to the method of the present invention.
Figure 3 illustrates agarose gel electrophoresis pattern of amplified DNA fragments amplified according to the method of the present invention.
Figure 4 illustrates agarose gel electrophoresis pattern of amplified DNA fragments amplified according to the method of the present invention.
Figure 5 illustrates agarose gel electrophoresis pattern of amplified DNA fragments amplified according to the method of the present invention.
Figure 6 illustrates agarose gel electrophoresis pattern of amplified DNA fragments amplified according to the method of the present invention.
Figure 7 illustrates agarose gel electrophoresis pattern of amplified DNA fragments amplified according to the method of the present invention.
Figure 8 illustrates agarose gel electrophoresis pattern of amplified DNA fragments amplified according to the method of the present invention.
Figure 9 illustrates agarose gel electrophoresis pattern of amplified DNA fragments amplified according to the method of the present invention.
Figure 10 illustrates agarose gel electrophoresis pattern of amplified DNA fragments amplified according to the method of the present invention. A: ICAN; B: PCR.
Figure 11 illustrates agarose gel electrophoresis pattern of amplified DNA fragments amplified according to the method of the present invention.
Figure 12 illustrates agarose gel electrophoresis pattern of amplified DNA fragments amplified according to the method of the present invention.

Figure 13 illustrates agarose gel electrophoresis pattern of amplified DNA fragments amplified according to the method of the present invention.

Figure 14 illustrates agarose gel electrophoresis pattern of amplified DNA fragments amplified according to the method of the present invention.

Figure 15 illustrates agarose gel electrophoresis pattern of amplified DNA fragments amplified according to the method of the present invention.

Figure 16 illustrates agarose gel electrophoresis pattern of amplified DNA fragments amplified according to the method of the present invention.

Figure 17 illustrates agarose gel electrophoresis pattern of amplified DNA fragments amplified according to the method of the present invention.

Figure 18 illustrates agarose gel electrophoresis pattern of amplified DNA fragments amplified according to the method of the present invention.

Figure 19 illustrates agarose gel electrophoresis pattern of amplified DNA fragments amplified according to the method of the present invention.

Figure 20 illustrates agarose gel electrophoresis pattern of amplified DNA fragments amplified according to the method of the present invention.

Figure 21 illustrates agarose gel electrophoresis pattern of amplified DNA fragments amplified according to the method of the present invention.

Figure 22 illustrates agarose gel electrophoresis pattern of amplified DNA fragments amplified according to the method of the present invention.

Figure 23 illustrates agarose gel electrophoresis pattern of amplified DNA fragments amplified according to the method of the present invention.

Figure 24 illustrates agarose gel electrophoresis pattern of amplified DNA fragments amplified according to the method of the present invention.

Figure 25 illustrates agarose gel electrophoresis pattern of amplified DNA fragments amplified according to the method of the present invention.

Figure 26 illustrates agarose gel electrophoresis pattern of amplified DNA fragments amplified according to the method of the present invention.

Figure 27 illustrates agarose gel electrophoresis pattern of amplified DNA fragments amplified according to the method of the present invention.

Figure 28 illustrates agarose gel electrophoresis pattern of amplified DNA fragments amplified according to the method of the present invention and dot blot hybridization pattern.

Figure 29 illustrates agarose gel electrophoresis pattern of amplified DNA fragments amplified according to the method of the present invention.

Figure 30 is a graph which compares the amounts of amplification products amplified according to the method of the present invention and the PCR.

Figure 31 illustrates agarose gel electrophoresis pattern of amplified DNA fragments amplified according to the method of the present invention.

Figure 32 illustrates polyacrylamide gel electrophoresis pattern of amplified DNA fragments amplified according to the method of the present invention.

Figure 33 illustrates one aspect of the method for amplifying a nucleic acid of the present invention.

Figure 34 illustrates one aspect of the method for amplifying a nucleic acid of the present invention.

Figure 35 illustrates one aspect of the method for amplifying a nucleic acid of the present invention.

Figure 36 illustrates one aspect of the method for amplifying a nucleic acid of the present invention.

Figure 37 illustrates agarose gel electrophoresis pattern of amplified DNA fragments amplified according to the method of the present invention.

Figure 38 illustrates agarose gel electrophoresis pattern of amplified DNA fragments amplified according to the method of the present invention.

Figure 39 illustrates agarose gel electrophoresis pattern of amplified DNA fragments amplified according to the method of the present invention.

Detailed Description of the Invention

As used herein, a deoxyribonucleotide (also referred to as a dN) refers to a nucleotide of which the sugar portion is composed of D-2-deoxyribose. The deoxyribonucleotides include, for example, ones having adenine, cytosine, guanine or thymine as the base portion. Furthermore, the deoxyribonucleotides also include a deoxyribonucleotide having a modified base such as 7-deazaguanosine and a deoxyribonucleotide analog such as deoxyinosine nucleotide.

As used herein, a ribonucleotide (also referred to as an N) refers to a nucleotide of which the sugar portion is composed of D-ribose. The ribonucleotides include ones having adenine, cytosine, guanine or uracil as the base portion. The ribonucleotides also include modified ribonucleotides such as a modified ribonucleotide in which the oxygen atom of the phosphate group at the α-position is replaced by a sulfur atom (also referred to as an (α-S) ribonucleotide or an (α-S) N) or other derivatives.

As used herein, a chimeric oligonucleotide primer refers to a primer that contains a deoxyribonucleotide and a ribonucleotide. The primer may contain a nucleotide analog and/or a modified ribonucleotide.

The chimeric oligonucleotide primers used in the present invention include any chimeric oligonucleotide primer that has a ribonucleotide being positioned at the 3'-terminus or on the 3'-terminal side of the primer, can be used to extend a nucleic acid strand in the method of the present invention, can be cleaved with an endonuclease, and can be used to effect a strand displacement reaction.

As used herein, 3'-terminal side refers to a portion from the center to the 3'-terminus of a nucleic acid such as a primer. Likewise, 5'-terminal side refers to a portion from the center to the 5'-terminus of a nucleic acid.

As used herein, an endonuclease may be any one that acts on a double-stranded DNA generated by extending a DNA from the chimeric oligonucleotide primer which have been annealed to a nucleic acid as a template, and specifically cleaves -it at a portion of the primer that contains a ribonucleotide.

As used herein, a DNA polymerase refers to an enzyme that synthesizes a DNA strand de novo using a DNA strand as a template. The DNA polymerases include naturally occurring DNA polymerases and variant enzymes having the above-mentioned activity. For example, such enzymes include a DNA polymerase having a strand displacement activity, a DNA polymerase lacking a 5'→3' exonuclease activity and a DNA polymerase having a reverse transcriptase activity or an endonuclease activity.

As used herein, "a strand displacement activity" refers to an activity that can effect a strand displacement, that is, that can proceed DNA duplication on the basis of the sequence of the nucleic acid as the template while displacing the DNA strand to release the complementary strand that has been annealed to the template strand. In addition, a DNA strand released from a nucleic acid as a template as a result of a strand displacement is referred to as "a displaced strand" herein.

Hereinafter, the present invention will be described in detail.

(1) Chimeric oligonucleotide primer used in the present invention.

The primer used in the method of the present invention is a chimeric oligonucleotide primer that contains a ribonucleotide as well as at least one selected from the group consisting of a deoxyribonucleotide and a nucleotide analog. Such primers also include an oligoribonucleotide primer that contains an unmodified ribonucleotide and/or a modified ribonucleotide.

A chimeric oligonucleotide primer used in the method of the present invention is a chimeric oligonucleotide primer that has a nucleotide sequence substantially complementary to a part of the nucleotide sequence of a nucleic acid as a template. It can contribute to extension of a DNA strand under conditions used. Furthermore, a ribonucleotide is positioned at the 3'-terminus or on the 3'-terminal side of the chimeric oligonucleotide primer. The primer is usually designed such that it is complementary to a portion upstream of the region to be amplified, that is, a portion 3' to the nucleotide sequence corresponding to a region to be amplified in a nucleic acid as a template. As used herein, "a substantially complementary nucleotide sequence" means a nucleotide sequence that can anneal to a DNA as a template under reaction conditions used.

The chimeric oligonucleotide primer used in the method of the present invention may contain one or more modified ribonucleotide. As used herein, a ribonucleotide may be an unmodified ribonucleotide and/or a modified ribonucleotide that can be positioned at the 3'-terminus or on the 3'-terminal side of a chimeric oligonucleotide primer and that is recognized by or cleaved with an endonuclease. The ribonucleotides include both of the unmodified ribonucleotide and the modified ribonucleotide as described above. An unmodified ribonucleotide, a modified ribonucleotide or a combination thereof can be used for the chimeric oligonucleotide primer of the present invention as long as it does not abolish the function of the primer. Examples of the modified ribonucleotides include, but are not limited to, an (α-S) ribonucleotide in which the oxygen atom bound to the phosphate group is replaced by a sulfur atom, and a ribonucleotide in which the hydroxy group at the 2-position of the ribose is replaced by a methoxy group. Such a chimeric oligonucleotide primer containing a modified ribonucleotide can be produced by using, for example, an (α-S) ribonucleotide triphosphate, which is prepared by a method using a sulfuration reaction reagent (Glen Research) as described in United States Patent No. 5,003,097, or a 2-OMe-RNA-CE phosphoramidite reagent (Glen Research).

A chimeric oligonucleotide primer that can be used in the amplification method of the present invention may be designed to contain a modified ribonucleotide that confers resistance to the cleavage with an endonuclease. Such a primer is useful in that one can control the cleavage site with an endonuclease during amplification reaction steps.

One or two chimeric oligonucleotide primer may be used in the method of the present invention depending on the desired form of a DNA fragment after amplification (single-stranded or double-stranded). Specifically, one chimeric oligonucleotide primer is used when a single-stranded DNA is desired, whereas two primers are used when a double-stranded DNA is desired.

The length of the chimeric oligonucleotide primer used in the method of the present invention is not specifically limited, but is preferably about 12 nucleotides to about 100 nucleotides, more preferably about 15 nucleotides to about 40 nucleotides. It is preferable that the nucleotide sequence of the chimeric oligonucleotide is substantially complementary to a nucleic acid as a template such that it anneals to the nucleic acid as the template under reaction conditions used. The primer contains a sequence recognized by an endonuclease, which is utilized in a step as described below, at the 3'-terminus or on the 3'-terminal side.

For example, an oligonucleotide having a structure represented by the following general formula can be used in the DNA synthesis method of the present invention as a primer, although it is not intended to limit the present invention:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns, and the nucleotide at the 3'-terminus may be modified such that extension from the 3'-terminus by the action of the DNA polymerase does not take place).

For example, a chimeric oligonucleotide primer represented by the general formula in which a = an integer of 11 or more; and b = 1 and c =0, b = 2 and c = 0, b = 3-5 and c = 0, or b = 2 and c = 0-5 can be preferably used in the present invention. The length of the ribonucleotides at the 3'-terminus or on the 3'-termunal side of the chimeric oligonucleotide primer used in the method of the present invention is preferably 1-mer to 15-mer, more preferably 1-mer to 10-mer, most preferably 1-mer to 5-mer. The number of c in the general formula is not specifically limited, but any number that can be used in the method of the present invention may be selected. Usually, 5 or less is preferably. Better results are obtained in a reaction by selecting 3 rather than 4, 2 rather than 3, and 1 rather than 2 for c. In particular, the most efficient reaction can be accomplished in case of c = 0.

The chimeric oligonucleotide primer used in the present invention has a structure in which an endonuclease recognizes or cleaves a DNA strand extended from the primer using a DNA polymerase (a primer-extended strand) at a site that contains a ribonucleotide, which ribonucleotide being positioned at the 3'-terminus or on the 3'-terminal side of the chimeric oligonucleotide primer. Although it is not intended to limit the present invention, for example, when an RNase H acts on a double-stranded DNA generated by extending a DNA from a chimeric oligonucleotide primer represented by the general formula that has been annealed to a nucleic acid as a template, the chimeric oligonucleotide primer is cleaved at the ribonucleotide portion. A double-stranded DNA in which a nick is introduced between the oligonucleotide primer and the DNA strand synthesized by the extension is then generated. Then, a strand displacement reaction with a DNA polymerase proceeds from the nicked site. Thus, any chimeric oligonucleotide primer that can be used to extend a nucleic acid strand from the 3'-terminus of the primer, that can be cleaved with an endonuclease, and with which a DNA polymerase can effect a strand displacement reaction can be used in the method of the present invention. Furthermore, the chimeric oligonucleotide primers of the present invention include one whose 3'-terminus is modified such that extension by the action of the DNA polymerase can not take place, and DNA extension takes place from a 3'-terminus generated upon cleavage by the endonuclease.

In addition, a promoter sequence for an RNA polymerase may be included on the 5'-terminal side of the chimeric oligonucleotide primer. Such RNA polymerases are exemplified by T7 RNA polymerase and SP6 RNA polymerase.

Furthermore, the chimeric oligonucleotide primer used in the method of the present invention may contain a nucleotide analog or other substances. That is, one or more nucleotide analog(s) can be contained in the chimeric oligonucleotide primer of the present invention as long as the function of the primer for effecting a polymerization extension reaction from the 3'-terminus by the action of a DNA polymerase is not abolished. Plural types of the nucleotide analogs can be used in combination. Examples of the nucleotide analogs include, but are not limited to, deoxyinosine nucleotide, deoxyuracil nucleotide, a nucleotide analog having a modified base such as 7-deazaguanine, a nucleotide analog having a ribose derivative and the like. Furthermore, the chimeric oligonucleotide primers used in the present invention may contain deoxynucleotides, ribonucleotides or nucleotide analogs having various modifications such as addition of labeled compounds as long as they retain the functions as described above.

Incorporation of a nucleotide analog into a primer is effective for suppressing the formation of high-order structure of the primer itself and stabilization of annealing formation with the template. A ribonucleotide may be incorporated into a primer for the same purpose. Although it is not intended to limit the present invention, a modified ribonucleotide such as ($\alpha$-S) ribonucleotide can be preferably used in order to prevent the digestion of the primer by a non-specific endonuclease (RNase).

The chimeric oligonucleotide primer can be synthesized to have desired nucleotide sequence using, for example, the 394 type DNA synthesizer from Applied Biosystems Inc. (ABI) according to a phosphoramidite method. Alternatively, any methods including a phosphate triester method, an H-phosphonate method and a thiophosphonate method may be used to synthesize the chimeric oligonucleotide primer.

(2) Endonuclease used in the present invention.

Any endonuclease that can act on a double-stranded DNA generated by DNA extension from the chimeric oligonucleotide primer as described above in (1) that has been annealed to a nucleic acid as a template and cleaves the extended strand to effect a strand displacement reaction may be used in the present invention. That is, the endonuclease is an enzyme that can generate a nick in the chimeric oligonucleotide primer portion of the double-stranded DNA. Examples of endonucleases that can be used in the present invention include, but are not limited to, ribonucleases. Among these, endoribonuclease H (RNase H) that acts on an RNA portion of a double-stranded nucleic acid composed of a DNA and an RNA can be preferably used. Any ribonuclease that has the above-mentioned activities can be preferably used in the present invention, including mesophilic and heat-resistant ones. For example, an RNase H from E. coli can be used for a reaction at about 50°C to about 70°C in the method of the present invention as described below in Examples. A heat-resistant ribonuclease can be preferably used in the method of the present invention. Examples of the heat-resistant ribonucleases which can be preferably used include, but are not limited to, a commercially available ribonuclease, Hybridase™ Thermostable RNase H (Epicenter Technologies) as well as an RNase H from a thermophilic bacterium of genus Bacillus, a bacterium of genus Thermus, a bacterium of genus Pyrococcus, a bacterium of genus Thermotoga, a bacterium of genus Archaeoglobus or the like. Furthermore, both of naturally occurring ribonucleases and variants can be preferably used. The enzymatic unit of RNase H indicated herein is a value expressed according to a method of measuring an enzymatic unit as described in Referential Examples.

The RNase H is not limited to a specific one as long as it can be used in the method of the present invention. For example, the RNase H may be derived from various viruses, phages, prokaryotes or eukaryotes. It may be either a cellular RNase H or a viral RNase H. The cellular RNase H is exemplified by Escherichia coli RNase HI and the viral RNase H is exemplified by HIV-1. Type I, type II or type III RNase H can be used in the method of the present invention. For example, RNase HI from Escherichia coli, or RNase HII from a bacterium of genus Pyrococcus or a bacterium of genus Archaeoglobus is preferable, without limitation.

The efficiency of the cleavage reaction with an endonuclease such as RNase H used in the method of the present invention may vary depending on the nucleotide sequence around the 3'-terminus of the primer and influence the amplification efficiency of the desired DNA.. Therefore, it is natural to design the optimal primer for the RNase H used.

As used herein, the term "introducing a nick" or "nicking" means internally cleaving one of the two strands of a double-stranded nucleic acid. For example, an RNase H acts on a hybrid double-stranded nucleic acid composed of a DNA and a ribonucleotide-containing DNA to selectively cleave the ribonucleotide-containing strand among the two strands at the ribonucleotide portion, thereby introducing a nick into the hybrid double-stranded nucleic acid.

(3) DNA polymerase used in the present invention.

A DNA polymerase having a strand displacement activity on a DNA can be used in the present invention. Particularly, a DNA polymerase substantially lacking a 5'→3' exonuclease activity can be preferably used.

As used herein, "a strand displacement activity" refers to an activity that can effect a strand displacement, that is, that can proceed DNA duplication on the basis of a sequence of a nucleic acid as a template while displacing a DNA strand to release a complementary strand that has been annealed to the template strand. Additionally, a DNA strand released from a nucleic acid as a template as a result of a strand displacement is referred to as "a displaced strand" herein.

Any DNA polymerases having the strand displacement activity can be used in the present invention. Examples thereof include variants of DNA polymerases lacking their 5'→3' exonuclease activities derived from thermophilic bacteria of genus Bacillus such as Bacillus caldotenax (hereinafter referred to as B. ca) and Bacillus stearothermophilus (hereinafter referred to as B. st), as well as large fragment (Klenow fragment) of DNA polymerase I from Escherichia coli (E. coli). Both of mesophilic and heat-resistant DNA polymerases can be preferably used in the present invention.

B. ca is a thermophilic bacterium having an optimal growth temperature of about 70°C. Bca DNA polymerase from this bacterium is known to have a DNA-dependent DNA polymerase activity, an RNA-dependent DNA polymerase activity (a reverse transcription activity), a 5'→3' exonuclease activity and a 3'→5' exonuclease activity. The enzyme may be either an enzyme purified from its original source or a recombinant protein produced by using genetic engineering techniques. The enzyme may be subjected to modification such as substitution, deletion, addition or insertion by using genetic engineering techniques or other means. Examples of such enzymes include BcaBEST DNA polymerase (Takara Shuzo), which is Bca DNA polymerase lacking its 5'→3' exonuclease activity.

It is known that some DNA polymerases have an endonuclease activity such as an RNase H activity under specific conditions. Such a DNA polymerase can be used in the method of the present invention. In one aspect, the DNA polymerase may be used under conditions that allow the RNase H activity to express, e.g., in the presence of $Mn^{2+}$. In this case, the method of the present invention can be conducted without the addition of an RNase H. The present inventors have demonstrated that the Bca DNA polymerase exhibits an RNase activity in a buffer containing $Mn^{2+}$ for the first time, and that the method for amplifying a nucleic acid of the present invention can be carried out in a reaction mixture containing no enzyme other than the Bca DNA polymerase. The above-mentioned aspect is not limited to the use of the Bca DNA polymerase. DNA polymerases that are known to have an RNase H activity such as Tth DNA polymerase from Thermus thermophilus can be used in the present invention.

(4) Composition of reaction buffer used in the present invention.

A reaction buffer that contains a buffering component, a magnesium salt or another metal salt and dNTPs is used in the present invention. Naturally, the type and the concentration of the salt are optimized depending on the metal requirement or the like of the enzyme to be used. Examples of the buffering components that can be preferably used include, but are not limited to, Bicine, Tricine, HEPES, tris and a phosphate (such as sodium phosphate and potassium phosphate). Among these, a buffer that contains Bicine, Tricine, HEPES or a phosphate as a buffering component is preferable for the present invention. Although it is not intended to limit the present invention, for example, when the reaction temperature is high, a Bicine buffer of which the change in pH due to the change in temperature is little is preferably used. A HEPES buffer may be preferable depending on the type of RNase H used. Thus, the optimal buffer may be selected depending on the reaction temperature, the endonuclease or the DNA polymerase to be used and the like. The final concentration of the buffering component ranges 5-100 mM, preferably 20-50 mM. The pH ranges 6.0-9.5, preferably 7.0-9.2. For example, a buffer containing 22-46 mM Tricine at pH 7.5-9.2 or a buffer containing 25-50 mM potassium phosphate at pH 7.0-8.0 is preferably used. Examples of magnesium salts that can be preferably used include, but are not limited to, magnesium chloride, magnesium acetate or magnesium sulfate. The final concentration of the magnesium salt ranges 1-20 mM, preferably 2-10 mM. The final concentrations of dNTPs (a mixture of dATP, dCTP, dGTP and dTTP) in a mixture as substrates for a DNA extension reaction range 0.1-3.0 mM, preferably 0.2-1.2 mM. The amount of the primers used in a reaction volume of 50 μl ranges 1-1000 pmol, preferably 10-150 pmol. Additionally, the reaction mixture may contain an additive, for example, in order to stabilize the amplification reaction. Bovine serum albumin (BSA) at a final concentration of 0.1% or less, dimethyl sulfoxide (DMSO) at a final concentration of 10% or less, putrescine dihydrochloride at a final concentration of 4 mM or less, or propylenediamine at a final concentration of 0.01% or less may be added. Additionally, NMP (1-methyl-2-pyrrolidinone), glycerol, polyethylene glycol, dimethyl sulfoxide and/or formamide may be contained. It is expected that the addition of such an organic solvent reduces the non-specific annealing of oligonucleotide primers.

The method of the present invention may be carried out by adding a substance that inhibits the reverse transcription activity of the DNA polymerase such as phosphonoformic acid (PFA). If a substance that inhibits the reverse transcription activity is added, the amplification of non-specific products other than the target nucleic acid is reduced.

In another aspect, annealing of the nucleic acid as the template to the chimeric oligonucleotide primer used in the present invention before the amplification reaction is effective to reduce non-specific annealing of an oligonucleotide primer in the detection, amplification or production method of the present invention. It is preferable to conduct the annealing using an annealing solution that contains a substance that enhances the annealing such as a polyamine (e.g., spermine or spermidine) or propylenediamine. Preferably, the annealing solution containing the polyamine also contains a salt. For example, without limitation, the annealing solution may contain sodium chloride, potassium chloride, potassium acetate, sodium acetate or the like and a polyamine.

The annealing is usually conducted by incubating the annealing solution containing the primer and the nucleic acid as the template at a temperature. at which a double-stranded nucleic acid is denatured (e.g., 90°C or above) and then cooling the solution to a reaction temperature used for the method of the present invention or below.

After annealing, the nucleic acid amplification reaction of the present invention is initiated by further adding other necessary components such as a DNA polymerase, an RNase H and dNTPs to the mixture.

The amount of an RNase H from Escherichia coli as an example of endonucleases in a reaction volume of 50 μl ranges preferably 3-200 U, more preferably 15-60 U. Similarly, the amount of an RNase H from a bacterium of genus Pyrococcus or a bacterium of genus Archaeoglobus as an example of endonuclease in a reaction volume of 50 μl ranges preferably 3-200 U, more preferably 4-40 U. The amount of BcaBEST DNA polymerase (Takara Shuzo) as an example of DNA polymerases in a reaction volume of 50 μl ranges preferably 0.5-100 U, more preferably 1-22 U.

When an endonuclease and a DNA polymerase is used in combination in the method of the present invention, for example, without limitation, a combination of an RNase H from Escherichia coli, a bacterium of genus Pyrococcus or a bacterium of genus Archaeoglobus and BcaBEST DNA polymerase is preferably used. It is considered that the preferable units of the endonuclease and the DNA polymerase may vary depending on the types the enzymes. In such a case, the composition of the buffer used and the amount of the enzymes added may be adjusted using the increase in detection sensitivity or the amount of amplification product as an index. In either case, it is natural to optimize the composition of the reaction buffer and the like depending on the type of the enzyme to be used.

(5) Method for amplifying nucleic acid of the present invention.

The method of the present invention can be conducted by using at least one oligonucleotide primer as described above in (1) in combination with the endonuclease as described above in (2) and the DNA polymerase as described above in (3). Alternatively, a DNA polymerase having an RNase H activity can be used under conditions that allow the RNase H activity to express as described above.

dNTPs used for the PCR or the like (a mixture of dATP, dCTP, dGTP and dTTP) can be preferably used as nucleotide triphosphates that serve as substrates in the extension reaction in the method. In addition, dUTP may be used as a substrate. The dNTPs may contain a dNTP (deoxyribonucleotide triphosphate) analog such as 7-deaza-dGTP, triphosphate of dITP or the like as long as it serves as a substrate for the DNA polymerase used. A derivative of a dNTP or a dNTP analog may be used. A derivative having a functional group such as a dUTP having an amino group may be contained. A chimeric oligonucleotide primer is used in the method. The primer can be prepared, for example, using a DNA synthesizer according to a conventional synthesis method. A combination of the chimeric oligonucleotide primer and a normal oligonucleotide primer can be used in the method of the present invention.

If the activity of the enzyme used may be decreased in the course of the reaction, the enzyme can be further added during the reaction in the method of the present invention. Although it is not intended to limit the present invention, for example, an RNase H from Escherichia coli may be further added during a reaction in which the RNase H is used. The added enzyme may be the same as that contained in the reaction mixture at the beginning of the reaction or it may be a different enzyme that exhibits the same activity. Thus, the type or the property of the enzyme to be added is not limited to a specific one as long as the addition during the reaction provides effects such as increase in the detection sensitivity or increase in the amount of amplification product.

The nucleic acid (DNA or RNA) used as a template in the method of the present invention may be prepared or isolated from any sample that may contain the nucleic acid. Alternatively, the sample may be used directly in the nucleic acid amplification reaction according to the present invention. Examples of the samples that may contain the nucleic acid include, but are not limited to, samples from organisms such as a whole blood, a serum, a buffy coat, a urine, feces, a cerebrospinal fluid, a seminal fluid, a saliva, a tissue (e.g., a cancerous tissue or a lymph node) and a cell culture (e.g., a mammalian cell culture or a bacterial cell culture), samples that contain a nucleic acid such as a viroid, a virus, a bacterium, a fungi, a yeast, a plant and an animal, samples suspected to be contaminated or infected with a microorganism such as a virus or a bacterium (e.g., a food or a biological formulation), and samples that may contain an organism such as a soil and a waste water. The sample may be a preparation containing a nucleic acid obtained by processing the above-mentioned samples according to a known method. Examples of the preparations that can be used in the present invention include a cell destruction product or a sample obtained by fractionating the product, the nucleic acid in the sample, or a sample in which specific nucleic acid molecules such as mRNAs are enriched. Furthermore, a nucleic acid such as a DNA or an RNA obtained amplifying a nucleic acid contained in the sample by a known method can be preferably used.

The preparation containing a nucleic acid.can be prepared from the above-mentioned materials by using, for example, lysis with a detergent, sonication, shaking/stirring using glass beads or a French press, without limitation. In some cases, it is advantageous to further process the preparation to purify the nucleic acid (e.g., in case where an endogenous nuclease exists). In such cases, the nucleic acid is purified by a know method such as phenol extraction, chromatography, ion exchange, gel electrophoresis or density-gradient centrifugation.

When it is desired to amplify a nucleic acid having a sequence derived from an RNA, the method of the present invention may be conducted using as a template a cDNA synthesized by a reverse transcription reaction that uses the RNA as a template. Any RNA for which one can make a primer to be used in a reverse transcription reaction can be applied to the method of the present invention, including total RNA in a sample, RNA molecules such as, mRNA, tRNA and rRNA as well as specific RNA molecular species.

Any primer that anneals to an RNA as a template under reaction conditions used can be used in the reverse transcription reaction. The primer may be a primer having a nucleotide sequence that is complementary to a specific RNA as a template (a specific primer), an oligo-dT (deoxythymine) primer and a primer having a random sequence (a random primer). In view of specific annealing, the length of the primer for reverse transcription is preferably 6 nucleotides or more, more preferably 9 nucleotides or more. In view of oligonucleotide synthesis, the length is preferably 100 nucleotides or less, more preferably 30 nucleotides or less. A chimeric oligonucleotide primer can be used as a primer for reverse transcription. The chimeric oligonucleotide primer can also be utilized as a primer for a strand displacement reaction in the method for amplifying a nucleic acid of the present invention using a cDNA obtained after reverse transcription as a template. Such a primer may be any one that has the properties as described above in (1) and that can be used in a reverse transcription reaction from an RNA.

Any enzyme that has an activity of synthesizing a cDNA using an RNA as a template can be used in the reverse transcription reaction. Examples thereof include reverse transcriptases originating from various sources such as avian myeloblastosis virus-derived reverse transcriptase (AMV RTase), Molony murine leukemia virus-derived reverse transcriptase (MMLV RTase) and Rous-associated virus 2 reverse transcriptase (RAV-2 RTase). In addition, a DNA polymerase that also has a reverse transcription activity can be used. An enzyme having a reverse transcription activity at a high temperature such as a DNA polymerase from a bacterium of genus Thermus (e.g., Tth DNA polymerase) and a DNA polymerase from a thermophilic bacterium of genus Bacillus is preferable for the present invention. For example, DNA polymerases from thermophilic bacteria of genus Bacillus such as a DNA polymerase from B. st (Bst DNA polymerase) and Bca DNA polymerase are preferable, although it is not intended to limit the present invention. For example, Bca DNA polymerase does not require a manganese ion for the reverse transcription reaction. Furthermore, it can synthesize a cDNA while suppressing the formation of a secondary structure of an RNA as a template under high-temperature conditions. Both a naturally occurring one and a variant of the enzyme having a reverse transcriptase activity can be used as long as they have the activity.

In another aspect, after duplicating a DNA or an RNA containing a nucleotide sequence to be amplified beforehand, the duplicated product may be used as a nucleic acid as a template in the method of the present invention. Examples of the methods for duplication include, but are not limited to, a method in which an appropriate host is transformed with a vector into which the nucleic acid fragment containing the nucleotide sequence to be amplified is inserted, the resulting transformant is cultured, the vector into which the nucleic acid fragment containing the nucleotide sequence to be amplified is inserted is extracted therefrom and used. Any vectors can be used as long as they are stably replicated in the host. For example, pUC series, pBluescript series, pGEM series, cosmid type vectors and phage type vectors are preferably used. Any hosts can be used as long as they can maintain the vectors used. For example, Escherichia coli, which is readily cultured, is exemplified.

In another aspect of the duplication method, after an RNA having a nucleotide sequence to be amplified is transcribed using an RNA polymerase and a nucleic acid fragment containing the nucleotide sequence as a template, the RNA may be used as a template for the method of the present invention directly or after converting it into a cDNA by reverse transcription reaction. The nucleic acid fragment containing the nucleotide sequence to be amplified is not limited to a specific one as long as it has a promoter sequence for an RNA polymerase. It may be inserted into a vector having a promoter sequence for an RNA polymerase, ligated with an adapter or a cassette having a promoter sequence for an RNA polymerase at its end or enzymatically synthesized using a primer having a promoter sequence for an RNA polymerase and an appropriate template. Thus, a nucleic acid fragment containing a nucleotide sequence to be amplified can be duplicated or amplified in a form of an RNA using a promoter sequence for an RNA polymerase being positioned as described above. Any vectors can be used as long as they have promoter sequences for RNA polymerases. For example, pUC series, pBluescript series, pGEM series, cosmid type vectors and phage type vectors are preferably used. The vector can be preferably used in its circular form or after being linearized. Any RNA polymerases can be used for the duplication or amplification method. For example, SP6 RNA polymerase, T7 RNA polymerase, T3 RNA polymerase or the like can be preferably used.

Both of a double-stranded DNA such as a genomic DNA isolated as described above or a PCR fragment and a single-stranded DNA such as a cDNA prepared by a reverse transcription reaction from a total RNA or an mRNA can be preferably used as a template DNA in the present invention. The double-stranded DNA is preferably used after denaturing it into single-stranded DNAs or without such denaturation.

The denaturing step may be eliminated in the nucleic acid amplification method of the present invention if a linear double-stranded DNA such as a PCR amplification product is used as a template. The elimination may be accomplished by locating the annealing site for the primer used in the method of the present invention about 50 bases inside from the terminus of the DNA. If a nucleic acid having a sequence from an RNA is to be amplified, amplification reaction can be initiated by adding an RNA-cDNA double-stranded nucleic acid obtained by reverse transcription reaction using an RNA as a template to the amplification reaction mixture of the present invention containing an RNase H to digest the RNA strand and convert the nucleic acid into a single-stranded cDNA. Furthermore, a reverse transcription reaction using an RNA as a template and a DNA amplification reaction using a cDNA generated by the reverse transcription reaction as a template can be conducted with one DNA polymerase in the DNA synthesis method of the present invention. Such a DNA polymerase has a reverse transcriptase activity and a strand displacement activity.

The suitable length of the template is one that provides a sufficient binding of the primer sequence due to the presence of the whole target sequence or at least a sufficient part of the target sequence in the fragment.

Without limitation, if a DNA as a template is a double-stranded DNA, the DNA can be denatured into single-stranded DNAs to allow a primer to bind to the DNA strand as the template in the method of the present invention. Incubating the double-stranded DNA at a temperature at which it is denatured (e.g., about 95°C) is preferable for the denaturation. Other processes include one in which an elevated pH is used. In this case, the pH should be lowered upon the amplification reaction in order to allow an oligonucleotide primer to bind to a target. A nucleic acid is successively amplified under isothermal conditions after denaturing a double-stranded DNA into single-stranded DNAs or, if an RNA is used as a template, preparing a cDNA (a single-stranded DNA) by a reverse transcription reaction.

"Successively" means that a reaction proceeds without a change in the reaction temperature or the composition of the reaction mixture. As used herein, "isothermal" means conditions of a substantially constant temperature under which an enzyme and a nucleic acid strand function in each step.

The nucleic acid amplification reaction of the present invention may be conducted at a normal temperature (e.g., 37°C) by using a mesophilic DNA polymerase such as Klenow fragment. It can also be conducted at a high temperature (e.g., 50°C or higher, or 60°C or higher) by using heat-resistant enzymes (an endonuclease and a DNA polymerase). In this case, non-specific annealing of a primer is suppressed, resulting in increase in the specificity of DNA amplification. Furthermore, the problem of forming secondary structure of a DNA as a template is solved, resulting in increase in the ability of extension of a DNA polymerase. A reverse transcription reaction and the nucleic acid amplification can be successively conducted in the method. In this case, a DNA having a sequence derived from an RNA can be amplified by combining the use of a reverse transcriptase with the above-mentioned reaction or by using a DNA polymerase having a reverse transcription activity.

In each aspect of the present invention, preferably, a chimeric oligonucleotide primer that is complementary to a single-stranded DNA as a template is first annealed to the DNA, although it is not intended to limit the present invention. A DNA that is complementary to the DNA as the template (a primer-extended strand) is then extended along the remained sequence of the DNA as the template from the 3'-terminus of the primer by the action of a DNA polymerase to synthesize a double-stranded DNA. An endonuclease acts on the double-stranded DNA and begins to reextend a DNA from the primer portion of the primer-extended strand de novo. In one aspect of the present invention, the endonuclease acts as a nicking enzyme that introduces a nick into the double-stranded DNA or it alters the structure of the double-stranded DNA composed of the chimeric oligonucleotide primer and the DNA as the template, although the present invention is not restricted by a theory. A DNA polymerase having a strand displacement activity re-extends a DNA strand from the 3'-terminus of the nick introduced in the double-stranded DNA to generate a new primer-extended strand while releasing the DNA downstream from the 3'-terminus of the nick. Thus, the new primer-extended strand replaces the previously synthesized primer-extended strand.

The method for amplifying a nucleic acid of the present invention can be carried out using two primers, i.e., a chimeric oligonucleotide primer that is complementary to a nucleic acid as a template and another chimeric oligonucleotide primer that is complementary to a displaced strand. In this case, one primer binds to a DNA strand as a template to cause a strand displacement reaction, whereas another primer binds to a displaced strand released as a result of the strand displacement reaction to initiate another strand displacement reaction. It is clear that a reaction product with one primer can function as a template for another primer if this aspect is used. Thus, the amount of amplification product increases in a non-linear manner as the amount of the template increases.

When the method for amplifying a nucleic acid of the present invention is conducted using a double-stranded DNA as a template, both strands can serve as templates in the amplification reaction by using chimeric oligonucleotide primers that anneal to the respective two strands. If the reaction is initiated after denaturing the double-stranded DNA, a chimeric oligonucleotide primer, four deoxyribonucleotide triphosphates (dNTPs), a DNA polymerase and an endonuclease are added to a reaction mixture before or after the double-stranded DNA is denatured. If heat treatment is used for denaturing the double-stranded DNA and a heat-resistant enzyme is not used, it is preferable to add the enzyme after the denaturation.

In the aspect of the nucleic acid amplification method of the present invention in which a double-stranded DNA as a template and two chimeric oligonucleotide primers are used, switching of templates may occur among the template-extended strand intermediates during the extension reaction from the primers to generate a double-stranded nucleic acid consisting of the synthesized primer-extended strands being annealed each other, although it depends on the reaction conditions or the like. The double-stranded nucleic acid has chimeric oligonucleotide primers at both ends. Then, reaction of extending complementary strands comprising strand displacement can be initiated from both of the ends again. As a result of the reaction, an amplification product having the primer sequence at one end is generated. Furthermore, if switching of templates occurs during the reaction, a double-stranded nucleic acid similar to one that described above is generated again.

The present invention provides a method for amplifying a nucleic acid which comprises using a DNA polymerase having a strand displacement activity to effect a template switching reaction. In the template switching reaction in the presence of a double-stranded nucleic acid as a template, two chimeric oligonucleotide primers substantially complementary to the nucleotide sequences of the respective strands and a DNA polymerase having a strand displacement activity, two primer-extended strands complementary to the template are synthesized. Template switching of each of the primer-extended strands from the template to the other primer-extended strand takes place during the synthesis of the primer-extended strands.

As used herein, a template switching reaction refers to a reaction in which when complementary strands are synthesized by strand displacement reactions from the both sides of a double-stranded nucleic acid, a DNA polymerase switches the template and synthesizes a complementary strand thereafter using the other complementary strand newly synthesized by another DNA polymerase as a template. In other words, a template switching reaction refers to a reaction in which a double-stranded nucleic acid as a template is treated with primers and a DNA polymerase having a strand displacement activity to generate extended strands complementary to the template, wherein a DNA polymerase that synthesized the primer-extended strands actively switches the template from the original templates to the other primer-extended strands during the synthesis of the extended strands. The ability of the DNA polymerase to effect a template switching reaction can be determined, for example, according to the method as described in Example 32 below, although it is not intended to limit the present invention.
A DNA polymerase capable of an effect the template switching reaction during strand displacement reaction can be preferably used for the present invention. For example, a variant enzyme of Bca DNA polymerase lacking a 5'→3' exonuclease activity is preferably used in particular. Such an enzyme is commercially available as BcaBEST DNA polymerase (Takara Shuzo). It can also be prepared from Escherichia coli HB101/pU1205 (FERM BP-3720) which contains the gene for the enzyme according to the method as described in Japanese Patent No. 2978001. Escherichia coli HB101/pU1205 (FERM BP-3720) has been deposited at International Patent Organism Depositary, National Institute of Advanced Industrial Science and Technology, AIST Tsukuba Central 6, 1-1, Higashi 1-chome, Tsukuba-shi, Ibaraki 305-8566, Japan since May 10, 1991 (date of original deposit).
Although it is not intended to limit the present invention, the mode of reaction of the method for amplifying a nucleic acid of the present invention is considered as follows, for example.
In the method for amplifying a nucleic acid of the present invention, a double-stranded nucleic acid as a template is treated in the presence of an RNase H with two chimeric oligonucleotide primers that are substantially complementary to the nucleotide sequences of the respective strands and a DNA polymerase having a strand displacement activity to synthesize primer-extended strands that are complementary to the template. A double-stranded nucleic acid consisting of the synthesized primer-extended strands being annealed each other and a double-stranded nucleic acid consisting of the templates being annealed each other to which the two primers are annealed as a result of template switching reaction can be obtained. The latter double-stranded nucleic acid is reused as a template.
The double-stranded nucleic acid consisting of the primer-extended strands being annealed each other is cleaved with an RNase H at sites that contain the ribonucleotides. Nucleic acids that are complementary to the template are extended using a DNA polymerase having a strand displacement activity from the 3'-termini of the respective primer portions of the double-stranded nucleic acid to effect strand displacements. A double-stranded nucleic acid consisting of the primer-extended strands being annealed each other and a double-stranded nucleic acid consisting of the templates being annealed each other to which the two primers are annealed as a result of template switching reaction can be obtained.
If the template switching reaction does not take place, two types of double-stranded nucleic acids each consisting of the template and the primer-extended strand can be obtained.
Nucleic acids that are complementary to the template are extended to effect strand displacements using a DNA polymerase having a strand displacement activity from the 3'-termini of the respective primer portions of the double-stranded nucleic acid to which the two primers are annealed. As a result of template switching reaction, a double-stranded nucleic acid consisting of the primer-extended strands being annealed each other and a double-stranded nucleic acid consisting of the templates being annealed each other to which the two primers are annealed can be obtained. The double-stranded nucleic acid to which the two primers are annealed is reused as a template.
If the template switching reaction does not take place, two types of double-stranded nucleic acids each consisting of the template and the primer-extended strand can be obtained.
The two types of double-stranded nucleic acids are cleaved with an RNase H at sites that contain the ribonucleotides. Nucleic acids that are complementary to the template are extended using a DNA polymerase having a strand displacement activity from the 3'-termini of the respective primer portions of the double-stranded nucleic acid to effect strand displacements.
In the method for amplifying a nucleic acid of the present invention, a double-stranded nucleic acid as a template is treated in the presence of an RNase H with two chimeric oligonucleotide primers that are substantially complementary to the nucleotide sequences of the respective strands and a DNA polymerase having a strand displacement activity, and primer-extended strands that are complementary to the template are synthesized. If the template switching reaction does not take place, two types of double-stranded nucleic acids each consisting of the template and the primer-extended strand can be obtained.
In the amplification method of the present invention, the chimeric oligonucleotide primer-extended strand may be cleaved at a site that contains the ribonucleotide such that the 5' fragment (primer portion) resulting from the cleavage does not contain the ribonucleotide. A primer-extended strand extended from the thus generated primer portion is no longer cleaved by an endonuclease. As a result, an amplification product having the primer sequence at its end is generated.

As described above, an amplification product without the primer sequence and a product having the primer sequence(s) at one or both of the ends may be generated in the nucleic acid amplification method of the present invention. These products are included in the amplification products herein.

An example of the method for amplifying a nucleic acid of the present invention is illustrated in Figures 33 to 36. In other words, Figures 33 to 36 illustrate an exemplary nucleic acid amplification in the nucleic acid amplification method of the present invention.

Figures 33 to 36 illustrate an exemplary nucleic acid amplification in the presence of a DNA as a template which is a double-stranded nucleic acid, a pair of chimeric oligonucleotide primers synthesized on the basis of the nucleotide sequence information of the DNA as the template (in the figures, the chimeric oligonucleotide primers have three ribonucleotides at their 3'-termini; open circles in the figures represent the ribonucleotides), a strand displacement type DNA synthetase (DNA polymerase) having a strand displacement activity, an RNase H which is a ribonuclease that cleaves at a DNA-RNA hybrid site, and dNTPs which are substrates to be incorporated into the extended strands.

As shown in Figure 33, the pair of the chimeric oligonucleotide primers synthesized on the basis of the nucleotide sequence information of the DNA as the template are annealed to the specific portions of the DNA as the template. DNA strands are extended from the 3'-termini of the respective chimeric oligonucleotide primers as a result of the strand displacement reaction as indicated with Step 1.

Next, as shown in Figure 34, some of the primer-extended strands extended from upstream and downstream are released from the original templates as a result of the template switching reaction as indicated with Step 2. The primer-extended strands are annealed each other at their 3' portions. Complementary strands are extended from the annealed extended strands, forming a double-stranded DNA consisting of the primer-extended strands being annealed each other. Additionally, a double-stranded DNA consisting of the displaced strands being annealed each other to which the above-mentioned pair of the chimeric oligonucleotide primers are annealed is generated. This is utilized as the starting material in Figure 34.

As indicated with Step 3 in Figure 35, only one strand containing an RNA derived from the chimeric oligonucleotide primer of the double-stranded DNA in Figure 34 consisting of the primer-extended strands being annealed each other is cleaved by the action of an RNase H at the DNA/RNA hybrid site of the double-stranded DNA, resulting in introduction of a nick in the double-stranded DNA.

Subsequently, the strand displacement reaction takes place from the nick in the double-stranded DNA and a DNA is extend as indicated with Step 4 in Figure 35. Next, a template switching reaction like that in Step 2 in Figure 34 takes place in some degree or at some ratio as indicated with Step 5 in Figure 35, resulting in a double-stranded DNA consisting of the amplification products, i.e., the primer-extended strands being annealed each other.

In addition, a double-stranded DNA consisting of the displaced strands being annealed each other to which the above-mentioned pair of chimeric oligonucleotide primers are annealed are generated.

Next, as shown in Figure 36, DNA strands are extended as a result of the strand displacement reaction from the 3'-termini of the respective chimeric oligonucleotide primers in the double-stranded DNA in Figure 35 which consists of the displaced strands being annealed each other to which the pair of the chimeric oligonucleotide primers are annealed. A template switching reaction similar to that in Step 2 and Step 5 takes place in some degree, resulting in a double-stranded DNA consisting of the primer-extended strands being annealed each other. This double-stranded DNA is brought back to Step 3 in Figure 35. The reaction starts on Step 3 again. A double-stranded DNA which consists of the displaced strands being annealed each other to which the pair of the chimeric oligonucleotide primers are annealed is generated and utilized as a starting material in Figure 36. As a result, a chain reaction, in which these double-stranded nucleic acids are repeatedly generated, takes place to specifically amplify and produce a region bounded by a pair of chimeric oligonucleotide primers.

In the nucleic acid amplification method of the present invention using a chimeric oligonucleotide, a polymer in which the regions to be amplified are connected each other may be generated. The polymer has a structure in which plural regions to be amplified are repeated in the same direction. The polymers are observed upon electrophoretic analysis of amplification products as laddered bands. It is considered that the generation of such polymers is influenced by the region to be amplified, the size of the region, the flanking regions, the nucleotide sequence of the chimeric oligonucleotide primer to be used, the reaction conditions and the like.

The polymer as described above contains plural regions to be amplified. For example, the polymer is useful when detection of a nucleic acid containing a region to be amplified is intended because it hybridizes to a number of probes upon hybridization using an appropriate probe and generates a intensive signal. The region to be amplified or a portion thereof can be obtained from the polymer as a monomer by using digestion with a restriction enzyme or the like in combination.

The DNA polymerase used in the present invention should synthesize an extended strand from the 3'-terminus of the primer portion towards the downstream while displacing a previously extended DNA strand. It is important that the DNA polymerase should not exhibit a 5'→3' exonuclease activity that may digest the displaced strand. For example, Klenow fragment, which is an exonuclease-deficient variant of DNA polymerase I from Escherichia coli, a similar fragment derived from Bst DNA polymerase (New England Biolabs), and BcaBEST DNA polymerase from B. ca (Takara Shuzo) are useful as such a DNA polymerase. Sequenase 1.0 and Sequenase 2.0 (United States Biochemical), and T5 DNA polymerase and φ29 DNA polymerase as described in Gene, 97:13-19 (1991) can also be used. A polymerase that normally has a 5'→3' exonuclease activity can be used in the DNA synthesis method of the present invention if addition of an appropriate inhibitor can inhibit the activity.

The method for amplifying a nucleic acid of the present invention may be conducted at varying temperatures or it may be conducted isothermally. Varying temperatures means that the reaction temperatures are changed for respective steps such that the change does not interfere with the reactions in the steps. Specifically, varying temperatures refers to change in temperature to that suitable for, for example, each of annealing of a primer, synthesis reaction of a complementary strand, nicking of a complementary strand and a displacement reaction.

On the other hand, isothermal means that the reaction temperature for each step is unchanged and each step is conducted at a substantially constant temperature. It is natural to select the temperature to optimize the reaction conditions in both cases.

One feature of the method for amplifying a nucleic acid of the present invention is that the method does not require adjusting the temperature up and down during the nucleic acid synthesis. Thus, the present invention provides a method for isothermally synthesizing a nucleic acid. Many of conventional nucleic acid amplification methods require adjusting the temperature up and down to dissociate a target from a synthesized strand. These methods require a special reaction equipment such as a thermal cycler for this purpose. However, the method of the present invention can be conducted only using an equipment that can keep a constant temperature. As described above, the method of the present invention can be conducted at a single temperature. Preferably, it is conducted by selecting the reaction temperature and the stringency level such that non-specific annealing of a primer is reduced and such that the primer specifically anneals to a nucleic acid as a template. Although it is not intended to limit the present invention, the method of the present invention can be conducted under high-temperature conditions by using a heat-resistant enzyme as described above. In addition, it is preferable to conduct the method of the present invention at an appropriate temperature for sufficiently retaining the activity of the enzyme used in order to maintain the reaction efficiency at high level. Thus, the reaction temperature is preferably about 20°C to about 80°C, more preferably about 30°C to about 75°C, most preferably about 50°C to about 70°C although it varies depending on the enzyme used. When the reaction is conducted under high-temperature conditions in particular, it is preferable to use a longer primer than that for a reaction at a normal temperature. The sequence and the length of the primer appropriate for the reaction temperature may be determined, for example, with reference to its Tm value. Alternatively, a commercially available software for designing a primer such as OLIGO™ Primer Analysis software (Takara Shuzo) may be used. For example, when a reaction temperature of 55°C to 60°C or 65°C is used, the primer used for the method of the present invention may be, for example, without limitation, 12-100 nucleotides in length, preferably 14-50 nucleotides in length, more preferably 15-40 nucleotides in length. An example of effects brought by the elevated reaction temperature is the solution of a problem of forming secondary structure of a DNA as a template. The elevated reaction temperature enables amplification of a desired nucleic acid even if a nucleic acid having a high GC content is used as a template. Furthermore, it is similarly effective in amplifying a region of a long chain length. Such effect is observed in a range between about 60 bp and about 20 kbp, specifically between about 110 bp and about 4.3 kbp, more specifically about 130 bp and about 1500 bp.

The amplification efficiency can be increased by adjusting the reaction temperature in accordance with the GC content of the nucleic acid as the template. For example, if a nucleic acid having a low GC content is used as a template, the amplification reaction of the present invention can be conducted at 50 to 55°C, although the temperature depends on the chain length to be amplified and the Tm value of the primer.

Use of a DNA polymerase having a reverse transcriptase activity (e.g., BcaBEST DNA polymerase) in the method of the present invention can make the amplification of a nucleic acid from an RNA, which comprises a step of preparing a cDNA from an RNA (a reverse transcription reaction), be conveniently conducted. Alternatively, a product obtained by independently conducting a step of preparing a cDNA from an RNA, i.e., a cDNA, can be used in the method of the present invention as the DNA as a template.

In each case, the reaction in the method of the present invention is repeated until it is terminated by appropriate means, for example, by inactivating the enzyme or by lowering the reaction temperature, or until the reaction is deprived of one of the substrates.

The method for amplifying a nucleic acid of the present invention can be used for various experimental procedures that utilize amplification of a nucleic acid including detection, labeling and sequencing of a nucleic acid.

Furthermore, the method for amplifying a nucleic acid of the present invention can be used for an in situ nucleic acid amplification method, a method for amplifying a nucleic acid on a solid substrate such as a DNA chip, or a multiplex nucleic acid amplification method in which plural regions are simultaneously amplified.

One of the features of the method for amplifying a nucleic acid of the present invention is its ability to prepare a single-stranded DNA. One or two chimeric oligonucleotide primers can be used in the method for this purpose. For example, if two oligonucleotide primers are used, the method of the present invention can be conducted applying a similar primer ratio to the so-called asymmetric-PCR in which an amplification reaction is carried out: by using an excess amount of one oligonucleotide primer relative to another. The primer ratio is, without limitation, preferably in a range of 1:10 to 1:500, more preferably in a range of 1:10 to 1:100. As a result, the amount of the replacement product from one strand becomes excessive relative to that from another.

According to the method for amplifying a nucleic acid of the present invention, a single-stranded DNA substantially free of a complementary strand thereto can be prepared. For example, a single-stranded DNA for producing a material having an immobilized nucleic acid such as a DNA chip, a single-stranded DNA probe for detecting a target nucleic acid, or a mega-primer for the long-chain PCR can be readily produced in a short time. Only a sense sequence or an antisense sequence can be selectively amplified by using the method of the present invention. Thus, the present invention is also useful as a method for producing a nucleic acid having a sense sequence or a antisense sequence.

A region of a nucleic acid of interest can be amplified even from a trace amount of a nucleic acid as a template by conducting the method of the present invention in a buffer of Bicine, Tricine, HEPES, phosphate or tris.

Furthermore, the methods for amplifying a nucleic acid of the present invention does not require the specific reaction equipment that can adjust a temperature with the passage of time. Therefore, an amplification reaction can be conducted in a large volume of reaction mixture. Thus, a nucleic acid (e.g., for medical use) can be industrially produced in large quantities.

The utilization efficiency of the primer in the method for amplifying a nucleic acid of the present invention is about 100%, which may be 5- to 10-fold higher than that in a conventional method such as the PCR.

The nucleic acid amplification method of the present invention can produce an amplification product with high fidelity to the nucleotide sequence of the template nucleic acid. When the frequency of error in the DNA synthesis in the method of the present invention was confirmed by analyzing the nucleotide sequences of resulting amplification products, the frequency of error found in amplification products obtained by the method of the present invention was equivalent to that by LA-PCR which is known to be able to amplify a nucleic acid with high fidelity. In other words, the method of the present invention has fidelity equivalent to that of the LA-PCR.

(6) Method for detecting target nucleic acid of the present invention and kit for the method.

A target nucleic acid in a sample can be detected by using the method for amplifying a nucleic acid of the present invention. The detection method comprises:

(a) amplifying a target nucleic acid by the method for amplifying a nucleic acid of the present invention as described above; and
(b) detecting the target nucleic acid amplified in the step above.

In step (a) above, if an RNA is used as a template, the reverse transcription reaction and the nucleic acid amplification reaction may be conducted in one step. Although it is not intended to limit the present invention, for example, a combination of AMV RTase, MMLV RTase or RAV-2 RTase and Bca DNA polymerase can be preferably used as a combination of a reverse transcriptase and a strand displacement-type DNA polymerase.

The method can be utilized to detect or quantify a specific gene in a sample. In other words, a specific gene can be detected or quantified from all samples suspected to contain a nucleic acid such as a DNA or an RNA. Examples of the samples from which a specific gene can be detected or quantified include, but are not limited to, samples from organisms such as a whole blood, a serum, a buffy coat, a urine, feces, a cerebrospinal fluid, a seminal fluid, a saliva, a tissue (e.g., a cancerous tissue or a lymph node) and a cell culture (e.g., a mammalian cell culture or a bacterial cell culture), samples that contain a nucleic acid such as a viroid, a virus, a bacterium, a fungi, a yeast, a plant and an animal, samples suspected to be contaminated or infected with a microorganism such as a virus or a bacterium (e.g., a food or a biological formulation), and samples that may contain an organism such as a soil and a waste water. For example, a viroid, a virus, a fungi, a bacterium or other microorganisms in a sample can be detected or quantified on the basis of the presence or the content of a specific gene derived from these microorganisms as a target. Particularly, a method for detecting a pathogenic microorganism is useful in fields of sanitation and environment. Furthermore, the method of the present invention can be used to distinguish a genotype of an organism or to determine the expression level of a gene. Particularly, detection or confirmation of the expression state of a disease-related gene, e.g., a gene related to canceration of cells is useful in a field of medicine. Both of an RNA and a DNA can be preferably used as the nucleic acid as the template in the detection.

The method for detecting a target nucleic acid of the present invention can be used to distinguish the difference in the nucleotide sequence of the target nucleic acid. In this aspect, the chimeric oligonucleotide primer to be used is designed such that the 3'-terminal portion of the primer is positioned close to the specific base of the target nucleotide sequence to be distinguished. For example, it is designed such that a hydrogen bond is formed between the base and the 3'-terminal base of the primer. If a mismatch exists between the nucleotide sequence of the 3'-terminal portion of the primer and the nucleotide sequence of the template, amplification from the target nucleic acid does not take place and no amplification product is generated using the above-mentioned chimeric oligonucleotide primer for amplification reaction. Information concerning a specific base in a gene such as a point mutation or a single nucleotide polymorphism (SNP) can be obtained using the method.

The method for detecting a target nucleic acid of the present invention can be conducted by amplifying the target nucleic acid directly from a sample containing the nucleic acid. In this case, the chain length of the target nucleic acid to be amplified is not limited to a specific one. For example, a region of 200 bp or shorter, preferably 150 bp or shorter is effective for sensitive detection of the target nucleic acid. The target nucleic acid in the sample can be detected with high sensitivity by designing the chimeric oligonucleotide primers of the present invention to result in the chain length to be amplified as described above.

In addition, a target nucleic acid can be detected with more sensitivity even from a trace amount of a nucleic acid sample in the detection method of the present invention by using a reaction buffer containing Bicine, Tricine, HEPES, phosphate or tris as a buffering component and an annealing solution containing spermidine or propylenediamine as exemplified in (4) above. In this case, the endonuclease and the DNA polymerase to be used are not limited to specific ones. For example, a combination of an RNase H from Escherichia coli, a bacterium of genus Pyrococcus or a bacterium of genus Archaeoglobus and BcaBEST DNA polymerase is preferable. It is considered that the preferable units of the endonuclease and the DNA polymerase may vary depending on the types the enzymes. In such a case, the composition of the buffer and the amount of the enzymes added may be adjusted using the increase in detection sensitivity or the amount of amplification product as an index.

In the detection method of the present invention, dUTP may be incorporated as a substrate during amplification of a target nucleic acid. Thus, if dUTP is used as a substrate, it is possible to prevent carry-over contamination of amplification products by degrading amplification products utilizing uracil N-glycosidase (UNG).

Known methods for detecting a nucleic acid can be used for step (b). Examples of such methods include detection of a reaction product having a specific size by electrophoresis, and detection by hybridization with a probe. Furthermore, a detection method in which magnetic beads are combined can be preferably used. A fluorescent substance such as ethidium bromide is usually used in the detection by electrophoresis. The hybridization with a probe may be combined with the detection by electrophoresis. The probe may be labeled with a radioisotope or with a nonradioactive substance such as biotin or a fluorescent substance. Additionally, use of a labeled nucleotide in step (a) may facilitate the detection of amplification product into which the labeled nucleotide is incorporated, or may enhance the signal for detection utilizing the label. A fluorescence polarization method, a fluorescence energy transition or the like can also be utilized for the detection. The target nucleic acid can be detected automatically or quantified by constructing a suitable detection system. In addition, detection with naked eyes by a hybrid chromatography method can be preferably used.

A ribonucleotide (RNA) probe labeled with two or more fluorescent substances positioned at a distance that results in a quenching state can be used in the detection method of the present invention. The probe does not emit fluorescence. When it is annealed to a DNA amplified from a target nucleic acid that is complementary to the probe, RNase H digests the probe. The distance between the fluorescent substances on the probe then increases, resulting in the emission of fluorescence. Thus, the emission reveals the presence of the target nucleic acid. If RNase H is used in the method for amplifying a nucleic acid of the present invention, a target nucleic acid can be detected only by adding the probe to the reaction mixture. For example, a combination of fluorescent substances, 6-carboxyfluorescein (6-FAM) and N,N,N',N'-tetramethyl-6-carboxyrhodamine (TAMRA), can be preferably used for labeling the probe.

The present invention further provides a probe used in the above-mentioned method for detecting a target nucleic acid. The probe of the present invention is not limited to specific one as long as it can hybridize to a target nucleic acid amplified by the nucleic acid amplification method of the present invention under normal hybridization conditions. In view of specific detection of amplification product, a probe that hybridizes under conditions, for example, known to those skilled in the art as being stringent is preferable. The stringent hybridization conditions are described in, for example, T. Maniatis et al. (eds.), Molecular Cloning: A Laboratory Manual 2nd ed., 1989, Cold Spring Harbor Laboratory. Specifically, the stringent conditions are exemplified by the following: incubation at a temperature about 25°C lower than the Tm of the probe to be used for 4 hours to overnight in 6 x SSC (1 x SSC: 0.15 M NaCl, 0.015 M sodium citrate, pH 7.0) containing 0.5% SDS, 5 x Denhardt's (0.1% bovine serum albumin (BSA), 0.1% polyvinylpyrrolidone, 0.1% Ficoll 400) and 100 μg/ml salmon sperm DNA. A probe having a label as described above may be used as the probe for facilitating the detection of the target nucleic acid.

The method for amplifying a nucleic acid under isothermal conditions of the present invention does not require the use of an equipment such as a thermal cycler. The number of primers used in the amplification method of the present invention can be one or two, which is less than that used in a conventional method. Since reagents such as dNTPs used for PCR and the like can be applied to the method of the present invention, the running cost can be reduced as compared with a conventional method. Therefore, the method of the present invention can be preferably used, for example, in a field of genetic test in which the detection is routinely conducted. The method of the present invention provides a greater amount of an amplification product in a shorter time than the PCR. Therefore, the method of the present invention can be utilized as a convenient, rapid and sensitive method for detecting a gene.

In genetic analysis on a genomic level, attempts are made to make the reaction system small and to increase the degree of integration in order to analyze a large amount of nucleotide sequences. An system has been developed for this purpose by utilizing the latest hyperfine processing techniques in which basic processes for genome analysis (e.g., extraction of DNA from cells, DNA amplification reaction, electrophoresis, hybridization, detection of the DNA of interest, etc.) are integrated on a microchip of several square centimeters to fingertip size. Such a system is called as a microchip or a nanochip.

Application of the PCR to the system as a gene amplification reaction is currently considered. However, since the PCRs requires means for repeatedly controlling temperature up and down over time, the system would become complicated. By contrast, since the system can be simplified using the method of the present invention which can amplify a nucleic acid under isothermal conditions, the method is quite suitably utilized for the integrated system as described above. A highly integrated system can be constructed by utilizing the techniques of the present invention.

(7) Kit of the present invention

The present invention provides a kit used for the method for amplifying or detecting a nucleic acid of present -invention as described above. In one embodiment, the kit is in a packaged form and contains instructions regarding the use of a DNA polymerase and an endonuclease in a strand displacement reaction. Also, a kit that contains a DNA polymerase having a strand displacement activity, an endonuclease, and a buffer for a strand displacement reaction is preferably used for the method of the present invention. Alternatively, a commercially available DNA polymerase having a strand displacement activity and/or endonuclease may be selected and used according to the instructions. Additionally, the kit may contain a reagent for a reverse transcription reaction that is used when an RNA is used as a template. The DNA polymerase can be selected from the DNA polymerases to be used in the present invention as described above in (3). The endonuclease can be selected from the endonucleases as described above in (2). One having the reaction buffer composition as described above in (4) can be preferably used as the buffer for the strand displacement reaction.

"Instructions" are printed matters describing a method of using the kit, e.g., a method for preparing a reagent solution for a strand displacement reaction, recommended reaction conditions and the like. The instructions include an instruction manual in a form of a pamphlet or a leaflet, a label stuck to the kit, and description on the surface of the package containing the kit. The instructions also include information disclosed or provided through electronic media such as the Internet.

The kit of the present invention may further contain a reaction buffer containing Bicine, Tricine, HEPES, phosphate or tris as a buffering component and an annealing solution as exemplified in (4) above. Additionally, it may contain a DNA polymerase having a strand displacement activity and an RNase H. Furthermore, the kit may contain a modified deoxyribonucleotide or a deoxynucleotide triphosphate analog.

The kit used in the method for detecting a target nucleic acid may further contain an appropriate chimeric oligonucleotide primer for amplifying the target nucleic acid and a reagent for detecting the amplified target nucleic acid such as a probe in addition to the instructions and the reagents for amplification reaction as described above.

In addition, the kits of the present invention include a kit containing the chimeric oligonucleotide primer used in the present invention and/or the probe of the present invention as described above.

(8) Composition of the present invention.

The present invention provides a composition used for the method for amplifying a nucleic acid of the present invention or the method for detecting a nucleic acid of the present invention as described above. For example, the composition may contain the endonuclease as described in (2) above and the DNA polymerase as described in (3) above. The composition may further contain a buffering component, a magnesium salt, dNTPs and the like as components for conducting an amplification reaction. Furthermore, it may contain a modified deoxyribonucleotide or a deoxynucleotide triphosphate analog. Those as described in (4) above can be used as the buffering component and other additives.

In a particularly preferable aspect, the composition may contain suitable amounts of the various components as listed above for the nucleic acid amplification method of the present invention. Amplification reaction can be conducted only by adding an appropriate template and chimeric oligonucleotide primer(s) to the composition. If the object to be amplified is known beforehand, the composition preferably contains chimeric oligonucleotide primer(s) suitable for amplifying the object.

(9) Material having immobilized nucleic acid arrayed in predefined region of the present invention and method for producing the same.

A DNA chip (also referred to as a DNA microarray or a DNA array) is a material having an immobilized nucleic acid in which various fragments of genes or DNAs are arrayed and immobilized in a predefined region or at a predefined position on a solid substrate such as a slide glass. The DNA chip is used for examining the presence of a nucleic acid in a nucleic acid sample that has a sequence complementary to an arrayed and immobilized DNA in a predefined region on the DNA chip. The examination is carried out by contacting the DNA chip with the nucleic acid sample prepared from a sample, preferably a labeled nucleic acid sample, for hybridization. Since the DNA chip can be used to detect or quantify a number of nucleic acids in a sample in one procedure, it is a very useful means that dramatically promotes the analysis of gene expression, or the analysis of a mutation or polymorphism. A DNA chip in which a double-stranded nucleic acid is arrayed and immobilized in a predefined region is used for hybridization after it is subjected to appropriate denaturation. A DNA chip in which a single-stranded DNA complementary to a target nucleic acid to be detected is arrayed and immobilized in a predefined region is particularly preferable for the detection of a target nucleic acid.

As described above, a desired DNA can be amplified in a single-stranded form by the method of the present invention. Although any method for purifying an amplification product can be used, purification using isopropanol precipitation is preferable. The thus obtained DNA, preferably a single-stranded DNA substantially free of a complementary strand thereto, can be preferably used as a DNA fragment to be immobilized onto a DNA chip. Thus, the method of the present invention is preferably used as a method for preparing a DNA to be arrayed and immobilized in a predefined region for producing a DNA chip. Any insoluble substrate can be used as a substrate onto which the thus obtained DNA is arrayed and immobilized in a predefined region, but a plate-shaped substrate made from glass or plastic, and a membrane-shaped substrate made from nitrocellulose or nylon are preferably used. A known method for immobilizing a nucleic acid can be used for the immobilization. The DNA may be directly immobilized onto a substrate. Alternatively, the DNA may be immobilized through a suitable linker or after ligating plural DNA molecules. Furthermore, since a modified deoxyribonucleotide can be incorporated into an amplified nucleic acid by the production method of the present invention, the material having an immobilized nucleic acid can be produced utilizing the modification group.

A target nucleic acid that hybridizes with a nucleic acid on a material having an immobilized nucleic acid in which a DNA amplified by the method of the present invention is arrayed and immobilized in a predefined region (e.g., a DNA chip) can be detected or quantified. Such detection or quantification can be accomplished by contacting the material with a nucleic acid sample prepared from a sample suspected to contain the target nucleic acid for hybridization. Among these, a DNA chip in which a single-stranded DNA amplified by the method of the present invention is arrayed and immobilized in a predefined region allows the detection of a target nucleic acid with more convenient operation, higher sensitivity and higher reproducibility as compared with a conventional material.

(10) Method for producing nucleic acid in large quantities of the present invention.

As described above, one aspect of the present invention provides a method for amplifying a nucleic acid that can be carried out under isothermal conditions. A desired nucleic acid can be produced in the method by mixing a nucleic acid as a template for the nucleic acid to be amplified and various components required for a reaction and reacting the mixture under isothermal conditions. Since the PCR requires changing the temperature of the reaction mixture over time, the reaction volume is limited to one in which the temperature can be controlled (usually, 200 μl or less). Therefore, it is difficult to scale up the volume. On the other hand, there is no such limitation in the method of the present invention. A large amount of nucleic acid can be produced by increasing the volume of the reaction mixture. In the method of the present invention, the volume is, for example, preferably more than 200 μl, more preferably 300 μl or more, and most preferably 500 μl or more, although it is not intended to limit the present invention. In the method of the present invention, a number of complementary strand molecules are synthesized from one template molecule. Furthermore, nucleic acids can be synthesized using these complementary strand molecules as templates. Thus, a desired nucleic acid can be efficiently produced in large quantities by suitably selecting the template and the primer. Additionally, the fact that, unlike the PCR, the method of the present invention does not require a special equipment or a complicated temperature change makes it advantageous in view of the cost of equipment and energy. Therefore, the method is an excellent industrial method for producing a nucleic acid in large quantities.

The nucleic acid of interest can be amplified or produced even from a trace amount of a nucleic acid as a template in the production method of the present invention by using the reaction buffer and the annealing solution as exemplified in (4) above. In this case, the endonuclease and the DNA polymerase to be used are not limited to specific ones. For example, a combination of an RNase H from Escherichia coli and BcaBEST DNA polymerase is preferable. It is considered that the preferable units of the endonuclease and the DNA polymerase may vary depending on the types of the enzymes. In such a case, the composition of the buffer and the amount of the enzymes added may be adjusted using the maximal amount of amplification product as an index.

Furthermore, the method of the present invention is useful as a method for supplying a variety of DNA fragments in large quantities, such as those to be immobilized onto the DNA chip. Specifically, DNA fragments can be obtained in large quantities in simple reaction steps in one embodiment. In another embodiment, a limited number of primers can be used to obtain a variety of DNA fragments. A step of amplifying- the nucleic acid that serves as the template in the method of the present invention beforehand by a known nucleic acid amplification method such as the PCR can be incorporated in the latter embodiment. All kinds of nucleic acids as templates can be amplified using a limited number of primers, for example, based on the method for amplifying a nucleic acid using a random primer having a tag sequence (Nucleic Acids Research, 24(19):3778-3783 (1996)) or the degenerate oligonucleotide-primed PCR (DOP-PCR; Genomics, 13:718-725 (1992)), which uses a degenerate primer. The nucleic acid amplification method of the present invention can be conducted using one or several primers for all of the nucleic acids as templates produced in the above-mentioned step. This can be accomplished by designing the primer used in the nucleic acid amplification method of the present invention such that it corresponds to the tag sequence added to the random or degenerate primer. Thus, a combination of a suitable step for preparing a nucleic acid as a template and the method of the present invention can supply a variety of DNA fragments in larger quantities and at a lower cost as compared with a conventional method.

A pharmaceutical composition containing a nucleic acid may contain a double-stranded DNA for expressing a useful polypeptide in a cell or a single-stranded antisense DNA for suppressing the expression of a gene of interest. Such a nucleic acid is administered into an organism using suitable means, for example, a carrier for gene transfer such as liposome. The method for producing a nucleic acid of the present invention is preferable for producing a single-stranded or double-stranded nucleic acid for medical use in large quantities. Additionally, a nucleic acid containing a dNTP analog that, for example, suppresses the degradation of the nucleic acid in vivo can be readily produced by the method of the present invention.

Since the DNA fragment amplified in the present invention is composed of normal nucleotides, the amplified DNA can be, for example, subcloned into a suitable vector utilizing a restriction enzyme site in the DNA. Furthermore, the DNA can be treated with a restriction enzyme for RFLP without a problem, for example. Therefore, the DNA can be widely utilized in a field of genetic test. In addition, a promoter sequence for an RNA polymerase can be incorporated into the amplified fragment. The amplified fragment can be used as a template to synthesize an RNA, which can be used as a probe, for example. Of course, a fluorescence-labeled DNA probe can be produced by conducting the method for amplifying a nucleic acid of the present invention using a fluorescence-labeled dNTP instead of a normal dNTP.

Features of the method for amplifying a nucleic acid of the present invention are listed below.

1. It can amplify a large amount of a nucleic acid from a small amount of a template. The amplification product increases quadratically when two primers are used.
2. It can be conducted isothermally. In this case, it does not require the use of an equipment such as a thermal cycler. Therefore, the reaction volume can be readily scaled up.
3. Usually, the amplification reaction is conducted using one or two chimeric oligonucleotide primer and two enzymes (a DNA polymerase and an endonuclease).
4. Since a number of DNA strands are synthesized from one molecule of a primer, the amount of the primer does not restrict the amount of the amplification product. Furthermore, the primer utilization efficiency is about 100%, which is very higher than that of the PCR.
5. A single-stranded or double-stranded DNA can be selectively amplified depending on the purpose.
6. Since it does not require a dNTP analog such as an ($\alpha$-S) dNTP for the amplification reaction, the cost of reagents is low. Furthermore, a nucleic acid in a natural form without a dNTP analog can be obtained.
7. It can supply an amplified DNA fragment at low cost and in large quantities by combining the method of the present invention with another nucleic acid duplication method.
8. The detection method of the present invention has an equal or higher detection sensitivity as compared with that of a conventional method. The detection method of the present invention can be conducted in a shorter time than that required for a conventional method having the equal sensitivity.
9. The method is suitable for amplification of a nucleic acid, automated detection, detection in a small amount and highly integrated detection on a microchip or a nanochip.

As described above, the method of the present invention is suitable for detection of a gene and industrial production of a nucleic acid.

Examples

The following Examples illustrate the present invention in more detail, but are not to be construed to limit the scope thereof.

Referential Example 1: Preparation of RNase H from thermophile Bacillus caldotenax

Bacillus caldotenax YT-G (purchased from Deutsche Sammlung von Mikroorganismen; DSM406) was inoculated into 100 ml of a medium containing 0.2% Tryptone (Difco Laboratories) and 1.5% yeast extract (Difco Laboratories) (pH 6.5), cultured at 60°C for 140 minutes with shaking and used as a pre-culture. 30 ml of the pre-culture was inoculated into 3 L of a medium having the same composition and cultured with aeration at 2.5 L/minute and stirring at 250 rpm at a temperature of 60°C for 5 hours.

The cells were collected by centrifuging the culture at 5000 x g for 15 minutes. 402 g (wet weight) of the cells were suspended in 1000 ml of 50 mM tris-HCl buffer (pH 7.5) containing 10 mM mercaptoethanol, 0.5 M NaCl, 1 mM EDTA and 20 μM PAPMSF and disrupted using MINI-Lab (APV GAULIN/RANNIE). Cell debris were removed by centrifugation to recover a supernatant.

A polyethylene imine solution was added to the resulting supernatant at a final concentration of 0.1%. After stirring, the mixture was allowed to stand for 1 hour. A supernatant was then recovered by centrifugation. Ammonium sulfate was added to the supernatant to 50% saturation. A precipitate obtained by centrifugation was dissolved in 20 mM tris-HCl buffer (pH 7.5) containing 10 mM mercaptoethanol, 0.1 mM EDTA, 50 mM NaCl and 10% glycerol. The solution -was dialyzed against the same buffer. The dialyzed sample was loaded onto a 280-ml DE52 column (Whatman) equilibrated with the same buffer and non-adsorptive fractions were collected.

The column was further washed with 420 ml of the buffer used for the equilibration and washing fractions were collected. The non-adsorptive fractions and the washing fractions from the DE52 column chromatography were mixed together and loaded onto a 240-ml P-11 column (Whatman) equilibrated with 20 mM tris-HCl buffer (pH 7.5) containing 10 mM mercaptoethanol, 0.1 mM EDTA, 50 mM NaCl and 10% glycerol. Elution was then carried out using the equilibration buffer containing 0 to 0.5 M NaCl.

The resulting active fractions were placed in a dialysis tube. The tube was placed on solid polyethylene glycol 20000 for dehydration-concentration at 4°C. The enzyme concentrate was then loaded onto a 300-ml Superdex G-200 column (Amersham Pharmacia Biotech) equilibrated with 25 mM tris-HCl buffer (pH 7.5) containing 5 mM mercaptoethanol, 0.5 mM EDTA, 30 mM NaCl and 50% glycerol. Elution was carried out using the buffer used for equilibration to obtain active fractions. The active fractions were loaded onto a 15-ml Heparin-Sepharose column (Amersham Pharmacia Biotech) equilibrated with 20 mM tris-HCl buffer (pH 7.5) containing 10 mM mercaptoethanol, 0.1 mM EDTA, 50 mM NaCl and 10% glycerol. Elution was carried out using the equilibration buffer containing 0 to 0.5 M NaCl.

The resulting active fractions were loaded onto a 5-ml Hitrap-SP column (Amersham Pharmacia Biotech) equilibrated with 20 mM tris-HCl buffer (pH 7.5) containing 10 mM mercaptoethanol, 0.1 mM EDTA, 50 mM NaCl and 10% glycerol. Elution was carried out using the equilibration buffer containing 0 to 0.5 M NaCl. The resulting active fractions were loaded onto a 300-ml Superdex G-200 column (Amersham Pharmacia Biotech) equilibrated with 25 mM tris-HCl buffer (pH 7.5) containing 5 mM mercaptoethanol, 0.5 mM EDTA, 30 mM NaCl and 50% glycerol again. The resulting active fractions were used as an RNase H preparation (an enzyme solution).

A heat-resistant RNase H activity was measured as follows.

1 mg of poly(rA) or poly(dT) (both from Amersham Pharmacia Biotech) was dissolved in 1 ml of 40 mM tris-HCl (pH 7.7) containing 1 mM EDTA to prepare a poly(rA) solution and a poly(dT) solution.

The poly(rA) solution (to a final concentration of 20 μg/ml) and the poly(dT) solution (to a final concentration of 30 μg/ml) were then added to 40 mM tris-HCl (pH 7.7) containing 4 mM $MgCl_2$, 1 mM DTT, 0.003% BSA and 4% glycerol. The mixture was reacted at 37°C for 10 minutes and then cooled to 4°C at prepare a poly(rA)-poly(dT) solution.

1 μl of an enzyme solution was added to 100 μl of the poly(rA)-poly(dT) solution. The mixture was reacted at 40°C for 10 minutes. 10 μl of 0.5 M EDTA was added thereto to terminate the reaction. Absorbance at 260 nm was then measured. As a control, 10 μl of 0.5 M EDTA was added to the reaction mixture, the resulting mixture was reacted at 40°C for 10 minutes, and the absorbance was then measured. A value (difference in absorbance) was obtained by subtracting the absorbance for the control from the absorbance for the reaction in the absence of EDTA. Thus, the concentration of nucleotide released from poly(rA)-poly(dT) hybrid by the enzymatic reaction was determined on the basis of the difference in absorbance. One unit of an RNase H was defined as an amount of enzyme that increases $A_{260}$ corresponding to release of 1 nmol of ribonucleotide in 10 minutes calculated according to the following equation. If a diluted enzyme solution is used, the value obtained using the following equation was corrected based on the dilution rate:

$$\text{Unit} = [\text{Difference in Absorbance} \times \text{Reaction Volume (ml)}] / 0.0152$$

Referential Example 2: Cloning of Bacillus caldotenax RNase HII gene

(1) Preparation of genomic DNA from Bacillus caldotenax

Bacillus caldotenax YT-G (DSM406) was inoculated into 60 ml of LB medium (1% Tryptone, 0.5% yeast extract and 0.5% NaCl, pH 7.2) and cultured at 65°C for 20 hours. After culturing, the culture was centrifuged to collect cells. The cells were suspended in 2 ml of 25% sucrose and 50 mM tris-HCl (pH 8.0). 0.2 ml of 10 mg/ml lysozyme chloride (Nacalai Tesque) in water was added thereto. The mixture was reacted at 20°C for 1 hour. After reaction, 12 ml of a mixture containing 150 mM NaCl, 1 mM EDTA and 20 mM tris-HCl (pH 8.0), 0.1 ml of 20 mg/ml proteinase K (Takara Shuzo) and 1 ml of a 10% aqueous solution of sodium lauryl sulfate were added to the reaction mixture. The mixture was incubated at 37°C for 1 hour.

2.1 ml of 5 M NaCl and 2 ml of a CTAB-NaCl solution [10% cetyltrimethylammonium bromide (Nacalai Tesque) and 0.7 M NaCl] were then added to the mixture and the resulting mixture was mixed thoroughly and incubated at 65°C for 10 minutes. An equal volume of a mixture of chloroform/isoamyl alcohol (24 : 1, v/v) was added thereto. The resulting mixture was gently mixed for 10 minutes and then centrifuged for 10 minutes at 10000 x g. After centrifugation, an equal volume of a mixture of phenol saturated with 100 mM tris-HCl (pH 8.0)/chloroform/isoamyl alcohol (25 : 24: 1, v/v) was added to the resulting supernatant. The resulting mixture was gently mixed for 10 minutes and then centrifuged for 10 minutes at 10000 x g. After centrifugation, a 0.6 volume of 2-propanol was added to the resulting supernatant. The resulting fibrous precipitate was wound using a glass bar, washed with 70% ethanol in water, air-dried and then dissolved in 0.5 ml of TE buffer to obtain a genomic DNA solution.

(2) Cloning of middle portion of RNase HII gene

Oligonucleotides BsuII-3 and BsuII-6 represented by SEQ ID NOS:1 and 2 were synthesized on the basis of Motif I and Motif III, portions conserved among amino acid. sequences of RNase HIIs from various organisms (Biochemistry, 38:605-608 (1999)).

A PCR was carried out in a volume of 100 µl using 1 µl of the Bacillus caldotenax genomic DNA solution as prepared in Referential Example 2-(1) as a template, and 100 pmol of BsuII-3 and 100 pmol of BsuII-6 as primers. TaKaRa Taq polymerase (Takara Shuzo) was used as a DNA polymerase for the PCR according to the attached protocol. The PCR was carried out as follows: 50 cycles of 94°C for 30 seconds, 45°C for 30 seconds and 72°C for 1 minute. After reaction, the reaction mixture was subjected to phenol treatment followed by ethanol precipitation to purify a DNA. The resulting DNA was blunt-ended using T4 DNA polymerase (Takara Shuzo) and then subjected to agarose gel electrophoresis to recover an amplified DNA fragment of about 0.4 kb from the gel. The about 0.4-kb DNA fragment was ligated with pUC119 (Takara Shuzo) digested with Sma I (Takara Shuzo) using T4 DNA ligase (Takara Shuzo). The ligation mixture was used to transform Escherichia coli JM109. The resulting transformants were cultured to obtain a plasmid 21-12 into which the about 0.4-kb DNA was inserted.

(3) Cloning of upstream portion of RNase II gene

The nucleotide sequence of the inserted fragment of about 0.4 kb in the plasmid 21-12 obtained in Referential Example 2-(2) was determined. Oligonucleotides RNII-S1 and RNII-S2 represented by SEQ ID NOS:3 and 4 were synthesized on the basis of the determined nucleotide sequence.

The Bacillus caldotenax genomic DNA as prepared in Referential Example 2-(1) was digested with BamHI (Takara Shuzo) and ligated with a Sau3AI cassette (Takara Shuzo) using T4 DNA ligase. A procedure was carried out according to the protocol attached to TaKaRa LA PCR in vitro cloning kit (Takara Shuzo) using the ligation mixture as a template, RNII-S2 as a primer for a primary PCR and RNII-S1 as a primer for a secondary PCR. A DNA was purified from the solution after the secondary PCR by phenol extraction followed by ethanol precipitation. The DNA was blunt-ended using T4 DNA polymerase and then subjected to agarose gel electrophoresis to recover an amplified DNA fragment of about 1.5 kb from the gel. The about 1.5-kb DNA fragment was ligated with pUC119 digested with Sma I using T4 DNA ligase. The ligation mixture was used to transform Escherichia coli JM109.

The resulting transformants were cultured to obtain a plasmid B25N16 into which the about 1.5-kb DNA was inserted.

(4) Cloning of entire RNase II gene

Oligonucleotides RNII-S5 and RNII-S6 represented by SEQ ID NOS:5 and 6 were synthesized on the basis of the nucleotide sequence of the inserted fragment of about 0.4 kb in the plasmid 21-12 as determined in Referential Example 2-(3).

A PCR was carried out using the plasmid 21-12 as prepared in Referential Example 2-(2) as a template, and RNII-S5 and RNII-S6 as primers. TaKaRa Ex Taq polymerase (Takara Shuzo) was used as a DNA polymerase for the PCR according to the attached protocol. The PCR was carried out as follows: 25 cycles of 94°C for 30 seconds, 55°C for 30 seconds and 72°C for 30 seconds. After reaction, the reaction mixture was subjected to agarose gel electrophoresis. An amplified DNA fragment of about 0.3 kb was recovered from the gel. The about 0.3-kb DNA-fragment was labeled with digoxigenin using DIG High-prime (Roche Diagnostics).

Southern hybridization was carried out for digests of the Bacillus caldotenax genomic DNA as prepared in Referential Example 2-(1) with HindIII (Takara Shuzo), Sac I (Takara Shuzo), or HindIII and SacI using the digoxigenin-labeled DNA as a probe. Hybridization and detection were carried out using DIG Luminescent Detection Kit (Roche Diagnostics) according to the protocol attached thereto. As a result, DNA fragments of about 4.5 kb, about 5.8 kb and about 1.3 kb were hybridized with the probe for the digests with HindIII, SacI, and HindIII and SacI, respectively.

Based on these results, the Bacillus caldotenax genomic DNA was digested with HindIII and subjected to agarose gel electrophoresis to recover DNAs of about 4.5 kb from the gel. The resulting DNA fragments were digested with SacI and subjected to agarose gel electrophoresis to recover DNAs of about 1.3 kb from the gel. The resulting DNAs were ligated with pUC19 (Takara Shuzo) digested with HindIII and SacI using T4 DNA ligase. The ligation mixture was used to transform Escherichia coli HB101.

The resulting transformants were replica-plated onto Hybond-N™ (Amersham Pharmacia Biotech). Colony hybridization was then carried out using the above-mentioned digoxigenin-labeled probe according to a conventional method. A plasmid pRHB1 was prepared from the thus obtained positive clone.

The nucleotide sequence of the DNA inserted into pRHB1 was then determined. Comparison of the amino acid sequence deduced from the nucleotide sequence with the amino acid sequence of the RNase HII from Bacillus subtilis suggested that a region of about 40 bp from the initiation codon was missing in the DNA in pRHB1. Then, the full-length RNase H gene was constructed as follows.

B25N16 as prepared in Referential Example 2-(3) was digested with HindIII and subjected to agarose gel electrophoresis to recover a DNA fragment of about 160 bp from the gel. The about 160-bp DNA fragment was ligated with pRHB1 digested with HindIII using T4 DNA ligase. The ligation mixture was used to transform Escherichia coli HB101. Plasmids were prepared from the resulting transformants.

Next, an oligonucleotide RNII-Nde represented by SEQ ID NO:7 was synthesized on the basis of the presumed nucleotide sequence around the initiation codon. PCRs were carried out using the plasmids prepared from the transformants as templates, and RNII-Nde and RNII-S6 as primers. A plasmid from which a DNA fragment of about 0.7 kb was amplified was selected and designated as pRHB11.

The nucleotide sequence of the DNA fragment inserted into the thus obtained plasmid pRHB11 was determined. Analysis of the results revealed an open reading frame presumably encoding RNase HII. This nucleotide sequence is shown in SEQ ID NO:8. The amino acid sequence of RNase HII deduced from the nucleotide sequence is shown in SEQ ID NO:9.

Escherichia coli HB101 transformed with the plasmid pRHB11 is designated and indicated as Escherichia coli JM109/pRHB11, and deposited on September 5, 2000 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM BP-7655.

(5) Expression of Bacillus caldotenax RNase HII gene

Escherichia coli HB101 transformed with pRHB11 or pRHB1 was inoculated into 5 ml of LB medium containing 100 μg/ml of ampicillin and cultured with shaking at 37°C overnight. After cultivation, cells collected by centrifugation were suspended in 0.5 ml of TE buffer and sonicated. A supernatant obtained by centrifugation was used as a crude cell extract.

10 mM tris-HCl (pH 8.0), 1 mM dithiothreitol (Nacalai Tesque), 0.003% bovine serum albumin (fraction V, Sigma), 4% glycerol, 20 μg/ml poly(dT) (Amersham Pharmacia Biotech) and 30 μg/ml poly(rA) (Amersham Pharmacia Biotech) were mixed together. The mixture was incubated at 37°C for 10 minutes and used as a substrate solution for measuring an RNase H activity.

1 μl of 1 M $MnCl_2$ was added to 100 μl of the substrate solution. The mixture was incubated at 40°C. 10 μl of a 10-fold dilution of the crude cell extract was added to the mixture to initiate a reaction. After reacting at 40°C for 30 minutes, 10 μl of 0.5 M EDTA was added thereto to terminate the reaction. Absorbance at 260 nm was then measured. As a result, the absorbance at 260 nm from a reaction in which a crude cell extract prepared from Escherichia coli HB101 harboring pRHB1 was used was clearly higher than that from a reaction in which a crude cell extract prepared from Escherichia coli HB101 harboring pRHB1 was used. Thus, it was demonstrated that pRHB11 contained an RNase H gene and that Escherichia coli harboring pRHB11 expressed an RNase H activity.

(6) Preparation of purified RNase HII preparation

Escherichia coli HB101 transformed with pRHB11 obtained in Referential Example 2-(4) was inoculated into 1 L of LB medium containing 100 μg/ml of ampicillin and cultured with shaking at 37°C for 16 hours. After cultivation, cells collected by centrifugation were suspended in 52.3 ml of a sonication buffer [50 mM tris-HCl (pH 8.0), 2 mM 2-mercaptoethanol, 10% glycerol, 2 mM phenylmethanesulfonyl fluoride] and sonicated. A supernatant obtained by centrifuging the sonicated suspension at 12000 rpm for 10 minutes was heated at 60°C for 15 minutes. It was then centrifuged at 12000 rpm for 10 minutes again to collect a supernatant. Thus, 50.0 ml of a heated supernatant was obtained.

The solution was subjected to RESOURSE Q column (Amersham Pharmacia Biotech) equilibrated with Buffer C [50 mM tris-HCl (pH 8.0), 2 mM 2-mercaptoethanol, 10% glycerol] and chromatographed using FPLC system (Amersham Pharmacia Biotech). As a result, RNase HII flowed through the RESOURSE Q column. 51 ml of the flow-through RNase HII fraction was subjected to RESOURSE S column (Amersham Pharmacia Biotech) equilibrated with Buffer C and eluted with a linear gradient of 0 to 500 mM NaCl using FPLC system. A fraction containing RNase II eluted with about 240 mM NaCl was obtained. 3.0 ml of the RNase II fraction was subjected in two portions to PD-10 column (Amersham Pharmacia Biotech) equilibrated with Buffer C containing 50 mM NaCl. 7.0 ml the resulting eluate was subjected to HiTrap-heparin column (Amersham Pharmacia Biotech) equilibrated with Buffer C containing 50 mM NaCl and eluted with a linear gradient of 50 to 550 mM NaCl using FPLC system. A fraction containing RNase II eluted with about 310 mM NaCl was obtained. 4.4 ml of the RNase II fraction was concentrated by ultrafiltration using Centricon-10 (Amicon). 280 μl of the concentrate was subjected to Superdex 200 gel filtration column (Amersham Pharmacia Biotech) equilibrated with 50 mM tris-HCl (pH 8.0) containing 100 mM NaCl and 0.1 mM EDTA and eluted with the same buffer. As a result, RNase HII was eluted at a position corresponding to a molecular weight of 35 kilodalton. This molecular weight corresponds to that of the RNase HII in the monomeric form. The thus eluted RNase HII was used as Bca RNase HII preparation.

The enzymatic activity of the thus obtained Bca RNase HII preparation was measured as follows.

100 μl of a reaction mixture [20 mM HEPES-potassium hydroxide (pH 7.8), 0.01% bovine serum albumin (Takara Shuzo), 1% dimethyl sulfoxide, 10 mM manganese chloride, 20 μg/ml poly(dT) (Amersham Pharmacia Biotech), 30 μg/ml poly(rA) (Amersham Pharmacia Biotech)] which had been incubated at 40°C was added to 1 μl of the Bca RNase HII preparation. The mixture was reacted at 40°C for 10 minutes. The reaction was then terminated by adding 10 μl of 0.5 M EDTA (pH 8.0). Absorbance at 260 nm was then measured.

As a result, an RNase H activity was observed for the Bca RNase HII preparation.

Referential Example 3: Cloning of Bacillus caldotenax RNase HIII gene

(1) Cloning of fragment of RNase HIII gene

Primers BsuIII-1, BsuIII-3, BsuIII-6 and BsuIII-8 represented by SEQ ID NOS:10 to 13 for screening a gene encoding RNase HIII were synthesized based on the amino acid sequences of regions well conserved among Bacillus subtilis and other organisms determined on the basis of the homology among the amino acid sequences of RNase HIIIs from Bacillus subtilis [Biochemistry, 38:605-608 (1999)] and other organisms.

A first PCR was carried out in a volume of 50 μl using 200 ng of the Bacillus caldotenax genomic DNA as prepared in Referential Example 2-(1) as a template, and 100 pmol of BsuIII-1 and 100 pmol of BsuIII-8 as primers. A second PCR was then carried out in a volume of 100 μl using 1 μl of the reaction mixture as a template, and 100 pmol of BsuIII-3 and 100 pmol of BsuIII-6 as primers. TaKaRa Taq polymerase (Takara Shuzo) was used as a DNA polymerase for the two PCRs according to the attached protocol. The PCRs were carried out as follows: 25 (the first PCR) or 30 (the second PCR) cycles of 94°C for 30 seconds, 45°C for 30 seconds and 72°C for 1 minute.

An amplified DNA fragment of about 450 bp was blunt-ended using T4 DNA polymerase (Takara Shuzo) and then subjected to agarose gel electrophoresis to recover the amplified DNA fragment of about 450 bp. The about 450-bp DNA fragment was ligated with pUC119 (Takara Shuzo) digested with Sma I (Takara Shuzo) using T4 DNA ligase (Takara Shuzo). The ligation mixture was used to transform Escherichia coli JM109. The resulting transformants were cultured to obtain a plasmid pBCA3204 into which the about 450-bp DNA fragment was inserted.

(2) Cloning of RNase HIII gene using Southern hybridization method

The nucleotide sequence of the DNA fragment inserted in pBCA3204 obtained in Referential Example 3-(1) was determined. Primers RNIII-S3 and BcaRNIII-3 represented by SEQ ID NOS:14 and 15 were synthesized on the basis of the determined nucleotide sequence. A PCR was carried out in a volume of 100 μl using RNIII-S3 and BcaRNIII-3 as primers and pBCA3204 as a template. TaKaRa Z-Taq (Takara Shuzo) was used as a DNA polymerase for the PCR according to the attached protocol. The PCR was carried out as follows: 30 cycles of 98°C for 0 second, 55°C for 0 second and 72°C for 20 seconds. After reaction, the reaction mixture was subjected to phenol-chloroform extraction, ethanol precipitation and agarose gel electrophoresis to recover a DNA fragment of about 0.4 kb from the gel. The about 0.4-kb DNA fragment was labeled using DIG DNA Labeling Kit (Boehringer Mannheim) to prepare a probe.

20 μg of the Bacillus caldotenax genomic DNA prepared in Referential Example 2-(1) was completely digested with BamHI, EcoRI, HindIII, PstI or XbaI (all from Takara Shuzo). The half of each of the digests was then subjected to agarose gel electrophoresis. The DNAs were transferred from the agarose gel to a nylon membrane using 0.4 N sodium hydroxide and fixed at 120°C for 30 minutes. The membrane was pre-incubated in a sealed bag containing 30 ml of a hybridization buffer [43.4 g/L sodium chloride, 17.6 g/L sodium citrate, 1% blocking agent (Boehringer Mannheim), 0.1% N-lauroyl sarcosine, 0.02% sodium lauryl sulfate (SDS)] at 60°C for 4 hours and then incubated in a sealed bag containing 5 ml of a hybridization buffer containing the probe at 60°C for 16 hours.

The membrane was washed twice in 50 ml of 2 x SSC (17.5 g/L NaCl, 8.8 g/L sodium citrate) containing 0.1% SDS at room temperature, and twice in 50 ml of 0.5 x SSC (4.3 g/L sodium chloride, 1.9 g/L sodium citrate) containing 0.1% SDS at 45°C. Then, an EcoRI fragment of about 8 kb, a PstI fragment of about 4.5 kb and a HindIII fragment of about 1 kb which have sequences complementary to the probe were detected using DIG nucleic acid detection kit (Boehringer Mannheim).

The remaining half of the Bacillus caldotenax genomic DNA completely digested with PstI was subjected to agarose gel electrophoresis. PstI fragments of about 4.5 kb were recovered from the gel. The DNA fragments were then ligated with a plasmid vector pTV119N, which had been digested with PstI and dephosphorylated with alkaline phosphatase (Takara Shuzo). The ligation mixture was used to transform Escherichia coli JM109.

A PCR was carried out in a volume of 50 μl using RNIII-S3 and BcaRNIII-3 as primers, and one of the colonies as a template to select a colony presumably harboring an RNase HIII gene. TaKaRa-Z Taq (Takara Shuzo) was used for the PCR according to the attached protocol. The PCR was carried out as follows: 30 cycles of 98°C for 0 second, 55°C for 0 second and 72°C for 20 seconds. As a result, it was found that the gene of interest was contained in the colony No. 88.

A PCR was carried out using a plasmid prepared from the colony No. 88 as a template, and a primer pair RN-N (Takara Shuzo) and BcaRNIII-3 or a primer pair M4 (Takara Shuzo) and RNIII-S3 to examine whether or not the entire RNase HIII gene was contained in the plasmid. As a result, it was found that the entire RNase HIII gene was contained in the plasmid, which was designated as pBCA3P88.

(3) Determination of nucleotide sequence of DNA fragment containing RNase HIII gene

The nucleotide sequence of the DNA fragment inserted into the plasmid pBCA3P88 obtained in Referential Example 3-(2) was determined according to a dideoxy method.

Analysis of the determined nucleotide sequence revealed the existence of an open reading frame encoding an amino acid sequence including the N-terminal amino acid sequence of RNase HIII. The nucleotide sequence of the open reading frame and the amino acid sequence of RNase HIII deduced from the nucleotide sequence are shown in SEQ ID NO:16 and SEQ ID NO:17, respectively.

(4) Construction of plasmid for expressing RNase HIII

A PCR was carried out in a volume of 100 μl using the plasmid pBCA3P88 as described in Referential Example 3-(2) as a template, BcaRNIIINde represented by SEQ ID NO:18 designed with reference to the sequence around the above-mentioned open reading frame for RNase HIII and M13 primer M4 (Takara Shuzo). Pyrobest DNA polymerase (Takara Shuzo) was used as a DNA polymerase for the PCR according to the attached protocol. The PCR was carried out as follows: 30 cycles of 94°C for 30 seconds, 55°C for 30 seconds and 72°C for 3 minutes. An amplified DNA fragment of about 4 kb was digested with NdeI (Takara Shuzo) and subjected to agarose gel electrophoresis to recover an NdeI fragment of about 1.4 kb from the gel. The about 1.4-kb DNA fragment was ligated with pTV119Nd (a plasmid in which the NcoI site in pTV119N is converted into a NdeI site) which had been digested with NdeI and dephosphorylated with alkaline phosphatase (Takara Shuzo). The ligation mixture was used to transform Escherichia coli JM109.

Next, a PCR was carried out in a volume of 50 μl using one of the colonies as a template, and RN-N (Takara Shuzo) and BcaRNIII-3 as primers in order to screen for a plasmid in which the RNase HIII gene in the NdeI fragment was linked downstream from the lac promoter in the vector pTV119Nd. A colony presumably harboring the RNase HIII gene was then selected. TaKaRa-Z Taq (Takara Shuzo) was used as a DNA polymerase for the PCR according to the attached protocol. The PCR was carried out as follows: 30 cycles of 98°C for 0 second, 55°C for 0 second and 72°C for 20 seconds. As a result, it was found that the colony No. 2 contained a plasmid in which the RNase HIII gene in the NdeI fragment was linked downstream from the lac promoter in the vector pTV119Nd. This plasmid was designated as pBCA3Nd2.

The determination of the nucleotide sequence of the DNA fragment inserted into the plasmid by a dideoxy method revealed that there was no mutation due to the PCR except that the initiation codon GTG was changed to ATG.
Escherichia coli JM109 transformed with the plasmid pBCA3Nd2 is designated and indicated as Escherichia coli JM109/pBCA3Nd2, and deposited on September 5, 2000 (date of original deposit) at International Patent Organism Depositary, National Institute of Advanced Industrial Science and Technology, AIST Tsukuba Central 6, 1-1, Higashi 1-chome, Tsukuba-shi, Ibaraki-ken 305-8566, Japan under accession number FERM BP-7653.

(5) Preparation of purified RNase HIII preparation

Escherichia coli JM109 transformed with pBCA3Nd2 obtained in Referential Example 3-(4) was inoculated into 2 L of LB medium containing 100 μg/ml of ampicillin and cultured with shaking at 37°C for 16 hours. After cultivation, cells collected by centrifugation were suspended in 39.6 ml of a sonication buffer [50 mM tris-HCl (pH 8.0), 1 mM EDTA, 2 mM phenylmethanesulfonyl fluoride] and sonicated. A supernatant obtained by centrifuging the sonicated suspension at 12000 rpm for 10 minutes was heated at 60°C for 15 minutes. It was then centrifuged at 12000 rpm for 10 minutes again to collect a supernatant. Thus, 39.8 ml of a heated supernatant was obtained.
The heated supernatant was subjected to RESOURSE Q column (Amersham Pharmacia Biotech) equilibrated with Buffer A [50 mM tris-HCl (pH 8.0), 1 mM EDTA] and chromatographed using FPLC system (Amersham Pharmacia Biotech). As a result, RNase HIII flowed through the RESOURSE Q column.
45 ml of the flow-through RNase HIII fraction was dialyzed against 2 L of Buffer B [50 mM tris-HCl (pH 7.0), 1 mM EDTA] for 2 hours. The dialysis was repeated for two more times under the same conditions. 55.8 ml of the dialyzed enzyme solution was subjected to RESOURSE S column (Amersham Pharmacia Biotech) equilibrated with Buffer B and eluted with a linear gradient of 0 to 500 mM NaCl using FPLC system. A fraction containing RNase HIII eluted with about 105 mM NaCl was obtained.
Buffer B containing 1 M NaCl was added to 7.0 ml of the fraction to make the NaCl concentration to 150 mM. The mixture was subjected to HiTrap-heparin column (Amersham Pharmacia Biotech) equilibrated with Buffer B containing 150 mM NaCl. As a result, RNase HIII flowed through the HiTrap-heparin column.
7.5 ml of the flow-through RNase HIII fraction was concentrated by ultrafiltration using Centricon-10 (Amicon). 190 μl of the concentrate was subjected to Superdex 200 gel filtration column (Amersham Pharmacia Biotech) equilibrated with 50 mM tris-HCl (pH 7.0) containing 100 mM NaCl and 0.1 mM EDTA and eluted with the same buffer. As a result, RNase HIII was eluted at a position corresponding to a molecular weight of 33 kilodalton. This molecular weight corresponds to that of the RNase HIII in the monomeric form.
The thus eluted RNase HIII was used as Bca RNase HIII preparation.
The enzymatic activity of the thus obtained Bca RNase HIII preparation was measured as follows.
100 μl of a reaction mixture [20 mM HEPES-potassium hydroxide (pH 7.8), 0.01% bovine serum albumin (Takara Shuzo), 1% dimethyl sulfoxide, 4 mM magnesium acetate, 20 μg/ml poly(dT) (Amersham Pharmacia Biotech), 30 μg/ml poly(rA) (Amersham Pharmacia Biotech)] which had been incubated at 40°C was added to 1 μl of the Bca RNase HIII preparation. The mixture was reacted at 40°C for 10 minutes. The reaction was terminated by adding 10 μl of 0.5 M EDTA (pH 8.0). Absorbance at 260 nm was then measured. As a result, an RNase H activity was observed for the Bca RNase HIII preparation.

Referential Example 4: Cloning of Pyrococcus furiosus RNase HII gene

(1) Preparation of genomic DNA from Pyrococcus furiosus

2 L of a medium containing 1% Tryptone (Difco Laboratories), 0.5% yeast extract (Difco Laboratories), 1% soluble starch (Nacalai Tesque), 3.5% Jamarine S Solid (Jamarine Laboratory), 0.5% Jamarine S Liquid (Jamarine Laboratory), 0.003% $MgSO_4$, 0.001% NaCl, 0.0001% $FeSO_4 \cdot 7H_2O$, 0.0001% $CoSO_4$, 0.0001% $CaCl_2 \cdot 7H_2O$, 0.0001% $ZnSO_4$, 0.1 ppm $CuSO_4 \cdot 5H_2O$, 0.1 ppm $KAl(SO_4)_2$, 0.1 ppm $H_3BO_4$, 0.1 ppm $Na_2MoO_4 \cdot 2H_2O$ and 0.25 ppm $NiCl_2 \cdot 6H_2O$ was placed in a 2-L medium bottle, sterilized at 120°C for 20 minutes, bubbled with nitrogen gas to remove dissolved oxygen, then Pyrococcus furiosus (purchased from Deutsche Sammlung von Mikroorganismen; DSM3638) was inoculated into the medium and cultured at 95°C for 16 hours without shaking. After cultivation, cells were collected by centrifugation.
The resulting cells were then suspended in 4 ml of 25% sucrose, 50 mM tris-HCl (pH 8.0). 0.4 ml of 10 mg/ml lysozyme chloride (Nacalai Tesque) in water was added thereto. The mixture was reacted at 20°C for 1 hour. After reaction, 24 ml of a mixture containing 150 mM NaCl, 1 mM EDTA and 20 mM tris-HCl (pH 8.0), 0.2 ml of 20 mg/ml proteinase K (Takara Shuzo) and 2 ml of 10% aqueous solution of sodium lauryl sulfate were added to the reaction mixture. The mixture was incubated at 37°C for 1 hour. After reaction, the mixture was subjected to phenol-chloroform extraction followed by ethanol precipitation to prepare about 1 mg of genomic DNA.

(2) Cloning of RNase HII gene

The entire genomic sequence of Pyrococcus horikoshii was published [DNA Research, 5:55-76 (1998)]. The existence of one gene encoding a homologue of RNase HII (PH1650) in the genome was known (SEQ ID NO:19, the home page of National Institute of Technology and Evaluation: http://www.nite.go.jp/).
Homology between the PH1650 gene and the partially published genomic sequence of Pyrococcus furiosus (the home page of University of Utah, Utah Genome Center: http://www.genome.utah.edu/sequence.html) was searched. As a result, a highly homologous sequence was found. Primers 1650Nde (SEQ ID NO:20) and 1650Bam (SEQ ID NO:21) were synthesized on the basis of the homologous sequence.
A PCR was carried out in a volume of 100 μl using 200 ng of the Pyrococcus furiosus genomic DNA obtained in Referential Example 4-(1) as a template, and 20 pmol of 1650Nde and 20 pmol of 1650Bam as primers. TaKaRa Ex Taq (Takara Shuzo) was used as a DNA polymerase for the PCR according to the attached protocol. The PCR was carried out as follows: 30 cycles of 94°C for 30 seconds, 55°C for 30 seconds and 72°C for 1 minute. An amplified DNA fragment of about 0.7 kb was digested with NdeI and BamHI (both from Takara Shuzo). The resulting DNA fragment was inserted between the NdeI site and the BamHI site in a plasmid vector pET3a (Novagen) to make a plasmid pPFU220.

(3) Determination of nucleotide sequence of DNA fragment containing RNase HII gene

The nucleotide sequence of the DNA fragment inserted into pPFU220 obtained in Referential Example 4-(2) was determined according to a dideoxy method.
Analysis of the determined nucleotide sequence revealed the existence of an open reading frame presumably encoding RNase HII. The nucleotide sequence of the open reading frame is shown in SEQ ID NO:22. The amino acid sequence of RNase HII deduced from the nucleotide sequence is shown in SEQ ID NO:23.
Escherichia coli JM109 transformed with the plasmid pPFU220 is designated and indicated as Escherichia coli JM109/pPFU220, and deposited on September 5, 2000 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM BP-7654.

(4) Preparation of purified RNase HII preparation

Escherichia coli HMS174(DE3) (Novagen) was transformed with pPFU220 obtained in Referential Example 4-(2). The resulting Escherichia coli HMS174(DE3) harboring pPFU220 was inoculated into 2 L of LB medium containing 100 μg/ml of ampicillin and cultured with shaking at 37°C for 16 hours. After cultivation, cells collected by centrifugation were suspended in 66.0 ml of a sonication buffer [50 mM tris-HCl (pH 8.0), 1 mM EDTA, 2 mM phenylmethanesulfonyl fluoride] and sonicated. A supernatant obtained by centrifuging the sonicated suspension at 12000 rpm for 10 minutes was heated at 60°C for 15 minutes. It was then centrifuged at 12000 rpm for 10 minutes again to collect a supernatant. Thus, 61.5 ml of a heated supernatant was obtained.
The heated supernatant was subjected to RESOURSE Q column (Amersham Pharmacia Biotech) equilibrated with Buffer A [50 mM tris-HCl (pH 8.0), 1 mM EDTA] and chromatographed using FPLC system (Amersham Pharmacia Biotech). As a result, RNase HIT flowed through the RESOURSE Q column.
60.0 ml of the flow-through RNase HII fraction was subjected to RESOURSE S column (Amersham Pharmacia Biotech) equilibrated with Buffer A and eluted with a linear gradient of 0 to 500 mM NaCl using FPLC system. A fraction containing RNase HII eluted with about 150 mM NaCl was obtained. 2.0 ml of the RNase HII fraction was concentrated by ultrafiltration using Centricon-10 (Amicon). 250 μl of the concentrate was subjected to Superdex 200 gel filtration column (Amersham Pharmacia Biotech) equilibrated with 50 mM tris-HCl (pH 8.0) containing 100 mM NaCl and 0.1 mM EDTA and eluted with the same buffer. As a result, RNase HII was eluted at a position corresponding to a molecular weight of 17 kilodalton. This molecular weight corresponds to that of the RNase HII in the monomeric form.
The thus eluted RNase HII was used as Pfu RNase HII preparation.
The enzymatic activity of the thus obtained Pfu RNase HII preparation was measured as described in Referential Example 3-(5). As a result, an RNase H activity was observed for the Pfu RNase HII preparation.

Referential Example 5: Cloning of Thermotoga maritima RNase HII gene

(1) Preparation of genomic DNA from Thermotoga maritima

2 L of a medium containing 1% Tryptone, 0.5% yeast extract, 1% soluble starch, 3.5% Jamarine S Solid, 0.5% Jamarine S Liquid, 0.003% $MgSO_4$, 0.001% NaCl, 0.0001% $FeSO_4 \cdot 7H_2O$, 0.0001% $CoSO_4$, 0.0001% $CaCl_2 \cdot 7H_2O$,

0.0001% $ZnSO_4$, 0.1 ppm $CuSO_4 \cdot 5H_2O$, 0.1 ppm $KAl(SO_4)_2$, 0.1 ppm $H_3BO_3$, 0.1 ppm $Na_2MoO_4 \cdot 2H_2O$ and 0.25 ppm $NiCl_2 \cdot 6H_2O$ was placed in a 2-L medium bottle, sterilized at 120°C for 20 minutes, bubbled with nitrogen gas to remove dissolved oxygen, then Thermotoga maritima (purchased from Deutsche Sammlung von Mikroorganismen und Zellkulturen GmbH; DSM3109) was inoculated into the medium and cultured at 85°C for 16 hours without shaking.

Cells collected by centrifugation from 300 ml of the culture were then suspended in 3 ml of TE buffer [10 mM. tris-HCl (pH 7.5), 1 mM EDTA]. 150 μl of 10% aqueous solution of sodium lauryl sulfate (Nacalai Tesque) and 15 μl of 20 mg/ml proteinase K (Takara Shuzo) were added thereto. The mixture was incubated at 37°C for 1 hour. After reaction, 0.5 ml of 5 M NaCl was added to the mixture. After thoroughly mixing, 0.4 ml of a CTAB-NaCl solution [10% cetyltrimethylammonium bromide (Nacalai Tesque), 0.7 M NaCl] was added to the mixture. After thoroughly mixing, the mixture was incubated at 65°C for 10 minutes. 1.5 ml of a mixture of chloroform/isoamyl alcohol (24:1, v/v) was added thereto. The mixture was gently mixed for 10 minutes and centrifuged at 20000 x g for 5 minutes. After centrifugation, an equal volume of a mixture of phenol saturated with 100 mM tris-HCl (pH 8.0)/chloroform/isoamyl alcohol (25:24:1, v/v) was added to the resulting supernatant. The mixture was gently mixed for 10 minutes and then centrifuged at 20000 x g for 5 minutes. After centrifugation, a 0.6 volume of 2-propanol was added to the supernatant. The precipitate obtained by centrifugation at 10000 x g for 5 minutes was washed with 70% ethanol in water, air-dried and then dissolved in 200 μl of TE to obtain a genomic DNA solution.

(2) Cloning of RNase HII gene

Oligonucleotides 915-F1, 915-F2, 915-R1 and 915-R2 represented by SEQ ID NOS:24 to 27 were synthesized on the basis of the nucleotide sequence of a portion that had been identified as an RNase HII gene in the nucleotide sequence of the genomic DNA of Thermotoga maritima (http://www.tigr.org/tdb/CMR/btm/htmls/SplashPage.html) in order to obtain an amplified DNA fragment containing an RNase H gene by carrying out a PCR using the Thermotoga maritima genomic DNA as a template.

PCRs were carried out using the Thermotoga maritima genomic DNA as prepared in Referential Example 5-(1) as a template, and 915-F1 and 915-R1, 915-F1 and 915-R2, 915-F2 and 915-R1, or 915-F2 and 915-R2 as a primer pair. TaKaRa Ex Taq was used as a DNA polymerase for the PCRs according to the attached protocol. The PCRs were carried out as follows: 25 cycles of 95°C for 0.5 minute,. 55°C for 0.5 minute and 72°C for 1.5 minute. After reactions, the respective PCR products were subjected to agarose gel electrophoresis to extract and purify amplified DNA fragments of about 0.7 kb. The DNAs amplified using a primer pair 915-F1 and 915-R1 or 915-F1 and 915-R2 were digested with HindIII and XbaI (both from Takara Shuzo) and ligated with pUC19 digested with HindIII and XbaI using T4 DNA ligase. The ligation mixture was used to transform Escherichia coli JM109. The resulting transformants were cultured to prepare plasmid DNAs into which the about 0.7-kb DNAs were inserted. As a result, plasmids No. 1 and No. 2 having DNAs amplified using 915-F1 and 915-R1, and plasmids No. 3 and No. 4 having DNAs amplified using 915-F1 and 915-R2 were obtained.

In addition, the DNAs amplified using a primer pair 915-F2 and 915-R1 or 915-F2 and 915-R2 were doubly digested with NcoI (Takara Shuzo) and XbaI and ligated with pTV119N (Takara Shuzo) doubly digested with NcoI and XbaI using T4 DNA ligase. The ligation mixture was used to transform Escherichia coli JM109.

The resulting transformants were cultured to prepare plasmid DNAs into which the about 0.7-kb DNAs were inserted. As a result, plasmids No. 5 and No. 6 having DNAs amplified using 915-F2 and 915-R1, and a plasmid No. 7 having a DNA amplified using 915-F2 and 915-R2 were obtained.

Escherichia coli JM109 transformed with the plasmid No. 7 is designated and indicated as Escherichia coli JM109/pTM-RNH, and deposited on September 5, 2000 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM BP-7652.

(3) Expression of Thermotoga maritima RNase HII gene

Escherichia coli JM109 transformed with one of the plasmids No. 1 to 7 or pUC19 was inoculated into 5 ml of LB medium (10 g/L Tryptone, 5 g/L yeast extract, 5 g/L NaCl, pH 7.2) containing 100 μg/ml of ampicillin and cultured with shaking at 37°C. When the absorbance at 660 nm reached 0.5, isopropyl-β-D-thiogalactopyranoside was added thereto to a final concentration of 1 mM and the cells were cultured overnight. After cultivation, cells collected by centrifugation were suspended in 1 ml of TE buffer and sonicated. The sonicated suspension was heated at 80°C for 10 minute. A supernatant obtained by centrifugation was used as a crude cell extract. Absorbance was measured using the crude cell extract as -described in Referential Example 2-(5). As a result, when reactions were carried out in the presence of $MnCl_2$, the absorbance at 260 nm from each of the reactions in which crude cell extracts prepared from Escherichia coli JM109 harboring the plasmid No. 3, 5, 6 or 7 were used was clearly higher than that from a reaction in which a crude extract prepared from Escherichia coli JM109 harboring pUC19 was used. Thus, it was demonstrated that the plasmids No. 3, 5, 6 and 7 contained RNase H genes and that Escherichia coli harboring one of these plasmids expressed an RNase H activity.

The partial nucleotide sequences of the DNA fragments inserted into the plasmids which were demonstrated to express RNase activities in Escherichia coli were determined. Analysis of the determined nucleotide sequences revealed an open reading frame presumably encoding RNase HII. The nucleotide sequence of the open reading frame is shown in SEQ ID NO:143. The amino acid sequence of RNase HII deduced from the nucleotide sequence is shown in SEQ ID NO:144. Then, it was found that one base substitution that was presumably generated upon the PCR was observed in a portion of the nucleotide sequence of the DNA fragment inserted in the plasmid No. 7, resulting in the change in the encoded amino acid residue.

(4) Preparation of purified RNase HII preparation

Escherichia coli JM109 was transformed with pTM-RNH obtained in Referential Example 5-(2). The resulting Escherichia coli JM109 harboring pTM-RNH was inoculated into 1 L of LB medium containing 100 μg/ml of ampicillin and cultured with shaking at 37°C for 16 hours. After cultivation, cells collected by centrifugation were suspended in 31.0 ml of a sonication buffer [50 mM tris-HCl (pH 8.0), 2 mM 2-mercaptoethanol, 10% glycerol, 2 mM phenylmethanesulfonyl fluoride] and sonicated. A supernatant obtained by centrifuging the sonicated suspension at 12000 rpm for 10 minutes was heated at 70°C for 15 minutes. It was then centrifuged at 12000 rpm for 10 minutes again to collect a supernatant. Thus, 32.0 ml of a heated supernatant was obtained.

The heated supernatant was subjected to RESOURSE Q column (Amersham Pharmacia Biotech) equilibrated with Buffer C [50 mM tris-HCl (pH 8.0), 2 mM 2-mercaptoethanol, 10% glycerol] and chromatographed using FPLC system (Amersham Pharmacia Biotech). As a result, RNase HII flowed through the RESOURSE Q column. 32.5 ml of the flow-through RNase HII fraction was subjected to RESOURSE S column (Amersham Pharmacia Biotech) equilibrated with Buffer C and eluted with a linear gradient of 0 to 500 mM NaCl using FPLC system. A fraction containing RNase II eluted with about 240 mM NaCl was obtained. 2.0 ml of the RNase II fraction was subjected to PD-10 column (Amersham Pharmacia Biotech) equilibrated with Buffer C containing 50 mM NaCl. 3.5 ml of the resulting eluate was subjected to HiTrap-heparin column (Amersham Pharmacia Biotech) equilibrated with Buffer C containing 50 mM NaCl and eluted with a linear gradient of 50 to 550 mM NaCl using FPLC system. As a result, a fraction containing RNase II eluted with about 295 mM NaCl was obtained. The thus eluted RNase HII was used as Tma RNase HII preparation.

The enzymatic activity of the thus obtained Tma RNase HII preparation was measured as described in Referential Example 2-(6). As a result, an RNase H activity was observed for the Tma RNase HII preparation.

Referential Example 6: Cloning of RNase HII gene from Pyrococcus horikoshii

(1) Preparation of genomic DNA from Pyrococcus horikoshii

2 L of a medium containing 1% Tryptone (Difco Laboratories), 0.5% yeast extract (Difco Laboratories), 1% soluble starch (Nacalai Tesque), 3.5% Jamarine S Solid (Jamarine Laboratory), 0.5% Jamarine S Liquid (Jamarine Laboratory), 0.003% $MgSO_4$, 0.001% NaCl, 0.0001% $FeSO_4 \cdot 7H_2O$, 0.0001% $CoSO_4$, 0.0001% $CaCl_2 \cdot 7H_2O$, 0.0001% $ZnSO_4$, 0.1 ppm $CuSO_4 \cdot 5H_2O$, 0.1 ppm $KAl(SO_4)_2$, 0.1 ppm $H_3BO_4$, 0.1 ppm $Na_2MoO_4 \cdot 2H_2O$ and 0.25 ppm $NiCl_2 \cdot 6H_2O$ was placed in a 2-L medium bottle, sterilized at 120°C for 20 minutes, bubbled with nitrogen gas to remove dissolved oxygen, then Pyrococcus horikoshii OT3 (purchased from the Institute of Physical and Chemical Research (RIKEN); JCM9974) was inoculated into the medium and cultured at 95°C for 16 hours without shaking. After cultivation, cells were collected by centrifugation.

The cells were then suspended in 4 ml of 25% sucrose, 50 mM tris-HCl (pH 8.0). 0.4 ml of 10 mg/ml lysozyme chloride (Nacalai Tesque) in water was added thereto. The mixture was reacted at 20°C for 1 hour. After reaction, 24 ml of a mixture containing 150 mM NaCl, 1 mM EDTA and 20 mM tris-HCl (pH 8.0), 0.2 ml of 20 mg/ml proteinase K (Takara Shuzo) and 2 ml of 10% aqueous solution of sodium lauryl sulfate were added to the reaction mixture. The mixture was incubated at 37°C for 1 hour.

After reaction, the mixture was subjected to phenol-chloroform extraction followed by ethanol precipitation to prepare about 1 mg of genomic DNA.

(2) Cloning of RNase HII gene

The entire genomic sequence of the Pyrococcus horikoshii has been published [DNA Research, 5:55-76 (1998)]. The existence of one gene encoding a homologue of RNase HII (PH1650) was known (SEQ ID NO:145, the home page of National Institute of Technology and Evaluation: http://www.nite.go.jp/).

Primers PhoNde (SEQ ID NO:146) and PhoBam (SEQ ID NO:147) were synthesized on the basis of the sequence of the PH1650 gene (SEQ ID NO:145).

A PCR was carried out using 100 ng of the Pyrococcus horikoshii genomic DNA prepared in Referential Example 6-(1) as a template, and 20 pmol each of PhoNde and PhoBam as primers in a volume of 100 µl. TaKaRa Ex Taq (Takara Shuzo) was used as a DNA polymerase for the PCR according to the attached protocol. The PCR was carried out as follows: 40 cycles of 94°C for 30 seconds, 55°C for 30 seconds and 72°C for 1 minute. An amplified DNA fragment of about 0.7 kb was digested with NdeI and BamHI (both from Takara Shuzo). Then, a plasmid pPHO238 was constructed by incorporating the resulting DNA fragment between NdeI and BamHI sites in a plasmid vector pET3a (Novagen).

(3) Determination of nucleotide sequence of DNA fragment containing RNase HII gene

The nucleotide sequence of the DNA fragment inserted into pPHO238 obtained in Referential Example 6-(2) was determined according to a dideoxy method.

Analysis of the determined nucleotide sequence revealed an open reading frame presumably encoding RNase HII. The nucleotide sequence of the open reading frame is shown in SEQ ID NO:148. The amino acid sequence of RNase HII deduced from the nucleotide sequence is shown in SEQ ID NO:149.

Escherichia coli JM109 transformed with the plasmid pPHO238 is designated and indicated as Escherichia coli JM109/pPHO238, and deposited on February 22, 2001 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM BP-7692.

(4) Preparation of purified RNase HII preparation

Escherichia coli HMS174(DE3) (Novagen) was transformed with pPHO238 obtained in Referential Example 6-(2). The resulting Escherichia coli HMS174(DE3) harboring pPHO238 was inoculated into 1 L of LB medium containing 100 µg/ml of ampicillin and cultured with shaking at 37°C for 16 hours. After cultivation, cells collected by centrifugation were suspended in 34.3 ml of a sonication buffer [50 mM tris-HCl (pH 8.0), 1 mM EDTA, 2 mM phenylmethanesulfonyl fluoride] and sonicated. A supernatant obtained by centrifuging the sonicated suspension at 12000 rpm for 10 minutes was heated at 80°C for 15 minutes. It was then centrifuged at 12000 rpm for 10 minutes again to collect a supernatant. Thus, 33.5 ml of a heated supernatant was obtained.

The heated supernatant was subjected to RESOURSE Q column (Amersham Pharmacia Biotech) equilibrated with Buffer A [50 mM tris-HCl (pH 8.0), 1 mM EDTA] and chromatographed using FPLC system (Amersham Pharmacia Biotech). As a result, RNase HII flowed through the RESOURSE Q column.

35.0 ml of the flow-through RNase HII fraction was dialyzed against 2 L of Buffer B (50 mM tris-HCl (pH 7.0), 1 mM EDTA) for 2 hours. The dialysis was repeated two more times. 34.5 ml of the dialyzed enzyme solution was subjected to RESOURSE S column (Amersham Pharmacia Biotech) equilibrated with Buffer B and eluted with a linear gradient of 0 to 500 mM NaCl using FPLC system. A fraction containing RNase HII eluted with about 155 mM NaCl was obtained.

Buffer B was added to 4.0 ml of the fraction to make the NaCl concentration to 50 mM. The mixture was subjected to HiTrap-heparin column (Amersham Pharmacia Biotech) equilibrated with Buffer B containing 50 mM NaCl and eluted with a linear gradient of 50 to 550 mM NaCl using FPLC system. As a result, a fraction containing RNase HII eluted with about 160 mM NaCl was obtained.

6.9 ml of the RNase HIT fraction was concentrated by ultrafiltration using Centricon-10 (Amicon). Two portions each separated from 250 µl of the concentrate were subjected to Superose 6 gel filtration column (Amersham Pharmacia Biotech) equilibrated with 50 mM tris-HCl (pH 7.0) containing 100 mM NaCl and 0.1 mM EDTA and eluted with the same buffer. As a result, RNase HII was eluted at a position corresponding to a molecular weight of 24.5 kilodalton. This molecular weight corresponds to that of the RNase HII in the monomeric form.

The RNase HII eluted as described above was used as Pho RNase HII preparation.

The enzymatic activity of the thus obtained Pho RNase HII preparation was measured as described in Referential Example 3-(5). As a result, an RNase H activity was observed for the Pho RNase HII preparation.

Referential Example 7: Cloning of RNase HII gene from Archaeoglobus fulgidus

(1) Preparation of genomic DNA from Archaeoglobus fulgidus

Cells of Archaeoglobus fulgidus (purchased from Deutsche Sammlung von Mikroorganismen und Zellkulturen GmbH; DSM4139) collected from 8 ml of a culture was suspended in 100 µl of 25% sucrose, 50 mM tris-HCl (pH 8.0). 20 µl of 0.5 M EDTA and 10 µl of 10 mg/ml lysozyme chloride (Nacalai Tesque) in water was added thereto. The mixture was reacted at 20°C for 1 hour. After reaction, 800 μl of a mixture containing 150 mM NaCl, 1 mM EDTA and 20 mM tris-HCl (pH 8.0), 10 μl of 20 mg/ml proteinase K (Takara Shuzo) and 50 μl of 10% aqueous solution of sodium lauryl sulfate were added to the reaction mixture. The mixture was incubated at 37°C for 1 hour. After reaction, the mixture was subjected to phenol-chloroform extraction, ethanol precipitation and air-drying, and then dissolved in 50 μl of TE to obtain a genomic DNA solution.

(2) Cloning of RNase HII gene

The entire genomic sequence of the Archaeoglobus fulgidus has been published [Nature, 390:364-370 (1997)]. The existence of one gene encoding a homologue of RNase HII (AF0621) was known (SEQ ID NO:150, http://www.tigr.org/tdb/CMR/btm/htmls/SplashPage.htlm).

Primers AfuNde (SEQ ID NO:151) and AfuBam (SEQ ID NO:152) were synthesized on the basis of the sequence of the AF0621 gene (SEQ ID NO:150).

A PCR was carried out using 30 ng of the Archaeoglobus fulgidus genomic DNA prepared in Referential Example 7-(1) as a template, and 20 pmol each of AfuNde and AfuBam as primers in a volume of 100 μl. Pyrobest DNA polymerase (Takara Shuzo) was used as a DNA polymerase for the PCR according to the attached protocol. The PCR was carried out as follows: 40 cycles of 94°C for 30 seconds, 55°C for 30 seconds and 72°C for 1 minute. An amplified DNA fragment of about 0.6 kb was digested with NdeI and BamHI (both from Takara Shuzo). Then, a plasmid pAFU204 was constructed by incorporating the resulting DNA fragment between NdeI and BamHI sites in a plasmid vector pTV119Nd (a plasmid in which the NcoI site in pTV119N is converted into a NdeI site).

(3) Determination of nucleotide sequence of DNA fragment containing RNase HII gene

The nucleotide sequence of the DNA fragment inserted into pAFU204 obtained in Referential Example 7-(2) was determined according to a dideoxy method.

Analysis of the determined nucleotide sequence revealed an open reading frame presumably encoding RNase HII. The nucleotide sequence of the open reading frame is shown in SEQ ID NO:153. The amino acid sequence of RNase HII deduced from the nucleotide sequence is shown in SEQ ID NO:154.

Escherichia coli JM109 transformed with the plasmid pAFU204 is designated and indicated as Escherichia coli JM109/pAFU204, and deposited on February 22, 2001 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM BP-7691.

(4) Preparation of purified RNase HII preparation

Escherichia coli JM109 was transformed with pAFU204 obtained in Referential Example 7-(2). The resulting Escherichia coli JM109 harboring pAFU204 was inoculated into 2 L of LB medium containing 100 μg/ml of ampicillin and cultured with shaking at 37°C for 16 hours. After cultivation, cells collected by centrifugation were suspended in 37.1 ml of a sonication buffer [50 mM tris-HCl (pH 8.0), 1 mM EDTA, 2 mM phenylmethanesulfonyl fluoride] and sonicated. A supernatant obtained by centrifuging the sonicated suspension at 12000 rpm for 10 minutes was heated at 70°C for 15 minutes. It was then centrifuged at 12000 rpm for 10 minutes again to collect a supernatant. Thus, 40.3 ml of a heated supernatant was obtained.

The heated supernatant was subjected to RESOURSE Q column (Amersham Pharmacia Biotech) equilibrated with Buffer A [50 mM tris-HCl (pH 8.0), 1 mM EDTA] and chromatographed using FPLC system (Amersham Pharmacia Biotech). As a result, RNase HII flowed through the RESOURSE Q column.

The flow-through RNase HII fraction was subjected to RESOURSE S column (Amersham Pharmacia Biotech) equilibrated with Buffer A and chromatographed using FPLC system (Amersham Pharmacia Biotech). As a result, RNase HII flowed through the RESOURSE S column.

40.0 ml of the flow-through RNase HII fraction was dialyzed against 2 L of Buffer B (50 mM tris-HCl (pH 7.0), 1 mM EDTA) containing 50 mM NaCl for 2 hours. The dialysis was repeated two more times. 40.2 ml of the dialyzed enzyme solution was subjected to HiTrap-heparin column (Amersham Pharmacia Biotech) equilibrated with Buffer B containing 50 mM NaCl and eluted with a linear gradient of 50 to 550 mM NaCl using FPLC system. As a result, a fraction containing RNase HII eluted with about 240 mM NaCl was obtained.

7.8 ml of the RNase HII fraction was concentrated by ultrafiltration using Centricon-10 (Amicon). Four portions each separated from about 600 μl of the concentrate were subjected to Superose 6 gel filtration column (Amersham Pharmacia Biotech) equilibrated with 50 mM tris-HCl (pH 7.0) containing 100 mM NaCl and 0.1 mM EDTA and eluted with the same buffer. As a result, RNase. HII was eluted at a position corresponding to a molecular weight of 30.0 kilodalton. This molecular weight corresponds to that of the RNase HII in the monomeric form.

The RNase HIT eluted as described above was used as Afu RNase HIT preparation.
The enzymatic activity of the thus obtained Afu RNase HII preparation was measured as described in Referential Example 3-(5). As a result, an RNase H activity was observed for the Afu RNase HII preparation.

Referential Example 8

Unit value of RNase H from Escherichia coli used in the method of the present invention was measured according to the following method.

(1) Preparation of reagent solutions used

Reaction mixture for determining activity: The following substances at the indicated final concentrations were contained in sterile water: 40 mM tris-hydrochloride (pH 7.7 at 37°C), 4 mM magnesium chloride, 1 mM DTT, 0.003% BSA, 4% glycerol and 24 μM poly(dT).
Poly[8-$^3$H]adenylic acid solution: 370 kBq of a poly[8-$^3$H]adenylic acid solution was dissolved in 200 μl of sterile water.
Polyadenylic acid solution: Polyadenylic acid was diluted to a concentration of 3 mM with sterile ultrapure water.
Enzyme dilution solution: The following substances at the indicated final concentrations were contained in sterile water: 25 mM tris-hydrochloride (pH 7.5 at 37°C), 5 mM 2-mercaptoethanol, 0.5 mM EDTA (pH 7.5 at 37°C), 30 mM sodium chloride and 50% glycerol.
Preparation of heat-denatured calf thymus DNA: 200 mg of calf thymus DNA was suspended and allowed to swell in 100 ml of TE buffer. The solution was diluted to a concentration of 1 mg/ml with sterile ultrapure water based on the absorbance measured at UV 260 nm. The diluted solution was heated at 100°C for 10 minutes and then rapidly cooled in an ice bath.

(2) Method for measuring activity

7 μl of the poly[8-$^3$H]adenylic acid solution was added to 985 μl of the reaction mixture for determining activity prepared in (1) above. The mixture was incubated at 37°C for 10 minutes. 8 μl of polyadenylic acid was added to the mixture to make the final concentration to 24 μM. The mixture was further incubated at 37°C for 5 minutes. Thus, 1000 μl of a poly[8-$^3$H]rA-poly-dT reaction mixture was prepared. 200 μl of the reaction mixture was then incubated at 30°C for 5 minutes. 1 μl of an appropriate serial dilution of an enzyme solution was added thereto. 50 μl each of samples was taken from the reaction mixture over time for use in subsequent measurement. The period of time in minutes from the addition of the enzyme to the sampling is defined as Y. 50 μl of a reaction mixture for total CPM or for blank was prepared by adding 1 μl of the enzyme dilution solution instead of an enzyme solution. 100 μl of 100 mM sodium pyrophosphate, 50 μl of the heat-denatured calf thymus DNA solution and 300 μl of 10% trichloroacetic acid (300 μl of ultrapure water for measuring total CPM) were added to the sample. The mixture was incubated at 0°C for 5 minutes, and then centrifuged at 10000 rpm for 10 minutes. After centrifugation, 250 μl of the resulting supernatant was placed in a vial. 10 ml of Aquasol-2 (NEN Life Science Products) was added thereto. CPM was measured in a liquid scintillation counter.

(3) Calculation of units

Unit value for each enzyme was calculated according to the following equation.

$$\text{Unit/ml} = \{(\text{measured CPM} - \text{blank CPM}) \times 1.2^{*} \times 20 \times 1000 \times \text{dilution rate}\}\ 200\ (\mu l) / (\text{total CPM} \times Y\ (\text{min.}) \times 50\ (\mu l) \times 9^{**})$$

1.2*: Amount in nmol of poly[8-$^3$H]rA-poly-dT contained in total CPM per 50 μl.
9**: Correction coefficient.

Example 1

The method of the present invention was used to detect enterohemorrhagic E. coli 0-157.

Sequences of the primers used in this Example are shown in SEQ ID NOS:31 to 34 of the Sequence Listing. A combination of primers each having a sequence of SEQ ID NO:31 or 32 were constructed for detecting a sequence encoding vero toxin (VT) 1 of 0-157, and a combination of primers each having a sequence of SEQ ID NO:33 or 34 were constructed for detecting a sequence encoding vero toxin 2 of 0-157. A hot water-extract prepared by culturing enterohemorrhagic E. coli 0-157 (ATCC accession no. 43895), collecting cells, suspending the cells in sterile water at an appropriate cell density and treating it at 98°C for 10 minutes was used as a template. The composition of the reaction mixture was as follows.

27 mM phosphate buffer (pH 7.3), 0.01% bovine serum albumin (BSA), 5% dimethyl sulfoxide (DMSO), 1 mM each of dNTPs, 8 mM magnesium acetate, 60 pmol each of the primers, the DNA as the template (the hot water-extract) corresponding to $10^4$-$10^6$ cells and sterile distilled water to a reaction volume of 48 µl. The reaction mixture was heat-denatured at 98°C for 1 minute, and then cooled to 55°C. 5.5 U of BcaBEST DNA polymerase and 60 U of E. coli RNase H were added thereto. The reaction mixture was incubated at 55°C for 60 minutes. Thereafter, the mixture was heated at 90°C for 2 minutes to inactivate the enzymes. 3 µl each of the reaction mixtures was subjected to electrophoresis on 4% NuSieve 3:1 agarose (Takara Shuzo) gel. As a result, 0-157 vero toxin 1 and 2 could be detected using either one of the primer pairs and the DNA as the template corresponding to $10^4$ cells, confirming that the method of the present invention can be utilized as a method for detecting a virulent bacterium.

Example 2

(1) The effect of the type of the buffer used in the method of the present invention was examined. Primers for amplifying λ DNA having sequences represented by SEQ ID NOS:39 and 40 were used in this Example. The reaction was carried out as follows. Briefly, 10 µl of a mixture containing 120 pmol each of the primers, 0.01% aqueous solution of propylenediamine and 10 ng or 1 ng of a DNA as a template was heat-denatured at 98°C for 2 minutes, and then cooled on ice to anneal the primers to the template DNA. An amplified product (1005 bp) obtained by a PCR using λ DNA (Takara Shuzo) as a template and primers represented by SEQ ID NOS:41 and 42, which was then purified using Suprec02 (Takara Shuzo), was used as the template.

After annealing, 40 µl of one of three types of buffers for reaction (42.5 mM Tricine-potassium hydroxide buffer (pH 8.5), 42.5 mM Bicine-potassium hydroxide buffer (pH 8.3) and 42.5 mM HEPES-potassium hydroxide buffer (pH 7.8)) each containing 0.625 mM each of dNTPs, 5.0 mM magnesium acetate, 0.0125% bovine serum albumin (BSA), 1.25% dimethyl sulfoxide (DMSO), 30 U of E. coli RNase H and 11 U of BcaBEST DNA polymerase was added to the mixture to make the final volume to 50 µl. The reaction mixtures were incubated at 60°C for 1 hour. After reaction, 3 µl each of the reaction mixtures was subjected to electrophoresis on 3.0% agarose gel for confirmation. Then, the amplified fragments of interest were observed for both of the amounts of the template. In particular, a greater amount of amplification product was obtained for the reaction system containing Bicine-potassium hydroxide buffer (pH 8.5).

(2) Improvement of reactivity by the use of HEPES-potassium hydroxide buffer was examined. pUC19 plasmid DNA with a DNA fragment of about 150 bp being inserted into the multi-cloning site was used as a template. This template was prepared as follows.

A PCR was carried out using pUC19 upper 150 PCR primer and pUC19 lower PCR primer having sequences represented by SEQ ID NOS:134 and 135, and 100 pg of pUC19 plasmid DNA as a template. The resulting amplified fragment was purified using Microcon-100, blunt-ended using DNA blunting kit (Takara Shuzo) and subcloned into a HincII site of the plasmid pUC19. The plasmid with the. amplified fragment being inserted was used to transform Escherichia coli JM109. The transformant was cultured. The plasmid having the inserted DNA was purified from the cells using QIAGEN plasmid mini kit (Qiagen) and used as a template.

A DNA fragment PCR-amplified using the pUC19-150 plasmid DNA prepared as described above as a template, and primers MCS-F and MCS-R having nucleotide sequences represented by SEQ ID NOS:35 and 36 was used as a template. Primers MF2N3(24) and MR1N3(24) having nucleotide sequences represented by SEQ ID NOS:37 and 38 were used as chimeric oligonucleotide primers. The size of the amplified fragment obtained using the combination of these primers was about 350 bp.

An HEPES-potassium hydroxide buffer system was selected as a buffer to be examined. A potassium phosphate buffer system and a Tricine buffer system were used as controls. The compositions of the reaction mixtures are shown below.

Reaction mixture A: 10 ng of the PCR-amplified fragment, 50 pmol each of the primers MF2N3(24) and MR1N3 (24), 0.01% aqueous solution of propylenediamine and sterile distilled water to a reaction volume of 10 µl.

Reaction mixtures B: The following three types were prepared.

Potassium phosphate buffer system: 40 µl of a reaction mixture containing, at final concentrations, 35 mM potassium phosphate buffer (pH 7.5), 1.25% DMSO, 0.0125% BSA, 5 mM magnesium acetate, 0.625 mM each of dNTPs, 60 U of E. coli RNase H and 5.5 U of BcaBEST DNA polymerase was prepared.

Tricine buffer system: 40 μl of a reaction mixture containing, at final concentrations, 42.5 mM Tricine buffer (pH 8.7), 12.5 mM potassium chloride, 12.5 mM ammonium sulfate, 1.25% DMSO, 0.0125% BSA, 5 mM magnesium acetate, 0.625 mM each of dNTPs, 30 U of E. coli RNase H and 5.5 U of BcaBEST DNA polymerase was prepared.

HEPES-potassium hydroxide buffer system: 40 μl of a reaction mixture containing, at final concentrations, 25 mM HEPES-potassium hydroxide buffer (pH 7.8), 125 mM potassium acetate, 1.25% DMSO, 0.0125% BSA, 5 mM magnesium acetate, 0.625 mM each of dNTPs, 30 U of E. coli RNase H and 5.5 U of BcaBEST DNA polymerase was prepared.

The reaction mixture A was heat-denatured at 98°C for 2 minutes, cooled to 60°C or 65°C and then allowed to stand on ice. One of the reaction mixtures B was added to the reaction mixture A on ice and mixed to make the reaction volume to 50 μl. The reaction mixtures were incubated at 60°C or 65°C for 1 hour. After reaction, they were cooled to 4°C, and a 1/10 volume of 0.5 M EDTA was padded to each of the mixtures to terminate the reaction. 3 μl each of the reaction mixtures was subjected to electrophoresis on 3% NuSieve 3:1 agarose gel. As a result, the amplified fragments of interest were observed for the three buffer systems regardless of the reaction temperature used. In particular, the largest amount of the amplification product and the highest reactivity were observed for the HEPES-potassium hydroxide buffer system in this Example.

Example 3

(1) Conditions used in the method of the present invention for annealing primers to a template were examined. Primers having nucleotide sequences represented by SEQ ID NOS:43 and 44 based on the partial nucleotide sequence of a bacterium Flavobacterium sp. SA-0082 as described in WO 97/32010 (deposited at International Patent Organism Depositary, National Institute of Advanced Industrial Science and Technology, AIST Tsukuba Central 6, 1-1, Higashi 1-chome, Tsukuba-shi, Ibaraki 305-8566, Japan on March 29, 1995 under accession number FERM P-14872, and deposited at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM BP-5402 (date of request for transmission to international depositary authority: February 15, 1996)) were used. An amplified product (573 bp) obtained by a PCR using a genomic DNA from Flavobacterium sp. SA-0082 as a template and a combination of primers represented by SEQ ID NOS:45 and 46, which was then purified using Suprec02 (Takara Shuzo), was used as the DNA as the template in this Example. The reaction was carried out as follows. Briefly, 2 μl of one of two annealing solutions (500 mM potassium chloride and 8 μM spermidine, or 0.05% propylenediamine) was added to 120 pmol each of the primers. A final volume of 10 μl of each of the mixtures further containing 10 ng or 1 ng of the PCR-amplified fragment was heat-denatured at 98°C for 2 minute. After denaturation, the mixtures were rapidly cooled on ice to anneal the primers to the template.

After annealing, 40 μl of one of three types of buffers (42.5 mM Tricine-potassium hydroxide buffer (pH 8.5), 42.5 mM Bicine-potassium hydroxide buffer (pH 8.3) and 42.5 mM HEPES-potassium hydroxide buffer (pH 7.8)) each containing 0.625 mM each of dNTPs, 5.0 mM magnesium acetate, 0.0125% bovine serum albumin (BSA), 1.25% dimethyl sulfoxide (DMSO), 30 U of E. coli RNase H and 11 U of BcaBEST DNA polymerase was added to the mixture to make the final volume to 50 μl. The reaction mixtures were incubated at 52°C for 1 hour. After reaction, 3 μl each of the reaction mixtures was subjected to electrophoresis on 3.0% agarose gel. The results are shown in Figure 1. Figure 1 illustrates the results of electrophoresis of reactions using the respective combinations of the annealing solutions and the buffers. Lane 1: molecular weight marker (100 bp ladder, Takara Shuzo); lane 2: propylenediamine/Tricine (template: 10 ng); lane 3: propylenediamine/HEPES (template: 10 ng); lane 4: propylenediamine/HEPES (template: 1 ng); lane 5: propylenediamine/Bicine (template: 10 ng); lane 6: propylenediamine/Bicine (template: 1 ng); lane 7: 500 mM potassium chloride and 8 μM spermidine/Bicine (template: 10 ng); lane 8: 500 mM potassium chloride and 8 μM spermidine/Bicine (template: 1 ng); lane 9: molecular weight marker (100 bp ladder); lane 10: propylenediamine/Tricine (template: 1 ng); lane 11: 500 mM potassium chloride and 8 μM spermidine/Tricine (template: 1 ng); lane 12: propylenediamine/HEPES (template: 1 ng); lane 13: 500 mM potassium chloride and 8 μM spermidine/HEPES (template: 1 ng); lane 14: propylenediamine/Bicine (template: 1 ng); and lane 15: 500 mM potassium chloride and 8 μM spermidine/Bicine (template: 1 ng).

As shown in Figure 1, a greater amount of the amplification product of interest was obtained when the annealing solution containing 500 mM potassium chloride + 8 μM spermidine was used for the annealing of the primers to the template DNA for either of the three buffers regardless of the amount of the DNA as the template. In particular, the combination of the annealing solution containing 500 mM potassium chloride + 8 μM spermidine and the Bicine-potassium hydroxide buffer yielded good results in this Example.

(2) The effect of an annealing solution in case where a PCR-amplified fragment from λ DNA was used as a template was examined. The chimeric oligonucleotide primers as described in Example 2(1) were used in this Example.

The PCR-amplified fragment as prepared in Example 2(1) or λ DNA was used as a template DNA. The reaction was carried out as follows. Briefly, 2 μl of one of three types of annealing solutions (500 mM potassium chloride and 8 μM spermidine, 0.05% propylenediamine, or sterile water) was added to 120 pmol each of the primers. 10 μl of the mixtures further containing 10 ng or 1 ng of the PCR-amplified fragment were prepared. The mixtures were heat-denatured at 98°C for 2 minutes, and then rapidly cooled on ice to anneal the primers to the template.

After annealing, 40 μl of one of three types of buffers (42.5 mM Tricine-potassium hydroxide buffer (pH 8.5), 42.5 mM Bicine-potassium hydroxide buffer (pH 8.3) and 42.5 mM HEPES-potassium hydroxide buffer (pH 7.8)) each containing 0.625 mM each of dNTPs, 5.0 mM magnesium acetate, 0.0125% BSA, 1.25% DMSO, 30 U of E. coli RNase H and 11 U of BcaBEST DNA polymerase was added to the mixture to make the final volume to 50 μl. The reaction mixtures were incubated at 60°C for 1 hour. After reaction, 3 μl each of the reaction mixtures was subjected to electrophoresis on 3.0% agarose gel. The results are shown in Figure 2. Figure 2 illustrates the results of electrophoresis showing the results for the examination of combinations of the amounts of the template, the reaction buffers and the annealing solutions. Lane 1: marker (100 bp ladder); lane 2: 10 ng of the template, a combination of Tricine/500 mM potassium chloride and 8 μM spermidine; lane 3: 1 ng of the template, a combination of Tricine/500 mM potassium chloride and 8 μM spermidine; lane 4: 10 ng of the template, a combination of Bicine/500 mM potassium chloride and 8 μM spermidine; lane 5: 1 ng of the template, a combination of Bicine/500 mM potassium chloride and 8 μM spermidine; lane 6: 10 ng of the template, a combination of HEPES/500 mM potassium chloride and 8 μM spermidine; lane 7: 1 ng of the template, a combination of HEPES/500 mM potassium chloride and 8 μM spermidine; lane 8: molecular weight marker (100 bp ladder); lane 9: 10 ng of the template, a combination of Tricine/propylenediamine; lane 10: 1 ng of the template, a combination of Tricine/propylenediamine; lane 11: 10 ng of the template, a combination of Bicine/propylenediamine; lane 12: 1 ng of the template, a combination of Bicine/propylenediamine; lane 13: 10 ng of the template, a combination of HEPES/propylenediamine; lane 14: 1 ng of the template, a combination of HEPES/propylenediamine; lane 15: molecular weight marker (100 bp ladder); lane 16: 10 ng of the template, a combination of Tricine/water; lane 17: 1 ng of the template, a combination of Tricine/water; lane 18: 10 ng of the template, a combination of Bicine/water; lane 19: 1 ng of the template, a combination of Bicine/water; lane 20: 10 ng of the template, a combination of HEPES/water; and lane 21: 1 ng of the template, a combination of HEPES/water.

As shown in Figure 2, the amplified fragments of interest were observed for the respective combinations of the three buffers and the three annealing solutions regardless of the amount of the template DNA. In particular, it was confirmed that a greater amount of the amplified fragment was obtained using the combination of the Bicine buffer and the annealing solution containing 500 mM potassium chloride and 8 μM spermidine.

Example 4

The effect of the presence of an inhibitor of reverse transcriptase (RTase) on the method of the present invention was examined. Phosphonoformic acid (PFA) was used as an inhibitor of RTase. Primers represented by SEQ ID NOS:47 and 48 were used in this Example. An amplified product (576 bp) obtained by a PCR using a genomic DNA from enterohemorrhagic Escherichia coli O-157 as a template and primers represented by SEQ ID NOS:49 and 50, which was then purified using Suprec02 (Takara Shuzo), was used as the template DNA. The reaction was carried out as follows. Briefly, 10 μl of a mixture prepared by adding 1 ng of the PCR-amplified fragment to 120 pmol each of the primers and 2 μl of an annealing solutions containing 500 mM potassium chloride and 8 μM spermidine was heat-denatured at 98°C for 2 minutes, and then rapidly cooled on ice to anneal the primers to the template.

After annealing, 40 μl of a mixture containing 0.625 mM each of dNTPs, 42.5 mM Tricine-potassium hydroxide buffer (pH 8.5), 5.0 mM magnesium acetate, 0.0125% bovine serum albumin (BSA), 1.25% dimethyl sulfoxide (DMSO), 30 U of E. coli RNase H and 11 U of BcaBEST DNA polymerase as well as PFA to a concentration of 500 μg/ml or 50 μg/ml were added to the annealing mixture to make the final volume to 50 μl. The reaction mixtures were incubated at 55°C for 1 hour. A system without the addition of PFA was also prepared as a control. After reaction, 9 μl each of the reaction mixtures was subjected to electrophoresis on 3.0% agarose gel. The results are shown in Figure 3. Figure 3 illustrates the results of electrophoresis which shows the effect of the inhibitor of reverse transcriptase activity. Lane 1: molecular weight marker (100 bp ladder); lane 2: without the addition of PFA; lane 3: with the addition of PFA at a concentration of 500 μg/ml; lane 4: with the addition of PFA at a concentration of 50 μg/ml.

As shown in Figure 3, when PFA was added, non-specific amplification was suppressed and the amplified fragment of interest was observed. In particular, it was confirmed that the non-specific amplification product which was observed in the system without the addition of PFA was not observed and the amplified fragment of interest was clearly amplified in the system to which PFA was added at a concentration of 500 μg/ml.

Example 5

The relationship between the length of a fragment to be amplified and the detection sensitivity in the method of the present invention was examined.

(1) Primers for amplifying Escherichia coli 0-157 vero toxin represented by SEQ ID NOS:51 to 53 were synthesized. The chimeric oligonucleotide primers used in Example 4 were also used. The length of a fragment to be amplified using each of the combinations of primers was as follows: 247 bp (SEQ ID NOS:51 and 48); 168 bp (SEQ ID NOS:52 and 53); 206 bp (SEQ ID NOS:52 and 48); 135 bp (SEQ ID NOS:47 and 53); and 173 bp (SEQ ID NOS: 47 and 48). The purified PCR-amplified 576-bp fragment prepared in Example 4 was used as a template DNA in this Example. The reaction was carried out as follows. Briefly, 10 μl of a mixture containing 60 pmol each of the primers, 2 μl of 0.05% aqueous solution of propylenediamine and 10 fg to 10 ng of the PCR-amplified fragment was heat-denatured at 98°C for 2 minutes and then cooled to 55°C in Thermal Cycler Personal (Takara Shuzo) to anneal the primers to the template.

After annealing, 40 μl of a reaction mixture containing 0.625 mM each of dNTPs, 42.5 mM Tricine-potassium hydroxide buffer (pH 8.5), 5.0 mM magnesium acetate, 0.0125% bovine serum albumin (BSA), 1.25% dimethyl sulfoxide (DMSO), 30 U of E. coli RNase H, 5.5 U of BcaBEST DNA polymerase and sterile water was added to the mixture to make the final volume to 50 μl. The reaction mixtures were incubated at 55°C for 1 hour. After reaction, 5 μl each of the reaction mixtures was subjected to electrophoresis on 3.0% agarose gel. As a control, detection of 10 fg to 1 pg of the PCR-amplified fragment was carried out using primers represented by SEQ ID NOS:54 and 55. A 135-bp fragment is amplified using the combination of these primers. 50 μl of a PCR solution containing 60 pmol each of the primers, 5 μl of 10 x Ex Taq buffer (Takara Shuzo), 1.25 U of TaKaRa Ex Taq DNA polymerase (Takara Shuzo) and 0.2 mM each of dNTPs was prepared. The PCR was carried out as follows: 25 or 30 cycles of 94°C for 30 seconds, 55°C for 30 seconds and 72°C for 30 seconds (2 min. 38 sec./cycle). After reaction, 1 μl (ICAN) or 5 μl (PCR) each of the reaction mixtures was subjected to electrophoresis on 3.0% agarose gel. The results are shown in Figure 4 and Table 1.

Table 1

| Amplification size (bp) | Detection limit |
|---|---|
| ICAN (total time: 70 minutes) | |
| 247 | 100 pg |
| 168 | 100 fg |
| 206 | 100 pg |
| 135 | 10 fg |
| 173 | 100 fg |
| PCR (25 cycles; total time: about 66 minutes) | |
| 135 | 100 fg |
| PCR (30 cycles; total time: about 80 minutes) | |
| 135 | 10 fg |

Figure 4 illustrates the results of electrophoresis which shows the detection limits for amplifying a chain length of 135 bp accomplished by the ICAN (1/50 of the reaction mixture was loaded) and the PCR (1/10 of the reaction mixture was loaded). Lane 1: molecular weight marker (100 bp ladder); lane 2: ICAN using 1 pg of the template; lane 3: ICAN using 100 fg of the template; lane 4: ICAN using 10 fg of the template; lane 5: PCR (25 cycles) using 1 pg of the template; lane 6: PCR (25 cycles) using 100 fg of the template; lane 7: PCR (25 cycles) using 10 fg of the template; lane 8: PCR (30 cycles) using 1 pg of the template; lane 9: PCR (30 cycles) using 100 fg of the template; and lane 10: PCR (30 cycles) using 10 fg of the template.

As shown in Table 1, it was confirmed that almost the same detection sensitivity as that of the PCR was accomplished by the ICAN. Furthermore, the total reaction time for the PCR was about 80 minutes, whereas the reaction time required for accomplishing the same detection sensitivity using the method of the present invention was 70 minutes, confirming that the reaction time can be shortened by using the method of the present invention.

(2) Primers for amplifying λ DNA having nucleotide sequences represented by SEQ ID NOS:39, 40 and 56 were synthesized. The length of a fragment to be amplified using each of the combinations of primers was as follows: 151 bp (SEQ ID NOS:39 and 40); and 125 bp (SEQ ID NOS:56 and 40). The template DNA prepared in Example 2(1) was used in this Example. The reaction was carried out as follows. Briefly, 2 μl of an annealing solution (500 mM potassium chloride and 8 μM spermidine), 1 fg to 1 ng of the template were added to 120 pmol each of the primers and fill up to 10 μl by sterile water. The mixture was heat-denatured at 98°C for 2 minutes, and then rapidly cooled on ice to anneal the primers to the template.

After annealing, 40 μl of a mixture containing 0.625 mM each of dNTPs, 42.5 mM Tricine-potassium hydroxide buffer (pH 8.5), 5.0 mM magnesium acetate, 0.0125% BSA, 1.25% DMSO, 30 U of E. coli RNase H and 11 U of BcaBEST DNA polymerase was added to the mixture to make the final volume to 50 µl with sterile water. The reaction mixtures were incubated at 60°C for 1 hour. After reaction, 3 µl each of the reaction mixtures was subjected to electrophoresis on 3.0% agarose gel. The results are shown in Table 2.

Table 2

| Amplification size (bp) | Detection limit |
|---|---|
| 125 | 10 fg |
| 151 | 100 fg |

As shown in Table 2, it was confirmed that, when λ DNA was used as a template, a detection sensitivity of as low as 10 fg could be also accomplished by examining the optimal region.

(3) A plasmid was prepared by inserting an amplified fragment (length: 340 bp) into a plasmid T7Blue T-Vector (Takara Shuzo). The fragment was prepared as described in JP-A 9-140383 using synthetic primers for amplifying a chrysanthemum viroid gene having nucleotide sequences represented by SEQ ID NOS:57 and 58, and an RNA from chrysanthemum infected with the viroid as a template. The plasmid was used to transform Escherichia coli JM109 competent cells (Takara Shuzo). The transformant was cultured in 5 ml of LB medium at 37°C for 16 hour. The plasmid was purified from the collected cells using QIAGEN Plasmid Mini Kit (Qiagen) according to the manual. Dilutions containing 0 fg, 1 fg, 10 fg, 100 fg, 1 pg, 10 pg, 100 pg or 1 ng of the plasmid in 1 µl of sterile water were prepared based on the concentration of the plasmid as measured using Beckman DU-600 (Beckman). 1 µl of one of the thus prepared plasmid solutions was used as a template for 50 µl each of ICAN reaction systems. Primers CSVD-F2 and CSVD-R6 having nucleotide sequences represented by SEQ ID NOS:59 and 60 were used in this Example. The reaction was carried out as follows. Briefly, 10 µl of a mixture containing 50 pmol each of the primers, 1 µl of the prepared plasmid solution and propylenediamine at a final concentration of 0.01% was prepared. The mixture was heated at 98°C for 2 minutes, cooled to 60°C, incubated at the temperature for 1 minute in Thermal Cycler Personal (Takara Shuzo), and then placed on ice.

After annealing, at final concentrations, 20 mM HEPES-potassium hydroxide buffer (pH 7.8), 100 mM potassium acetate, 1% DMSO, 0.01% BSA, 4 mM magnesium acetate, 500 µM each of dNTPs, 30 U of E. coli RNase H and 5.5 U of BcaBEST DNA polymerase were added to the mixture to make the final volume to 50 µl with sterile water. The reaction mixtures were placed in Thermal Cycler Personal which had been set at 60°C and reacted for 60 minutes. After reaction, 3 µl each of the reaction mixtures was subjected to electrophoresis on 3% NuSieve 3:1 agarose. As a result, the amplification products of interest (about 90 bp, about 70 bp and about 50 bp) were observed using a template at a concentration of 10 fg.

Example 6

Primers to be used in the method of the present invention were examined.

(1) The Tm value of a primer and the reaction temperature were examined. Primers for amplifying Flavobacterium sp. SA-0082 having nucleotide sequences represented by SEQ ID NOS:43 and 61-63 were synthesized. These primers were constructed such that a region of 160 bp or shorter having a GC content of about 20% was amplified. The length of a fragment to be amplified using each of the combinations of primers was as follows: 126 bp (SEQ ID NOS:43 and 62); 158 bp (SEQ ID NOS:43 and 63); 91 bp (SEQ ID NOS:61 and 62); and 123 bp (SEQ ID NOS:61 and 63). The PCR-amplification product as prepared in Example 3(1) was used as a DNA as the template in this Example. The reaction was carried out as follows. Briefly, 10 µl of a mixture containing 120 pmol each of the primers, 2 µl of one of three types of annealing solutions (500 mM potassium chloride and 8 µM spermidine, 0.05% propylenediamine, or water) and 1 fg to 10 ng of the template was prepared. The mixture was heat-denatured at 98°C for 2 minutes, and then cooled on ice to anneal the primers to the template.

After annealing, 40 µl of one of three types of buffers (17 mM Tricine-potassium hydroxide buffer (pH 8.5), 17 mM Bicine-potassium hydroxide buffer (pH 8.3) and 20 mM HEPES-potassium hydroxide buffer (pH 7.8)) containing 0.625 mM each of dNTPs, 5.0 mM magnesium acetate, 0.0125% bovine serum albumin (BSA), 1.25% dimethyl sulfoxide (DMSO), 30 U of E. coli RNase H and 11 U of BcaBEST DNA polymerase was added to the mixture to make the final volume to 50 µl. The reaction mixtures were incubated at 52, 55 or 60°C for 1 hour. After reaction, 3 µl each of the reaction mixtures was subjected to electrophoresis on 3.0% agarose gel. As a result, the amplified fragment of interest was observed using the reaction temperature of 52°C. In particular, a greater amount of amplified fragment of interest was obtained using the combination of the annealing solution containing 500 mM potassium chloride and 8 µM spermidine and Tricine or Bicine buffer. The primer pair, the length of the amplified fragment and the detection sensitivity for the reaction temperature of 52°C are shown in Figure 5 and Table 3.

Table 3

| Amplification size (bp) | Detection limit |
|---|---|
| 126 | 100 fg |
| 158 | 1 pg |
| 91 | 1 fg |
| 123 | 100 fg |

Figure 5 illustrates the results of electrophoresis which shows the relationship between the length of the amplified fragment and the amount of the DNA as the template when an AT-rich region was amplified. Lane 1: molecular weight marker (100 bp ladder); lane 2: amplification of a fragment of 91 bp in length using 1 pg of the template; lane 3: amplification of a fragment of 91 bp in length using 100 fg of the template; lane 4: amplification of a fragment of 91 bp in length using 10 fg of the template; lane 5: amplification of a fragment of 91 bp in length using 1 fg of the template; lane 6: amplification of a fragment of 123 bp in length using 1 pg of the template; lane 7: amplification of a fragment of 123 bp in length using 100 fg of the template; lane 8: amplification of a fragment of 123 bp in length using 10 fg of the template; lane 9: amplification of a fragment of 126 bp in length using 1 pg of the template; lane 10: amplification of a fragment of 126 bp in length using 100 fg of the template; lane 11: amplification of a fragment of 126 bp in length using 10 fg of the template; lane 12: amplification of a fragment of 158 bp in length using 1 pg of the template; lane 13: amplification of a fragment of 158 bp in length using 100 fg of the template; and lane 14: amplification of a fragment of 158 bp in length using 10 fg of the template.

As shown in Figure 5 and Table 3, it was demonstrated that good results were yielded by lower the reaction temperature depending on the Tm value of the primer when the method of the present invention was carried out using an AT-rich template and a set of AT-rich primers.

(2) The higher-order structure of the primer may influence the reactivity in the method of the present invention. Then, modification of the primer for avoiding the formation of the higher-order structure of the primer and for making the primer readily anneal to the objective template was examined. Primers represented by SEQ ID NOS:47, 48 and 64-69 were used. Specifically, a primer having a nucleotide sequence represented by SEQ ID NO:47, primers 120I4, 121I5 and 122I6 which are primers having nucleotide sequences represented by SEQ ID NOS:64 to 66 and having an inosine deoxynucleotide at the fourth, fifth or sixth base from the 3'-terminus, a primer having a nucleotide sequence represented by SEQ ID NO:104, and primers 123I4, 124I5 and 125I6 which are primers having nucleotide sequences represented by SEQ ID NOS:67 to 69 and having an inosine deoxynucleotide at the fourth, fifth or sixth base from the 3'-terminus were used. The DNA as the template as prepared in Example 4 was used in this Example. The reaction was carried out as follows. Briefly, 10 µl of a mixture containing 50 pmol each of the primers, 2 µl of 0.05% aqueous solution of propylenediamine, 1 ng to 10 ng of the DNA as the template and sterile distilled water was heated at 98°C for 2 minutes, cooled to 55°C, and incubated at the temperature for 1 minute using a thermal cycler (GeneAmp PCR System 9600, Applied Biosystems).

After annealing, 0.625 mM each of dNTPs, 42.5 mM Tricine-potassium hydroxide buffer (pH 8.5), 5.0 mM magnesium acetate, 0.0125% BSA, 1.25% DMSO, 30 U of E. coli RNase H or 5 U of a heat-resistant RNase H from Thermus thermophilus (Tth) (Toyobo, hereinafter referred to as Tth RNase H) and 5.5 U of BcaBEST DNA polymerase were added to the mixture to make the final volume to 50 µl with sterile water. The reaction mixtures were incubated at 55°C for 1 hour. After reaction, 5 µl each of the reaction mixtures was subjected to electrophoresis on 3.0% agarose gel. The results are shown in Figure 6.

Figure 6 illustrates the results of electrophoresis which shows the effects of chimeric oligonucleotide primers containing inosine deoxynucleotides when E. coli RNase H and Tth RNase H were used. Lanes 2 to 9 represent the results obtained using E. coli RNase H. Lanes 10 to 17 represent the results obtained using Tth RNase H. Lane 1: molecular weight marker (100 bp ladder); lane 2: a pair of primers represented by SEQ ID NOS:47 and 48, 1 ng of the template; lane 3: a pair of primers 120I4 and 123I4, 1 ng of the template; lane 4: a pair of primers 121I5 and 124I5, 1 ng of the templates lane 5: a pair of primers 122I6 and 125I6, 1 ng of the template; lane 6: a pair of primers represented by SEQ ID NOS:47 and 48, 10 ng of the template; lane 7: a pair of primers 120I4 and 123I4, 10 ng of the template; lane 8: a pair of primers 121I5 and 124I5, 10 ng of the template; lane 9: a pair of primers 122I6 and 125I6, 1 ng of the template; lane 10: a pair of primers represented by SEQ ID NOS:47 and 48, 1 ng of the template; lane 11: a pair of primers 120I4 and 123I4, 1 ng of the template; lane 12: a pair of primers 121I5 and 124I5, 1 ng of the template; lane 13: a pair of primers 122I6 and 125I6, 1 ng of the template; lane 14: a pair of primers represented by SEQ ID NOS:47 and 48, 10 ng of the template; lane 15: a pair of primers 120I4 and 12314, 10 ng of the template; lane 16: a pair of primers 121I5 and 124I5, 10 ng of the template; and lane 17: a pair of primers 122I6 and 125I6, 10 ng of the template.

As shown in Figure 6, when primers having inosines being incorporated at fourth or fifth base from the 3'-termini of the primers were used, increase in the amplification product of interest was observed using either of E. coli RNase H and the heat-resistant RNase H from Thermus thermophilus regardless of the amount of the template. These results demonstrate that the reactivity of the ICAN is improved by incorporating inosine at an appropriate position.

(3) Primers were examined for the same purpose as that in (2) above. Oligonucleotide primers 1S and 4S having nucleotide sequences represented by SEQ ID NOS:84 and 85 in which the three bases at the 3'-terminus were $\alpha$-S (or alpha-thio) ribonucleotides, i.e., which had 5'-phosphothioate bonds in the RNA moieties, were synthesized. In addition, oligonucleotide primers 1N3N3 and 4N3N3 having nucleotide sequences represented by SEQ ID NOS:70 and 71 and having ribonucleotides at the three bases from the 3'-terminus and in a portion of the sequence of the deoxyribonucleotide moiety, i.e., ribonucleotides at eleventh to thirteenth bases from the 3'-terminus of the primer, were synthesized. The template DNA as prepared in Example 4 was used. The reaction was carried out as follows. Briefly, 10 μl of a mixture containing 50 pmol each of the primers, 2 μl of 0.05% aqueous solution of propylenediamine, 10 ng of the DNA as the template and sterile water was heated at 98°C for 2 minutes using a thermal cycler, and then placed on ice for cooling.

After annealing, 40 μl of a mixture containing 0.625 mM each of dNTPs, 42.5 mM Tricine-potassium hydroxide buffer (pH 8.5), 5.0 mM magnesium acetate, 0.0125% BSA, 1.25% DMSO, 30 U of E. coli RNase H or 5 U of Tth RNase H and 5.5 U of BcaBEST DNA polymerase was added to the mixture to make the final volume to 50 μl with sterile water. The reaction mixtures were incubated at 55°C for 1 hour in a thermal cycler.

After reaction, 5 μl each of the reaction mixtures was subjected to electrophoresis on 3.0% agarose gel. As a result, the amplification product was clearly observed at the expected position using the combination of primers 1S and 4S or 1N3N3 and 4N3N3 regardless of the type of the RNase H used. These results confirmed that modification of a primer at its 3'-terminus with 5'-phophothioate was effective for the method of the present invention. Additionally, it was confirmed that substitution with a ribonucleotide at an appropriate internal position in addition to the 3'-terminus of a primer was effective for improving the reactivity of the method of the present invention.

Example 7

Use of a DNA polymerase having E. coli RNase H activity in the presence of a specific metal ion in the method of the present invention was examined. 10 μl of a mixture containing 120 pmol each of the chimeric oligonucleotide primers used in Example 2(1), 2 μl of an annealing solution containing 500 mM potassium chloride and 8 μM spermidine, 1 ng of the DNA as the template used in Example 2(1) and sterile water was heat-denatured at 98°C for 2 minutes, and then rapidly cooled on ice to anneal the primers to the template. After annealing, 40 μl of a mixture containing 0.625 mM each of dNTPs, 42.5 mM Tricine-potassium hydroxide buffer (pH 8.5), 5.0 mM magnesium acetate, 0.0125% BSA, 1.0% DMSO and 11 U of BcaBEST DNA polymerase, and manganese chloride (Nacalai Tesque) at a final concentration of 0.5, 2.5, 5.0 or 10 mM were added to the mixture to make the final volume to 50 μl with sterile water. The reaction mixtures were incubated at 60°C for 1 hour. In addition, a mixture without the addition of manganese chloride, and a mixture to which 30 U of E. coli RNase H was added but manganese chloride was not added were prepared as controls. After reaction, 3 μl each of the reaction mixtures was subjected to electrophoresis on 3.0% agarose gel. The results are shown in Figure 7.

Figure 7 illustrates electrophoresis which shows the results of the ICAN utilizing the RNase H activity of BcaBEST DNA polymerase. Lane 1: molecular weight marker (100 bp ladder); lane 2: without the addition of manganese chloride/with the addition of E. coli RNase H; lane 3: without the addition of manganese chloride/without the addition of E. coli RNase H; lane 4: with the addition of 0.5 mM manganese chloride/without the addition of E. coli RNase H; lane 5: with the addition of 2.5 mM manganese chloride/without the addition of E. coli RNase H; lane 6: with the addition of 5.0 mM manganese chloride/without the addition of E. coli RNase H; and lane 7: with the addition of 10.0 mM manganese chloride/without the addition of E. coli RNase H.

As shown in Figure 7, the amplification product of interest was observed for a reaction system to which manganese chloride was added at a concentration of 2.5 mM in the absence of E. coli RNase H.

Example 8

The method of the present invention was examined using a practical biological sample.

(1) Detection was carried out using a hot water-extract prepared from a culture of enterohemorrhagic Escherichia coli 0-157 (ATCC accession number 43895) as a template. Enterohemorrhagic Escherichia coli O-157 was cultured in mEC medium containing novobiocin at 42°C for 18 hours, and then heated at 95°C for 10 minutes. Hot water-extracts of O-157 corresponding to 0, 1, 10, $10^2$, $10^3$, $10^4$ or $10^5$ cells were prepared by diluting the extract with sterile water. Vero toxin 2 (VT2) gene was amplified using one of these hot water-extracts of O-157 under the same conditions as those in Example 5(1). In addition, a PCR was carried out using the same template under the conditions as described in Example 5(1) as a control. After reaction, 1 μl (ICAN) or 5 μl (PCR) each of the reaction mixtures was subjected to electrophoresis on 3.0% agarose gel. The results are shown in Table 4 and Figure 8.

Table 4

| Amplification size (bp) | Detection limit (cells) |
|---|---|
| ICAN (total time: 70 minutes) | |
| 135<br>173 | $10^2$<br>$10^3$ |
| PCR (25 cycles; total time: about 66 minutes) | |
| 135 | $10^3$ |
| PCR (30 cycles; total time: about 80 minutes) | |
| 135 | $10^2$ |

Figure 8 illustrates the results of electrophoresis which shows the detection of Escherichia coli 0157 using ICAN or PCR. The chain length to be amplified was 135 bp. Lane 1: molecular weight marker (100 bp ladder); lane 2: ICAN for $10^4$ cells; lane 3: ICAN for $10^3$ cells; lane 4: ICAN for $10^2$ cells; lane 5: PCR of 25 cycles for $10^4$ cells; lane 6: PCR of 25 cycles for $10^3$ cells; lane 7: PCR of 25 cycles for $10^2$ cells; lane 8: PCR of .30 cycles for $10^4$ cells; lane 9: PCR of 30 cycles for $10^3$ cells; and lane 10: PCR of 30 cycles for $10^2$ cells.

As shown in Table 4 and Figure 8, it was confirmed that the detection sensitivity of the detection method of the present invention was equivalent to that of the PCR, and that the method of the present invention required shorter time for detection as compared with that required for the PCR.

(2) λ DNA was detected using the primers represented by SEQ ID NOS:40 and 56 used in Examples 2 and 4. The reaction was carried out as follows. Briefly, 10 μl of a mixture containing 120 pmol each of the primers, 2 μl of an annealing solution containing 500 mM potassium chloride and 8 μM spermidine, 10 fg to 1 ng of λ DNA (Takara Shuzo) and sterile water was prepared. The mixture was heat-denatured at 98°C for 2 minutes, and then rapidly cooled on ice to anneal the primers to the template.

After annealing, 40 μl of a mixture containing 0.625 mM each of dNTPs, 42.5 mM Tricine-potassium hydroxide buffer (pH 8.5), 5.0 mM magnesium acetate, 0.0125% BSA, 1.25% DMSO, 30 U of E. coli RNase H and 11 U of BcaBEST DNA polymerase was added to the mixture to make the final volume to 50 μl with sterile water. The reaction mixtures were incubated at 60°C for 1 hour. After reaction, 3 μl each of the reaction mixtures was subjected to electrophoresis on 3.0% agarose gel. The results are shown in Table 5.

Table 5

| Amplification size (bp) | Detection limit |
|---|---|
| 125 | 1 pg |

As shown in Table 5, it was confirmed that the method of the present invention was effective in detecting λ DNA.

(3) Detection was carried out using a genomic DNA from a bacterium Flavobacterium sp. SA-0082 as a template and the primers represented by SEQ ID NOS:61 and 62 used in Example 6(1). The genomic DNA as a template was prepared according to a conventional method from the bacterium of genus Flavobacterium cultured as described in WO 97/32010. The reaction was carried out as follows. Briefly, 10 μl of a mixture containing 120 pmol each of the primers, 2 μl of an annealing solution containing 500 mM potassium chloride and 8 μM spermidine, 10 fg to 1 ng of the genomic DNA and sterile water was prepared. The mixture was heat-denatured at 98°C for 2 minutes, and then rapidly cooled on ice to anneal the primers to the template.

After annealing, 40 μl of a mixture containing 0.625 mM each of dNTPs, 42.5 mM Tricine-potassium hydroxide buffer (pH 8.5), 5.0 mM magnesium acetate, 0.0125% BSA, 1.25% DMSO, 30 U of E. coli RNase H and 11 U of BcaBEST DNA polymerase was added to the mixture to make the final volume to 50 μl with sterile water. The reaction mixtures were incubated at 52°C for 1 hour. After reaction, 3 μl each of the reaction mixtures was subjected to electrophoresis on 3.0% agarose gel. The results are shown in Table 6 and Figure 9.

Table 6

| Amplification size (bp) | Detection limit |
|---|---|
| 91 | 100 fg |

Figure 9 illustrates the results of electrophoresis which shows the detection of a bacterium of genus Flavobacterium. Lane 1: molecular weight marker (100 bp ladder); lane 2: 1 ng of the template; lane 3: 10 pg of the template; lane 4: 1 pg of the template; lane 5: 100 fg of the template; and lane 6: 10 fg of the template.

As shown in Table 6 and Figure 9, it was confirmed that the method of the present invention was effective in detecting a bacterium.

Example 9

A method for detecting a target nucleic acid in which the amplification method of the present invention and a hybridization method were combined was examined. Enterohemorrhagic Escherichia coli O-157 was selected as a target. The DNA as the template was prepared as described in Example 8(1). A region of about 100 bp having a GC content of about 40% was selected as a fragment to be amplified. Primers VT2-IF20 and VT2-IR20-2 having nucleotide sequences represented by SEQ ID NOS:51 and 72 were used as primers. The reaction was carried out as follows. Briefly, 10 µl of a mixture containing 50 pmol each of the primers VT2-IF20 and VT2-IR20-2, an annealing solution containing propylenediamine at a final concentration of 0.01%, one of the hot water-extracts corresponding to 0 to $10^4$ cells and sterile water was prepared. The mixture was heat-denatured at 98°C for 2 minutes, cooled to 55°C and incubated at the temperature for 1 minute in Thermal Cycler Personal (Takara Shuzo), and then placed on ice for annealing.

After annealing, at final concentrations, 20 mM HEPES-potassium hydroxide buffer (pH 7.8), 100 mM potassium acetate, 1% DMSO, 0.01% BSA, 4 mM magnesium acetate, 500 µM each of dNTPs, 30 U of E. coli RNase H and 5.5 U of BcaBEST DNA polymerase was added to the mixture to make the final volume to 50 µl with sterile water. The reaction mixtures were placed on Thermal Cycler Personal which had been set at 55°C and incubated at the temperature for 60 minutes. As a control, a PCR was carried out using O-157 Typing Set (Takara Shuzo) according to the manual using Thermal Cycler Personal. The PCR was carried out as follows: 35 cycles of 94°C for 1 minute, 55°C for 1 minute and 72°C for 1 minute. This reaction required about 4 minutes per cycle and a total time of about 145 minutes. The expected size of the amplification product was 404 bp. After reaction, 3 µl each of the reaction mixtures was subjected to electrophoresis on 3% NuSieve 3:1 agarose. The results are shown in Figure 28A. Figure 28A shows the results of electrophoresis for the detection of enterohemorrhagic Escherichia coli 0157 vero toxin II gene using the ICAN or the PCR. Lane M1: molecular weight marker (50-2000 bp); lane M2: molecular weight marker (100 bp ladder); lane N: negative control; lane 1: the template corresponding to 1 cell; lane 2: the template corresponding to 10 cells; lane 3: the template corresponding to $10^2$ cells; lane 4: the template corresponding to $10^3$ cells; and lane 5: the template corresponding to $10^4$ cells. Furthermore, results of comparison between the amplification levels accomplished by the ICAN and the PCR for 1 cell or 10 cells are shown in Table 7.

Table 7

| | Number of E. coli O-157 cells | | |
|---|---|---|---|
| | 0 | 1 | 10 |
| ICAN | - | + | +++ |
| PCR | - | + | ++ |
| -: no amplification; + to +++ indicate the degree of amplification in three grades. | | | |

As shown in Figure 28A and Table 7, the amplification products of interest were obtained for the reaction systems using a hot water-extract corresponding to 1 cell according to both the detection method of the present invention and the PCR. Dot blot hybridization was further carried out for the amplification product obtained according to the ICAN using an oligonucleotide VT2 having a nucleotide sequence represented by SEQ ID NO:73 labeled with biotin at the 5'-terminus as a probe. Hybridization was carried out under as follows. Briefly, 1 µl of a reaction mixture was denatured at 98°C for 5 minutes, rapidly cooled on ice, and spotted onto Hybond-N™ (Amersham Pharmacia Biotech). After exposure to UV, the membrane was placed in a hybridization bag. 10 ml of a hybridization solution containing 0.5 M disodium hydrogenphosphate (pH 7.2), 1 mM ethylenediaminetetraacetic acid and 7% sodium lauryl sulfate was added thereto. Pre-hybridization was then carried out at 42°C for 30 minutes. 10 µl of the solution of the VT2 probe at a concentration of 100 ng/µl was heat-denatured and added to the pre-hybridization reaction system. After hybridization at 42°C for 60 minutes, the membrane was washed twice in a solution containing 66.6 mM sodium chloride, 66.6 mM sodium citrate and 0.1% sodium lauryl sulfate at room temperature for 5 minutes, incubated in 6 ml of a washing buffer (0.3 M sodium chloride, 17.3 mM disodium hydrogenphosphate dihydrate, 2.5 mM EDTA, 0.1% sodium lauryl sulfate) to which 2 µl of 5 mg/ml horseradish peroxidase streptoavidin conjugate (PIERCE) was added at 42°C for 12 minutes, and then washed twice in the washing buffer at room temperature. The membrane was then washed in 10 ml of 0.1 M citrate buffer (pH 5.0) at room temperature and reacted in a mixture of 5 ml of 0.1 M citrate buffer, 5 μl of 3% hydrogen peroxide and 250 μl of a solution of 2 mg/ml tetramethylbenzidine (TMB, Nacalai Tesque) in ethanol in the dark for about 10 minutes. After color development, the reaction was terminated with deionized water. The results are shown in Figure 28B. Figure 28B shows the results of dot blot hybridization for detecting a gene for vero toxin II from enterohemorrhagic Escherichia coli O-157 by the ICAN. The results were consistent with those obtained for the above-mentioned electrophoresis. Thus, the detection sensitivity of the method of the present invention was equivalent to that of the PCR. The total time required for the amplification reaction using the ICAN of the present invention was 1/2 or shorter as compared with that required for the PCR. Thus, the ICAN of the present invention was confirmed to be effective as a method for detecting a pathogen and the like.

Example 10

(1) Combination of a reverse transcription reaction and the method of the preset invention was examined using an RNA from cultured cells as a template. The reaction was carried out as follows. Briefly, RAW264.7 cells (ATCC TIB 71) were suspended in Dulbecco's modified Eagle's medium (Bio Whittaker, 12-604F) containing 10% fetal calf serum (Gibco) at a concentration of 1.5 x $10^5$ cells/ml. 5 ml of the suspension was added to each well of a 6-well microtiter plate and the plate was incubated at 37°C overnight in the presence of 5% $CO_2$. 50 μl of a 100 μg/ml solution of lipopolysaccharide (LPS, Sigma) in water and 50 μl of a 1000 U/μl solution of interferon-γ (IFN-γ, Genzyme Techne) in water were added to the well. The plate was incubated for additional 4 hours. An RNA was then prepared from the cells using RNeasy Mini Kit (Qiagen) according to the instructions attached to the kit. As a negative control, a group to which LPS or IFN-γ was not added was provided.

A cDNA was prepared by incubating 60 μl of a mixture containing 3 μg of the thus prepared RNA, 10 mM tris-hydrochloride buffer (pH 8.3), 50 mM KCl, 5 mM $MgCl_2$, 1 mM each of dNTPs, 150 pmol of random 6mers primer, 60 U of ribonuclease inhibitor (Takara Shuzo) and 15 U of Reverse Transcriptase XL (AMV) (Takara Shuzo, 2620A) at 30°C for 10 minutes, 42°C for 1 hour and then 99°C for 5 minutes for inactivating the enzyme using a thermal cycler (GeneAmp PCR System 9600, Applied Biosystems).

Primers having nucleotide sequences represented by SEQ ID NOS:74 and 75 were synthesized based on the nucleotide sequence of the mRNA for mouse inducible NO synthase (iNOS) (GeneBank accession no. NM-010927). As a control, primers for PCR represented by SEQ ID NOS:76 and 77 were also synthesized.

10 μl of a mixture containing 50 pmol each of the primers, 2 μl of an aqueous solution of propylenediamine at a concentration of 0.05%, 1 μl of the cDNA as a template (corresponding to 50 ng of the RNA) and sterile water was prepared. The mixture was heat-denatured at 98°C for 2 minutes, cooled to 55°C and incubated at the temperature for 1 minute in a thermal cycler to anneal the primers to the template.

After annealing, 40 μl of a mixture containing 0.625 mM each of dNTPs, 42.5 mM Tricine-potassium hydroxide buffer (pH 8.5), 5.0 mM magnesium acetate, 0.0125% BSA, 1.25% DMSO, 15 U of E. coli RNase H and 11 U of BcaBEST DNA polymerase was added to the mixture to make the final volume to 50 μl with sterile water. The reaction mixtures were incubated at 55°C for 1 hour in a thermal cycler. The reacted samples were stored by freezing at -20°C until analysis. The PCR as a control was carried out as follows. Briefly, 50 μl of a reaction mixture containing 50 pmol each of the primers, 1 μl of the cDNA (corresponding to 50 ng of the RNA), 5 μl of 10 x Ex Taq buffer (Takara Shuzo), 1.25 U of TaKaRa Ex Taq DNA polymerase (Takara Shuzo) and 0.2 mM each of dNTPs was reacted in a thermal cycler. The program was as follows: 1 cycle of 94°C for 2 minutes; 30 cycles of 94°C for 30 seconds, 55°C for 30 seconds and 72°C for 30 seconds; and 1 cycle of 72°C for 5 minutes. The reacted samples were stored by freezing at -20°C until analysis. After reaction, 5 μl each of the reaction mixtures was subjected to electrophoresis on 3.0% agarose gel. The results are shown in Figure 10.

Figure 10 illustrates the results of electrophoresis which shows the comparison between the RT-ICAN (A) and the RT-PCR (B). Lane 1: molecular weight marker (100 bp ladder); lane 2: negative control group; and lane 3: a group treated with LPS and IFN-γ.

As shown in Figure 10, the amplification products were observed for both of the method of the present invention and the PCR only when the cDNA prepared from cells treated with LPS and IFN-γ was used as a template. Thus, it was confirmed that, since the method of the present invention required a shorter period of time for reaction as compared with the PCR, the method of the present invention was more effective as a DNA amplification method after reverse transcription.

Example 11

E. coli RNase H of which the optimal temperature is 37°C may become inactivated during amplification reaction of the present invention. Then, the effect of addition of E. coli RNase H to the reaction mixture during the amplification reaction was examined. An amplified fragment (1071 bp) obtained by a PCR using primers GMO-PCR-F and GMO-PCR-R represented by SEQ ID NOS:78 and 79 from a genomic DNA extracted from recombinant soybeans into which cauliflower mosaic virus 35S promoter and EPSPS gene had been introduced was used as a template DNA. In addition, primers GMO-S1, S2, A1 and A2 having nucleotide sequences represented by SEQ ID NOS:80 to 83 were used. The reaction was carried out as follows. Briefly, 10 μl of a mixture containing 50 pmol each of the primers, propylenediamine at a final concentration of 0.01%, 1 pg to 10 ng of the DNA as the template and sterile water was prepared. The mixture was heat-denatured at 98°C for 2 minutes and cooled to 55°C for annealing.

After annealing, at final concentrations, 500 μM each of dNTPs, 34 mM Tricine-potassium hydroxide buffer (pH 8.7), 4.0 mM magnesium acetate, 0.01% BSA, 1% DMSO, 30 U of E. coli RNase H and 5.5 U of BcaBEST DNA polymerase were added to the mixture to make the final volume to 50 μl with sterile water. The reaction mixtures were incubated at 55°C for 25 minutes in a thermal cycler. 30 U of E. coli RNase H was further added thereto 25 minutes after the initiation of the reaction. The mixture was incubated at 55°C for 30 minutes. As a control, a reaction was carried out by incubating the mixture at 55°C for 55 minutes. After reaction, 3 μl each of the reaction mixtures was subjected to electrophoresis on 3% agarose. As a result, it was confirmed that the amplification efficiency was improved by the addition of E. coli RNase H during the reaction regardless of the concentration of the template DNA for either of the combinations of the primers, S1/A1, S1/A2, S2/A1 and S2/A2.

Example 12

The combination of a method for amplifying or duplicating a nucleic acid to be used as a template in the present invention and the method of the present invention was examined. The reaction was carried out as follows. Briefly, in vitro transcription was carried out using a plasmid containing the chrysanthemum viroid gene as prepared in Example 5(3) which was duplicated in Escherichia coli as a template and T7 RNA polymerase (Takara Shuzo) to obtain a fragment duplicated from an RNA. A cDNA was synthesized using primers having nucleotide sequences represented by SEQ ID NOS:57 and 58 and cDNA synthesis kit (Takara Shuzo). An amplification reaction was carried out as described in Example 5(3) using the cDNA fragment or the duplicated plasmid as a template. As a result, it was confirmed that both a nucleic acid duplicated in a form of a plasmid and a nucleic acid in a form of a cDNA duplicated from an RNA using an RNA polymerase can be used as templates for the method of the present invention.

Example 13

(1) Synthesis of primer

Oligonucleotide primers N31 and NS2 represented by SEQ ID NOS:86 and 87 were synthesized on the basis of the nucleotide sequence of the mRNA for mouse inducible NO synthase (iNOS).

(2) Amplification of DNA fragment according to ICAN using PCR product as template

10 μl of a mixture containing 50 pmol each of the synthetic oligonucleotide primers, 2 μl of a 0.05% aqueous solution of propylenediamine and 10 fg to 10 pg of the template was heated at 98°C for 2 minutes and then 60°C for 2 minutes using a thermal cycler (GeneAmp PCR System 9600, Applied Biosystems) to anneal the primers to the template. iNOS cDNA (741 bp) amplified using primers NS-PCR1 and NS-PCR2 represented by SEQ ID NOS:132 and 133, which was then purified using Suprec02 (Takara Shuzo), was used as the template. 40 μl of a reaction mixture containing 0.625 mM each of dNTPs, 40 mM HEPES-potassium hydroxide buffer (pH 7.8), 125 mM potassium acetate, 5.0 mM magnesium acetate, 0.0125% BSA, 1.25% DMSO, 0.0156 μg of Pfu RNase H and 0.66 U of BcaBEST DNA polymerase was added to the heated solution. The mixtures were at incubated at 60°C for 1 hour in a thermal cycler. 5 μl each of the reaction mixtures was analyzed by electrophoresis on 3.0% agarose gel. The results are shown in Figure 11. Figure 11 represents the results of the ICAN using Pfu RNase H. Lane 1: molecular weight marker (100 bp); lane 2: 10 fg of the template; lane 3: 100 fg of the template; lane 4: 1 pg of the template; and lane 5: 10 pg of the template.

As shown in Figure 11, the amplification product of interest was observed using 100 fg of the template.

Example 14

(1) Preparation of RNA

RAW264.7 cells (ATCC TIB 71) were suspended in Dulbecco's modified Eagle's medium (Bio Whittaker) containing 10% fetal calf serum (Gibco) at a concentration of 1.5 x $10^5$ cells/ml. 5 ml of the suspension was added to each well of a 6-well microtiter plate and the plate was incubated at 37°C overnight in the presence of 5% $CO_2$. 50 μl

of a 100 μg/ml solution of lipopolysaccharide (LPS, Sigma) in water and 50 μl of a 1000 U/ml solution of interferon-γ (IFN-γ, Genzyme Techne) in water were added to the well. The plate was incubated for additional 4 hours. An RNA was then prepared from the cells using RNeasy Mini Kit (Qiagen, 74104) according to the instructions attached to the kit. As a negative control, a group to which LPS or IFN-γ was not added was provided.

A cDNA was prepared by incubating 60 μl of a mixture containing 3 μg of the thus prepared RNA, 10 mM tris-hydrochloride buffer (pH 8.3), 50 mM KCl, 5 mM $MgCl_2$, 1 mM each of dNTPs, 150 pmol of random 6mers, 60 U of Ribonuclease Inhibitor (Takara Shuzo) and 15 U of Reverse Transcriptase XL (AMV) (Takara Shuzo) at 30°C for 10 minutes, 42°C for 1 hour and then 99°C for 5 minutes for inactivating the enzyme using a thermal cycler (GeneAmp PCR System 9600, Applied Biosystems).

Primers NS5 and NS6 having nucleotide sequences represented by SEQ ID NOS:92 and 93 were synthesized based on the nucleotide sequence of the mRNA for mouse inducible NO synthase (iNOS). Furthermore, primers NS3 and NS4 for PCR represented by SEQ ID NOS:88 and 89 were also synthesized.

50 μl of a mixture containing 50 pmol each of the primers NS5 and NS6, 1 μl of the cDNA solution synthesized as described above (corresponding to 50 ng of the RNA) or a 10-, 100-, 1000- or 10000-fold dilution with water thereof as a template, 0.5 mM each of dNTPs, 32 mM HEPES-potassium hydroxide buffer (pH 7.8), 100 mM potassium acetate, 4.0 mM magnesium acetate, 0.01% BSA, 1% DMSO, 0.0156 μg of Pfu RNase H and 0.66 U of BcaBEST DNA polymerase was incubated at 60°C for 1 hour in a thermal cycler. The reacted samples were stored by freezing at -20°C until analysis.

On the other hand, a PCR was carried out as a control. 50 μl of a reaction system containing 50 pmol each of the primers NS3 and NS4, 1 μl of the cDNA solution (corresponding to 50 ng of the RNA) or a 10-, 100-, 1000-or 10000-fold dilution with water thereof, 5 μl of 10 x Ex Taq buffer (Takara Shuzo), 1.25 U of TaKaRa Ex Taq polymerase (Takara Shuzo) and 0.2 mM each of dNTPs was reacted using a thermal cycler. The program was as follows: 1 cycle of 94°C for 2 minutes; 35 cycles of 94°C for 30 seconds, 55°C for 30 seconds and 72°C for 30 seconds; and 1 cycle of 72°C for 5 minutes. The reacted samples were stored by freezing at -20°C until analysis.

5 μl each of the reaction mixtures (ICAN or PCR) was analyzed by electrophoresis on 3.0% agarose gel. The results are shown in Figure 12. Figure 12 shows the results of the detection of iNOS gene according to the ICAN using Pfu RNase H or the PCR. Lane 1: a lane for 100 bp DNA ladder marker; lane 2: the 10000-fold dilution of the negative control cDNA; lane 3: the 1000-fold dilution of the negative control cDNA; lane 4: the 100-fold dilution of the negative control cDNA; lane 5: the 10-fold dilution of the negative control cDNA; lane 6: the original solution of the negative control cDNA; lane 7: the 10000-fold dilution of the cDNA from the group with the addition of LPS and IFN-γ; lane 8: the 1000-fold dilution of the cDNA from the group with the addition of LPS and IFN-γ; lane 9: the 100-fold dilution of the cDNA from the group with the addition of LPS and IFN-γ; lane 10: the 10-fold dilution of the cDNA from the group with the addition of LPS and IFN-γ; and lane 11: the original solution of the cDNA from the group with the addition of LPS and IFN-γ.

As shown in Figure 12, the amplification products were observed for both of the ICAN and the PCR only when the cDNA prepared from cells treated with LPS and IFN-γ was used as a template. For the ICAN, the increase in amplification product was observed using the 1000-fold dilution of the cDNA. For the PCR, the increase in amplification product was observed using the 100-fold dilution of the cDNA.

Example 15

(1) Oligonucleotide primers 4 and 5 represented by SEQ ID NOS:90 and 91 were synthesized based on the nucleotide sequence of λ DNA. The oligonucleotide primer 4 is a sense primer having 75% of a GC content. The oligonucleotide primer 5 is an antisense primer having 80% of a GC content.

10 μl of a reaction system containing 120 pmol each of the primers 4 and 5, 2 μl of a 0.05% propylenediamine solution and 10 ng of a template was heat-denatured at 98°C for 2 minutes, and then rapidly cooled on ice to anneal the primers to the template. The PCR product (1005 bp) purified using Suprec02 as described in Example 2 was used as the template.

(2) After annealing, 40 μl of a mixture containing 0.625 mM each of dNTPs, 42.5 mM Bicine-potassium hydroxide buffer (pH 8.3), 5.0 mM magnesium acetate, 0.0125% BSA, 1.25% DMSO, 0.5 μl of Thermotoga maritima RNase HII (0.58 μg/ml) and 2.2 U of BcaBEST DNA polymerase was added to the mixture to carry out the ICAN at 60, 65 or 70°C for 1 hour. 3 μl each of the reaction mixtures after the ICAN was confirmed by electrophoresis on 3.0% agarose gel. The results are shown in Figure 13. Figure 13 shows the results of the ICAN using Thermotoga maritima RNase HII. Lane 1: molecular weight marker (100 bp); lane 2: reaction temperature of 60°C; lane 3: reaction temperature of 65°C; and lane 4: reaction temperature of 70°C.

As shown in Figure 13, the amplification products of interest were observed for the respective reaction temperatures.

Example 16

(1) Amplification of a DNA fragment according to the ICAN using a PCR product as a template (alkali-denatured) was examined. 1 µl of a solution containing 10 fg to 10 pg of a template and 1 µl of 0.4 N NaOH were mixed together. The mixture was incubated at 37°C for 5 minutes to denature the template. The PCR-amplified iNOS cDNA (741 bp) purified using Suprec02 (Takara Shuzo) as described in Example 13 was used as a template. Each of the denatured templates was neutralized using 1 µl of 0.4 N HCl. 47 µl of a reaction mixture containing 50 pmol each of the primers NS1 and NS2, 0.5 mM each of dNTPs, 32 mM HEPES-potassium hydroxide buffer (pH 7.8), 100 mM potassium acetate, 4.0 mM magnesium acetate, 0.01% BSA, 1.0% DMSO, 0.0156 µg of Pfu RNase H and 0.66 U of BcaBEST DNA polymerase was then added thereto. The mixture was incubated at 60°C for 1 hour in a thermal cycler. 5 µl each of the reaction mixtures was analyzed by electrophoresis on 3.0% agarose gel. The results are shown in Figure 14. Figure 14 shows the results of the ICAN using an alkali-denatured template. Lane 1: molecular weight marker (100 bp); lane 2: 10 fg of the template; lane 3: 100 fg of the template; lane 4: 1 pg of the template; and lane 5: 10 pg of the template.

As shown in Figure 14, the amplification product was clearly increased using 1 pg of the template.

Example 17

(1) Amplification of a DNA fragment according to the ICAN without denaturing a template was examined. Primers NS5 and NS6 represented by SEQ ID NOS:92 and 93 were used as primers. The template as prepared in Example 13 was used as a template DNA.

50 µl of a reaction mixture containing 10 fg to 100 pg of the template or water for a negative control, 50 pmol each of the primers NS5 and NS6, 0.5 mM each of dNTPs, 32 mM HEPES-potassium hydroxide buffer (pH 7.8), 100 mM potassium acetate, 4.0 mM magnesium acetate, 0.01% BSA, 1.0% DMSO, 0.0156 µg of Pfu RNase H and 1 U of BcaBEST DNA polymerase (Takara Shuzo) was incubated at 60°C for 1 hour in a thermal cycler. After reaction, 5 µl each of the reaction mixture was analyzed by electrophoresis on 3.0% agarose gel. Results of the electrophoresis are shown in Figure 15. Figure 15 illustrates the results of electrophoresis for the amplification method of the present invention without denaturing a template DNA. Lane 1: 100 bp DNA ladder marker; lane 2: negative control (water); lane 3: 10 fg of the template; lane 4: 100 fg of the template; lane 5: 1 pg of the template; lane 6: 10 pg of the template; and lane 7: 100 pg of the template.

As shown in Figure 15, the amplification product of interest was observed using 1 pg of the template.

Example 18

(1) Primers pDON-AI-1 and pDON-AI-2 represented by SEQ ID NOS:94 and 95 were synthesized based the nucleotide sequence of the packaging region in a vector plasmid pDON-AI DNA (Takara Shuzo).

(2) Amplification of DNA fragment according to ICAN without denaturing template

50 µl of a reaction mixture containing 1 µl of a solution containing 10 fg to 1 ng of pDON-AI DNA or water for a negative control, 50 pmol each of the primers, 0.5 mM each of dNTPs, 32 mM HEPES-potassium hydroxide buffer (pH 7.8), 100 mM potassium acetate, 4.0 mM magnesium acetate, 0.01% BSA, 1.0% DMSO, 0.0156 µg of Pfu RNase H as prepared in Referential Example 4 and 1 U of BcaBEST DNA polymerase was incubated at 60°C for 1 hour in a thermal cycler. 5 µl each of the reaction mixtures was analyzed by electrophoresis on 3.0% agarose gel. The results are shown in Figure 16. Figure 16 illustrates the results of electrophoresis for the method of the present invention using a circular double-stranded DNA as a template without denaturation. Lane 1: 100 bp DNA ladder marker; lane 2: negative control (water); lane 3: 10 fg of the template; lane 4: 100 fg of the template; lane 5: 1 pg of the template; lane 6: 10 pg of the template; lane 7: 100 pg of the template; and lane 8: 1 ng of the template.

As shown in Figure 16, it was confirmed that the amplification product of interest was obtained using 10 fg of the template.

Example 19

Detection of human papilloma virus 16 gene utilizing the method of the present invention was examined. A DNA from cells infected with human papilloma virus 16, CaSki cells (Dainippon Pharmaceutical; containing 500 copies of human papilloma virus 16 in a cell), was used as a template. Primers HPV16 S3 and HPV16 A2 having nucleotide sequences represented by SEQ ID NOS:96 and 97 were used as primers for detecting HPV16. The expected size of the amplification product obtained using the primer pair was about 120 bp. The reaction was carried out as follows.

10 µl of a mixture containing 1 pg, 3 pg, 30 pg, 100 pg, 300 pg, 1 ng, 3 ng or 10 ng of the template DNA, 50 pmol each of the primers HPV16 S3 and HPV16 A2 and propylenediamine at a final concentration of 0.01% was prepared. The mixtures were incubated at 98°C for 2 minutes and at 55°C for 1 minute in Thermal Cycler Personal, and then placed on ice. At final concentrations, 20 mM HEPES-potassium hydroxide buffer (pH 7.8), 100 mM potassium acetate, 1% DMSO, 0.01% BSA, 4 mM magnesium acetate, 500 µM each of dNTPs, 30 U of E. coli RNase H and 5.5 U of BcaBEST DNA polymerase were added to the mixture to make the final volume to 50 µl. The reaction mixtures were placed in a thermal cycler which had been set at 55°C and reacted for 60 minutes. As a control, a PCR was carried out using Human Papillomavirus Primers HPVp16 (forward, reverse) (Takara Shuzo) according to the manual in Thermal Cycler Personal. The expected size of the amplification product was 140 bp.

After reaction, 3 µl each of the reaction mixtures was subjected to electrophoresis on 4% NuSieve 3:1 agarose. The results are shown in Figure 17A. Figure 17A shows the results of the detection of HPV16 gene utilizing the ICAN and the PCR. Lane M1: molecular weight marker (100 bp ladder); lane M2: molecular weight marker (50-2000 bp); lane 1: no template; lane 2: 1 pg of the template; lane 3: 3 pg of the template; lane 4: 30 pg of the template; lane 5: 100 pg of the template; lane 6: 300 pg of the template; lane 7: 1 ng of the template; lane 8: 3 ng of the template; and lane 9: 10 ng of the template.

As shown in Figure 17A, it was confirmed that the amplification products of interest were obtained using 3 pg of the template DNA for the ICAN and 1 pg of the template DNA for the PCR, respectively.

Furthermore, dot blot hybridization for the reaction products was carried out using an oligonucleotide HPV16 probe having a nucleotide sequence represented by SEQ ID NO:98. Hybridization was carried out as described in Example 9. The results are shown in Figure 17B. Figure 17B shows the results of dot blot hybridization detection of HPV16 gene according to the PCR and the ICAN. Lane 1: no template; lane 2: 1 pg of the template; lane 3: 3 pg of the template; lane 4: 30 pg of the template; lane 5: 100 pg of the template; lane 6: 300 pg of the template; lane 7: 1 ng of the template; lane 8: 3 ng of the template; and lane 9: 10 ng of the template.

As shown in Figure 17B, the detection sensitivities of the ICAN and the PCR were almost equivalent. Thus, it was confirmed that these methods were effective for detecting a virus or the like.

Example 20

Detection of human papilloma virus 16 gene from a clinical specimen DNA sample was examined. DNAs prepared according to a conventional method from 6 clinical specimens obtained with informed consent were used as templates. The types of the infecting HPVs in the samples prepared from these clinical specimens had been proved by the PCR. The primers HPV16 S3 and HPV16 A2 as described in Example 19 were used as primers for detection. The concentrations of the DNA samples from the clinical specimens to be used as templates were adjusted to 100 ng/µl with TE buffer. The composition of the reaction mixture and the reaction conditions as described in Example 19 were used except for the amount of the template. In addition, similar reactions were carried out using a reaction mixture without the addition of the template DNA as a negative control and a reaction mixture containing 500 pg of the DNA from CaSki cells infected with HPV 16 as a positive control. After reaction, 3 µl each of the reaction mixtures was subjected to electrophoresis on 4% NuSieve 3:1 agarose. The results are shown in Figure 18A. Figure 18A shows the results of the detection of HPV16 gene from clinical specimens. Lane M: molecular weight marker; lanes 1 to 6: clinical specimens; lane 7: negative control; and lane 8: positive control.

As shown in Figure 18A, the amplification products of about 120 bp were observed according to the ICAN for the samples which had been proved to be infected with HPV16 by the conventional PCR. No amplification was observed for samples infected with other types of HPVs or non-infected samples.

Furthermore, dot blot hybridization for the amplification products was carried out as described in Example 9. The results are shown in Figure 18B and Table 8. Figure 18B shows the results of dot blot hybridization detection of HPV16 gene from the clinical specimens. Lanes 1 to 6: clinical specimens; lane 7: negative control; and lane 8: positive control.

As shown in Figure 18, results consistent with those obtained by the electrophoresis were obtained, confirming that similar results with those of the PCR could be obtained by using electrophoresis as well as dot blot hybridization. Thus, it was confirmed that HPV16 could be detected from practical clinical specimens according to the method of the present invention and that the method was effective for detecting a virus or the like.

Table 8

| Sample | Typing by PCR | ICAN amplification using HPV16 detection primer |
|---|---|---|
| No.3 | Non-infected | |
| No.4 | Non-infected | - |

Table 8 (continued)

| Sample | Typing by PCR | ICAN amplification using HPV16 detection primer |
|---|---|---|
| No.6 | Type 18 | - |
| No.7 | Type 16 | + |
| No.8 | Type 67 | - |
| No.9 | Type 16 | + |
| No template | * | - |
| Positive control | * | + |
| -: no amplification; +: amplification observed. | | |

Example 21

Detection of HCV from clinical specimens was examined. Specimen samples were prepared from 300 µl each of 5 serum specimens from patients with HCV obtained with informed consent using TRIzol reagent (Life Technologies) according to the instructions attached to the reagent and finally dissolved in 6 µl of injectable water (Otsuka Pharmaceutical) to obtain RNA samples. An RNA similarly extracted from 300 µl of a serum from a healthy individual was used as a negative control. First, 4 µl of a reaction mixture for reverse transcription containing 1 x RNA PCR Buffer, 5 mM $MgCl_2$, 1 mM dNTPs, 1 U of AMV Reverse Transcriptase XL, 10 pmol each of primers HCV-F and HCV-R represented by SEQ ID NOS:99 and 100, and 2 µl of one the RNA samples was prepared using RNA PCR kit (AMW) ver 2.1 (Takara Shuzo). The mixtures were warmed at 30°C for 10 minutes and then reacted at 50°C for 30 minutes. After reverse transcription, the ICAN was carried out. Primers HCV-F2 and HCV-R1 having nucleotide sequences represented by SEQ ID NOS:101 and 102 were used for the ICAN. The reaction was carried as follows.

10 µl of a mixture containing 50 pmol each of the primers, 3 µl one of the reverse transcription reaction mixtures and propylenediamine at a final concentration of 0.01% was prepared. 3 µl of sterile water was used for a blank. The mixtures were heated at 98°C for 2 minutes, rapidly cooled to 60°C and incubated at the temperature for 1 minute in Thermal Cycler Personal, and stored on ice.

After annealing, at final concentrations, 20 mM HEPES-potassium hydroxide buffer (pH 7.8), 100 mM potassium acetate, 1% DMSO, 0.01% BSA, 4 mM magnesium acetate, 500 µM each of dNTPs, 30 U of E. coli RNase H and 5.5 U of BcaBEST DNA polymerase were added to the mixture to make the final volume to 50 µl with sterile water. The reaction mixtures were placed in Thermal Cycler MP which had been set at 60°C and reacted for 60 minutes. After reaction, 3 µl each of the reaction mixtures was subjected to electrophoresis on 3% NuSieve 3:1 agarose. The results are shown in Figure 19A. Figure 19A shows the results of HCV detection from clinical specimens. Lane B: sterile water as a template; lane 1: a sample from a healthy individual; lanes 2 to 6: samples from patients with HCV; and lane M: molecular weight marker (50-2000 bp).

As shown in Figure 19A, the amplification products of about 107 bp expected from the nucleotide sequence of HCV genome were observed only for the RNA samples from patients with HCV, whereas such an amplification product was not observed for the serum from the healthy individual and the blank. Furthermore, dot blot hybridization for the ICAN-amplified products was carried out as described in Example 9 using a probe for HCV represented by SEQ ID NO:103 biotinylated at 5'-terminus. The results are shown in Figure 19B. The lanes for the samples in Figure 19B are as those indicated for the electrophoresis.

As shown in Figure 19, it was confirmed that the results from the electrophoresis were consistent with those from the dot blot hybridization. These results confirms that the method of the present invention can be used to detect HCV from practical clinical specimens and is effective for detecting a virus or the like.

Example 22

A method for detecting adenovirus was examined.

Primers for amplifying E1A (tumor gene), E1A-1 (sense), E1A-2 (antisense) and E1A-3 (antisense), represented by SEQ ID NOS:104 to 106 were constructed based on the nucleotide sequence of adenovirus (GenBank accession no. J01917). Adenovirus (ATCC accession no. VR-5) was used A template was prepared as follows. 100 µl of a solution containing adenovirus at a concentration of 8.73 x $10^{10}$ PFU/ml was incubated in the presence of SDS at a final concentration of 0.1% and proteinase K at a final concentration of 0.2 mg/ml at 37°C for 1 hour. The DNA was purified by adsorption to silica gel. Adenoviral DNAs corresponding to $10^3$, $10^4$, $10^5$ or $10^6$ PFU prepared by diluting the purified DNA with sterile water were used. The reaction was carried out as follows. Briefly, 10 µl of a reaction system containing 60 pmol each of the primers E1A-1 and E1A-2 (chain length to be amplified: 112 bp) or E1A-1 and E1A-3 (chain length to be amplified: 91 bp), 2 µl of 0.05% propylenediamine and the template was heat-denature at 98°C for 2 minutes and then rapidly cooled on ice to anneal the primers to the template.

After annealing, 40 μl of a mixture containing 0.625 mM each of dNTPs, 42.5 mM Tricine-potassium hydroxide buffer (pH 8.5), 5.0 mM magnesium acetate, 0.0125% BSA, 1.25% DMSO, 30 U of E. coli RNase H and 5.5 U of BcaBEST DNA polymerase was added to the mixture to make the final volume to 50 μl. The reaction mixtures were incubated at 60°C for 1 hour. As a control, a detection by a PCR was carried out using the same template as the above and primers constructed for PCR-amplifying E1A (tumor gene), E1A-1P (sense), E1A-2P (antisense) and E1A-3P (antisense) having nucleotide sequences represented by SEQ ID NOS:107, 108 and 142. The PCR was carried out as follows. Briefly, 50 μl of a PCR solution containing 60 pmol each of the primers E1A-1P and E1A-2P (chain length to be amplified: 112 bp) or E1A-1P and E1A-3P (chain length to be amplified: 91 bp), 5 μl of 10 x Ex Taq buffer (Takara Shuzo), 1.25 U of TaKaRa Ex Taq DNA polymerase (Takara Shuzo) and 0.2 mM each of dNTPs was prepared. The conditions for the PCR were as follows: 30 cycles of 94°C for 30 seconds, 55°C for 30 seconds and 72°C for 30 seconds.

After reaction, 3 μl each of the reaction mixtures of the ICAN and the PCR was subjected to electrophoresis on 3.0% agarose gel. The results are shown in Figure 20 and Table 9. Figure 20 shows the results of the detection of viral E1A gene from adenoviral particles. Lanes 1 to 10 show the results obtained using the combination of the primers E1A-1 and E1A-2. Lanes 11 to 20 show the results obtained using the combination of the primers E1A-1 and E1A-3. Lane 1: molecular weight marker (100 bp ladder); lane 2: ICAN using DNA corresponding to $10^6$ PFU; lane 3: ICAN using DNA corresponding to $10^5$ PFU; lane 4: ICAN using DNA corresponding to $10^4$ PFU; lane 5: ICAN using DNA corresponding to $10^3$ PFU; lane 6: molecular weight marker (100 bp ladder); lane 7: PCR using DNA corresponding to $10^6$ PFU; lane 8: PCR using DNA corresponding to $10^5$ PFU; lane 9: PCR using DNA corresponding to $10^4$ PFU; and lane 10: PCR using DNA corresponding to $10^3$ PFU. In addition, lane 11: molecular weight marker (100 bp ladder); lane 12: ICAN using DNA corresponding to $10^6$ PFU; lane 13: ICAN using DNA corresponding to $10^5$ PFU; lane 14: ICAN using DNA corresponding to $10^4$ PFU; lane 15: ICAN using DNA corresponding to $10^3$ PFU; lane 16: molecular weight marker (100 bp ladder); lane 17: PCR using DNA corresponding to $10^6$ PFU; lane 18: PCR using DNA corresponding to $10^5$ PFU; lane 19: PCR using DNA corresponding to $10^4$ PFU; and lane 20: PCR using DNA corresponding to $10^3$ PFU.

Table 9

| Amplification size (bp) | Detection limit | |
|---|---|---|
| | ICAN | PCR |
| 112 | $10^4$ | $10^4$ |
| 91 | $10^4$ | $10^4$ |

As shown in Figure 20 and Table 9, it was confirmed that the detection sensitivity for the detection of adenovirus E1A gene by the ICAN was equivalent to that by the PCR.

Example 23

Detection of an integrated viral gene from cells infected with a retrovirus vector was examined. Cells infected with a retrovirus and a genomic DNA were prepared as follows. Briefly, a vector plasmid pDON-AI (Takara Shuzo) was introduced into packaging cells GPE+86 according to the calcium phosphate method. An ecotropic vector was prepared from the culture supernatant of the introduced cells. Cells infected with a virus vector were prepared by infecting NIH/3T3 cells with the ecotropic vector and culturing the infected cells for 14 days in a medium containing G418. 27 μg of a genomic DNA was obtained according to a conventional method from 4 x $10^4$ of the prepared cells infected with the retrovirus. The primers pDON-AI-1 and pDON-AI-2 as described in Example 18(1) were used as primers. The reaction was carried out as follows. Briefly, 10 μl of a reaction system containing 60 pmol each of the primers, 2 μl of a 0.25% aqueous solution of propylenediamine and 0.1 ng to 1000 ng of the genomic DNA as a template was heated at 98°C for 2 minutes and then at 60°C in a thermal cycler (Takara Shuzo) to anneal the primers to the template.

After annealing, 40 μl of a reaction mixture containing 0.625 mM each of dNTPs, 40 mM HEPES-potassium hydroxide buffer (pH 7.8), 125 mM potassium acetate, 5 mM magnesium acetate, 0.0125% BSA, 1.25% DMSO, 30 U of E. coli RNase H and 5.5 U of BcaBEST DNA polymerase was added to the mixture to make the final volume to 50 μl. The reaction mixtures were incubated at 60°C for 1 hour in a thermal cycler. After reaction, 5 μl each of the reaction mixtures was subjected to electrophoresis on 3.0% agarose gel. In addition, a PCR was carried out using primers pDON-AI-3 and pDON-AI-4 represented by SEQ ID NOS:111 and 112 in order to compare the sensitivities of detecting a DNA by the ICAN and the PCR. The PCR was carried out as follows. 50 μl of a reaction mixture containing 0.1 ng to 100 ng of the template, 60 pmol each of the primers, 5 μl of 10 x Ex Taq buffer, 1.25 U of TaKaRa Ex Taq polymerase and 0.2 mM each of dNTPs was prepared. The mixtures were subjected to reactions using Thermal Cycler Personal as follows: 35 cycles of 94°C for 30 seconds, 55°C for 30 seconds and 72°C for 30 seconds. After reaction, 5 µl each of the reaction mixtures was subjected to electrophoresis on 3.0% agarose gel. The results are shown in Figure 21. Figure 21 shows the results of the detection of an integrated viral gene from cells infected with a retrovirus vector according to the ICAN and the PCR. Lane 1: molecular weight marker (100 bp ladder); lane 2: 1000 ng of the template; lane 3: 100 ng of the template; lane 4: 10 ng of the template; lane 5: 1 ng of the template; and lane 6: 0.1 ng of the template.

As shown in Figure 21, the amplification products of interest were observed for the ICAN using 1 ng of the template DNA and for the PCR of 35 cycles using 1 ng of the template.

Example 24

A method for detecting a target nucleic acid in which the amplification method of the present invention and a hybridization method were combined was examined for the detection of Escherichia coli 0-157 vero toxin I gene. Vero toxin I gene from enterohemorrhagic Escherichia coli 0-157 was selected as a target. The template DNA was prepared as described in Example 8(1). A region of about 80 bp having a GC content of about 40% was selected as a region to be amplified. Primers VT1-IF4 and VT1-IR1 having nucleotide sequences represented by SEQ ID NOS:113 and 114 were used as primers. The reaction was carried out as follows. Briefly, 5 µl of a mixture containing 60 pmol each of the primers VT1-IF4 and VT1-IR1, propylenediamine at a final concentration of 0.01%, one of hot water-extracts corresponding to 0 to $10^5$ cells and sterile water was prepared. The mixtures were heat-denatured at 98°C for 2 minutes, rapidly cooled to 55°C and incubated at the temperature for 1 minute in Thermal Cycler Personal, and then placed on ice for annealing.

After annealing, at final concentrations, 20 mM HEPES-potassium hydroxide buffer (pH 7.8), 100 mM potassium acetate, 1% DMSO, 0.01% BSA, 4 mM magnesium acetate, 500 µM each of dNTPs, 15 U of E. coli RNase H and 2.75 U of BcaBEST DNA polymerase. were added to the mixture to make the final volume to 25 µl with sterile water. The reaction mixtures were placed in Thermal Cycler Personal which had been set at 55°C and incubated at the temperature for 60 minutes. As a control, a PCR for the hot water-extract was carried out using 0-157 Typing Set (Takara Shuzo) according to the manual in Thermal Cycler Personal. The PCR was carried out as follows: 35 cycles of 94°C for 1 minute, 55°C for 1 minute and 72°C for 1 minute. The total time required for the reaction was about 145 minutes. The expected size of the amplification product was 349 bp. After reaction, 3 µl each of the reaction mixtures was subjected to electrophoresis on 3% NuSieve 3:1 agarose. The results for the ICAN are shown in Figure 22. Figure 22 shows the results of the detection of O-157 vero toxin I gene. Lane M: molecular weight marker (50-2000 bp); lane N: sterile water as template; lane 1: the template corresponding to 1 cell; lane 2: the template corresponding to 10 cells; lane 3: the template corresponding to $10^2$ cells; and lane 4: the template corresponding to $10^3$ cells. Furthermore, the results of the detection by the ICAN and the PCR are shown in Table 10.

Table 10

| | Number of Escherichia coli 0-157 cells | | |
|---|---|---|---|
| | 0 | 1 | 10 |
| ICAN | - | + | +++ |
| PCR | - | + | ++ |
| -: no amplification; + to +++ indicate the degree of amplification in three grades. | | | |

As shown in Table 10, the amplification products of interest were obtained for the reaction systems in which the hot water-extract corresponding to 1 cell was used for both of the ICAN and the PCR. Furthermore, dot blot hybridization for the amplification products was carried out using an oligonucleotide probe VT1 having a nucleotide sequence represented by SEQ ID NO labeled with biotin at the 5'-terminus. The hybridization was carried out as described in Example 19. The results were consistent with those obtained by the electrophoresis. Thus, it was confirmed that the detection sensitivities of the ICAN and the PCR were equivalent. In addition, the total time required for the amplification reaction using the ICAN of the present invention was 1/2 or shorter as compared with that required for the PCR. Thus, the ICAN of the present invention was confirmed to be effective as a method for detecting a pathogenic bacterium and the like.

Example 25

A method for detecting a gene for botulinum toxin type A was examined. A DNA prepared from Clostridium botulinum, a strain from a food poisoning case, type A-190 was used as a template. This strain is preserved in Department of Hygiene, Kagawa Nutrition University. Primers BotA S2 and BotA A2 having nucleotide sequences represented by SEQ IOD NOS:116 and 117 were synthesized as primers for detection. The size of the amplification product obtained using the primer pair was about 150 bp. Solutions containing 100 fg, 1 pg, 10 pg or 100 pg of the DNA from type A toxin-producing Clostridium botulinum in 1 μl of sterile water to be used as templates were prepared. The reaction was carried out as follows.

10 μl of a mixture containing 50 pmol each of the primers, propylenediamine at a final concentration of 0.01% and 1 μl of one of the DNA solutions as a template was prepared. The mixtures were subjected to the ICAN using the composition of the reaction mixture and the reaction conditions as described in Example 19. As a control, a PCR was carried out in using a primer set for detecting botulinum toxin type A gene, BAS-1 and BAS-2 (Takara Shuzo), according to the manual in Thermal Cycler Personal. The expected size of the amplification product was 284 bp.

After reaction, 3 μl each of the reaction mixtures was subjected to electrophoresis on 4% NuSieve 3:1 agarose. The results are shown in Figure 23A. Figure 23A shows the results of the detection of the gene for botulinum toxin type A according to the ICAN and the PCR. Lane M1: molecular weight marker (100 bp ladder); lane M2: molecular weight marker (50-2000 bp marker); lane 1: no template; lane 2: 100 fg of the template; lane 3: 10 pg of the template; and lane 4: 100 pg of the template.

As shown in Figure 23A, the amplification product of interest was observed for the reaction in which 100 fg of the template DNA was used according to the ICAN. On the other hand, the amplification product of interest was not observed for the reaction in which 100 fg of the template DNA was used according to the PCR. Furthermore, dot blot hybridization for the reaction products was carried out using BotA probe having a nucleotide sequence represented by SEQ ID NO:118. Dot blot hybridization was carried out as described in Example 9. The results are shown in Figure 23B. As shown in Figure 23B, the signals were observed using 100 fg of the template for the ICAN and 10 pg of the template for the PCR, respectively. These results were consistent with those of the electrophoresis.

Example 26

Detection of chrysanthemum viroid was examined. 10-fold serial dilutions of low molecular weight RNA obtained according to the method for extracting low molecular weight RNAs from chrysanthemum infected with chrysanthemum stunt viroid (CSVd) as described in Example 1 of JP-A 9-140383 were prepared. A reverse transcription reaction was carried out using RNA PCR kit (AMW) ver 2.1 (Takara Shuzo). Specifically, 20 μl of a reaction mixture for reverse transcription containing 1 x RNA PCR Buffer, 5 mM $MgCl_2$, 1 mM each of dNTPs, 20 U of RNase Inhibitor, 5 U of AMV Reverse Transcriptase XL, 50 pmol of Random 9mers, 1 μl of one of the serial dilutions of the RNA was prepared. The mixtures were warmed at 30°C for 10 minutes, reacted at 55°C for 30 minutes and then heated at 99°C for 5 minutes for inactivating the reverse transcriptase. After cooling, the ICAN was carried out. 1 μl of the reverse transcription reaction mixture was used as a template for 50 μl of a reaction mixture for the ICAN. Primers CSVD-F4 and CSVD-R3 having nucleotide sequences represented by SEQ ID NOS:119 and 120 were used as primers in this Example. The reaction was carried out as described in Example 19 except that reaction temperature was 60°C and that Thermal Cycler MP was used. After reaction, 3 μl each of the reaction mixtures was subjected to electrophoresis on 3% NuSieve 3:1 agarose.

On the other hand, PCR amplification was carried out in a reaction system of 50 μl using 1 μl of the same reverse transcription reaction mixture as a template. Primers F94 and R264 represented by SEQ ID NOS:109 and 110 were used as primers. The reaction was carried out as follows. Briefly, a reaction mixture was prepared using TaKaRa PCR Amplification kit according to the protocol. 10 pmol each of the primers and 1 μl of one of the reverse transcription reaction mixture were added thereto to make the total volume to 50 μl. An amplification reaction was carried out using Thermal Cycler MP. The reaction was carried out as follows: 30 cycles of 94°C for 30 seconds, 55°C for 30 seconds and 72°C for 30 seconds. After reaction, 5 μl each of the reaction mixtures was subjected to electrophoresis on 3% NuSieve 3:1 agarose. The results are shown in Table 11.

Table 11

| Dilution rate of RNA as template | x $10^2$ | x $10^3$ |
|---|---|---|
| RT-ICAN | ++ | + |
| RT-PCR | + | - |
| -: not amplified; +: amplified; ++: amplified well. | | |

As shown in Table 11, the amplification product was observed for the reaction in which the $10^3$-fold-diluted RNA sample was used as a template according to the ICAN. On the other hand, the amplification product was observed for the reaction in which the $10^2$-fold-diluted RNA sample was used as a template according to the PCR.

Furthermore, the ICAN-amplified products and the PCR-amplified products were confirmed to be the products of interest by dot blot hybridization. Dot blot hybridization was carried out using CSVd probe having a nucleotide sequence represented by SEQ ID NO:121 labeled with biotin at the 5'-terminus. Hybridization was carried out as described in Example 9. The results were consistent with those of the electrophoresis. The signals were observed using the $10^3$-fold-diluted RNA sample for the ICAN and the $10^2$-fold-diluted RNA sample for the PCR, respectively, demonstrating that the ICAN was more sensitive than the PCR.

Example 27

Detection of a viroid gene from chrysanthemum infected with chrysanthemum dwarfing viroid (CSVd) using Pfu RNase H was examined. 60 μl of a mixture containing 3 μl of one of the 10-fold serial dilutions of the RNA as prepared in Example 26, 10 mM tris-hydrochloride buffer (pH 8.3), 50 mM potassium chloride, 5 mM magnesium chloride, 1 mM each of dNTPs, 150 pmol of Random 6mers, 60 U of Ribonuclease Inhibitor (Takara Shuzo) and 15 U of Reverse Transcriptase XL (AMV) (Takara Shuzo) was incubated at 30°C for 10 minutes and at 42°C for 1 hour and then heated at 99°C for 5 minutes for inactivating the enzyme using a thermal cycler (GeneAmp PCR System 9600, Applied Biosystems) to prepare a cDNA. Primers Vd1, Vd2, Vd3 and Vd4 represented by SEQ ID NOS:122 to 125 were synthesized based on the nucleotide sequence of the mRNA for the viroid.

50 μl of a mixture containing 50 pmol each of the primers Vd1 and Vd2, 1 μl of the cDNA solution synthesized as described above, a 10-, 100-, 1000- or 10000-fold dilution thereof with water or water for a negative control as a template, 0.5 mM each of dNTPs, 32 mM HEPES-potassium hydroxide buffer (pH 7.8), 100 mM potassium acetate, 4.0 mM magnesium acetate, 0.01% BSA, 1% DMSO, 0.0156 μg of Pfu RNase H and 1 U of BcaBEST DNA polymerase was incubated at 57°C for 1 hour in a thermal cycler. The reacted samples were stored by freezing at -20°C until analysis.

A PCR was carried out as a control. Briefly, 50 μl of a reaction system containing 50 pmol each of the primers Vd3 and Vd4, 1 μl of the cDNA solution, a 10-, 100-, 1000- or 10000-fold dilution thereof with water or water for a negative control, 5 μl of 10 x Ex Taq buffer, 1.25 U of TaKaRa Ex Taq polymerase and 0.2 mM each of dNTPs was subjected to a reaction in a thermal cycler. The program was as follows: 1 cycle of 94°C for 2 minute; 35 cycles of 94 C for 30 seconds, 55°C for 30 seconds and 72°C for 30 seconds; and 1 cycle of 72°C for 5 minutes. The reacted samples were stored by freezing at -20°C until analysis.

5 μl each of the reaction mixtures of the ICAN and the PCR was subjected to electrophoresis on 3.0% agarose gel. The results are shown in Figure 24. Figure 24 shows the results of the detection of a viroid according to the ICAN using Pfu RNase H and the PCR. Lane 1: 100 bp DNA ladder marker; lane 2: negative control; lane 3: 10000-fold dilution of the cDNA; lane 4: 1000-fold dilution of the cDNA; lane 5: 100-fold dilution of the cDNA; lane 6: 10-fold dilution of the cDNA; and lane 7: the original solution of the cDNA.

As shown in Figure 24, the amplification products of interest were observed using the 100-fold dilution of the cDNA for both of the ICAN and the PCR.

Example 28

Detection of K-ras gene was examined.

(1) Detection from genomic DNA

Primers c-Ki-ras-1 and c-Ki-ras-2 represented by SEQ ID NOS:126 and 127 were constructed based on the nucleotide sequence of human c-Ki-ras.

10 μl of a mixture containing 60 pmol each of the primers, 2 μl of a 0.25% aqueous solution of propylenediamine and 1 ng to 100 ng of human genomic DNA (Clontech) as a template was prepared. The mixtures were heated at 98°C for 2 minutes and then 53°C in Thermal Cycler Personal to anneal the primers to the template.

After annealing, 40 μl of a reaction mixture containing 0.625 mM each of dNTPs, 40 mM HEPES-potassium hydroxide buffer (pH 7.8), 125 mM potassium acetate, 5 mM magnesium acetate, 0.0125% BSA, 1.25% DMSO, 30 U of E. coli RNase H and 5.5 U of BcaBEST DNA polymerase was added to the mixture to make the final volume to 50 μl. The reaction mixtures were incubated at 53°C for 1 hour. After reaction, 5 μl each of the reaction mixtures was subjected to electrophoresis on 3.0% agarose gel.

On the other hand, a PCR was carried out as a control. Primers c-Ki-ras-3 and c-Ki-ras-4 represented by SEQ ID NOS:128 and 129 were used as primers. 50 μl of a a solution containing 60 pmol each of the primers, 0.1 ng to 100 ng of the template, 5 μl of 10 x Ex Taq buffer, 1.25 U of TaKaRa Ex Taq polymerase and 0.2 mM each of dNTPs was prepared. The mixtures were subjected to reactions using Thermal Cycler Personal as follows: 30 or 35 cycles of 94°C for 30 seconds, 55°C for 30 seconds and 72°C for 30 seconds. After reaction, 5 μl each of the reaction mixtures was subjected to electrophoresis on 3.0% agarose gel. The results are shown in Figure 25. Figure 25 shows the results of the detection of c-Ki-ras gene from human genomic DNA according to the ICAN and the PCR. For the ICAN, lane 1: molecular weight marker; lane 2: 100 ng of the template; lane 3: 10 ng of the template; lane 4: 1 ng of the template; and lane 5: no template. For the PCR, lane 1: 100 ng of the template; lane 2: 10 ng of the template; lane 3: 1 ng of the template; and lane 4: no template.

As shown in Figure 25, the amplified products of interest were observed using 1 ng of the template for the ICAN and 10 ng of the template for the PCR of 30 cycles. The comparative results for the amounts of the amplified products obtained by the ICAN and the PCR using 1 ng to 100 ng of the template are shown in Figure 30. In the figure, the shaded bar represents the results for the PCR of 30 cycles, the dotted bar represents the results for the PCR of 35 cycles, and the closed bar represents the results for the ICAN, respectively. As shown in Figure 30, it was confirmed that the amount of the amplified product obtained by the ICAN was greater than that by the PCR.

(2) Detection from blood sample

Genomic DNAs were prepared using Dr. GenTLE™ (for Whole Blood) (Takara Shuzo) from 100 μl each of blood samples collected from a healthy individual using sodium citrate or heparin as an anticoagulating agent. Detection of c-Ki-ras gene by the ICAN was carried out as described in (1) above using the prepared DNA corresponding to 0.04 to 5 μl of the blood. Furthermore, detection using the DNA from 0.04 to 5 μl of the blood sample was carried out as described in (1) above in order to compare the sensitivities for detecting a DNA by the ICAN and the PCR. The results are shown in Figure 26. Figure 26 shows the results of the detection of c-Ki-ras gene from blood samples according to the ICAN and the PCR. For the ICAN, lane 1: molecular weight marker; lane 2: 5 μl of citrated blood; lane 3: 1 μl of citrated blood; lane 4: 0.2 μl of citrated blood; lane 5: 0.04 μl of citrated blood; lane 6: 5 μl of heparinized blood; lane 7: 1 μl of heparinized blood; lane 8: 0.2 μl of heparinized blood; and lane 9: 0.04 μl of heparinized blood. For the PCR, lane 1: molecular weight marker; lane 2: 5 μl of citrated blood, 30 cycles; lane 3: 1 μl of citrated blood, 30 cycles; lane 4: 0.2 μl of citrated blood, 30 cycles; lane 5: 0.04 μl of citrated blood, 30 cycles; lane 6: 5 μl of citrated blood, 35 cycles; lane 7: 1 μl of citrated blood, 35 cycles; lane 8: 0.2 μl of citrated blood, 35 cycles; lane 9: 0.04 μl of citrated blood, 35 cycles; lane 10: 5 μl of heparinized blood, 30 cycles; lane 11: 1 μl of heparinized blood, 30 cycles; lane 12: 0.2 μl of heparinized blood, 30 cycles; lane 13: 0.04 μl of heparinized blood, 30 cycles; lane 14: 5 μl of heparinized blood, 35 cycles; lane 15: 1 μl of heparinized blood, 35 cycles; lane 16: 0.2 μl of heparinized blood, 35 cycles; and lane 17: 0.04 μl of heparinized blood, 35 cycles.

As shown in Figure 26, the amplification products of interest were observed using the genomic DNA corresponding to 0.2 μl of either of the blood samples for the ICAN, and the genomic DNA corresponding to 0.2 μl of either of the blood samples (citrated or heparinized) for the PCR of 30 cycles, respectively.

Example 29

Detection of Escherichia coli O-157 vero toxin 2 (VT-2) gene using Bca RNase HIII was examined.. Enterohemorrhagic Escherichia coli O-157 was cultured in mEC medium containing novobiocin at 42°C for 18 hours, and then heated at 95°C for 10 minutes. Dilutions corresponding to 0, 1, 10, $10^2$ or 103 cells in sterile water were prepared and used as templates. Primers VT-2 IF4 and VT-2 IR3 having nucleotide sequences represented by SEQ ID NOS: 130 and 131 were synthesized as primers for detection. The size of the amplification product obtained using the primer pair was about 146 bp. The reaction was carried out as follows. Briefly, 10 μl of a mixture containing 50 pmol each of the primers, propylenediamine at a final concentration of 0.01% and one of the hot water-extracts was prepared. The mixtures were heated at 98°C for 2 minutes and incubated at 55°C for 1 minute in Thermal Cycler Personal, and then placed on ice. At final concentrations, 34 mM Tricine buffer (pH 8.7), 10 mM potassium chloride, 10 mM ammonium sulfate, 1% DMSO, 0.01% BSA, 4 mM magnesium acetate, 500 μM each of dNTPs, 32 U of Bca RNase HIII as prepared in Referential Example 3(5) and 5.5 U of BcaBEST DNA polymerase were added to the mixture to make the final volume to 50 μl. The reaction mixtures were placed in a thermal cycler which had been set at 55°C and reacted at the temperature for 60 minutes. After reaction, 3 μl each of the reaction mixtures was subjected to electrophoresis on 4% NuSieve 3:1 agarose. The results are shown in Figure 27. Figure 27 shows the results of the detection of Escherichia coli O-157 vero toxin II (VT2) gene using Bca RNase HIII. Lane M: molecular weight marker (100 bp ladder); lane N: sterile water as template; lane 1: the template corresponding to 1 cell; lane 2: the template corresponding to 10 cells; lane 3: the template corresponding to $10^2$ cells; and lane 4: the template corresponding to $10^3$ cells.

As shown in Figure 27, VT2 gene was detected using the hot water-extract corresponding to 1 cell according to the ICAN. These results were equivalent to those of the detection reactions according to the ICAN using E. coli RNase H and the PCR as described in Example 9. Thus, it was confirmed that the ICAN using a heat-resistant RNase H, Bca RNase HIII, was also effective in detecting a virus, a bacterium and the like.

Example 30

Detection of Staphylococcus aureus enterotoxin A gene was examined. Primers SEA-1 and SEA-2 represented by SEQ ID NOS:136 and 137 were synthesized based on the nucleotide sequence of the enterotoxin A gene region of Staphylococcus aureus. 50 μl of a reaction mixture containing 1 μl of a solution containing 115 pg or 1.15 ng of a genomic DNA from Staphylococcus aureus (ATCC accession no. 13565) or 1 μl of water for a negative control, 50 pmol each of the primers, 0.5 mM each of dNTPs, 32 mM HEPES-potassium hydroxide buffer (pH 7.8), 100 mM potassium acetate, 4.0 mM magnesium acetate, 0.01% BSA, 1.0% DMSO, 0.0156 μg of Pfu RNase H and 1 U of BcaBEST DNA polymerase was incubated at 58°C for 1 hour in a thermal cycler. After reaction, 5 μl each of the reaction mixtures was analyzed by electrophoresis on 3.0% agarose gel. The results are shown in Figure 29. Figure 29 shows the results of electrophoresis for the detection of Staphylococcus aureus enterotoxin A gene. Lane 1: molecular weight marker (100 bp ladder); lane 2: negative control (sterile water); lane 3: 115 pg of the template; and lane 4: 1.15 ng of the template.

As shown in Figure 29, the amplification product of interest was observed using about 1.15 ng of the template.

Example 31

Detection of hepatitis C virus (HCV) was examined. Primers HCV-F3 and HCV-R1 having nucleotide sequences represented by SEQ ID NOS:138 and 139 were synthesized based on the nucleotide sequence of HCV. A template DNA was prepared as follows. Briefly, 4 μl of a mixture containing RNA prepared as described in Example 21 from 100 μl of a serum from a healthy individual or a patient with HCV, 10 mM tris-hydrochloride buffer (pH 8.3), 5 mM $MgCl_2$, 1 mM each of dNTPs, 10 pmol of Random 6mers and 10 U of Reverse Transcriptase XL (Takara Shuzo) was incubated at 30°C for 10 minutes and at 42°C for 1 hour and then heated at 99°C for 5 minutes for inactivating the enzyme using a thermal cycler (Gene Amp PCR System 9600, Applied Biosystems) to prepare a cDNA.

An ICAN was carried out at 55°C for 1 hour as described in Example 13 except that 1 μl of the cDNA reaction mixture and 100 pmol each of the primers HCV-F3 and HCV-R1 were used. After reaction, 2.5 μl each of the reaction mixtures was subjected to electrophoresis on 3.0% agarose gel. The results are shown in Figure 31. Figure 31 shows the results of electrophoresis for the detection of hepatitis C virus. Lane 1: molecular weight marker (100 bp); lane 2: the template prepared from a serum from a healthy individual; and lanes 3 to 6: the templates prepared from sera from patients infected with HCV.

As shown in Figure 31, it was confirmed that HCV could be specifically detected from the serum samples from patients infected with HCV.

Example 32

The amplification method of the present invention was examined.

(1) A PCR was carried out using the pUC19-150 plasmid DNA as prepared in Example 2(2) as a template and primers MCS-F and MCS-R represented by SEQ ID NOS:35 and 36. A PCR-amplified fragment of 534 bp was obtained by purifying the reaction mixture using Microcon-100. (Millipore). A reaction mixture containing 15 ng of the PCR fragment and 30 pmol of a primer MR2 having a nucleotide sequence represented by SEQ ID NO:140 labeled with [$\gamma$-$^{32}$P]ATP by phosphorylation at the 5'-terminus and sterile distilled water to 5 μl and a reaction mixture further containing 30 pmol of a primer MR1 having a nucleotide sequence represented by SEQ ID NO:141 were prepared. The reaction mixtures were heat-denatured at 98°C for 2 minutes and then cooled to 55°C. 20 μl of a reaction mixture (42.5 mM Tricine buffer (pH 8.7), 12.5 mM potassium chloride, 12.5 mM ammonium sulfate, 0.0125% BSA, 1.25% DMSO, 5 mM magnesium acetate, 0.625 mM each of dNTPs) containing 1 U of BcaBEST DNA polymerase was added to the reaction mixture. The resulting mixture was reacted at 55°C for 15 minutes. After reaction, 2.5 μl of a reaction termination solution (95% formamide, 20 mM EDTA, 0.05% Bromophenol Blue, 0.5% xylene cyanol) was added to 5 μl of the reaction mixture. The mixture was heat-denatured at 94°C for 3 minutes. 1.6 μl each of the reaction mixtures was subjected to electrophoresis on 6% polyacrylamide gel containing 8 M urea and the signals were read using BAS2000 (Fujix) to detect products extended from the primer MR1. The results are shown in Figure 32A. The sequence ladder in Figure 32A was prepared by sequencing M13mp18 single strand DNA (Takara Shuzo) using the primer MF2 labeled with [$\gamma$-$^{32}$P]ATP by phosphorylation and used for the determination of the length of the extension product. Lane 1: a combination of the primers MF2 and MR1; and lane 2: MR1.

As shown in Figure 32A, a band of 448 bp extended from the primer MR1 to the end of the template was detected when the extension reaction was carried out by adding only the primer MR1 to the template. On the other hand, a band of 373 bp bounded by the primers MR1 and MF2 was detected in addition to the above-mentioned band by further adding the primer MF2. Thus, it was confirmed that the extension from the MR1 primer using the PCR-amplified fragment as a template by the action of BcaBEST DNA polymerase was switched due to template switching to the extension using a strand extended from the primer MF2 as a template. Furthermore, template switching was observed when Klenow DNA polymerase was used as a mesophilic DNA polymerase having a strand displacement activity under similar conditions. On the other hand, template switching was not observed when TaKaRa Taq DNA polymerase (Takara Shuzo) or PyroBEST DNA polymerase (Takara Shuzo) without a strand displacement activity was used.

(2) The template switching reaction was examined using a template DNA strand with a primer being annealed thereto. DNA fragments to which the primers MF2 and MR1 could be annealed were prepared as follows. PCRs were carried out using the plasmid pUC19 as a template and primers MCSF and RV (Takara Shuzo) or primers M4 (Takara Shuzo) and MCSR. The reaction mixtures were purified using Microcon-100 to obtain PCR-amplified fragments MSCF-RV (236 bp) and M4-MCSR (271 bp). The region bounded by the primers M4 and RV was commonly present in the two PCR-amplified fragments.

Next, a template-primer (1) in which template DNA strands with primers being annealed thereto were not annealed each other, and a template-primer (2) in which template DNA strands with primers being annealed thereto were annealed each other were prepared as follows.

(1) A reaction mixture containing 30 ng of the fragment MCSF-RV, 40 pmol of the primer MF2 labeled with [$\gamma$-$^{32}$P]ATP by phosphorylation at the 5'-terminus, propylenediamine at a final concentration of 0.01% and sterile distilled water to 5 μl, and a mixture containing 30 ng of the fragment M4-MCSR, 40 pmol of the primer MR1, propylenediamine at a final concentration of 0.01% and sterile distilled water to 5 μl were separately heat-denatured at 98°C for 2 minutes and then cooled to 55°C. 2.5 μl each of the reaction mixtures were mixed together to prepare a template-primer.

(2) A reaction mixture containing 15 ng of the fragment MCSF-RV, 15 ng of the fragment M4-MCSR, 20 pmol of the primer MF2 labeled with [$\gamma$-$^{32}$P]ATP by phosphorylation at the 5'-terminus, 20 pmol of the primer MR1, propylenediamine at a final concentration of 0.01% and sterile distilled water to 5 μl was heat-denatured at 98°C for 2 minutes and then cooled to 55°C to prepare a template-primer.

20 μl of a reaction mixture (42.5 mM Tricine buffer (pH 8.7), 12.5 mM potassium chloride, 12.5 mM ammonium sulfate, 0.0125% BSA, 1.25% DMSO, 5 mM magnesium acetate, 0.625 mM each of dNTPs) containing 1 U of BcaBEST DNA polymerase was added to 5 μl of the template-primer reaction mixture. The resulting mixture was reacted at 55°C for 15 minutes. After reaction, 2.5 μl of a reaction termination solution (95% formamide, 20 mM EDTA, 0.05% Bromophenol Blue, 0.5% xylene cyanol) was added to 5 μl of the reaction mixture. The mixture was heat-denatured at 94°C for 3 minutes. 1.6 μl each of the reaction mixtures was subjected to electrophoresis on 6% polyacrylamide gel containing 8 M urea and the signals were read using BAS2000 (Fujix) to detect products extended from the primer MF2. The results are shown in Figure 32B. The sequence ladder in Figure 32B was prepared by sequencing M13mp18 single strand DNA using the primer MR1 labeled with [$\gamma$-$^{32}$P]ATP by phosphorylation and used for the determination of the length of the extension product. Lane 1: template DNA strands not being annealed each other; and lane 2: template DNA strands being annealed each other.

As shown in Figure 32B, only a band of 161 bp extended from the primer MF2 to the end of the template was detected for the template-primer in which template DNA strands with primers being annealed thereto were not annealed each other. On the other hand, a band of 223 bp bounded by the primers MF2 and MR1 in addition to the above-mentioned band was detected for the template-primer in which template DNA strands with primers being annealed thereto were annealed each other. Thus, it was confirmed that a template switching reaction took place if template DNA strands with primers being annealed thereto were annealed each other.

Example 33

(1) Detection of Mycobacterium tuberculosis was examined using the RNase H derived from Archaeoglobus fulgidus (Afu) described in Referential Example 7. Primers MTIS2F (SEQ ID NO:155) and MTIS2R (SEQ ID NO:156) were synthesized on the basis of the nucleotide sequence of the Mycobacterium tuberculosis genome registered in GenBank under accession number AL123456. The length of the region bordered by the primer pair including the primer portions is 103 bp. Mycobacterium tuberculosis genomic DNA as a template was extracted from dried BCG vaccine (Nippon BCG Seizo) according to a conventional method. Solutions containing 100 pg, 10 pg, 1 pg, 100 fg, 10 fg or 1 fg of the genomic DNA per μl of sterile water were prepared. Reactions were carried out as follows. Briefly, at final concentrations, 32 mM HEPES-potassium hydroxide buffer (pH 7.8), 100 mM potassium acetate, 1% DMSO, 0.01% BSA, 4 mM magnesium acetate, 500 uM each of dNTPs, 50 pmol each of the primers MTIS2F and MTIS2R, 8.75 U of RNase H from Afu, 8 U of BcaBEST DNA polymerase and 1 μl of one of the templates were mixed and the final volume was made to 50 μl with sterile water. The reaction mixtures were placed in Thermal Cycler Personal which had been set at 60°C and incubated for 60 minutes. After reaction, 3 μl each of the reaction mixtures was subjected to electrophoresis on 3.0% agarose gel.

On the other hand, a PCR was carried out as a control. Primers MTIS PCR-F and MTIS PCR-R as described in Rinsho Byori (the Japanese Journal of Clinical Pathology), 43(9):941-947 (1995) were used as primers. An amplification product of 276 bp is obtained using the primer pair. A reaction mixture of 50 µl was prepared using 10 pmol each of the primers according to the instruction manual attached to Ex Taq DNA polymerase (Takara Shuzo). The reaction mixture was placed in a thermal cycler and subjected to a reaction of 40 cycles each cycle consisting of 94°C for 30 seconds, 50°C for 30 seconds and 72°C for 30 seconds. After reaction, 3 µl each of the reaction mixtures was subjected to electrophoresis on 3.0% agarose gel.

As a result, the amplification products of interest were observed in either case using 100 fg of the template.

(2) Detection of Chlamydia trachomatis was examined using RNase H from Afu or RNase H from Pyrococcus horikoshii (Pho). Primers CT2F (SEQ ID NO:157) and CT2R (SEQ ID NO:158) were synthesized on the basis of the nucleotide sequence of the Chlamydia trachomatis plasmid registered in GenBank under accession number X06707. The length of the region bordered by the primer pair including the primer portions is 109 bp. A samples as a template DNA was prepared by subjecting a clinical specimen obtained from a patient agreed with the informed consent to phenol-chloroform treatment and ethanol precipitation. Reactions were carried out as follows. Briefly, at final concentrations, 32 mM HEPES-potassium hydroxide buffer (pH 7.8), 100 mM potassium acetate, 1% DMSO, 0.01% BSA, 4 mM magnesium acetate, 500 µM each of dNTPs, 50 pmol each of the primers CT2F and CT2R, 46.4 U of RNase H from Pho or 8.75 U of RNase H from Afu, 8 U of BcaBEST DNA polymerase and 1 µl of the sample were mixed and the final volume was made to 50 µl with sterile water. The reaction mixtures were placed in Thermal Cycler Personal which had been set at 55°C and incubated for 60 minutes. After reaction, 3 µl each of the reaction mixtures was subjected to electrophoresis on 3.0% agarose gel. As a result, the amplification products of interest was observed. These results demonstrate that Chlamydia trachomatis can be detected using RNase H from Afu or Pho in the method of the present invention.

(3) Furthermore, detection using magnetic beads in a commercially available detection instrument was examined. Briefly, an amplification reaction was carried out as described in (1) above using the primer MTIS2R used in (1) above labeled at its 5'-terminus with biotin as a primer and 100 ng of the Mycobacterium tuberculosis genomic DNA as a template. The resulting amplified fragment was diluted 30-fold, 300-fold or 3000-fold and subjected to detection using streptoavidin-coated magnetic beads (Pierce) in an automated detection instrument Lumipuls (Fujirebio).

Magnetic beads having streptoavidin capable of binding 100 pmol of biotin being immobilized was reacted with the biotinylated amplified fragment for 5 minutes on the first layer of a cuvette. 0.1 N NaOH was then added. thereto. Hybridization with a FITC-labeled probe MTISBF was carried out for 5 minutes. After washing, a POD-labeled anti-FITC antibody was added thereto. After reacting for 5 minutes and washing, a luminescent substrate was added thereto. As a result, it was shown that detection can be carried out semi-quantitatively in a short time (20 minutes) using magnetic beads in a conventional automated detection instrument. The detection was carried out by measuring the luminescence level by photocounting. The results are shown in Table 12.

Table 12

| Mycobacterium tuberculosis amplicon | Photocounting | S/N ratio |
|---|---|---|
| x 30 | $3.55 \times 10^7$ | 29.6 |
| x 300 | $1.21 \times 10^7$ | 10.0 |
| x 3000 | $0.21 \times 10^7$ | 1.75 |
| 0 | $0.12 \times 10^7$ | - |

The results in Table 12 demonstrate that detection can be carried out with sensitivity equivalent to that of the conventional plate luminescence method.

(4) A hybrid chromatography method was examined as a method for detecting the above-mentioned amplified fragment. Streptoavidin (Nacalai Tesque) was immobilized onto a nitrocellulose membrane. A water-absorptive pad was connected therewith to construct a hybrid chromatography strip. This strip was used to detect the amplified fragment used in (3) above according to a hybrid chromatography method. Detection was carried out by color development using 1-step TMB-Blotting (Pierce). Specifically, a reaction mixture containing the amplified fragment was developed on the nitrocellulose membrane. Then, 0.1 N NaOH solution, FITC-labeled probe, washing solution and color development solution were developed in this order. As a result, a blue band was detected for the amplified fragment derived from a Mycobacterium tuberculosis-positive sample. It was demonstrated that this method is useful as a rapid gene examination method because, using this method, results are obtained with naked eyes in 5 to 10 minutes by the method of the present invention.

Example 34

(1) Southern hybridization analysis of amplified products containing laddered bands

In some cases, plural high molecular weight laddered bands other than three bands of interest may be observed in the amplification method of the present invention. The laddered bands were examined. Enterohemorrhagic E. coli 0-157 was selected as a target. A template DNA, chimeric primers and reaction conditions for ICAN were as described in Example 9. After reaction, 5 μl of the reaction mixture was subjected to electrophoresis on 3% NuSieve 3:1 agarose gel. The results are shown in Figure 37. In Figure 37, the lanes represent results for the following: lane M: 100 bp DNA ladder marker; lane 1: negative control; lane 2: the heated extract corresponding to 1 cell; lane 3: the heated extract corresponding to 10 cells; lane 4: the heated extract corresponding to $10^2$ cells; lane 5: the heated extract corresponding to 103 cells; lane 6: the heated extract corresponding to $10^4$ cells; and lane 7: the heated extract corresponding to $10^5$ cells. Ladder bands were observed as shown in Figure 37.

(2) Analysis of laddered amplified fragments

The nucleotide sequences of the laddered bands obtained in (1) above were analyzed. Briefly, 50 μl of the reaction mixture prepared in (1) was subjected to electrophoresis on 3% agarose gel. After electrophoresis, the laddered bands' were excised from the gel. The amplified DNA fragments were then recovered from the gel using EASYTRAP Ver.2 (Takara Shuzo). The recovered amplified fragments were blunt-ended using DNA Blunting kit (Takara Shuzo).

The blunt-ended DNA fragments were ligated with a vector pGEM-3Z (Promega) digested with a restriction enzyme HincII (Takara Shuzo) using DNA ligation Kit (Takara Shuzo). The ligation mixtures were used to transform competent cells of JM109 (Takara Shuzo). After transformation, the cells were cultured on LB agar plate containing 0.1 mM ampicillin, 1 mM IPTG and 0.02% X-gal at 37°C overnight.

After cultivation, several white colonies were selected from the plates. Colony PCRs were carried out using primers M13-M4 and M13-RV (both from Takara Shuzo) to determine the presence of an insert. Colonies having inserts were cultured with shaking in LB liquid medium containing 0.1 mM ampicillin at 37°C overnight. After cultivation, plasmids were purified from cells using QIAGEN plasmid mini Kit (Qiagen). The sequences of fragments cloned at the HincII sites of the plasmids were analyzed in both directions using primers M13-M4 and M13-RV according to a conventional method.

As a result, it was demonstrated that the laddered fragment obtained by the method of the present invention has a structure in which the region to be amplified is repeated. Furthermore, it was confirmed that the repetition is in the same direction from 5' to 3'.

(3) The laddered amplified fragments other than three amplified fragments of interest formed in the method of the present invention for detecting Mycobacterium tuberculosis, HCV or Chlamydia trachomatis were examined. HCV was detected under conditions as described in Example 31. Mycobacterium tuberculosis or Chlamydia trachomatis was detected under conditions as described in Example 33. The resulting laddered amplified fragments were subcloned and sequenced as described in (2) above. As a result, it was demonstrated that the laddered fragment obtained by the method of the present invention has a structure in which the region to be amplified is repeated. Furthermore, it was confirmed that the repetition is in the same direction from 5' to 3'.

Example 35

(1) The detection method of the present invention for Mycobacterium tuberculosis was examined. First, primers K-F-1033(60) (SEQ ID NO:159) and K-F-1133(62) (SEQ ID NO:160) were synthesized for amplifying a region with relatively low GC content in the Mycobacterium tuberculosis genome. Serial dilutions containing 100 fg to 10 pg of the Mycobacterium tuberculosis genomic DNA as described in Example 33(1) were used as templates. The reaction was carried our as follows. Briefly, at final concentrations, 32 mM HEPES-potassium hydroxide buffer (pH 7.8), 100 mM potassium acetate, 1% DMSO, 0.01% BSA, 4 mM magnesium acetate, 500 μM each of dNTPs, 50 pmol each of the primers K-F-1033(60) and K-F-1133(62), 9.375 U of RNase HII from Pfu or 4.375 U of RNase H from Afu, 2.75 U of BcaBEST DNA polymerase and 1 μl of one of the templates were mixed and the final volume was made to 25 μl with sterile water. The reaction mixtures were placed in Thermal Cycler Personal which had been set at 62°C and incubated for 60 minutes. After reaction, 3 μl each of the reaction mixtures was subjected to electrophoresis on 3.0% agarose gel. As a result, it was demonstrated that the amplification products could be detected using 100 fg to 10 pg of the genomic DNA as a template and either RNase H.

(2) The method as described in (1) above was examined using primers having higher Tm values. First, primers K-F-1033(68) (SEQ ID NO:161) and K-F-1133(68) (SEQ ID NO:162) were synthesized. The amplification reaction was carried out under the same conditions as those described in (1) above except that the reaction temperature was 63°C.

After reaction, 3 µl each of the reaction mixtures was subjected to electrophoresis on 3.0% agarose gel. The results are shown in Figure 38. Figure 38 illustrates results of electrophoresis of Mycobacterium tuberculosis genome amplified using RNase H from Pfu or RNase H from Afu. Lanes 1 to 4 represent results obtained using RNase HII from Pfu and the following amount of template DNA: lane 1: 10 pg; lane 2: 1 pg; lane 3: 100 fg; and lane 4: negative control. Lanes 5 to 8 represent results obtained using RNase HII from Afu and the following amount of template DNA: lane 5: 10 pg; lane 6: 1 pg; lane 7: 100 fg; and lane 8: negative control. Lane M represents 100 bp DNA ladder marker.

As shown in Figure 38, it was demonstrated that the amplification fragment of interest could be detected using 100 fg of the template DNA and either RNase H. It was shown that more amplification product is obtained when RNase H from Afu is used. In addition, it was shown that more stable detection sensitivity is accomplished when RNase H from Afu is used.

(3) Amplification using a combination of the primers K-F-1033(68) and K-F-1133(68) and a plasmid containing a region to be amplified as a template was examined. First, primers F26 (SEQ ID NO:163) and R1310 (SEQ ID NO: 164) were synthesized in order to prepare a plasmid containing a region to be amplified. A PCR was carried out using these primers and BCG vaccine strain. The resulting amplification product was then introduced into pT7-Blue-T vector (Takara Shuzo) to prepare the plasmid. The composition of the reaction mixture was as described in (2) above except that 4 U of Bca DNA polymerase was used. As a result, it was confirmed that detection can be carried out using 1 fg of the template.

(4) The detection sensitivity accomplished when a combination of primers MTIS2F and MTIS2R, which results in the formation of laddered amplified fragments containing the three amplified fragments of interest, was used was compared with that accomplished when a combination of primers K-F-1033(68) and K-F-1133(68), which results in the three amplified fragments of interest, was used. Mycobacterium tuberculosis was selected as a target. Reactions were carried out as described in Example 33(2) when the combination of the primers MTIS2F and MTIS2R was used or in (2) above when the combination of the primers K-F-1033(68) and K-F-1133(68) was used. As a result, the same detection sensitivity was observed in either case.

Example 36

The amplification method of the present invention that does not comprise denaturation of a genomic DNA as a template was examined.

(1) Primers pDON-AI-68-1 (SEQ ID NO:165) and pDON-AI-68-2 (SEQ ID NO:166) were synthesized in accordance with the nucleotide sequence of the packaging region in a plasmid pDON-AI (Takara Shuzo).

(2) A reaction mixture of a total volume of 50 µl containing 1 µl of a solution containing 10 fg or 1 pg of pDON-AI, 1 µl of a solution containing 1 ng, 10 ng or 100 ng of the genomic DNA derived from NIH/3T3 cells having pDON-AI being incorporated prepared in Example 23, or 1 µl of water as a negative control, 50 pmol each of the primers as described in (1) above, 0.5 mM each of dNTPs, 32 mM HEPES-potassium hydroxide buffer (pH 7.8), 100 mM potassium acetate, 4 mM magnesium acetate, 0.01% BSA, 1% DMSO, 18.5 U of RNase HII from Pfu and 4 U of BcaBEST DNA polymerase was prepared. The reaction mixtures were placed in a thermal cycler and incubated at 64°C for 1 hour. After reaction, 5 µl each of the reaction mixtures was subjected to electrophoresis on 3% agarose gel to observe amplification products. The results are shown in Figure 39. In Figure 39, the lanes represent results for the following templates: lane M: 100 bp DNA ladder marker; lane 1: negative control; lane 2: 1 ng of genomic DNA having pDON-AI being incorporated; lane 3: 10 ng of genomic DNA having pDON-AI being incorporated; lane 4: 100 ng of genomic DNA having pDON-AI being incorporated; lane 5: 10 fg of pDNA-AI DNA; and lane 6: 1 pg of pDON-AI DNA.

As shown in Figure 39, amplification of specific DNA fragments was observed for either pDON-AI or the genomic DNA having pDON-AI being incorporated. Thus, it was confirmed that a DNA fragment of interest can be amplified without denaturing the DNA as a template prior to the reaction even if a genomic DNA is used as a template.

Example 37

The fidelity (accuracy) of the present invention was compared with that of a PCR utilizing LA technology in which TaKaRa Ex Taq polymerase (Takara Shuzo) was used. First, a plasmid as a template was prepared as follows.

Specifically, the regions each consisting of 300 bp represented by SEQ ID NOS:167 to 170 were amplified by PCR from human proto-oncogene, Wnt-5a gene (GenBank accession no. L20861), ribosomal protein S5 gene (GenBank accession no. XM_009371), human NADH gene (GenBank accession no. NM_000903) and human protocadherin 43 gene (GenBank accession no. AU077347). In this case, the PCRs were carried out using specific primers each having a site for a restriction enzyme SfiI at the 5'-terminus of the primer. After reaction, the amplified fragments were digested with a restriction enzyme SfiI (Takara Shuzo). pUC19 (Takara Shuzo) was digested with restriction enzymes Af1III (NEB) and NdeI (Takara Shuzo) and subjected to agarose gel electrophoresis. A fragment of about 2 kbp was excised from the gel and recovered using EASYTRAP (Takara Shuzo). A DNA having the sequence of SEQ ID NO:171 and a DNA complementary thereto were synthesized using a DNA synthesizer. These DNAs were heat-denatured and then annealed each other to form a double-stranded DNA. This double-stranded DNA has cohesive ends for restriction enzymes AflIII and NdeI at its termini. The double-stranded synthetic DNA was inserted into the fragment obtained by digesting pUC19 with restriction enzymes using DNA Ligation kit ver. 2 (Takara Shuzo). The resulting plasmid was designated as pIC62. pIC62 has sequences to which primers ICAN2 (SEQ ID NO:172) and ICAN6 (SEQ ID NO:173) anneal as well as a site for a restriction enzyme SfiI. The plasmid pIC62 was digested with the restriction enzyme SfiI. The above-mentioned PCR-amplified fragment digested with the restriction enzyme SfiI was ligated with the plasmid using Ligation kit ver. 2 (Takara Shuzo). The ligation mixture was used to transform Escherichia coli JM109 (Takara Shuzo). The resulting transformants were cultured, plasmids having the about 300-bp DNAs being inserted were obtained, and the sequences of the inserted DNAs were determined. The plasmids were then used as templates in this Example.

(2) ICAN amplification products were prepared as follows. Briefly, a solution of 10 μl containing 10 ng of one of the plasmids as a template, 50 pmol each of chimeric primers ICAN2 (SEQ ID NO:172) and ICAN6 (SEQ ID NO: 173) and 0.01% propylenediamine was prepared. The solution was denatured at 98°C for 2 minutes and incubated at 60°C for 1 minute in Thermal Cycler Personal, and then transferred onto ice. Next, at final concentrations, 20 mM HEPES-potassium hydroxide buffer (pH 7.8), 100 mM potassium acetate, 1% DMSO, 0.01% BSA, 4 mM magnesium acetate, 500 μM each of dNTPs, 30 U of RNase H from E. coli and 5.5 U of BcaBEST DNA polymerase were added thereto and the final volume was made to 50 μl with sterile water. The reaction mixtures were placed in Thermal Cycler Personal which had been set at 60°C and incubated for 60 minutes. After reaction, the reaction mixtures were subjected to electrophoresis on 2% SeaKem GTG agarose gel (Takara Shuzo). After electrophoresis, bands for the amplification products of interest were recovered by excision from the agarose gel, recovery using SUPREC-01 (Takara Shuzo), treatment with phenol-chloroform and ethanol precipitation.

(3) PCR amplification products were prepared as follows using TaKaRa Ex Taq DNA polymerase. Briefly, a reaction mixture was prepared using 10 ng of the above-mentioned plasmid as a template as well as 10 pmol each of DNA primers ICAN2 (SEQ ID NO:174) and ICAN6 (SEQ ID NO:175) according to the instruction manual attached to TaKaRa Ex Taq DNA polymerase (Takara Shuzo). The reaction mixture was placed in a thermal cycler and subjected to a reaction of 30 cycles, each cycle consisting of 94°C for 30 seconds, 55°C for 30 seconds and 72°C for 30 seconds. After reaction, the reaction mixture was subjected to agarose gel electrophoresis as described in (2) above. The amplification products of interest were recovered by recovery using Microcon-100 (Takara Shuzo); treatment with phenol-chloroform and ethanol precipitation.

(4) The amplification products obtained in (2) and (3) above were subcloned as follows. Briefly, the ICAN amplification products and the PCR amplification products were introduced into a vector pT7 Blue (Takara Shuzo) using Perfectly Blunt Cloning kit (Takara Shuzo) according to the instruction manual. The ligation mixture was used to transform NovaBlue Singles Competent Cell (Takara Shuzo). For each clone, 10 colonies were selected from the resulting transformants and cultured to obtain plasmids each having about 0.4-kb DNA being inserted. The fragments inserted in the plasmids were sequenced using T7 promoter primer (Takara Shuzo) and M3 primer (Takara Shuzo).

A total of about 16,000 bases were analyzed by sequencing as described above. As a result, one mutation was found in about 2500 bases for both of the fragments amplified according to the method of the present invention and the fragments amplified by PCR using TaKaRa Ex Taq DNA polymerase. Thus, it was confirmed that the fidelity (accuracy) of the method of the present invention is equivalent to that of LA-PCR whose fidelity is high.

Example 38

(1) Preparation of template for ICAN reaction by PCR

A double-stranded cDNA was prepared from polyA+ RNA derived from mouse brain (OriGene) using cDNA synthesis kit (Takara Shuzo). PCR fragments were amplified using the double-stranded cDNA as a template and combinations of the primers having nucleotide sequences of SEQ ID NOS:176-189. The fragments were introduced into pT7 Blue T-vector (Takara Shuzo) by TA cloning to obtain plasmid clones. A reaction mixture of a total volume of 50 μl containing 1 μl (1 ng) of one the plasmid clones, 10 pmol each of primers MCS-F (SEQ ID NO:190) and MCS-R (SEQ ID NO:191), 1.25 U of Ex Taq (Takara Shuzo), 5 μl of 10 x Ex Taq buffer (Takara Shuzo) and 0.2 mM each of dNTPs was subjected to the following reaction using TaKaRa PCR Thermal Cycler Personal (Takara Shuzo): 94°C for 2 minutes; 30 cycles of 94°C for 30 seconds, 55°C for 30 seconds and 72°C for 1 minute. The resulting amplified DNA fragment was used as a template for an ICAN reaction.

(2) Amplification of DNA fragment by ICAN using PCR product as template

An ICAN reaction was carried out using Aminoallyl dUTP (Sigma) in order to introduce an amino group into an ICAN amplification product. The ratio of amino group introduced into an ICAN amplification product was examined by changing the ratio of the amount of dTTP to the amount of Aminoallyl dUTP in the ICAN reaction as follows: 10 : 0, 9 : 1, 8 : 2, 7 : 3 and 6 : 4. The reaction was carried out as follows.

First, a solution of a total volume of 10 μl containing 1 μl of the PCR reaction mixture prepared in (1) above, 50 pmol each of primers MF2N3(24) (SEQ ID NO:192) and MR1N3(24) (SEQ ID NO:193) and 2 μl of 0.05% aqueous solution of propylenediamine was heated at 98°C for 2 minutes followed by 65°C for 30 seconds in TaKaRa PCR Thermal Cycler Personal and rapidly cooled on ice to anneal the primers to the template. A reaction mixture of a total volume of 40 μl containing 0.625 mM each of dATP, dCTP and dGTP, 0.625 mM of a dTTP + Aminoallyl dUTP mixture, 32 mM HEPES-potassium hydroxide buffer (pH 7.8), 5.0 mM magnesium acetate, 0.6 U of RNase H from E. coli (Takara Shuzo) and 2.75 U of BcaBEST DNA polymerase was added to the heated solution. The resulting mixture was incubated in Thermal Cycler at 65°C for 1 hour.

50 μl of isopropanol and 5 μl of 3 M sodium acetate solution (pH 5.2) were added to the reaction mixture of 50 μl. The resulting mixture was cooled at-80°C for 20 minutes and then centrifuged to remove a supernatant. 200 μl of 70% ethanol solution was added thereto. The supernatant was removed by centrifugation. The precipitate was air-dried. The resulting DNA was redissolve in water. $OD_{260/280}$ was measured to determined the amount of the product.

(3) Confirmation of introduction of Aminoallyl dUTP into ICAN amplification product

Introduction of an amino group into an ICAN product was confirmed by fluorescence-label the amino group in the ICAN product using 5-carboxyfluorecein succinimidil ester (Molecular Probe). The above-mentioned DNA solution was diluted to make the concentration to 2 μg/50 μl. 20 μl of 1 M sodium carbonate buffer (pH 9.0) was added thereto. Then, 4 μl of a solution of FITC (Nacalai Tesque) in N,N-dimethylformamide at a concentration of 10 mM was further added. The resulting mixture was reacted at 20°C for 16 hours. After removing excess FITC using a commercially available spin column, 10 μl of the reaction mixture was applied onto 2.0% agarose gel and electrophoresed. After electrophoresis, fluorescent dye was detected using FM-BIO. Furthermore, the ICAN-amplified fragment was detected by staining with EtBr. As a result, it was confirmed that an amino group can be introduced into an ICAN amplification product by conducting an ICAN using Aminoallyl dUTP. It was also confirmed that the detection sensitivity can be further increased by using a modified nucleotide having a functional group and fluorescent label for an amplification product in combination.

(4) The amplification method of the present invention was examined using deoxyUTP. Mycobacterium tuberculosis was selected as a target. First, primers MTIS2F-16 (SEQ ID NO:194) and MTIS2R-ACC (SEQ ID NO:195) were synthesized in accordance with the nucleotide sequence of Mycobacterium tuberculosis genome registered in GenBank under accession no. AL123456. The length of the region to be amplified using this primer pair including the primer portions is 98 bp. A template was prepared as follows. Briefly, a product obtained by PCR-amplifying the Mycobacterium tuberculosis genome using primers MTIS-PCR-F-2 (SEQ ID NO:196) and MTIS-PCR-R-2 (SEQ ID NO: 197) was inserted into pT7 Blue T-Vector (Takara Shuzo) using DNA Ligation kit Ver. 2. The ligated plasmid was used to transform Escherichia coli JM109. The resulting transformants were cultured to obtain a plasmid having an about 400-bp DNA being introduced. A solution containing $10^3$ copies of the plasmid per μl was prepared based on the concentration determined by measuring OD260.

At final concentrations, 32 mM HEPES-potassium hydroxide buffer (pH 7.8), 100 mM potassium acetate, 1% DMSO, 0.01% BSA, 4 mM magnesium acetate, 500 μM each of dATP, dCTP and dGTP, a dTTP/dUTP mixture (500/0, 400/100, 300/200, 200/300, 100/400 or 0/500 μM), 50 pmol each of the primers MTIS 2F-16 and MTIS 2R-AAC, 8.75 U of RNase H from Afu, 8 U of BcaBEST DNA polymerase and 1 μl of the template ($10^3$ copies) were mixed and the final volume was made to 50 μl with sterile water. The reaction mixtures were placed in Thermal Cycler Personal which had been set at 60°C and incubated for 60 minutes. After reaction, 3 μl each of the reaction mixtures was subjected to electrophoresis on 3% agarose gel.

As a result, the amplification product of interest was observed for each of the dTTP/dUTP ratios. Based on these results, it was confirmed that a modified nucleotide can be used as a substrate in the method of the present invention.

Example 39

Application of the method of the present invention to a one-step amplification method was examined. HCV was selected as a target.

(1) Preparation of transcript RNA

A transcript RNA as a template was prepared. Preparation was carried out from 300 μl of a serum derived from a patient with hepatitis C virus agreed with the informed consent using TRIzol reagent (Life Technologies) according to the instructions attached to the reagent followed by final dissolution in 20 μl of injectable water (Otsuka Pharmaceutical). An RT-PCR was carried out using the RNA sample above as a template. The reaction was carried out as follows. A reaction mixture of 50 μl was prepared using 2 μl of the RNA sample and 20 pmol each of primers SP6-HCV-F (SEQ ID NO:19 and T7-HCV-R (SEQ ID NO:199) according to a manual attached to One-Step RNA PCR kit (Takara Shuzo). The reaction mixture was placed in Thermal Cycler Personal and subjected to the following reaction: 50°C for 15 minutes; 94°C for 2 minutes; and 40 cycles of 94°C for 30 seconds, 60°C for 30 seconds and 72°C for 30 seconds. After reaction, the reaction mixture was subjected to electrophoresis on 2% SeaPlaque GTG agarose gel. A 350-bp amplification product of interest was excised from the gel. The DNA was then recovered using EASYTRAP Ver. 2 according to the instructions attached to the kit. A transcript RNA was synthesized using the recovered DNA as a template and Competitive RNA Transcription kit (Takara Shuzo) according to the instructions attached to the kit. The transcript RNA was used as a template for examination of a one-step RT-ICAN.

(2) Examination of one-step RT-ICAN

The concentration of the transcript RNA prepared in (1) above was determined based on the $OD_{260}$ value and dilutions containing $10^4$, $10^5$, $10^6$ or $10^7$ copies per μl were prepared. A reaction mixture of 50 μl containing, at final concentrations, 32 mM HEPES-potassium hydroxide buffer (pH 7.8), 100 mM potassium acetate, 1% DMSO, 0.01% BSA, 4 mM magnesium acetate, 500 μM each of dNTPs, 50 pmol each of the primers HCV-A S (SEQ ID NO: 200) and HCV-A A (SEQ ID NO:201), 30 U of RNase H from Pfu, 8 U of BcaBEST DNA polymerase, 20 U of RNase inhibitor, 0, 1, 2.5, 3 or 5 U of AMV RTase XL (Takara Shuzo) and 1 μl of one of the dilutions containing varying copies of the transcript RNA was prepared. The reaction mixtures were placed in Thermal Cycler Personal which had been set at 60°C and incubated for 60 minutes. After reaction, 2 μl each of the reaction mixtures was subjected to electrophoresis on 3% agarose gel.

As a result, no amplification product of interest was observed using each of the dilutions of the template when AMV RTase was not added. On the other hand, the amplification product of interest was observed using the dilution containing $10^7$ copies (1 U of AMV RTase XL added), $10^6$ copies (2.5 U of AMV RTase XL added), $10^6$ copies (3 U of AMV RTase XL added) or $10^6$ copies (5 U of AMV RTase XL added). When the reaction was carried out at a temperature of 57°C, the amplification product of interest was observed using the dilution containing $10^5$ copies and 2.5 U of AMV RTase XL. Furthermore, when 1 U of BcaBEST DNA polymerase and 10 U of RNase H from Pfu were used, the amplification product of interest was observed using the dilution containing $10^6$ copies even if no AMV RTase was added.

Industrial Applicability

The present invention provides a method for amplifying a target nucleic acid comprising amplifying a region suitable for specific amplification in the nucleotide sequence of the target nucleic acid by a DNA synthesis reaction using a chimeric oligonucleotide primer. The present invention also provides a method for detecting a target nucleic acid comprising detecting a fragment amplified from the target nucleic acid obtained by the method for amplifying a target nucleic acid. Furthermore, the amplification method of the present invention can be utilized as an efficient method for producing a nucleic acid by combining it with another method for amplifying a nucleic acid or a method for duplicating a nucleic acid. The present invention also provides a method for detecting a target nucleic acid for the specific detection and quantification of a microorganism, particularly a pathogenic microorganism, such as a virus, a bacterium, a fungi, a yeast or the like with high sensitivity as well as a chimeric oligonucleotide primer for the method. The present invention further provides a system for amplifying and detecting a nucleic acid which is automated, in a small amount and highly integrated.

Sequence Listing Free Text

SEQ ID NO:1: PCR primer BsuII-3 for cloning a gene encoding a polypeptide having a RNaseHII activity from Bacillus caldotenax.

SEQ ID NO:2: PCR primer BsuII-6 for cloning a gene encoding a polypeptide having a RNaseHII activity from Bacillus caldotenax.

SEQ ID NO:3: PCR primer RNII-S1 for cloning a gene encoding a polypeptide having a RNaseHII activity from Bacillus caldotenax.

SEQ ID NO:4: PCR primer RNII-S2 for cloning a gene encoding a polypeptide having a RNaseHII activity from Bacillus caldotenax.
SEQ ID NO:5: PCR primer RNII-S5 for cloning a gene encoding a polypeptide having a RNaseHII activity from Bacillus caldotenax.
SEQ ID NO:6: PCR primer RNII-S6 for cloning a gene encoding a polypeptide having a RNaseHII activity from Bacillus caldotenax.
SEQ ID NO:7: PCR primer RNII-Nde for cloning a gene encoding a polypeptide having a RNaseHII activity from Bacillus caldotenax.
SEQ ID NO:8: Nucleotide sequence of ORF in RNaseHII gene from Bacillus caldotenax.
SEQ ID NO:9: Amino acid sequence of RNaseHII from Bacillus caldotenax.
SEQ ID NO:10: PCR primer BsuIII-1 for cloning a gene encoding a polypeptide having a RNaseHIII activity from Bacillus caldotenax.
SEQ ID NO:11: PCR primer BsuIII-3 for cloning a gene encoding a polypeptide having a RNaseHIII activity from Bacillus caldotenax.
SEQ ID NO:12: PCR primer BsuIII-6 for cloning a gene encoding a polypeptide having a RNaseHIII activity from Bacillus caldotenax.
SEQ ID NO:13: PCR primer BsuIII-8 for cloning a gene encoding a polypeptide having a RNaseHIII activity from Bacillus caldotenax.
SEQ ID NO:14: PCR primer RNIII-S3 for cloning a gene encoding a polypeptide having a RNaseHIII activity from Bacillus caldotenax.
SEQ ID NO:15: PCR primer BcaRNIII-3 for cloning a gene encoding a polypeptide having a RNaseHIII activity from Bacillus caldotenax.
SEQ ID NO:16: Nucleotide sequence of ORF in RNaseHIII from Bacillus caldotenax.
SEQ ID NO:17: Amino acid sequence of RNaseHIII from Bacillus caldotenax.
SEQ ID NO:18: PCR primer BcaRNIIINde for amplifying a gene encoding a polypeptide having a RNaseHIII activity from Bacillus caldotenax.
SEQ ID NO:19: Nucleotide sequence conserving between PH1650 and a portion of Pyrococcus furiosus genome sequence.
SEQ ID NO:20: PCR primer 1650Nde for cloning a gene encoding a polypeptide having a RNaseHII activity from Pyrococcus furiosus.
SEQ ID NO:21: PCR primer 1650Bam for cloning a gene encoding a polypeptide having a RNaseHII activity from Pyrococcus furiosus.
SEQ ID NO:22: Nucleotide sequence of ORF in RNaseHII from Pyrococcus furiosus.
SEQ ID NO:23: Amino acid sequence of RNaseHII from Pyrococcus furiosus.
SEQ ID NO:24: PCR primer 915-F1 for cloning a gene encoding a polypeptide having a RNaseHII activity from Thermotoga maritima.
SEQ ID NO:25: PCR primer 915-F2 for cloning a gene encoding a polypeptide having a RNaseHII activity from Thermotoga maritima.
SEQ ID NO:26: PCR primer 915-R1 for cloning a gene encoding a polypeptide having a RNaseHII activity from Thermotoga maritima.
SEQ ID NO:27: PCR primer 915-R2 for cloning a gene encoding a polypeptide having a RNaseHII activity from Thermotoga maritima.
SEQ ID NO:28: Designed chimeric oligonucleotide primer designated as pUC19 upper 150 to amplify a portion of plasmid pUC19. "nucleotides 24 to 25 are ribonucleotides-other nucleotides are deoxyribonucleotides"
SEQ ID NO:29: Designed chimeric oligonucleotide primer designated as MR1N3 to amplify a portion of plasmid pUC19. "nucleotides 28 to 30 are ribonucleotides-other nucleotides are deoxyribonucleotides"
SEQ ID NO:30: Designed oligonucleotide primer designated as M13M4
SEQ ID NO:31: Designed chimeric oligonucleotide primer to amplify a portion of vero toxin 1-encoding sequence from hemorrhagic Escherichia coli O-157. "nucleotides 16 to 18 are ribonucleotides-other nucleotides are deoxyribonucleotides"
SEQ ID NO:32: Designed chimeric oligonucleotide primer to amplify a portion of vero toxin 1-encoding sequence from hemorrhagic Escherichia coli O-157. "nucleotides 15 to 17 are ribonucleotides-other nucleotides are deoxyribonucleotides"
SEQ ID NO:33: Designed chimeric oligonucleotide primer to amplify a portion of vero toxin 2-encoding sequence from hemorrhagic Escherichia coli O-157. "nucleotides 16 to 18 are ribonucleotides-other nucleotides are deoxyribonucleotides"
SEQ ID NO:34: Designed chimeric oligonucleotide primer to amplify a portion of vero toxin 2-encoding sequence from hemorrhagic Escherichia coli O-157. "nucleotides 16 to 18 are ribonucleotides-other nucleotides are deoxyribonucleotides"

SEQ ID NO:35: Designed oligonucleotide primer designated as MCR-F to amplify a long DNA fragment
SEQ ID NO:36: Designed oligonucleotide primer designated as MCR-R to amplify a long DNA fragment
SEQ ID NO:37: Designed chimeric oligonucleotide primer designated as MF2N3(24) to amplify a long DNA fragment. "nucleotides 22 to 24 are ribonucleotides-other nucleotides are deoxyribonucleotides"
SEQ ID NO:38: Designed chimeric oligonucleotide primer designated as MR1N3(24) to amplify a long DNA fragment. "nucleotides 22 to 24 are ribonucleotides-other nucleotides are deoxyribonucleotides"
SEQ ID NO:39: Designed oligonucleotide primer to amplify a portion of lambda DNA. "nucleotides 18 to 20 are ribonucleotides-other nucleotides are deoxyribonucleotides"
SEQ ID NO:40: Designed chimeric oligonucleotide primer to amplify a portion of lambda DNA. "nucleotides 18 to 20 are ribonucleotides-other "nucleotides are deoxyribonucleotides"
SEQ ID NO:41: Designed oligonucleotide primer to amplify a portion of lambda DNA
SEQ ID NO:42: Designed oligonucleotide primer to amplify a portion of lambda DNA
SEQ ID NO:43: Designed chimeric oligonucleotide primer to amplify a portion of Flavobacterium species DNA. "nucleotides 18 to 20 are ribonucleotides-other nucleotides are deoxyribonucleotides"
SEQ ID NO:44: Designed chimeric oligonucleotide primer to amplify a portion of Flavobacterium species DNA. "nucleotides 18 to 20 are ribonucleotides-other nucleotides are deoxyribonucleotides"
SEQ ID NO:45: Designed oligonucleotide primer to amplify a portion of Flavobacterium species DNA.
SEQ ID NO:46: Designed oligonucleotide primer to amplify a portion of Flavobacterium species DNA.
SEQ ID NO:47: Designed chimeric oligonucleotide primer to amplify a portion of vero toxin 2-encoding sequence from hemorrhagic Escherichia coli O-157. "nucleotides 19 to 21 are ribonucleotides-other nucleotides are deoxyribonucleotides"
SEQ ID NO:48: Designed chimeric oligonucleotide primer to amplify a portion of vero toxin 2-encoding sequence from hemorrhagic Escherichia coli O-157. "nucleotides 18 to 20 are ribonucleotides-other nucleotides are deoxyribonucleotides"
SEQ ID NO:49: Designed oligonucleotide.primer to amplify a portion of vero toxin 2-encoding sequence from hemorrhagic Escherichia coli O-157.
SEQ ID NO:50: Designed oligonucleotide primer to amplify a portion of vero toxin 2-encoding sequence from hemorrhagic Escherichia coli O-157.
SEQ ID NO:51: Designed chimeric oligonucleotide primer to amplify a portion of vero toxin 2-encoding sequence from hemorrhagic Escherichia coli O-157. "nucleotides 18 to 20 are ribonucleotides-other nucleotides are deoxyribonucleotides"
SEQ ID NO:52: Designed chimeric oligonucleotide primer to amplify a portion of vero toxin 2-encoding sequence from hemorrhagic Escherichia coli O-157. "nucleotides 18 to 20 are ribonucleotides-other nucleotides are deoxyribonucleotides"
SEQ ID NO:53: Designed chimeric oligonucleotide primer to amplify a portion of vero toxin 2-encoding sequence from hemorrhagic Escherichia coli O-157. "nucleotides 18 to 20 are ribonucleotides-other nucleotides are deoxyribonucleotides"
SEQ ID NO:54: Designed oligonucleotide primer to amplify a portion of vero toxin 2-encoding sequence from hemorrhagic Escherichia coli O-157.
SEQ ID NO:55: Designed oligonucleotide primer to amplify a portion of vero toxin 2-encoding sequence from hemorrhagic Escherichia coli O-157.
SEQ ID NO:56: Designed chimeric oligonucleotide primer to amplify a portion of lambda DNA. "nucleotides 18 to 20 are ribonucleotides-other nucleotides are deoxyribonucleotides"
SEQ ID NO:57: Designed oligonucleotide primer to amplify a portion of viroid CSVd.
SEQ ID NO:58: Designed oligonucleotide primer to amplify a portion of viroid CSVd.
SEQ ID NO:59: Designed chimeric oligonucleotide primer to amplify a portion of viroid CSVd. "nucleotides 16 to 18 are ribonucleotides-other nucleotides are deoxyribonucleotides"
SEQ ID NO:60: Designed chimeric oligonucleotide primer to amplify a portion of viroid CSVd. "nucleotides 18 to 20 are ribonucleotides-other nucleotides are deoxyribonucleotides"
SEQ ID NO:61: Designed chimeric oligonucleotide primer to amplify a portion of Flavobacterium species DNA. "nucleotides 18 to 20 are ribonucleotides-other nucleotides are deoxyribonucleotides"
SEQ ID NO:62: Designed chimeric oligonucleotide primer to amplify a portion of Flavobacterium species DNA. "nucleotides 18 to 20 are ribonucleotides-other nucleotides are deoxyribonucleotides"
SEQ ID NO:63: Designed chimeric oligonucleotide primer to amplify a portion of Flavobacterium species DNA. "nucleotides 18 to 20 are ribonucleotides-other nucleotides are deoxyribonucleotides"
SEQ ID NO:64: Designed chimeric oligonucleotide primer to amplify a portion of vero toxin 2-encoding sequence from hemorrhagic Escherichia coli O-157. "nucleotides 19 to 21 are ribonucleotides-nucleotide 18 is inosine-other nucleotides are deoxyribonucleotides"

SEQ ID NO:65: Designed chimeric oligonucleotide primer to amplify a portion of vero toxin 2-encoding sequence from hemorrhagic Escherichia coli O-157. "nucleotides 19 to 21 are ribonucleotides-nucleotide 17 is inosine other nucleotides are deoxyribonucleotides"

SEQ ID NO:66: Designed chimeric oligonucleotide primer to amplify a portion of vero toxin 2-encoding sequence from hemorrhagic Escherichia coli O-157. "nucleotides 19 to 21 are ribonucleotides-nucleotide 16 is inosine-other nucleotides are deoxyribonucleotides"

SEQ ID NO:67: Designed chimeric oligonucleotide primer to amplify a portion of vero toxin 2-encoding sequence from hemorrhagic Escherichia coli O-157. "nucleotides 18 to 20 are ribonucleotides-nucleotide 17 is inosine-other nucleotides are deoxyribonucleotides"

SEQ ID NO:68: Designed chimeric oligonucleotide primer to amplify a portion of vero toxin 2-encoding sequence from hemorrhagic Escherichia coli O-157. "nucleotides 18 to 20 are ribonucleotides-nucleotide 16 is inosine-other nucleotides are deoxyribonucleotides"

SEQ ID NO:69: Designed chimeric oligonucleotide primer to amplify a portion of vero toxin 2-encoding sequence from hemorrhagic Escherichia coli O-157. "nucleotides 18 to 20 are ribonucleotides-nucleotide 15 is inosine-other nucleotides are deoxyribonucleotides"

SEQ ID NO:70: Designed chimeric oligonucleotide primer to amplify a portion of vero toxin 2-encoding sequence from hemorrhagic Escherichia coli O-157. "nucleotides 9 to 11 and 19 to 21 are ribonucleotides-other nucleotides are deoxyribonucleotides"

SEQ ID NO:71: Designed chimeric oligonucleotide primer to amplify a portion of vero toxin 2-encoding sequence from hemorrhagic Escherichia coli O-157. "nucleotides 8 to 10 and 18 to 20 are ribonucleotides-other nucleotides are deoxyribonucleotides"

SEQ ID NO:72: Designed chimeric oligonucleotide primer to amplify a portion of vero toxin 2-encoding sequence from hemorrhagic Escherichia coli O-157. "nucleotides 18 to 20 are ribonucleotides-other nucleotides are deoxyribonucleotides"

SEQ ID NO:73: Designed oligonucleotide probe to detect a DNA fragment amplifying a portion of vero toxin 2-encoding sequence from hemorrhagic Escherichia coli O-157.

SEQ ID NO:74: Designed chimeric oligonucleotide primer to amplify a portion of iNOS-encoding sequence from mouse. "nucleotides 18 to 20 are ribonucleotides-other nucleotides are deoxyribonucleotides"

SEQ ID NO:75: Designed chimeric oligonucleotide primer to amplify a portion of iNOS-encoding sequence from mouse. "nucleotides 17 to 19 are ribonucleotides-other nucleotides are deoxyribonucleotides"

SEQ ID NO:76: Designed oligonucleotide primer to amplify a portion of iNOS-encoding sequence from mouse.

SEQ ID NO:77: Designed oligonucleotide primer to amplify a portion of iNOS-encoding sequence from mouse

SEQ ID NO:78: Designed oligonucleotide primer designated as GMO-PCR-F 20mer

SEQ ID NO:79: Designed oligonucleotide primer designated asGMO-PCR-R 20mer

SEQ ID NO:80: Designed chimeric oligonucleotide primer designated as GMO-S1 20mer. "nucleotides 19 to 20 are ribonucleotides-other nucleotides are deoxyribonucleotides"

SEQ ID NO:81: Designed oligonucleotide primer designated as GMO-S2 20mer. "nucleotides 19 to 20 are ribonucleotides-other nucleotides are deoxyribonucleotides"

SEQ ID NO:82: Designed oligonucleotide primer designated as GMO-A1 20mer. "nucleotides 19 to 20 are ribonucleotides-other nucleotides are deoxyribonucleotides"

SEQ ID NO:83: Designed oligonucleotide primer designated as GMO-A2 20 mer. "nucleotides 19 to 20 are ribonucleotides-other nucleotides are deoxyribonucleotides"

SEQ ID NO:84: Designed chimeric oligonucleotide primer to amplify a portion of vero toxin 2-encoding sequence from hemorrhagic Escherichia coli O-157. "nucleotides 18 to 20 are (alpha-thio)ribonucleotides-other nucleotides are deoxyribonucleotides"

SEQ ID NO:85: Designed chimeric oligonucleotide primer to amplify a portion of vero toxin 2-encoding sequence from hemorrhagic Escherichia coli O-157. "nucleotides 18 to 20 are (alpha-thio)ribonucleotides-other nucleotides are deoxyribonucleotides"

SEQ ID NO:86: Designed chimeric oligonucleotide primer to amplify a portion of INOS-encoding sequence from mouse."nucleotides 20 to 22 are ribonucleotides-other nucleotides are deoxyribonucleotides"

SEQ ID NO:87: Designed chimeric oligonucleotide primer to amplify a portion of INOS-encoding sequence from mouse."nucleotides 20 to 22 are ribonucleotides-other nucleotides are deoxyribonucleotides"

SEQ ID NO:88: Designed oligonucleotide primer to amplify a portion of INOS-encoding sequence from mouse.

SEQ ID NO:89: Designed oligonucleotide primer to amplify a portion of INOS-encoding sequence from mouse.

SEQ ID NO:90: Designed chimeric oligonucleotide primer to amplify a portion of lambda DNA."nucleotides 18 to 20 are ribonucleotides-other nucleotides are deoxyribonucleotides"

SEQ ID NO:91: Designed chimeric oligonucleotide primer to amplify a portion of lambda DNA."nucleotides 19 to 21 are ribonucleotides-other nucleotides are deoxyribonucleotides"

SEQ ID NO:92: Designed chimeric oligonucleotide primer to amplify a portion of INOS-encoding sequence from mouse."nucleotides 21 to 23 are ribonucleotides-other nucleotides are deoxyribonucleotides"
SEQ ID NO:93: Designed chimeric oligonucleotide primer to amplify a portion of INOS-encoding sequence from mouse."nucleotides 20 to 22 are ribonucleotides-other nucleotides are deoxyribonucleotides"
SEQ ID NO:94: Designed chimeric oligonucleotide primer to amplify a portion of pDON-AI DNA."nucleotides 17 to 19 are ribonucleotides-other nucleotides are deoxyribonucleotides"
SEQ ID NO:95: Designed chimeric oligonucleotide primer to amplify a portion of pDON-AI DNA."nucleotides 19 to 21 are ribonucleotides-other nucleotides are deoxyribonucleotides"
SEQ ID NO:96: Designed chimeric oligonucleotide primer to amplify a portion of HPV DNA."nucleotides 19 to 21 are ribonucleotides-other nucleotides are deoxyribonucleotides"
SEQ ID NO:97: Designed chimeric oligonucleotide primer to amplify a portion of HPV DNA."nucleotides 19 to 21 are ribonucleotides-other nucleotides are deoxyribonucleotides"
SEQ ID NO:98: Designed oligonucleotide probe to detect a DNA fragment amplifying a portion of HPV DNA.
SEQ ID NO:99: Designed oligonucleotide primer to amplify a portion of HCV.
SEQ ID NO:100: Designed oligonucleotide primer to amplify a portion of HCV.
SEQ ID N0:101: Designed chimeric oligonucleotide primer to amplify a portion of HCV."nucleotides 19 to 21 are ribonucleotides-other nucleotides are deoxyribonucleotides"
SEQ ID NO:102: Designed chimeric oligonucleotide primer to amplify a portion of HCV."nucleotides 16 to 18 are ribonucleotides-other nucleotides are deoxyribonucleotides"
SEQ ID NO:103: Designed oligonucleotide probe to detect a DNA fragment amplifying portion of HCV.
SEQ ID NO:104: Designed chimeric oligonucleotide primer to amplify a portion of adenovirus."nucleotides 19 to 21 are ribonucleotides-other nucleotides are deoxyribonucleotides"
SEQ ID NO:105: Designed chimeric oligonucleotide primer to amplify a portion of adenovirus."nucleotides 19 to 21 are ribonucleotides-other nucleotides are deoxyribonucleotides"
SEQ ID NO:106: Designed chimeric oligonucleotide primer to amplify a portion of adenovirus."nucleotides 19 to 21 are ribonucleotides-other nucleotides are deoxyribonucleotides"
SEQ ID NO:107: Designed oligonucleotide primer to amplify a portion of adenovirus
SEQ ID NO:108: Designed oligonucleotide primer to amplify a portion of adenovirus.
SEQ ID NO:109: Designed oligonucleotide primer to amplify a portion of viroid CSVd.
SEQ ID NO:110: Designed oligonucleotide primer to amplify a portion of viroid CSVd.
SEQ ID NO:111: Designed oligonucleotide primer to amplify a portion of pDON-AI DNA.
SEQ ID NO:112: Designed oligonucleotide primer to amplify a portion of pDON-AI DNA.
SEQ ID NO:113: Designed chimeric oligonucleotide primer to amplify a portion of verotoxin-1 encoding sequence from hemorrhagic Escherichia coli O-157."nucleotides 18 to 20 are ribonucleotides-other nucleotides are deoxyribonucleotides"
SEQ ID NO:114: Designed chimeric oligonucleotide primer to amplify a portion of verotoxin-1 encoding sequence from hemorrhagic Escherichia coli O-157."nucleotides 18 to 20 are ribonucleotides-other nucleotides are deoxyribonucleotides"
SEQ ID NO:115: Designed oligonucleotide probe to detect a DNA fragment amplifying a portion of verotoxin-1 encoding sequence from hemorrhagic Escherichia coli O-157.
SEQ ID NO:116: Designed chimeric oligonucleotide primer to amplify a portion of botulinum toxin A encoding sequence from Clostridium botulinum."nucleotides 19 to 21 are ribonucleotides-other nucleotides are deoxyribonucleotides"
SEQ ID NO:117: Designed chimeric oligonucleotide primer to amplify a portion of botulinum toxin A encoding sequence from Clostridium botulinum."nucleotides 21 to 23 are ribonucleotides-other nucleotides are deoxyribonucleotides"
SEQ ID NO:118: Designed oligonucleotide probe to detect a DNA fragment amplifying a portion of botulinum toxin A encoding sequence from Clostridium botulinum.
SEQ ID NO:119: Designed chimeric oligonucleotide primer to amplify a portion of viroid CSVd."nucleotides 19 to 21 are ribonucleotides-other nucleotides are deoxyribonucleotides"
SEQ ID NO:120: Designed chimeric oligonucleotide primer to amplify a portion of viroid CSVd."nucleotides 18 to 20 are ribonucleotides-other nucleotides are deoxyribonucleotides"
SEQ ID NO:121: Designed oligonucleotide probe to detect a DNA fragment amplifying a portion of viroid CSVd.
SEQ ID NO:122: Designed chimeric oligonucleotide primer to amplify a portion of viroid CSVd."nucleotides 19 to 21 are ribonucleotides-other nucleotides are deoxyribonucleotides"
SEQ ID NO:123: Designed chimeric oligonucleotide primer to amplify a portion of viroid CSVd."nucleotides 19 to 21 are ribonucleotides-other nucleotides are deoxyribonucleotides"
SEQ ID NO:124: Designed oligonucleotide primer to amplify a portion of viroid CSVd.

SEQ ID NO:125: Designed oligonucleotide primer to amplify a portion of viroid CSVd.
SEQ ID NO:126: Designed chimeric oligonucleotide primer to amplify a portion of c-ki-ras oncogene."nucleotides 18 to 20 are ribonucleotides-other nucleotides are deoxyribonucleotides"
SEQ ID NO:127: Designed chimeric oligonucleotide primer to amplify a portion of c-ki-ras oncogene."nucleotides 18 to 20 are ribonucleotides-other nucleotides are deoxyribonucleotides"
SEQ ID NO:128: Designed oligonucleotide primer to amplify a portion of c-ki-ras oncogene.
SEQ ID NO:129: Designed oligonucleotide primer to amplify a portion of c-ki-ras oncogene.
SEQ ID NO:130: Designed chimeric oligonucleotide primer to amplify a portion of verotoxin-1 encoding sequence from hemorrhagic Escherichia coli O-157."nucleotides 18 to 20 are ribonucleotides-other nucleotides are deoxyribonucleotides"
SEQ ID NO:131: Designed chimeric oligonucleotide primer to amplify a portion of verotoxin-1 encoding sequence from hemorrhagic Escherichia coli O-157."nucleotides 18 to 20 are ribonucleotides-other nucleotides are deoxyribonucleotides"
SEQ ID NO:132: Designed oligonucleotide primer to amplify a portion of INOS-encoding sequence from mouse.
SEQ ID NO:133: Designed oligonucleotide primer to amplify a portion of INOS-encoding sequence from mouse.
SEQ ID NO:134: Designed oligonucleotide primer designated as pUC19 upper 150 to amplify a portion of plasmid pUC19.
SEQ ID NO:135: Designed oligonucleotide primer designated as pUC19 lower NN to amplify a portion of plasmid pUC19.
SEQ ID NO:136: Designed chimeric oligonucleotide primer designated as SEA-1 to amplify a portion of Staphylococcus aureus. "nucleotides 19 to 21 are ribonucleotides-other nucleotides are deoxyribonucleotides"
SEQ ID NO:137: Designed chimeric oligonucleotide primer designated as SEA-2 to amplify a portion of Staphylococcus aureus. "nucleotides 19 to 21 are ribonucleotides-other nucleotides are deoxyribonucleotides"
SEQ ID NO:138: Designed chimeric oligonucleotide primer designated as HCV-F3 to amplify a portion of HCV. "nucleotides 17 to 19 are ribonucleotides-other nucleotides are deoxyribonucleotides"
SEQ ID NO:139: Designed chimeric oligonucleotide primer designated as HCV-R1 to amplify a portion of HCV. "nucleotides 16 to 18 are ribonucleotides-other nucleotides are deoxyribonucleotides"
SEQ ID NO:140: Designed oligonucleotide primer designated as MF2 to amplify a portion of pUC19 plasmid DNA.
SEQ ID NO:141: Designed oligonucleotide primer designated as MR1 to amplify a portion of pUC19 plasmid DNA.
SEQ ID NO:142:"Designed oligonucleotide primer to amplify a portion of adenovirus.
SEQ ID NO:143: Nucleotide sequence of ORF in RNaseHII gene from Thermotoga maritima.
SEQ ID NO:144: Amino acid sequence of RNaseHII from Thermotoga maritima.
SEQ ID NO:145: Nucleotide sequence of PH1650 from Pyrococcus horikoshii.
SEQ ID NO:146: PCR primer PhoNde for cloning a gene encoding a polypeptide having a RNaseHII activity from Pyrococcus horikoshii
SEQ ID NO:147: PCR primer PhoBam for cloning a gene encoding a polypeptide having a RNaseHII activity from Pyrococcus horikoshii
SEQ ID NO:148: Nucleotide sequence of ORF in RNaseHII gene from Pyrococcus horikoshii.
SEQ ID NO:149: Amino acid sequence of RNaseHII from Pyrococcus horikoshii.
SEQ ID NO:150: Nucleotide sequence of AF0621 from Archaeoglobus fulgidus.
SEQ ID NO:151: PCR primer AfuNde for cloning a gene encoding a polypeptide having a RNaseHII activity from Archaeoglobus fulgidus
SEQ ID NO:152: PCR primer AfuBam for cloning a gene encoding a polypeptide having a RNaseHII activity from Archaeoglobus fulgidus
SEQ ID NO:153: Nucleotide sequence of ORF in RNaseHII gene from Archaeoglobus fulgidus.
SEQ ID NO:154: Amino acid sequence of RNaseHII from Archaeoglobus fulgidus.
SEQ ID NO:155: Designed chimeric oligonucleotide primer designated as MTIS2F to amplify a portion of Mycobacterium tuberculosis DNA."nucleotides 16 to 18 are ribonucleotides-other nucleotides are deoxyribonucleotides.".
SEQ ID NO:156: Designed chimeric oligonucleotide primer designated as MTIS2R to amplify a portion of Mycobacterium tuberculosis DNA."nucleotides 19 to 21 are ribonucleotides-other nucleotides are deoxyribonucleotides.".
SEQ ID NO:157: Designed chimeric oligonucleotide primer designated as CT2F to amplify a portion of Chlamydia trachomatis cryptic plasmid DNA."nucleotides 18 to 20 are ribonucleotides-other nucleotides are deoxyribonucleotides.".
SEQ ID NO:158: Designed chimeric oligonucleotide primer designated as CT2R to amplify a portion of Chlamydia trachomatis cryptic plasmid DNA."nucleotides 16 to 18 are ribonucleotides-other nucleotides are deoxyribonucleotides.".

SEQ ID NO:159: Designed chimeric oligonucleotide primer designated as K-F-1033(60) to amplify a portion of Mycobacterium tuberculosis DNA."nucleotides 17 to 19 are ribonucleotides-other nucleotides are deoxyribonucleotides.".
SEQ ID NO:160: Designed chimeric oligonucleotide primer designated as K-R-1133(62) to amplify a portion of Mycobacterium tuberculosis DNA."nucleotides 18 to 20 are ribonucleotides-other nucleotides are deoxyribonucleotides.".
SEQ ID NO:161: Designed chimeric oligonucleotide primer designated as K-F-1033(68) to amplify a portion of Mycobacterium tuberculosis DNA."nucleotides 20 to 22 are ribonucleotides-other nucleotides are deoxyribonucleotides.".
SEQ ID NO:162: Designed chimeric oligonucleotide primer designated as K-R-1133(68) to amplify a portion of Mycobacterium tuberculosis DNA."nucleotides 20 to 22 are ribonucleotides-other nucleotides are deoxyribonucleotides.".
SEQ ID NO:163: Designed oligonucleotide primer designated as F26 to amplify a portion of Mycobacterium tuberculosis DNA.
SEQ ID NO:164: Designed oligonucleotide primer designated as R1310 to amplify a portion of Mycobacterium tuberculosis DNA.
SEQ ID NO:165: Designed chimeric oligonucleotide primer designated as pDON-AI-68-1 to amplify a portion of pDON-AI."nucleotides 20 to 22 are ribonucleotides-other nucleotides are deoxyribonucleotides.".
SEQ ID NO:166: Designed chimeric oligonucleotide primer designated as pDON-AI-68-2 to amplify a portion of pDON-AI."nucleotides 21 to 23 are ribonucleotides-other nucleotides are deoxyribonucleotides.".
SEQ ID NO:167: Nucleotide sequence of Homo sapiens proto-oncogene Wn t-5a
SEQ ID NO:168: Nucleotide sequence of Homo sapiens ribosomal protein S5
SEQ ID NO:169: Nucleotide sequence of Homo sapiens diaphorase
SEQ ID NO:170: Nucleotide sequence of Human protocadherin
SEQ ID NO:171: Designed oligonucleotide for making of pIC62.
SEQ ID NO:172: Designed chimeric oligonucleotide primer designated as ICAN2."nucleotides 19 to 20 are ribonucleotides-other nucleotides are deoxyribonucleotides.".
SEQ ID NO:173: Designed chimeric oligonucleotide primer designated as ICAN6."nucleotides 19 to 20 are ribonucleotides-other nucleotides are deoxyribonucleotides.".
SEQ ID NO:174: Designed oligonucleotide primer designated as ICAN2 DNA.
SEQ ID NO:175: Designed oligonucleotide primer designated as ICAN6 DNA.
SEQ ID NO:176: Designed oligonucleotide primer to amplify a portion of ribosomal protein S18-encoding sequence from mouse.
SEQ ID NO:177: Designed oligonucleotide primer to amplify a portion of ribosomal protein S18-encoding sequence from mouse.
SEQ ID NO:178: Designed oligonucleotide primer to amplify a portion of transferrin receptor (TFR)-encoding sequence from mouse.
SEQ ID NO:179: Designed oligonucleotide primer to amplify a portion of transferrin receptor (TFR)-encoding sequence from mouse.
SEQ ID NO:180: Designed oligonucleotide primer to amplify a portion of stromal cell derived factor 4 (Sdf4)-encoding sequence from mouse.
SEQ ID NO:181: Designed oligonucleotide primer to amplify a portion of stromal cell derived factor 4 (Sdf4)-encoding sequence from mouse.
SEQ ID NO:182: Designed oligonucleotide primer to amplify a portion of cytoplasmic beta-actin encoding sequence from mouse.
SEQ ID NO:183: Designed oligonucleotide primer to amplify a portion of cytoplasmic beta-actin encoding sequence from mouse.
SEQ ID NO:184: Designed oligonucleotide primer to amplify a portion of ornithine decarboxylase-encoding sequence from mouse.
SEQ ID NO:185: Designed oligonucleotide primer to amplify a portion of ornithine decarboxylase-encoding sequence from mouse.
SEQ ID NO:186: Designed oligonucleotide primer to amplify a portion of hypoxanthine guanine phosphoribosyl transferase (HPRT)-encoding sequence from mouse.
SEQ ID NO:187: Designed oligonucleotide primer to amplify a portion of hypoxanthine guanine phosphoribosyl transferase (HPRT)-encoding sequence from mouse.
SEQ ID NO:188: Designed oligonucleotide primer to amplify a portion of tyrosine 3-monooxygenase encoding sequence from mouse.
SEQ ID NO:189: Designed oligonucleotide primer to amplify a portion of tyrosine 3-monooxygenase encoding sequence from mouse.

SEQ ID NO:190: Designed oligonucleotide primer designated as MCS-F.

SEQ ID NO:191: Designed oligonucleotide primer designated as MCS-R

SEQ ID NO:192: Designed chimeric oligonucleotide primer designated as MF2N3(24). "nucleotides 22 to 24 are ribonucleoitdes-other nucleotides are deoxyribonucleotides"

SEQ ID NO:193: Designed chimeric oligonucleotide primer designated as MR1N3(24). "nucleotides 22 to 24 are ribonucleoitdes-other nucleotides are deoxyribonucleotides"

SEQ ID NO:194: Designed chimeric oligonucleotide primer designated as MTIS2F-16 to amplify a portion of Mycobacterium tuberculosis DNA."nucleotides 14 to 16 are ribonucleotides-other nucleotides are deoxyribonucleotides.

SEQ ID NO:195: Designed chimeric oligonucleotide primer designated as MTIS2R-ACC to amplify a portion of Mycobacterium tuberculosis DNA."nucleotides 18 to 20 are ribonucleotides-other nucleotides are deoxyribonucleotides."

SEQ ID NO:196: Designed oligonucleotide primer designated as MTIS-PCR-F-2 to amplify a portion of Mycobacterium tuberculosis DNA

SEQ ID NO:197: Designed oligonucleotide primer designated as MTIS-PCR-R-2 to amplify a portion of Mycobacterium tuberculosis DNA

SEQ ID NO:198: Designed oligonucleotide primer designated as SP6-HCV-F to amplify a portion of HCV

SEQ ID NO:199: Designed oligonucleotide primer designated as SP6-HCV-R to amplify a portion of HCV

SEQ ID NO:200: Designed chimeric oligonucleotide primer designated as HCV-A S to amplify a portion of HCV. "nucleotides 18 to 20 are ribonucleotides-other nucleotides are deoxyribonucleotides."

SEQ ID NO:201: Designed chimeric oligonucleotide primer designated as HCV-A A to amplify a portion of HCV. "nucleotides 18 to 20 are ribonucleotides-other nucleotides are deoxyribonucleotides.

## Claims

**1.** A method for amplifying a nucleic acid, the method comprising:

(a) preparing a reaction mixture by mixing a nucleic acid as a template, a deoxyribonucleotide triphosphate, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer; and
(b) incubating the reaction mixture for a sufficient time to generate a reaction product.

**2.** The method according to claim 1, wherein the reaction mixture further contains a chimeric oligonucleotide primer having a sequence substantially homologous to the nucleotide sequence of the nucleic acid as the template.

**3.** A method for amplifying a nucleic acid, the method comprising:

(a) treating a nucleic acid as a template with at least one primer that is substantially complementary to the nucleotide sequence of the nucleic acid and a DNA polymerase to synthesize a primer-extended strand that is complementary to the template and synthesize a double-stranded nucleic acid, wherein the primer is a chimeric oligonucleotide primer containing a ribonucleotide as well as at least one selected from the group consisting of a deoxyribonucleotide and a nucleotide analog, the ribonucleotide being positioned at the 3'-terminus or on the 3'-terminal side of the primer;
(b) extending a nucleotide sequence that is complementary to the double-stranded nucleic acid as a template obtained in the previous step using a DNA polymerase having a strand displacement activity in the presence of an RNase H to effect a strand displacement and synthesize a displaced strand and a double-stranded nucleic acid; and
(c) reusing in step (b) the double-stranded nucleic acid obtained in step (b) as a template.

**4.** A method for amplifying a nucleic acid using at least two primers, the method comprising:

(a) treating a nucleic acid as a template with at least one primer that is substantially complementary to the nucleotide sequence of the nucleic acid and a DNA polymerase to synthesize a primer-extended strand that is complementary to the template, wherein the primer is a chimeric oligonucleotide primer containing a ribonucleotide as well as at least one selected from the group consisting of a deoxyribonucleotide and a nucleotide analog, the ribonucleotide being positioned at the 3'-terminus or on the 3'-terminal side of the primer;
(b) extending a nucleotide sequence that is complementary to the double-stranded nucleic acid as a template obtained in the previous step using a DNA polymerase having a strand displacement activity in the presence of an RNase H to effect a strand displacement and synthesize a displaced strand and a double-stranded nucleic acid;
(c) reusing in step (b) the double-stranded nucleic acid obtained in step (b) as a template;
(d) treating a displaced strand obtained in step (b) as a template with at least one primer that is different from that used in step (a) and a DNA polymerase to synthesize a primer-extended strand that is complementary to the displaced strand, wherein the primer that is different from that used in step (a) is a chimeric oligonucleotide primer that is substantially complementary to the nucleotide sequence of the displaced strand and contains a ribonucleotide as well as at least one selected from the group consisting of a deoxyribonucleotide and a nucleotide analog, the ribonucleotide being positioned at the 3'-terminus or on the 3'-terminal side of the primer;
(e) extending a nucleotide sequence that is complementary to the double-stranded nucleic acid as a template obtained in the previous step using a DNA polymerase having a strand displacement activity in the presence of an RNase H to effect a strand displacement and synthesize a displaced strand and a double-stranded nucleic acid; and
(f) reusing in step (e) the double-stranded nucleic acid obtained in step (e) as a template.

**5.** The method according to claim 3 or 4, wherein the DNA polymerase is at least one DNA polymerase having a strand displacement activity.

**6.** A method for amplifying a nucleic acid, the method comprising:

(a) treating a double-stranded nucleic acid as a template with two primers that are substantially complementary to the nucleotide sequences of the respective strands of the double-stranded nucleic acid and a DNA polymerase having a strand displacement activity to synthesize primer-extended strands that are complementary to the template and obtain a double-stranded nucleic acid consisting of the synthesized primer-extended strands being annealed each other, wherein each primer is a chimeric oligonucleotide primer containing a ribonucleotide as well as at least one selected from the group consisting of a deoxyribonucleotide and a nucleotide analog, the ribonucleotide being positioned at the 3'-terminus or on the 3'-terminal side of the primer;
(b) cleaving the sites that contain the ribonucleotide of the double-stranded nucleic acid consisting of the primer-extended strands obtained in step (a) with the endonuclease; and
(c) extending nucleotide sequences that are complementary to the template using a DNA polymerase having a strand displacement activity from the 3'-termini of the respective primer portions of the double-stranded nucleic acid in which the primer-extended strands are cleaved obtained in step (b) to effect strand displacements and obtain a double-stranded nucleic acid consisting of the template and the primer-extended strand.

**7.** A method for amplifying a nucleic acid, the method comprising:

(a) treating a double-stranded nucleic acid as a template with two primers that are substantially complementary to the nucleotide sequences of the respective strands of the double-stranded nucleic acid and a DNA polymerase having a strand displacement activity to synthesize primer-extended strands that are complementary to the template and obtain a double-stranded nucleic acid consisting of the synthesized primer-extended strands being annealed each other, wherein each primer is a chimeric oligonucleotide primer containing a ribonucleotide as well as at least one selected from the group consisting of a deoxyribonucleotide and a nucleotide analog, the ribonucleotide being positioned at the 3'-terminus or on the 3'-terminal side of the primer;
(b) cleaving the sites that contain the ribonucleotide of the double-stranded nucleic acid consisting of the primer-extended strands obtained in step (a) with the endonuclease; and
(c) extending nucleotide sequences that are complementary to the template using a DNA polymerase having a strand displacement activity from the 3'-termini of the respective primer portions of the double-stranded nucleic acid in which the primer-extended strands are cleaved obtained in step (b) to effect strand displacements and obtain a double-stranded nucleic acid consisting of the primer-extended strands being annealed each other.

**8.** A method for amplifying a nucleic acid, the method comprising:

(a) treating a double-stranded nucleic acid as a template with two primers that are substantially complementary to the nucleotide sequences of the respective strands of the double-stranded nucleic acid and a DNA polymerase having a strand displacement activity to synthesize primer-extended strands that are complementary to the template and obtain a double-stranded nucleic acid consisting of the synthesized primer-extended strands being annealed each other, wherein each primer is a chimeric oligonucleotide primer containing a ribonucleotide as well as at least one selected from the group consisting of a deoxyribonucleotide and a nucleotide analog, the ribonucleotide being positioned at the 3'-terminus or on the 3'-terminal side of the primer;
(b) cleaving the sites that contain the ribonucleotide of the double-stranded nucleic acid consisting of the primer-extended strands obtained in step (a) with the endonuclease;
(c) extending nucleotide sequences that are complementary to the template using a DNA polymerase having a strand displacement activity from the 3'-termini of the respective primer portions of the double-stranded nucleic acid in which the primer-extended strands are cleaved obtained in step (b) to effect strand displacements and obtain a double-stranded nucleic acid consisting of the primer-extended strands being annealed each other and a double-stranded nucleic acid consisting of the templates being annealed each other to which the two primers in step (a) are annealed;
(d) extending nucleotide sequences that are complementary to the template using a DNA polymerase having a strand displacement activity from the 3'-termini of the respective primer portions of the double-stranded nucleic acid to which the two primers are annealed obtained in step (c) to effect strand displacements and obtain a double-stranded nucleic acid consisting of the primer-extended strands being annealed each other and a double-stranded nucleic acid consisting of the templates being annealed each other to which the two primers in step (a) are annealed; and
(e) reusing in step (d) the double-stranded nucleic acid to which the two primers are annealed obtained in step (d).

**9.** A method for amplifying a nucleic acid, the method comprising:

(a) treating a double-stranded nucleic acid as a template with two primers that are substantially complementary to the nucleotide sequences of the respective strands of the double-stranded nucleic acid and a DNA polymerase having a strand displacement activity to synthesize primer-extended strands that are complementary to the template and obtain a double-stranded nucleic acid consisting of the synthesized primer-extended strands being annealed each other, wherein each primer is a chimeric oligonucleotide primer containing a ribonucleotide as well as at least one selected from the group consisting of a deoxyribonucleotide and a nucleotide analog, the ribonucleotide being positioned at the 3'-terminus or on the 3'-terminal side of the primer;
(b) cleaving the sites that contain the ribonucleotide of the double-stranded nucleic acid consisting of the primer-extended strands obtained in step (a) with the endonuclease;
(c) extending nucleotide sequences that are complementary to the template using a DNA polymerase having a strand displacement activity from the 3'-termini of the respective primer portions of the double-stranded nucleic acid in which the primer-extended strands are cleaved obtained in step (b) to effect strand displacements and obtain a double-stranded nucleic acid consisting of the primer-extended strands being annealed each other and a double-stranded nucleic acid consisting of the templates being annealed each other to which the two primers in step (a) are annealed;
(d) extending nucleotide sequences that are complementary to the template using a DNA polymerase having a strand displacement activity from the 3'-termini of the respective primer portions of the double-stranded nucleic acid to which the two primers are annealed obtained in step (c) to effect a strand displacement and obtain a double-stranded nucleic acid consisting of the template and the primer-extended strand;
(e) cleaving the sites that contain the ribonucleotide of the double-stranded nucleic acid consisting of the template and the primer-extended strand obtained in step (d) with the endonuclease; and
(f) extending a nucleotide sequence that is complementary to the template using a DNA polymerase having a strand displacement activity from the 3'-terminus of the primer portion of the double-stranded nucleic acid in which the primer-extended strand is cleaved obtained in step (e) to synthesize a displaced strand.

**10.** The method according to any one of claims 6 to 9, wherein the endonuclease is an endoribonuclease.

**11.** The method according to claim 10, wherein the endoribonuclease is an RNase H.

**12.** The method according to any one of claims 1 to 5 and 11, wherein the RNase H is selected from the group consisting of an RNase H from Escherichia coli, an RNase H from a bacterium of genus Thermotoga, an RNase H from a bacterium of genus Thermus, an RNase H from a bacterium of genus Pyrococcus, an RNase H from a bacterium of genus Archaeoglobus and an RNase H from a bacterium of genus Bacillus.

**13.** The method according to any one of claims 1 to 12, wherein the length of the region of the nucleic acid to be amplified is 200 bp or shorter.

**14.** The method according to any one of claims 1 to 13, wherein a chimeric oligonucleotide primer represented by general formula below is used:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns, and the nucleotide at the 3'-terminus may be modified such that extension from the 3'-terminus by the action of the DNA polymerase does not take place).

**15.** The method according to claim 14, wherein c is 0.

**16.** The method according to claim 14 or 15, wherein the nucleotide analog is deoxyriboinosine nucleotide or deoxyribouracil nucleotide and the modified ribonucleotide is ($\alpha$-S) ribonucleotide.

**17.** The method according to any one of claims 14 to 16, wherein the DNA extension reaction is conducted at a DNA extension reaction temperature suitable for the chimeric oligonucleotide primer as defined in any one of claims 14 to 16.

**18.** The method according to any one of claims 1 to 17, which comprises annealing the nucleic acid as the template to the chimeric oligonucleotide primer that is substantially complementary to the nucleotide sequence of the nucleic acid in an annealing solution containing a substance that enhances the annealing of the nucleic acid to the primer.

**19.** The method according to claim 18, wherein the annealing solution contains spermidine and/or propylenediamine.

**20.** The method according to claim 18 or 19, wherein the annealing is conducted by incubating the annealing solution containing the nucleic acid as the template and the chimeric oligonucleotide primer that is substantially complementary to the nucleotide sequence of the nucleic acid at 90°C or above and then cooling the solution to a temperature at which the amplification reaction is conducted or below.

**21.** The method according to any one of claims 1 to 20, wherein the amplification reaction is conducted in a buffer containing a buffering component selected from the group consisting of Bicine and HEPES.

**22.** The method according to any one of claims 1 to 21, wherein the DNA polymerase having a strand displacement activity is selected from the group consisting of Klenow fragment of DNA polymerase I from Escherichia coli, Bst DNA polymerase lacking 5'→3' exonuclease from Bacillus stearothermophilus and Bca DNA polymerase lacking 5'→3' exonuclease from Bacillus caldotenax.

**23.** The method according to any one of claims 1 to 5 and 11 to 22, wherein the DNA polymerase having a strand displacement activity is Bca DNA polymerase lacking 5'→3' exonuclease from Bacillus caldotenax and the RNase H is selected from the group consisting of an RNase H from Escherichia coli, an RNase H from a bacterium of genus Pyrococcus and an RNase H from a bacterium of genus Archaeoglobus.

**24.** The method according to claim 23, wherein the RNase H is type I RNase H from Escherichia coli, or type II RNase H from a bacterium of genus Pyrococcus or a bacterium of genus Archaeoglobus.

**25.** The method according to any one of claims 1 to 24, wherein a DNA polymerase having an endonuclease activity is used.

**26.** The method according to claim 25, wherein the DNA polymerase is Bca DNA polymerase lacking 5'→3' exonuclease from Bacillus caldotenax and the Bca DNA polymerase is used in the presence of a substance that allows the endonuclease activity of the Bca DNA polymerase to express.

**27.** The method according to claim 26, wherein the substance that allows the endonuclease activity of the DNA polymerase to express is a manganese ion.

**28.** The method according to any one of claims 1 to 27, wherein the amplification reaction is conducted in the presence of a substance that inhibits the reverse transcription activity of the DNA polymerase.

**29.** The method according to claim 28, wherein the substance that inhibits the reverse transcription activity of the DNA polymerase is phosphonoformic acid.

**30.** The method according any one of claims 1 to 29, wherein the nucleic acid as the template is a single-stranded DNA or a double-stranded DNA.

**31.** The method according to claim 30, which is conducted after converting a double-stranded DNA as the template into single-stranded DNAs.

**32.** The method according to claim 30 or 31, wherein the nucleic acid as the template is a cDNA obtained by a reverse transcription reaction using an RNA as a template.

**33.** The method according to claim 32, which is conducted after synthesizing a cDNA by a reverse transcription reaction using an RNA as a template.

**34.** The method according to claim 32 or 33, wherein a DNA polymerase having a reverse transcriptase activity is used as a reverse transcriptase.

**35.** The method according to any one of claims 31 to 34, wherein the reverse transcription reaction and the synthesis of the extended strand that is complementary to the template are conducted using one DNA polymerase having both a reverse transcriptase activity and a strand displacement activity.

**36.** The method according to claim 35, wherein the DNA polymerase is Bst DNA polymerase lacking 5'→3' exonuclease from Bacillus stearothermophilus or Bca DNA polymerase lacking 5'→3' exonuclease from Bacillus caldotenax.

**37.** The method according to any one of claims 1 to 36, wherein the nucleic acid amplification reaction is conducted under isothermal conditions.

**38.** The method according to any one of claims 1 to 37, which is conducted in the presence of a deoxyribonucleotide triphosphate analog.

**39.** The method according to claim 38, wherein the deoxyribonucleotide triphosphate analog is deoxyuridine triphosphate or a derivative thereof.

**40.** A composition for amplifying a nucleic acid which contains:

(a) at least one primer that is substantially complementary to a nucleotide sequence of a nucleic acid as a template, wherein the primer is a chimeric oligonucleotide primer that contains a ribonucleotide as well as at least one selected from the group consisting of a deoxyribonucleotide and a nucleotide analog, the ribonucleotide being positioned at the 3'-terminus or on the 3'-terminal side of the primer;
(b) an endonuclease; and
(c) a DNA polymerase having a strand displacement activity.

**41.** A composition for amplifying a nucleic acid which contains:

(a) at least two primers that are substantially complementary to nucleotide sequences of respective strands of a double-stranded nucleic acid as a template, wherein each primer is a chimeric oligonucleotide primer that contains a ribonucleotide as well as at least one selected from the group consisting of a deoxyribonucleotide and a nucleotide analog, the ribonucleotide being positioned at the 3'-terminus or on the 3'-terminal side of the primer;
(b) an endonuclease; and
(c) a DNA polymerase having a strand displacement activity.

**42.** A composition for amplifying a nucleic acid obtained by mixing a nucleic acid as a template, a deoxyribonucleotide triphosphate, a DNA polymerase having a strand displacement activity, at least one primer and an endonuclease, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer.

**43.** A composition for amplifying a nucleic acid obtained by mixing a nucleic acid as a template, a deoxyribonucleotide triphosphate, a DNA polymerase having a strand displacement activity, at least two primers and an endonuclease, wherein each primer is a chimeric oligonucleotide primer that is substantially complementary to the nucleotide sequence of each strand of the double-stranded nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer.

**44.** The composition according to any one of claims 40 to 43, wherein the primer is a chimeric oligonucleotide primer represented by general formula below:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns, and the nucleotide at the 3'-terminus may be modified such that extension from the 3'-terminus by the action of the DNA polymerase does not take place).

**45.** The composition according to claim 44, wherein c is 0.

**46.** The composition according to claim 44 or 45, wherein the nucleotide analog is deoxyriboinosine nucleotide or deoxyribouracil nucleotide and the modified ribonucleotide is (α-S) ribonucleotide.

**47.** The composition according to any one of claims 40 to 46, which contains a buffering component suitable for a nucleic acid amplification reaction.

**48.** The composition according to claim 47, which contains a buffering component selected from the group consisting of Bicine and HEPES.

**49.** The composition according to any one of claims 40 to 48, wherein a DNA polymerase selected from the group consisting of Klenow fragment of DNA polymerase I from Escherichia coli, Bst DNA polymerase lacking 5'→3' exonuclease from Bacillus stearothermophilus and Bca DNA polymerase lacking 5'→3' exonuclease from Bacillus caldotenax is used as the DNA polymerase having a strand displacement activity.

**50.** The composition according to any one of claims 40 to 49, wherein the endonuclease is an endoribonuclease.

**51.** The composition according to claim 50, wherein the endoribonuclease is an RNase H.

**52.** The composition according to claim 51, wherein the RNase H is selected from the group consisting of an RNase H from Escherichia coli, an RNase H from a bacterium of genus Thermotoga, an RNase H from a bacterium of genus Thermus, an RNase H from a bacterium of genus Pyrococcus, an RNase H from a bacterium of genus Archaeoglobus and an RNase H from a bacterium of genus Bacillus.

**53.** The composition according to any one of claims 40 to 52, wherein the DNA polymerase having a strand displacement activity is Bca DNA polymerase lacking 5'→3' exonuclease from Bacillus caldotenax and the endonuclease is an RNase H selected from the group consisting of an RNase H from Escherichia coli, an RNase H from a bacterium of genus Pyrococcus and an RNase H from a bacterium of genus Archaeoglobus.

**54.** The composition according to claim 53, wherein the RNase H is type I RNase H from Escherichia coli, or type II RNase H from a bacterium of genus Pyrococcus or a bacterium of genus Archaeoglobus.

**55.** The composition according to any one of claims 40 to 54, wherein a DNA polymerase having an endonuclease activity is used.

**56.** The composition according to claim 55, wherein the DNA polymerase is Bca DNA polymerase lacking 5'→3' exonuclease from Bacillus caldotenax, the composition containing a substance that allows the endonuclease activity of the Bca DNA polymerase to express.

**57.** The composition according to claim 56, wherein the substance that allows the endonuclease activity of the DNA polymerase to express is a manganese ion.

**58.** The composition according to any one of claims 40 to 57, which contains a substance that inhibits the reverse transcription activity of the DNA polymerase.

**59.** The composition according to claim 58, wherein the substance that inhibits the reverse transcription activity of the DNA polymerase is phosphonoformic acid.

**60.** The composition according to any one of claims 40 to 59, which contains a deoxyribonucleotide triphosphate analog.

**61.** The composition according to claim 60, wherein the deoxyribonucleotide triphosphate analog is deoxyuridine triphosphate or a derivative thereof.

**62.** A composition for amplifying a nucleic acid used for the method for amplifying a nucleic acid defined by any one of claims 1 to 5, which contains:

(a) an RNase H; and
(b) a DNA polymerase having a strand displacement activity.

**63.** A composition for amplifying a nucleic acid used for the method for amplifying a nucleic acid defined by any one of claims 6 to 9, which contains:

(a) an endonuclease; and
(b) a DNA polymerase having a strand displacement activity.

**64.** The composition according to claim 63, wherein the endonuclease is an endoribonuclease.

**65.** The composition according to claim 64, wherein the endoribonuclease is an RNase H.

**66.** The composition according to claim 62 or 65, wherein the RNase H is selected from the group consisting of an RNase H from Escherichia coli, an RNase H from a bacterium of genus Thermotoga, an RNase H from a bacterium of genus Thermus, an RNase H from a bacterium of genus Pyrococcus, an RNase H from a bacterium of genus Archaeoglobus and an RNase H from a bacterium of genus Bacillus.

**67.** The composition according to any one of claims 62 to 66, which contains a buffering component suitable for a nucleic acid amplification reaction.

**68.** The composition according to claim 67, which contains a buffering component selected from the group consisting of Bicine and HEPES.

**69.** The composition according to any one of claims 62 to 68, wherein a DNA polymerase selected from the group consisting of Klenow fragment of DNA polymerase I from Escherichia coli, Bst DNA polymerase lacking 5'→3' exonuclease from Bacillus stearothermophilus and Bca DNA polymerase lacking 5'→3' exonuclease from Bacillus caldotenax is used as the DNA polymerase having a strand displacement activity.

**70.** The composition according to claim 62, wherein the DNA polymerase having a strand displacement activity is Bca DNA polymerase lacking 5'→3' exonuclease from Bacillus caldotenax and the RNase H is an RNase H selected from the group consisting of an RNase H from Escherichia coli, an RNase H from a bacterium of genus Pyrococcus and an RNase H from a bacterium of genus Archaeoglobus.

**71.** The composition according to claim 63, wherein the DNA polymerase having a strand displacement activity is Bca DNA polymerase lacking 5'→3' exonuclease from Bacillus caldotenax and the endonuclease is an RNase H selected from the group consisting of an RNase H from Escherichia coli, an RNase H from a bacterium of genus Pyrococcus and an RNase H from a bacterium of genus Archaeoglobus.

**72.** The composition according to any one of claims 62 to 71, wherein a DNA polymerase having an endonuclease activity is used.

**73.** The composition according to claim 72, wherein the DNA polymerase is Bca DNA polymerase lacking 5'→3' exonuclease from Bacillus caldotenax, the composition containing a substance that allows the endonuclease activity of the Bca DNA polymerase to express.

**74.** The composition according to claim 73, wherein the substance that allows the endonuclease activity of the DNA polymerase to express is a manganese ion.

**75.** The composition according to any one of claims 62 to 74, which contains a substance that inhibits the reverse transcription activity of the DNA polymerase.

**76.** The composition according to claim 75, wherein the substance that inhibits the reverse transcription activity of the DNA polymerase is phosphonoformic acid.

**77.** The composition according to any one of claims 62 to 76, which contains a deoxynucleotide triphosphate analog.

**78.** The composition according to claim 77, wherein the deoxynucleotide triphosphate analog is deoxyuridine triphosphate or a derivative thereof.

**79.** A kit for amplifying a nucleic acid used for the method for amplifying a nucleic acid defined by any one of claims 1 to 5, which contains:

(a) an RNase H; and
(b) a DNA polymerase having a strand displacement activity.

**80.** A kit for amplifying a nucleic acid used for the method for amplifying a nucleic acid defined by any one of claims 6 to 9, which contains:

(a) an endonuclease; and
(b) a DNA polymerase having a strand displacement activity.

**81.** The kit according to claim 80, wherein the endonuclease is an endoribonuclease.

**82.** The kit according to claim 81, wherein the endoribonuclease is an RNase H.

**83.** The kit according to claim 79 or 82, wherein the RNase H is selected from the group consisting of an RNase H from Escherichia coli, an RNase H from a bacterium of genus Thermotoga, an RNase H from a bacterium of genus Thermus, an RNase H from a bacterium of genus Pyrococcus, an RNase H from a bacterium of genus Archaeoglobus and an RNase H from a bacterium of genus Bacillus.

**84.** The kit according to any one of claims 79 to 83, which contains a buffer suitable for a nucleic acid amplification reaction.

**85.** The kit according to claim 84, which contains a buffer for nucleic acid amplification containing a buffering component selected from the group consisting of Bicine and HEPES.

**86.** The kit according to any one of claims 79 to 85, which contains an annealing solution containing a substance that enhances the annealing of the nucleic acid as the template to the primer that is substantially complementary to the nucleotide sequence of the nucleic acid.

**87.** The kit according to claim 86, wherein the annealing solution contains spermidine and/or propylenediamine.

**88.** The kit according to any one of claims 79 to 87, wherein a DNA polymerase selected from the group consisting of Klenow fragment of DNA polymerase I from Escherichia coli, Bst DNA polymerase lacking 5'→3' exonuclease from Bacillus stearothermophilus and Bca DNA polymerase lacking 5'→3' exonuclease from Bacillus caldotenax is used as the DNA polymerase having a strand displacement activity.

**89.** The kit according to claim 79, wherein the DNA polymerase having a strand displacement activity is Bca DNA polymerase lacking 5'→3' exonuclease from Bacillus caldotenax and the RNase H is an RNase H selected from the group consisting of an RNase H from Escherichia coli, an RNase H from a bacterium of genus Pyrococcus and an RNase H from a bacterium of genus Archaeoglobus.

**90.** The kit according to claim 80, wherein the DNA polymerase having a strand displacement activity is Bca DNA polymerase lacking 5'→3' exonuclease from Bacillus caldotenax and the endonuclease is an RNase H selected from the group consisting of an RNase H from Escherichia coli, an RNase H from a bacterium of genus Pyrococcus and an RNase H from a bacterium of genus Archaeoglobus.

**91.** The kit according to claim 89 or 90, wherein the RNase H is type I RNase H from Escherichia coli, or type II RNase H from a bacterium of genus Pyrococcus or a bacterium of genus Archaeoglobus.

**92.** The kit according to any one of claims 79 to 91, wherein a DNA polymerase having an endonuclease activity is used.

**93.** The kit according to claim 92, wherein the DNA polymerase is Bca DNA polymerase lacking 5'→3' exonuclease from Bacillus caldotenax, the kit containing a substance that allows the endonuclease activity of the Bca DNA polymerase to express.

**94.** The kit according to claim 93, wherein the substance that allows the endonuclease activity of the DNA polymerase to express is a manganese ion.

**95.** The kit according to any one of claims 79 to 94, which contains a substance that inhibits the reverse transcription activity of the DNA polymerase.

**96.** The kit according to claim 95, wherein the substance that inhibits the reverse transcription activity of the DNA polymerase is phosphonoformic acid.

**97.** The kit according to any one of claims 79 to 96, which contains a deoxynucleotide triphosphate analog.

**98.** The kit according to claim 97, wherein the deoxynucleotide triphosphate analog is deoxyuridine triphosphate or a derivative thereof.

**99.** A kit for amplifying a nucleic acid used for the method for amplifying a nucleic acid defined by any one of claims 1 to 5, which is in a packaged form and contains instructions that direct the use of a DNA polymerase having a strand displacement activity and an RNase H.

**100.** A kit for amplifying a nucleic acid used for the method for amplifying a nucleic acid defined by any one of claims 6 to 9, which is in a packaged form and contains instructions that direct the use of a DNA polymerase having a strand displacement activity and an endonuclease.

**101.** A product of a reagent for amplifying a nucleic acid consisting of a packing material and a reagent for amplifying a nucleic acid enclosed in the packing material, wherein the reagent for amplifying a nucleic acid contains a DNA polymerase having a strand displacement activity and/or an RNase H, and description that the reagent for amplifying a nucleic acid can be used for nucleic acid amplification under isothermal conditions is indicated in a label stuck to the packaging material or instructions attached to the packaging material.

**102.** A product of a reagent for amplifying a nucleic acid consisting of a packing material and a reagent for amplifying a nucleic acid enclosed in the packing material, wherein the reagent for amplifying a nucleic acid contains a DNA polymerase having a strand displacement activity and/or an endonuclease, and description that the reagent for amplifying a nucleic acid can be used for nucleic acid amplification under isothermal conditions is indicated in a label stuck to the packaging material or instructions attached to the packaging material.

**103.** A method for detecting a target nucleic acid in a sample, the method comprising:

(a) amplifying a nucleic acid by the method for amplifying a nucleic acid defined by any one of claims 1 to 39; and
(b) detecting a target nucleic acid amplified in step (a).

**104.** The method according to claim 103, which comprises detecting the amplified nucleic acid using a probe for detection.

**105.** The method according to claim 104, wherein the probe for detection is a probe that has been labeled with a labeling substance.

**106.** The method according to claim 105, wherein the probe is an RNA probe labeled with two or more fluorescent substances positioned at a distance that results in a quenching state.

**107.** A chimeric oligonucleotide primer used for the method for detecting a target nucleic acid defined by any one of claims 103 to 106.

**108.** The chimeric oligonucleotide primer according to claim 107 which is represented by general formula below:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns, and the nucleotide at the 3'-terminus may be modified such that extension from the 3'-terminus by the action of the DNA polymerase does not take place).

**109.** The chimeric oligonucleotide primer according to claim 108, wherein c is 0.

**110.** The chimeric oligonucleotide primer according to claim 108 or 109, wherein the nucleotide analog is deoxyriboinosine nucleotide or deoxyribouracil nucleotide and the modified ribonucleotide is ($\alpha$-S) ribonucleotide.

**111.** The chimeric oligonucleotide primer according to any one of claims 107 to 110, which is a chimeric oligonucleotide primer for detecting a pathogenic microorganism or a disease-related gene.

**112.** The chimeric oligonucleotide primer according to claim 111, wherein the pathogenic microorganism is enterohemorrhagic Escherichia coli, Clostridium botulinum, Staphylococcus aureus, Mycobacterium tuberculosis, Chlamydia, papilloma virus, hepatitis C virus or a viroid.

**113.** A chimeric oligonucleotide primer for detecting enterohemorrhagic Escherichia coli having a nucleotide sequence selected from the group consisting of SEQ ID NOS: 31-34, 47, 48, 51-53, 64-72, 84, 85, 113, 114, 130 and 131.

**114.** A chimeric oligonucleotide primer for detecting a viroid having a nucleotide sequence selected from the group consisting of SEQ ID NOS: 59, 60, 119, 120, 122 and 123.

**115.** A chimeric oligonucleotide primer for. detecting Clostridium botulinum having a nucleotide sequence represented by SEQ ID NO: 116 or 117.

**116.** A chimeric oligonucleotide primer for detecting papilloma virus having a nucleotide sequence represented by SEQ ID NO: 96 or 97.

**117.** A chimeric oligonucleotide primer for detecting hepatitis C virus having a nucleotide sequence selected from the group consisting of SEQ ID NOS: 101, 102, 138, 139, 200 and 201.

**118.** A chimeric oligonucleotide primer for detecting Staphylococcus aureus having a nucleotide sequence represented by SEQ ID NO: 136 or 137.

**119.** A chimeric oligonucleotide primer for detecting Mycobacterium tuberculosis having a nucleotide sequence selected from the group consisting of SEQ ID NOS: 155, 156, 159-162, 194 and 195.

**120.** A chimeric oligonucleotide primer for detecting Chlamydia having a nucleotide sequence represented by SEQ ID NO: 157 or 158.

**121.** A kit for amplifying a nucleic acid used for the method for amplifying a nucleic acid defined by any one of claims 1 to 39, which contains the chimeric oligonucleotide primer defined by any one of claims 107 to 120.

**122.** A kit for detecting a target nucleic acid used for the method for detecting a target nucleic acid defined by any one of claims 103 to 106, which contains the chimeric oligonucleotide primer defined by any one of claims 107 to 120.

**123.** A probe used in the method defined by any one of claims 103 to 106.

**124.** A probe which hybridizes to the nucleic acid amplified by the method defined by any one of claims 1 to 39.

**125.** A probe which hybridizes to a region amplified using the chimeric oligonucleotide primer defined by any one of claims 113 to 120.

**126.** The probe according to any one of claims 123 to 125, which has been labeled with a labeling substance.

**127.** The probe according to claim 126, which is an RNA probe labeled with two or more fluorescent substances positioned at a distance that results in a quenching state

**128.** A kit used in the method defined by any one of claims 103 to 106, which contains the probe defined by any one of claims 123 to 127.

**129.** A method for amplifying a nucleic acid, which comprises using a DNA polymerase having a strand displacement activity to effect a template switching reaction.

**130.** The method according to claim 129, wherein the DNA polymerase having a strand displacement activity is selected from the group consisting of Klenow fragment of DNA polymerase I from Escherichia coli, Bst DNA polymerase lacking 5'→3' exonuclease from Bacillus stearothermophilus and Bca DNA polymerase lacking 5'→3' exonuclease from Bacillus caldotenax.

**131.** A method for producing a material having an immobilized nucleic acid in which the nucleic acid is arrayed in a predefined region, the method comprising:

(a) amplifying a nucleic acid to be immobilized by the method for amplifying a nucleic acid defined by any one of claims 1 to 39; and
(b) arraying and immobilizing the nucleic acid amplified in step (a) in a predefined region.

**132.** A material having an immobilized nucleic acid in which the nucleic acid is arrayed in a predefined region produced by the method defined by claim 131.

**133.** A method for producing a nucleic acid in large quantities, the method comprising:

(a) amplifying a nucleic acid by the method for amplifying a nucleic acid defined by any one of claims 1 to 39; and
(b) collecting the nucleic acid amplified in step (a).

**134.** A method for amplifying a nucleic acid, the method comprising:

(a) duplicating a DNA or an RNA containing a sequence to be amplified to prepare a nucleic acid as a template; and
(b) amplifying the nucleic acid as the template obtained in step (a) by the method for amplifying a nucleic acid defined by any one of claims 1 to 39.

**135.** A method for determining a nucleotide sequence of a nucleic acid, the method comprising amplifying a nucleic acid according to the method defined by any one of claims 1 to 39, 133 and 134.

**136.** A method for preparing a single-stranded nucleic acid, the method comprising preparing a single-stranded nucleic acid using the method defined by any one of claims 1 to 39.

**137.** The method according to claim 136, wherein at least two primers at different concentrations are used.

Fig. 1

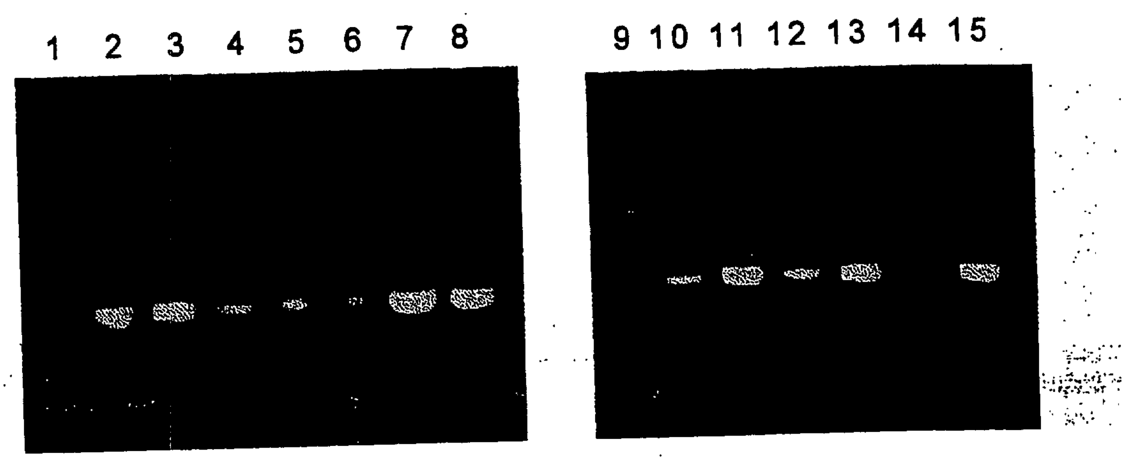

Fig.2

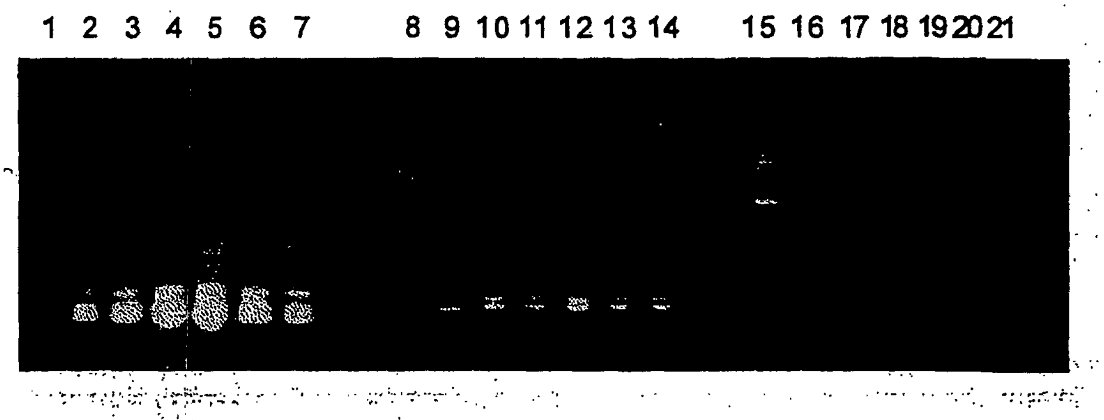

Fig.3

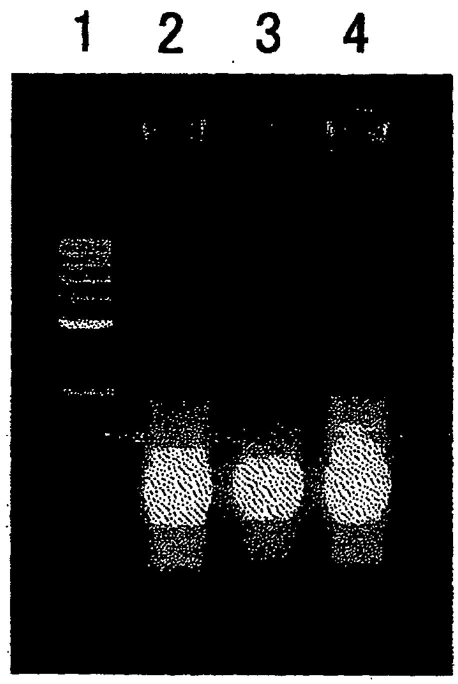

Fig.4

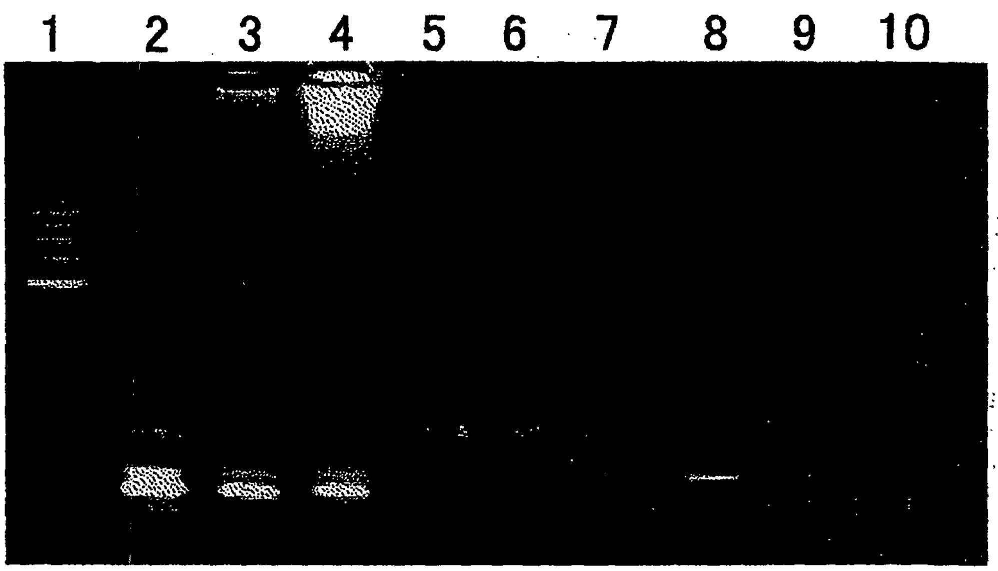

Fig.5

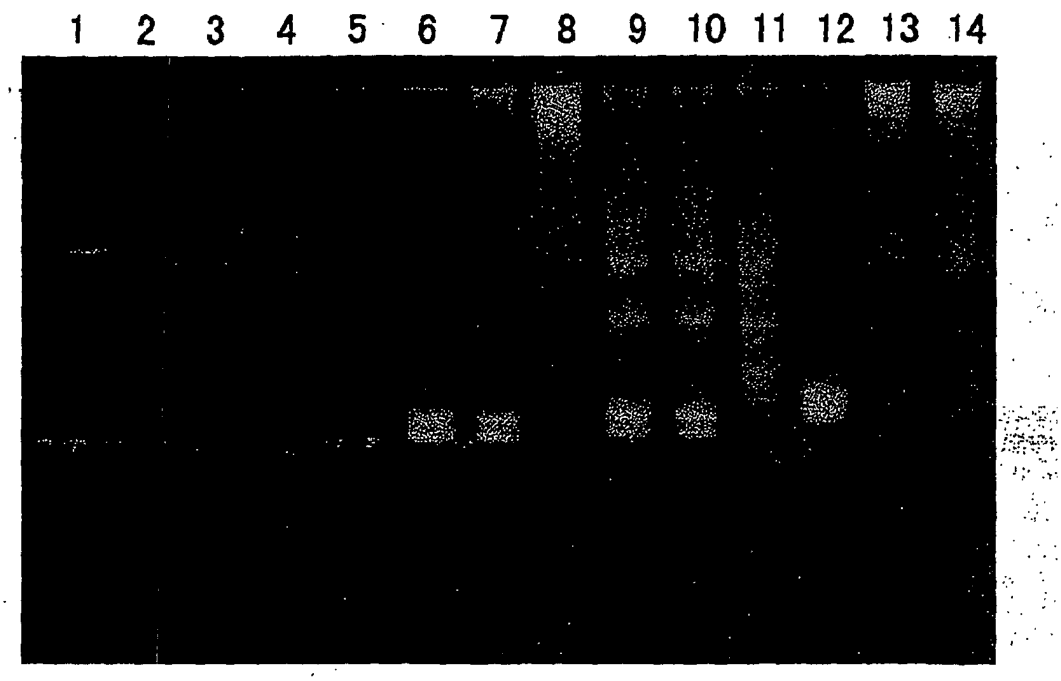

Fig.6

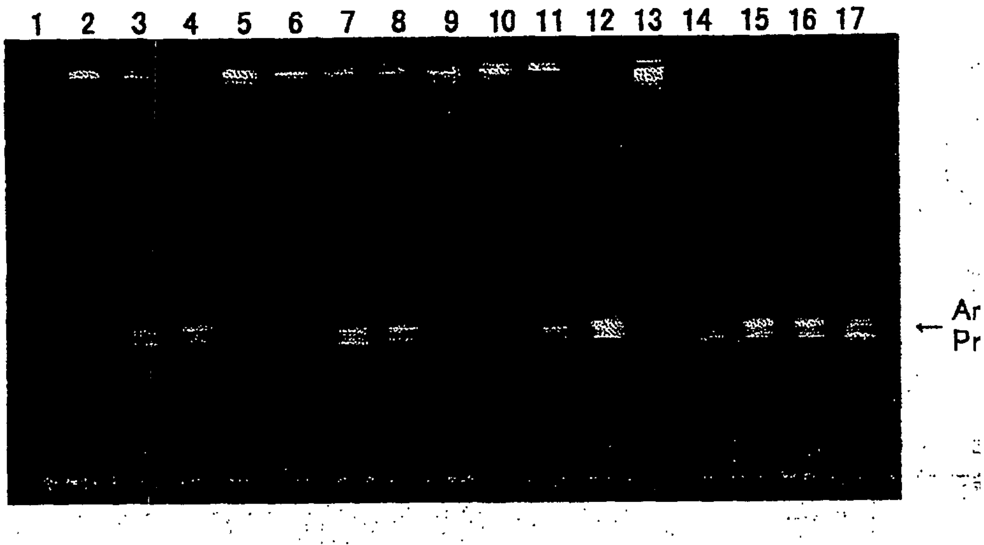

Fig.7

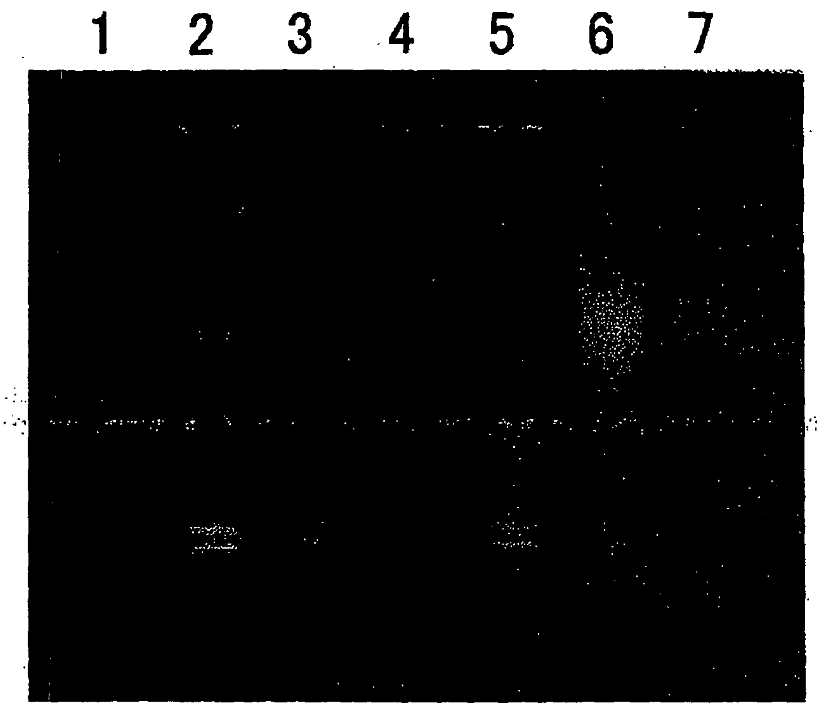

Fig.8

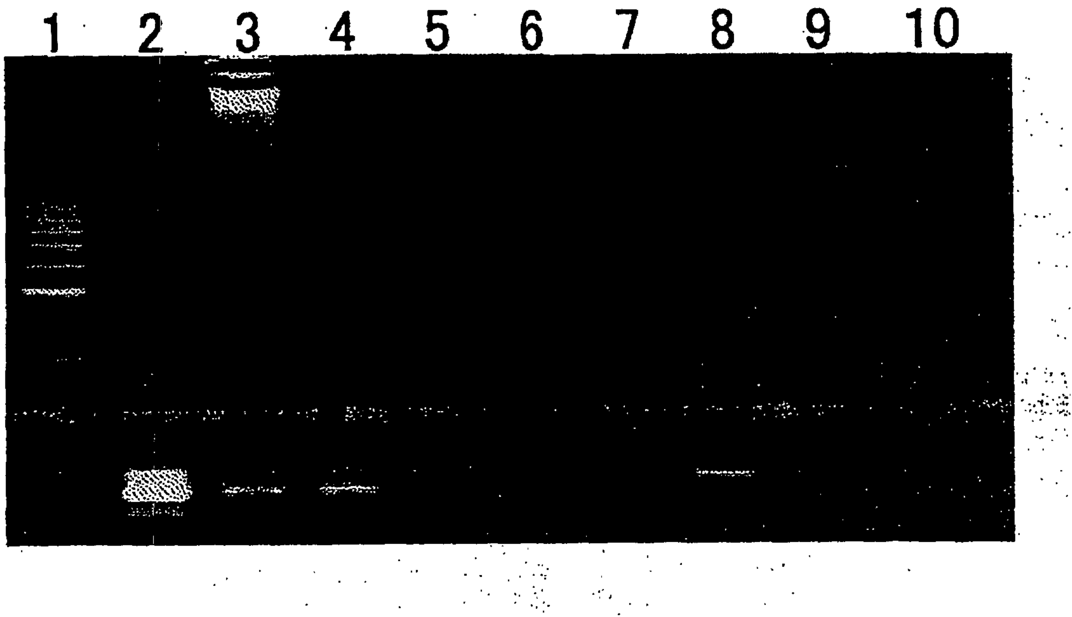

Fig.9

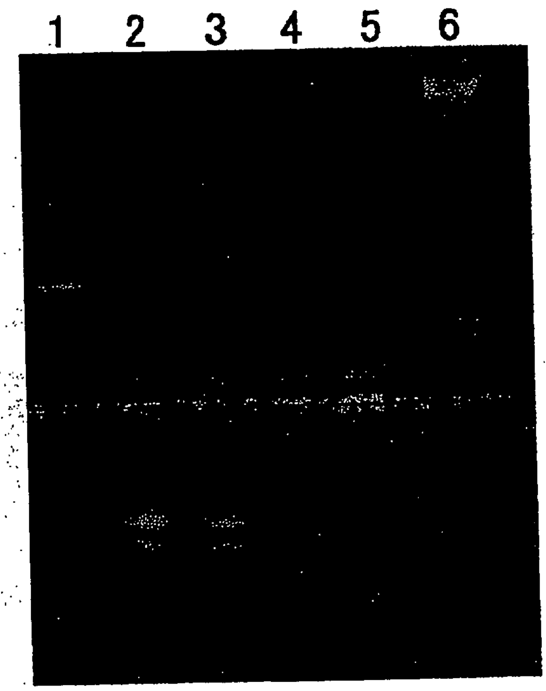

Fig. 10

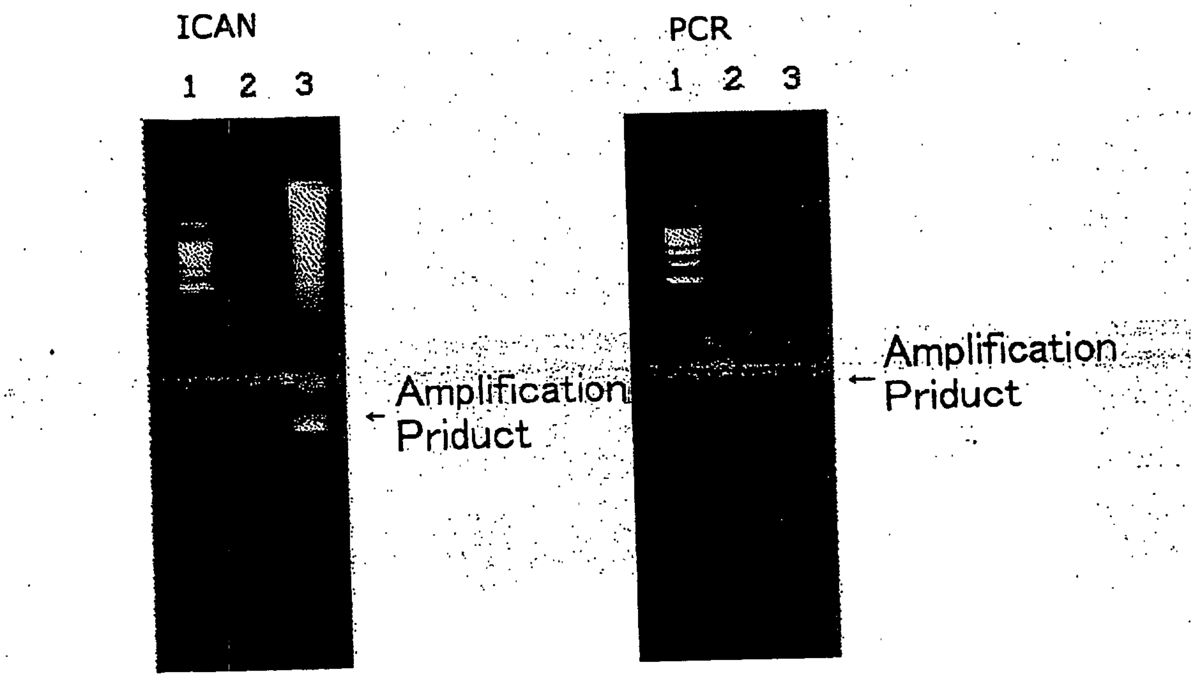
ICAN
1 2 3
Amplification
Priduct
PCR
1 2 3
Amplification
Priduct

Fig.11

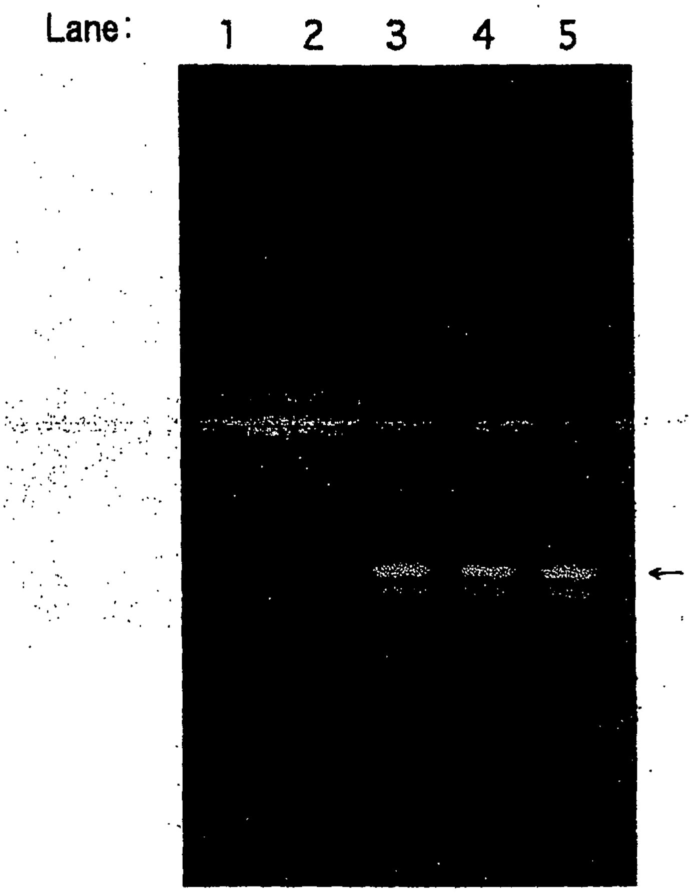

Fig.12

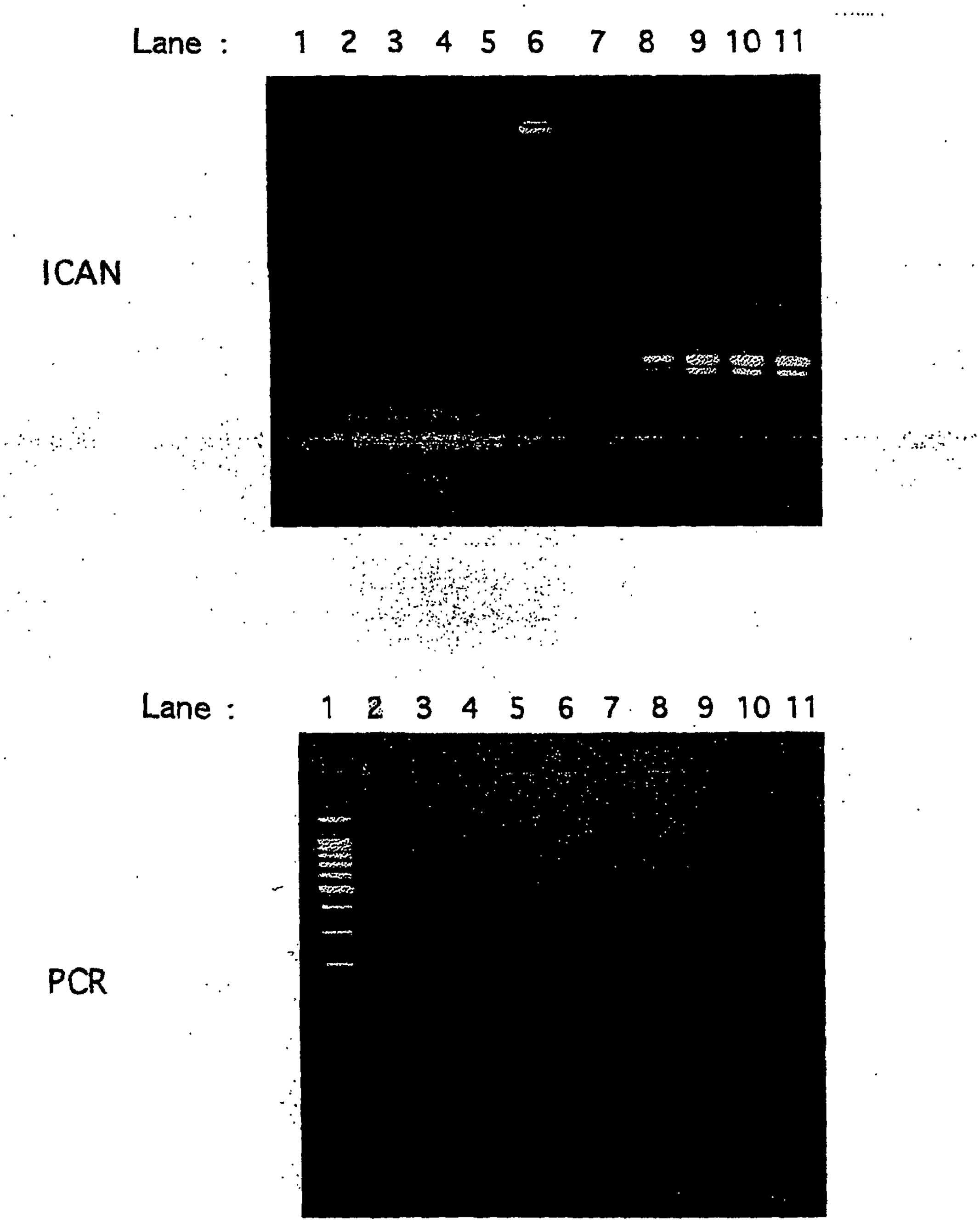

Fig.13

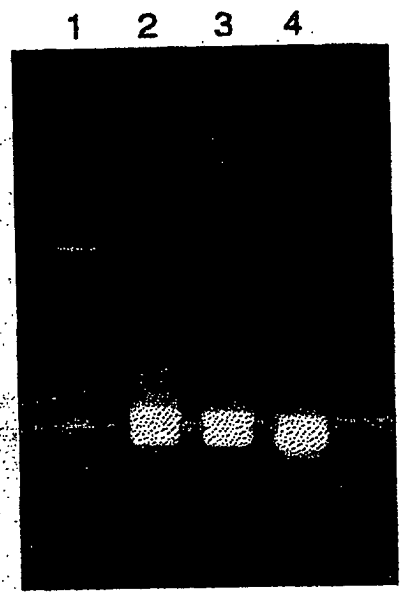

Fig.14

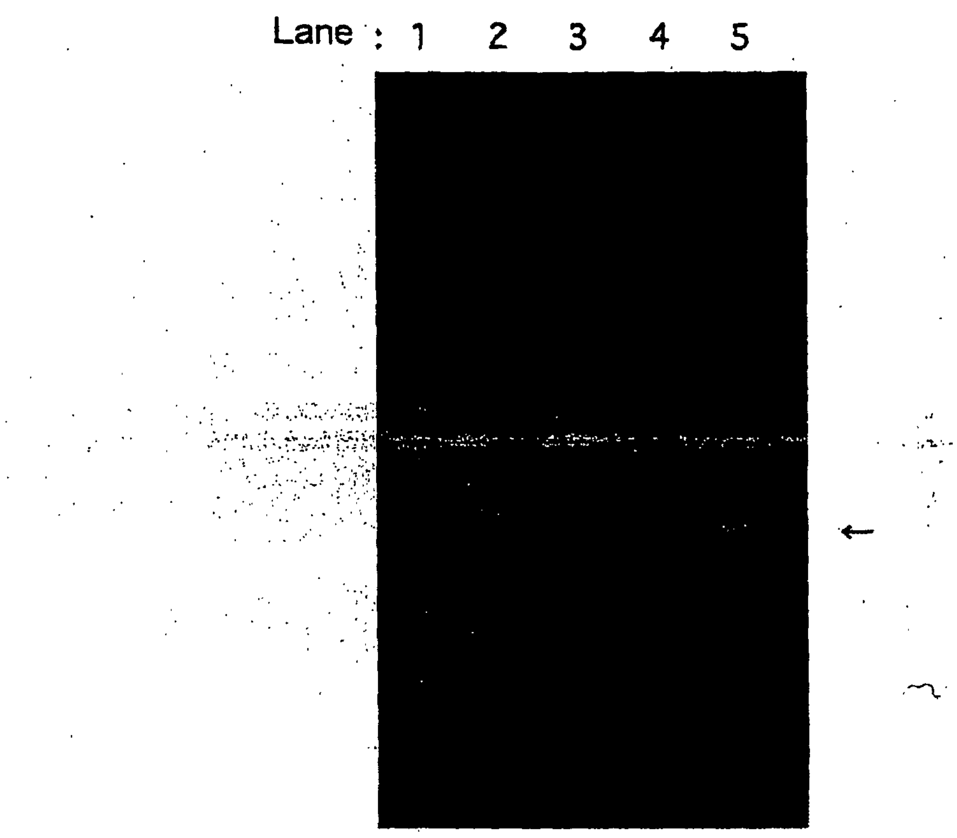

Fig.15

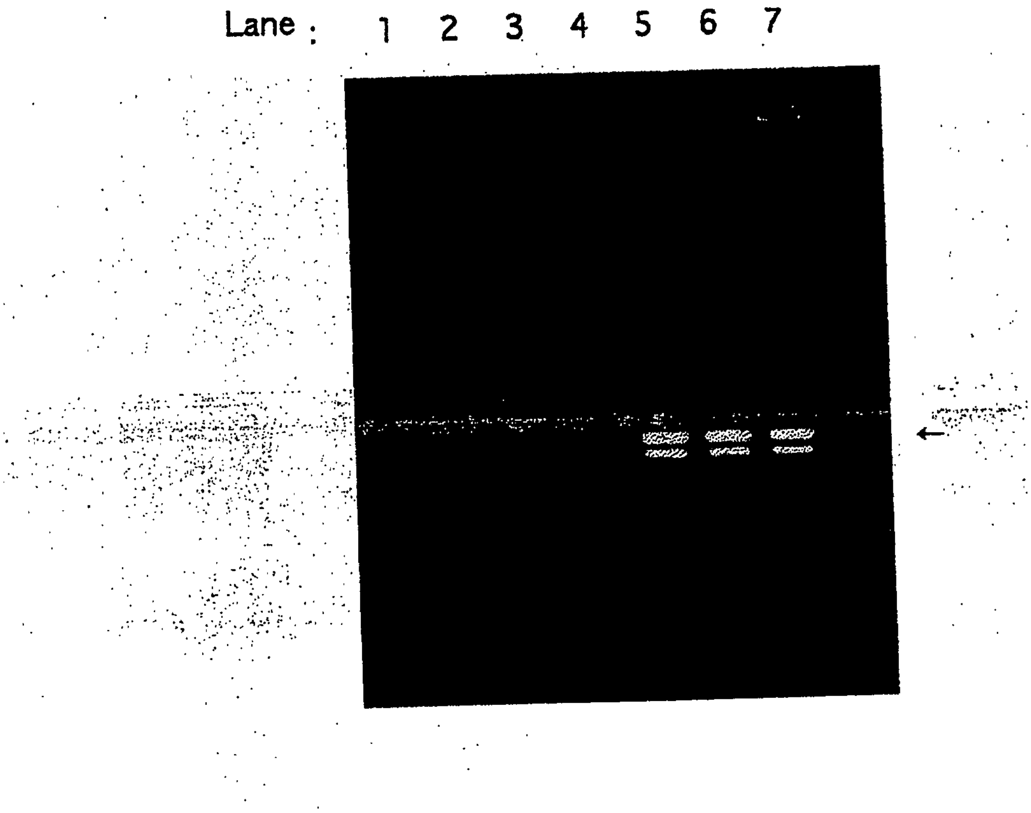

Fig. 16

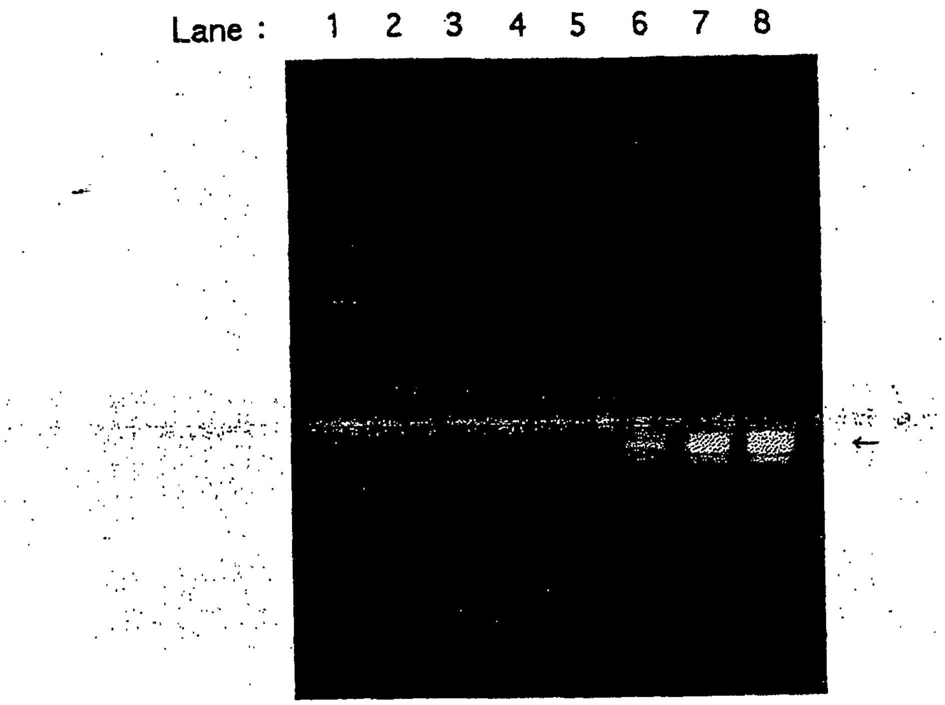

Fig.17

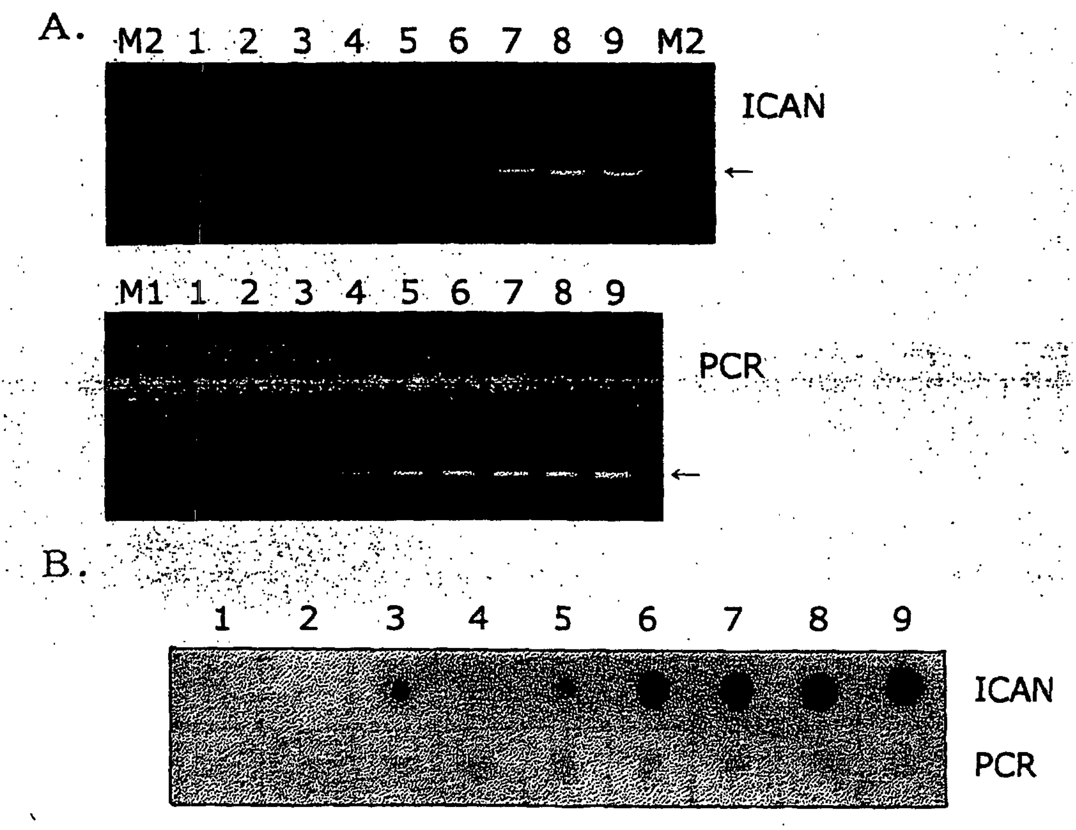

Fig. 18

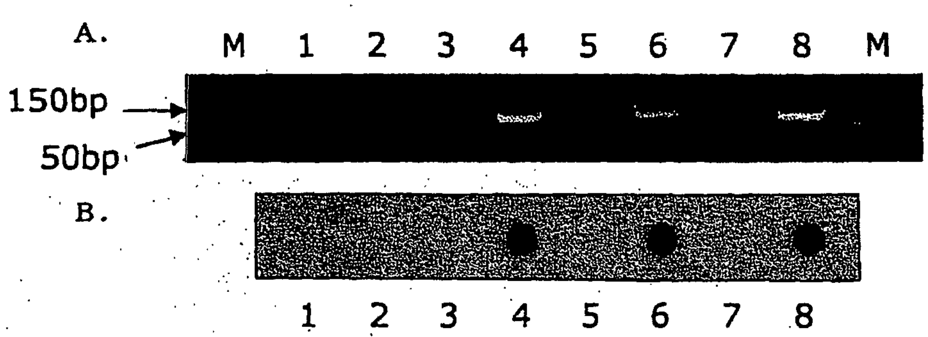

Fig.19

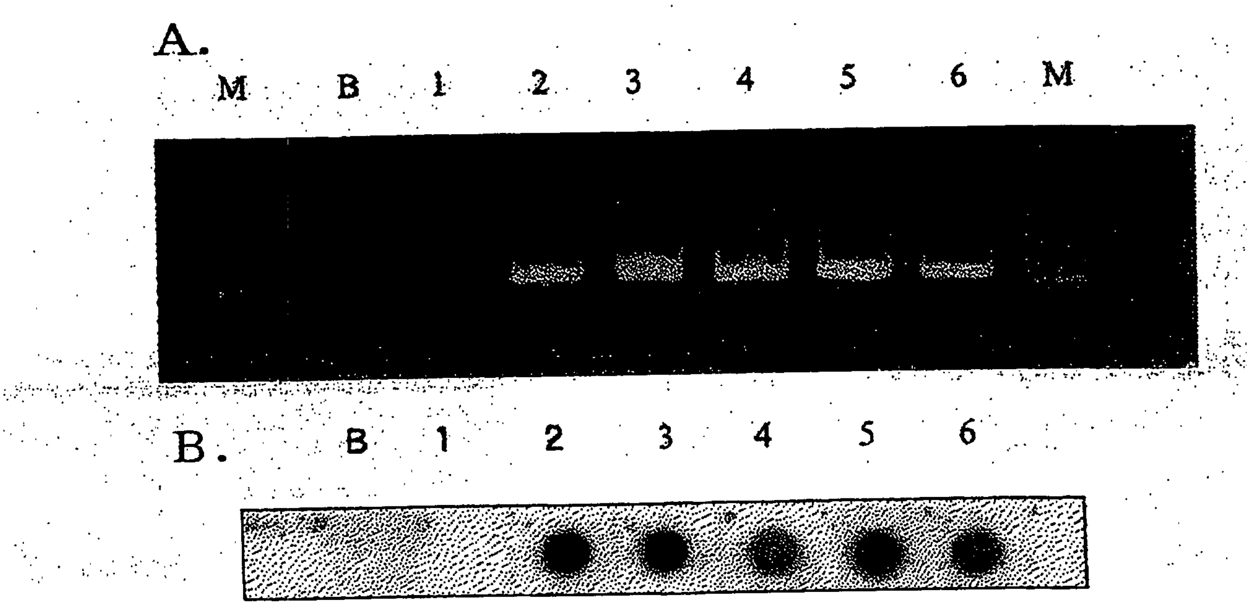

Fig.20

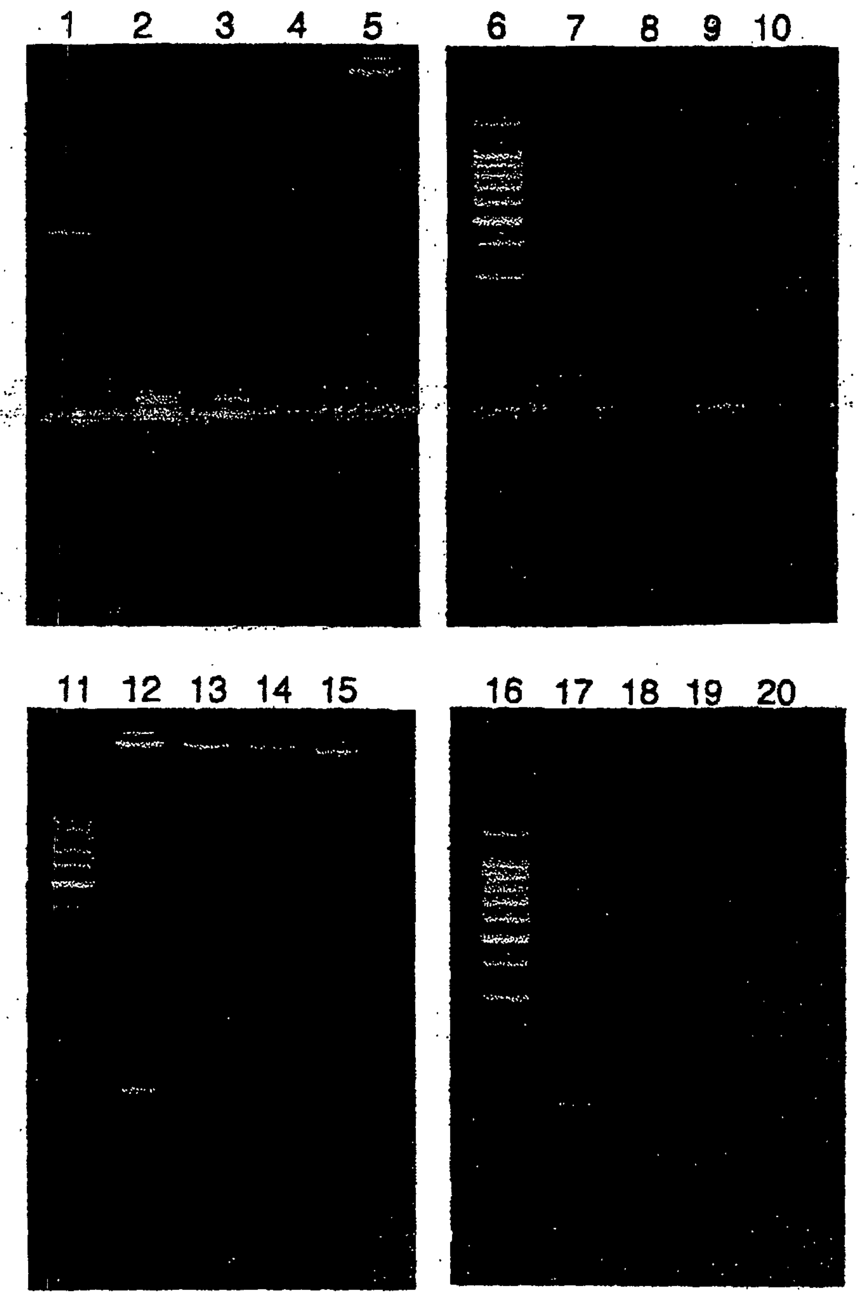

Fig.21

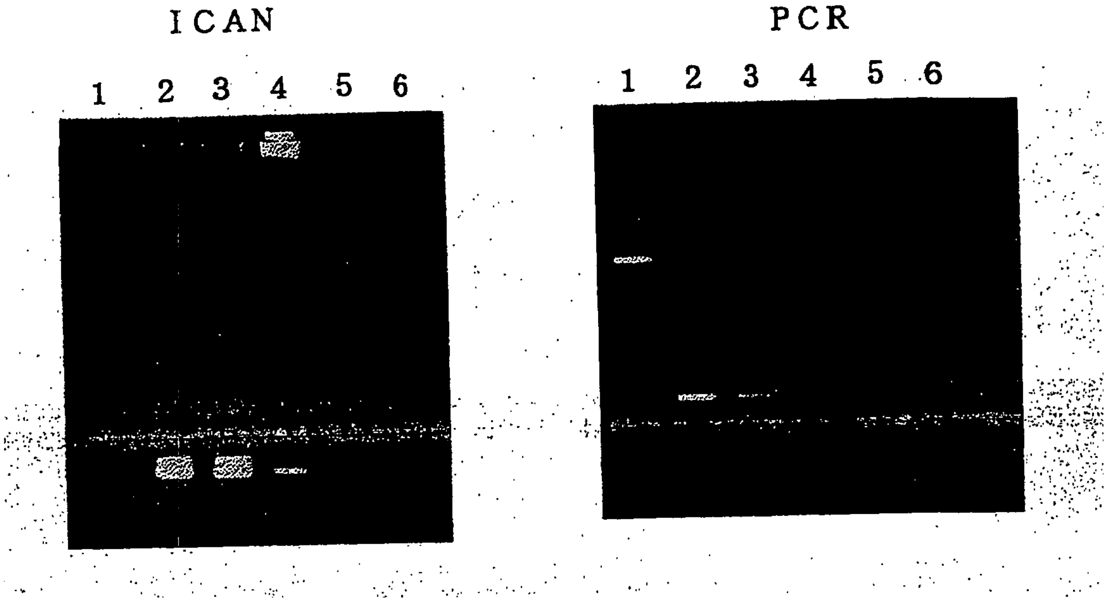

Fig.22

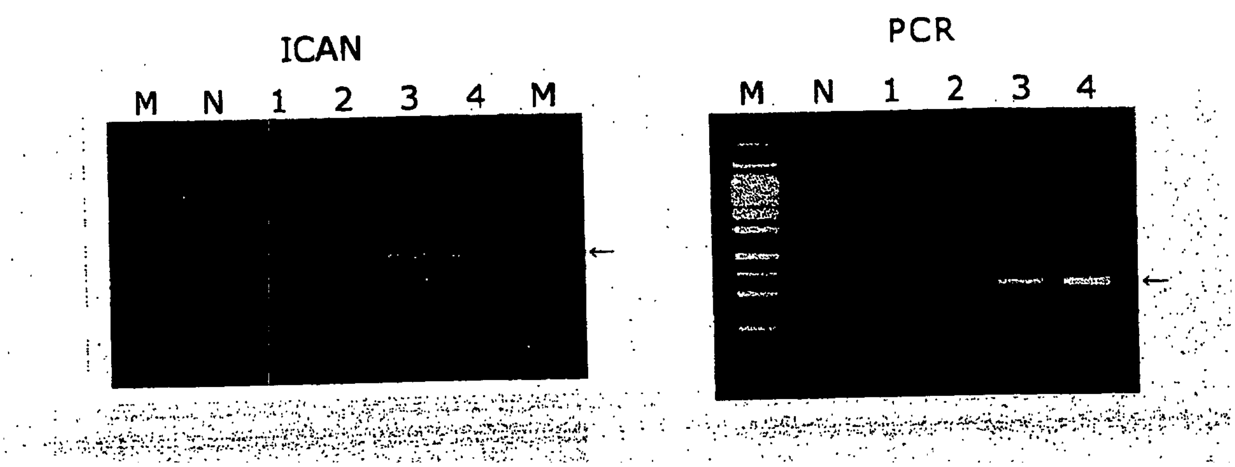

Fig.23

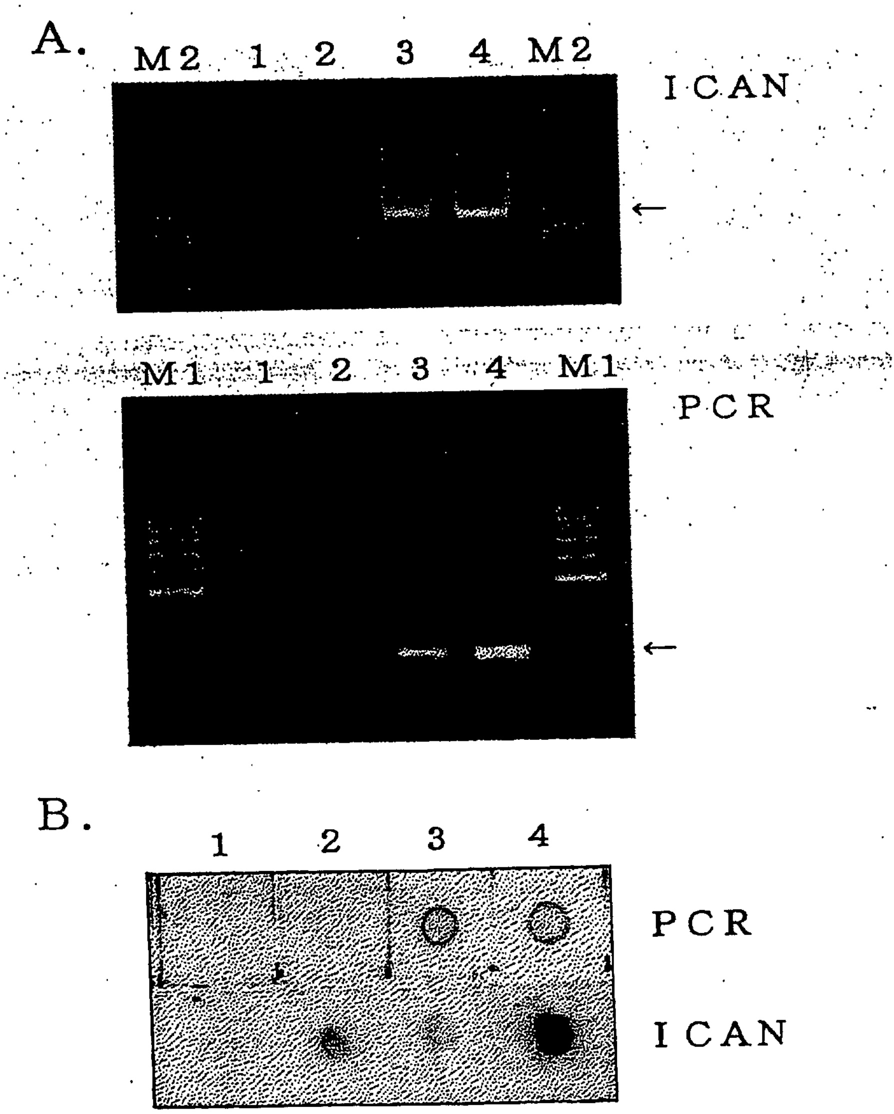

Fig.24

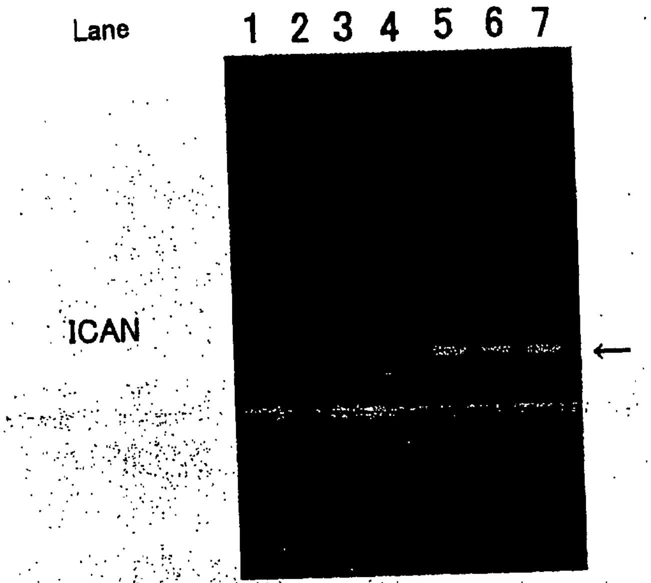


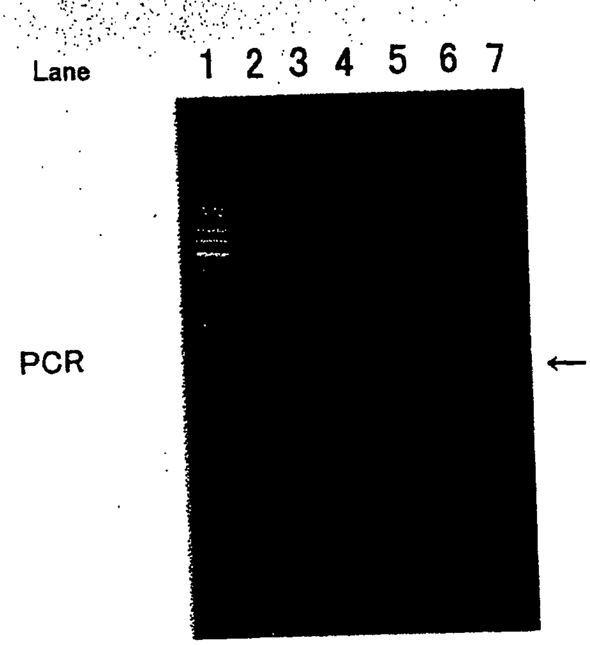

Fig.25

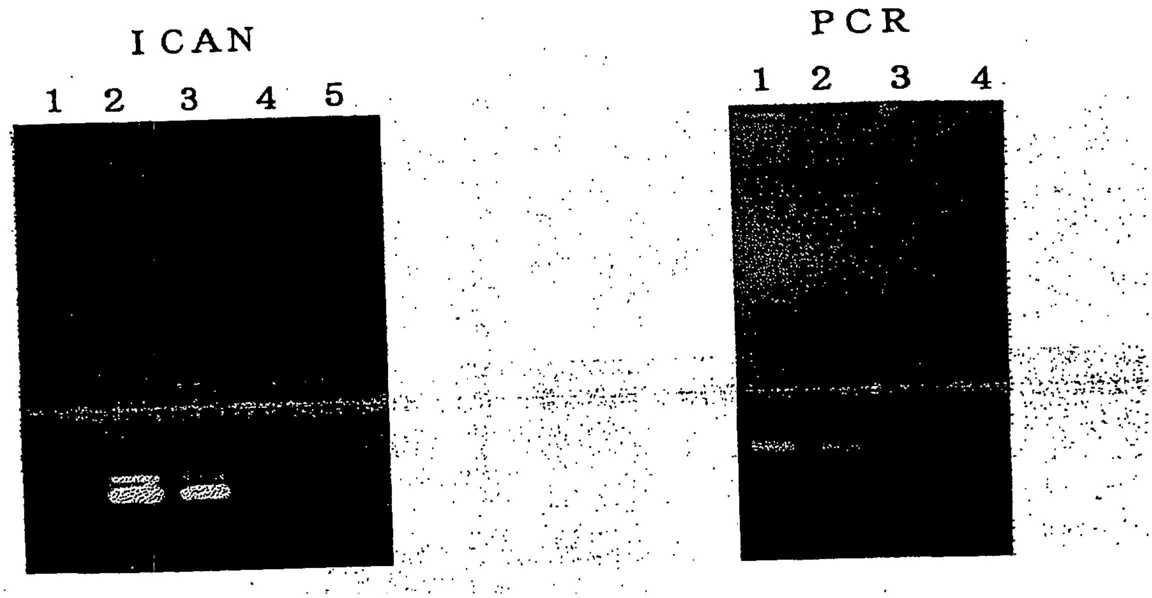

Fig.26

ICAN

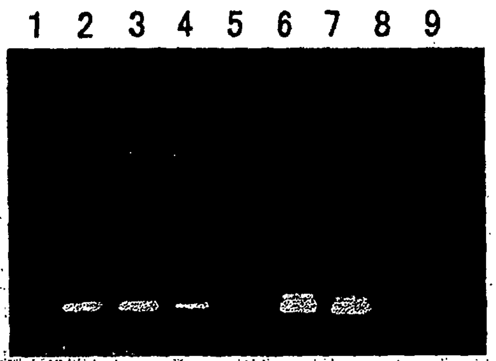


PCR

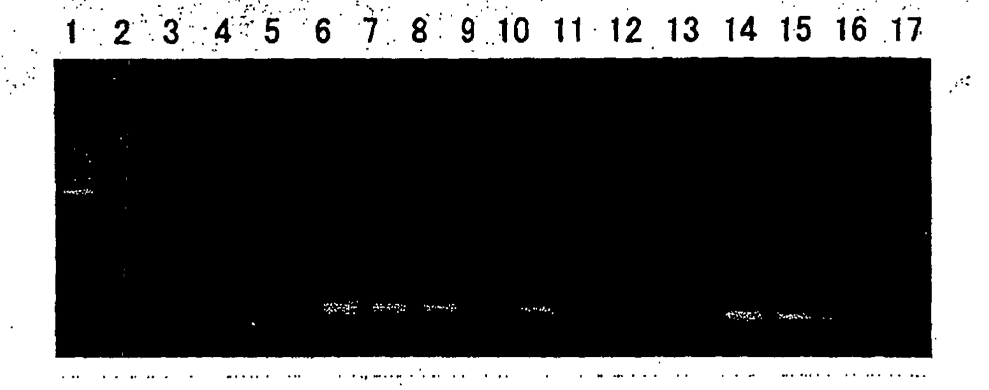

Fig.27

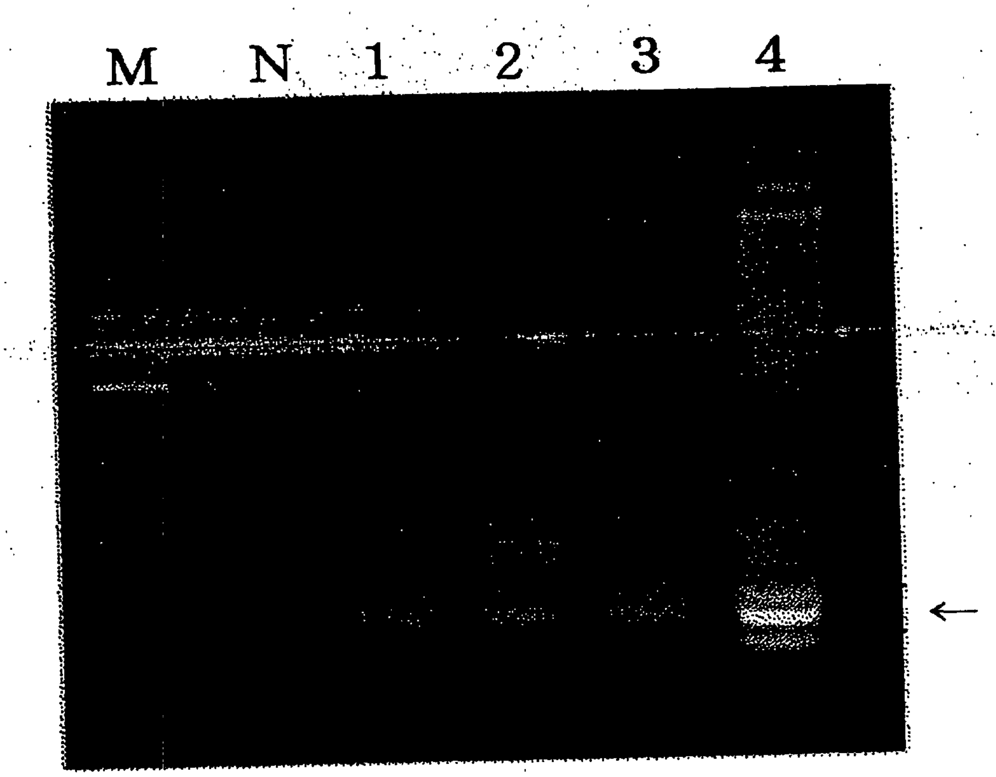

Fig.28

A.

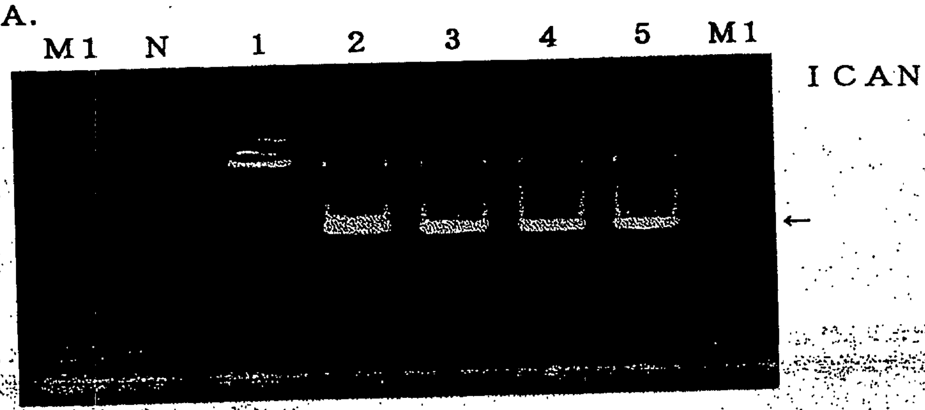


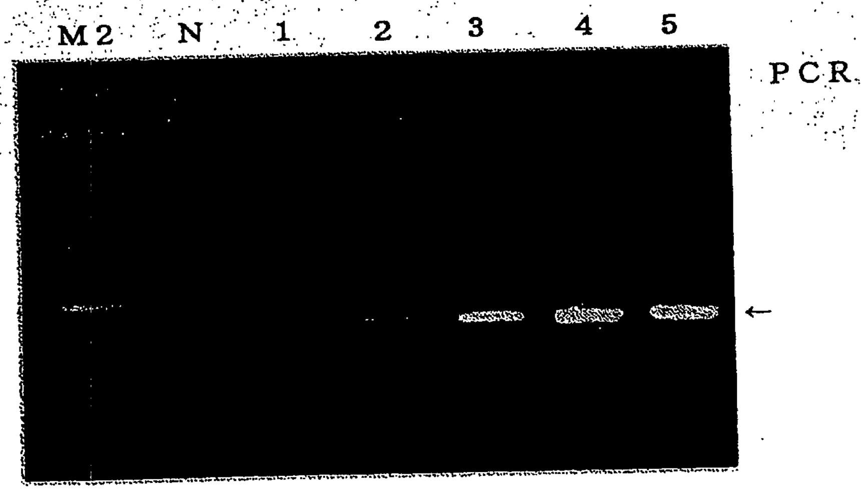


B.

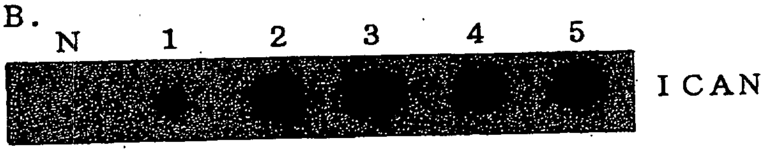

Fig.29

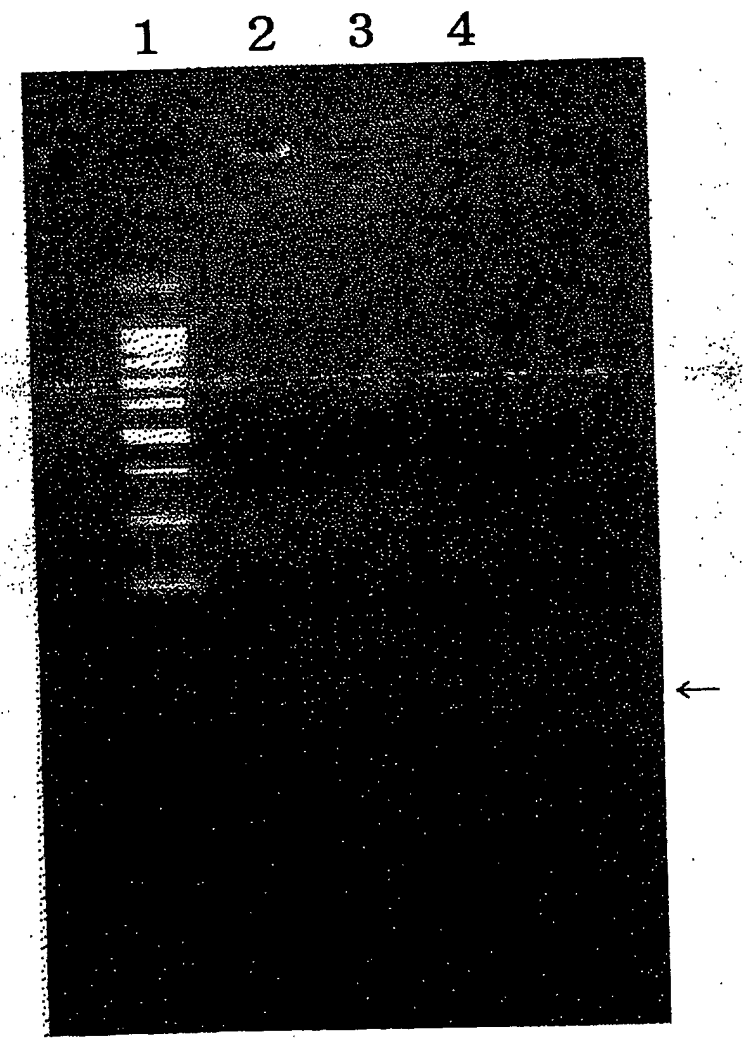

30/39

Fig.30

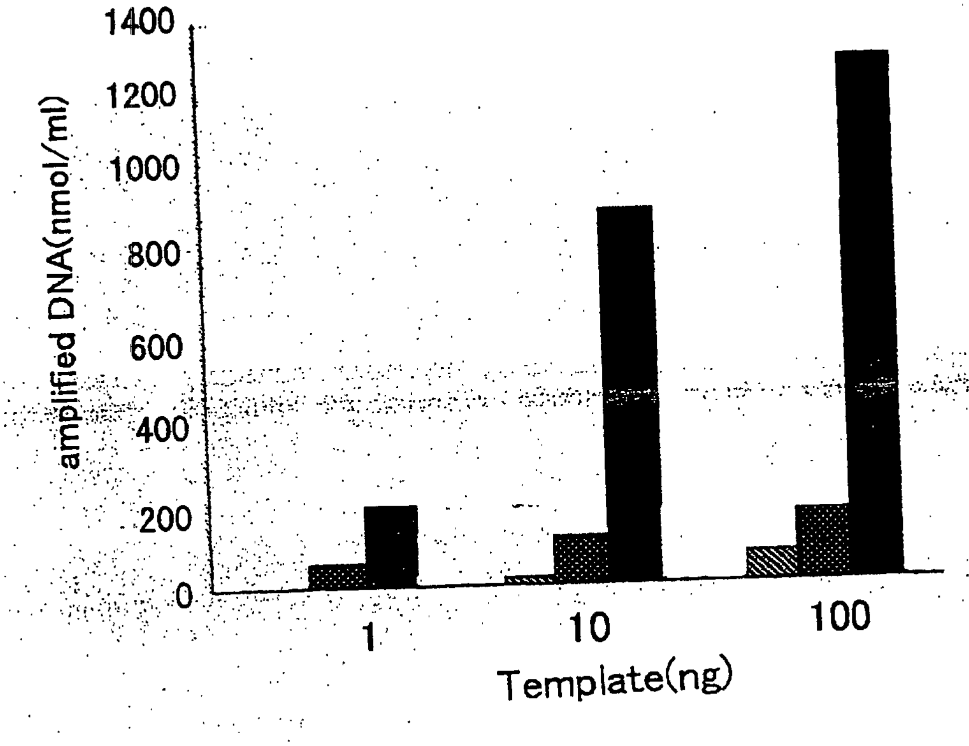
1400
1200
1000
800
600
400
200
0
amplified DNA(nmol/ml)
1
10
100
Template(ng)

Fig.31

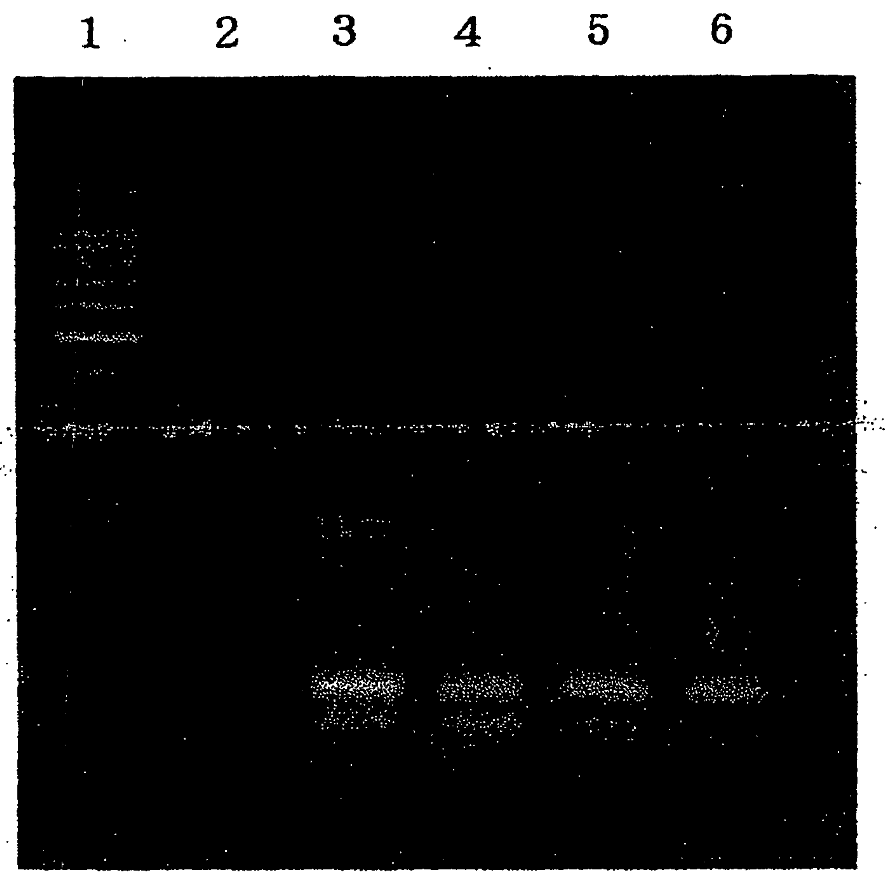

Fig.32

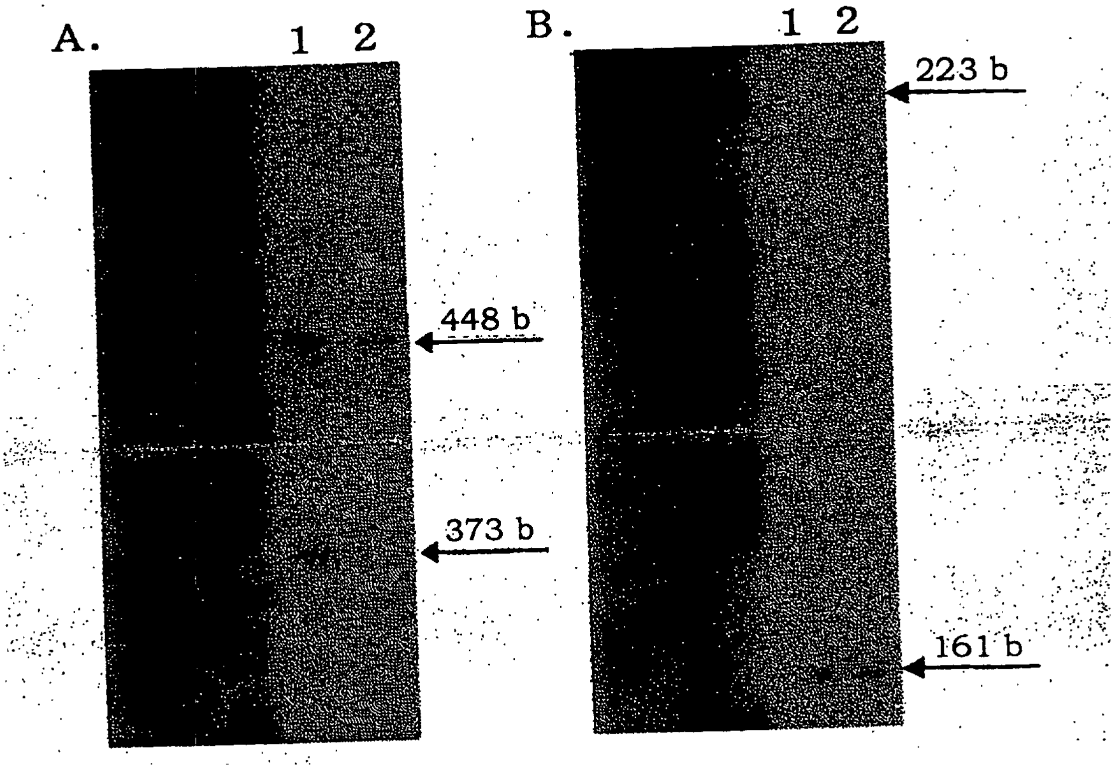

Fig.33

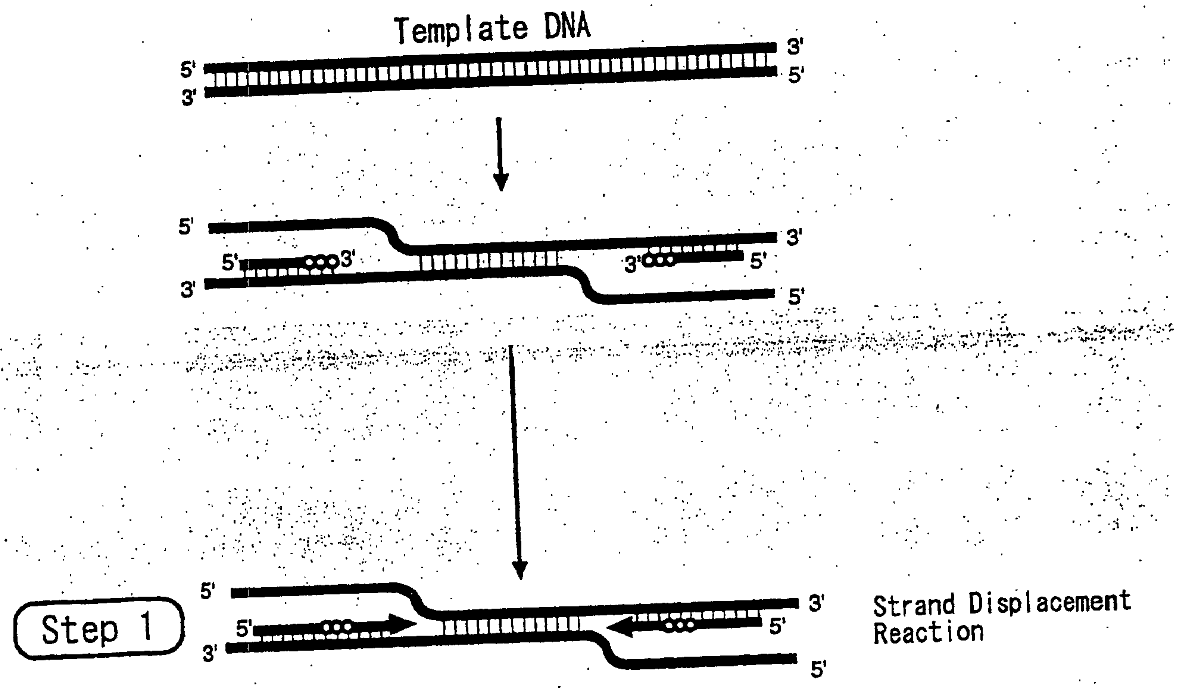
Template DNA
5'
3'
3'
5'
5'
3'
5'
3'
3'
5'
3'
5'
5'
3'
5'
3'
5'
3'
5'
5'
Step 1
Strand Displacement
Reaction

Fig.34

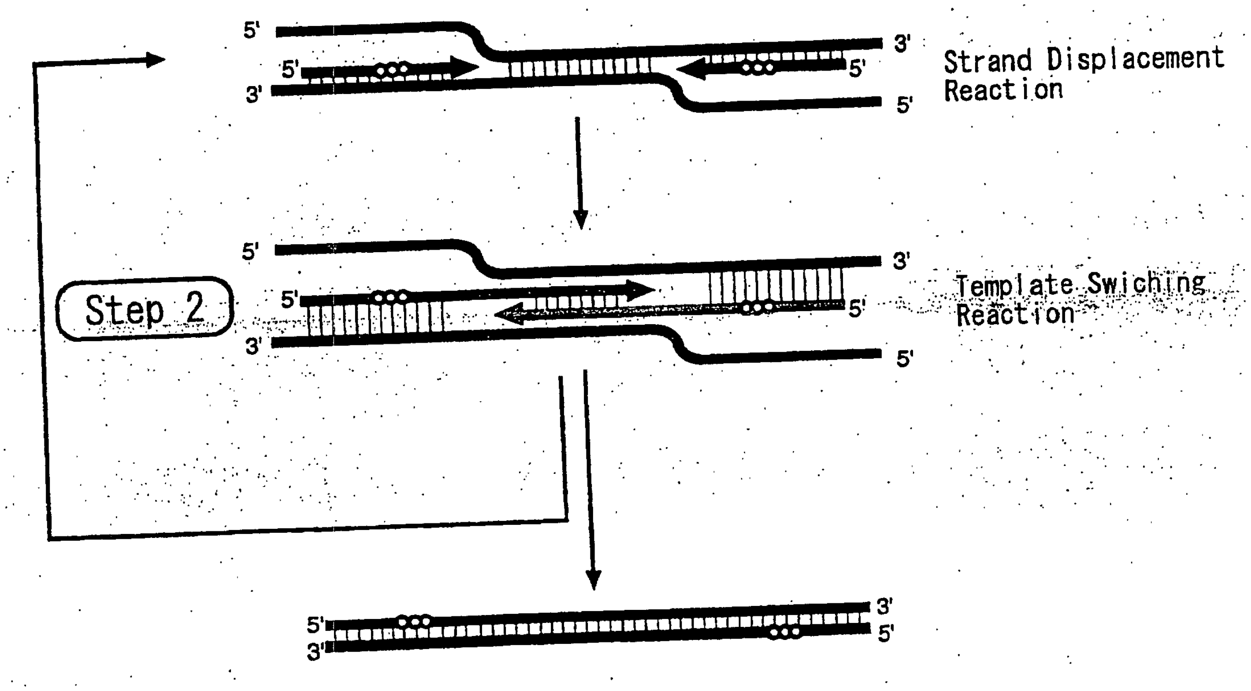
5'
3'
5'
5'
3'
5'
Strand Displacement
Reaction
5'
3'
Step 2
5'
5'
3'
5'
Template Swiching
Reaction
5'
3'
3'
5'

Fig.35

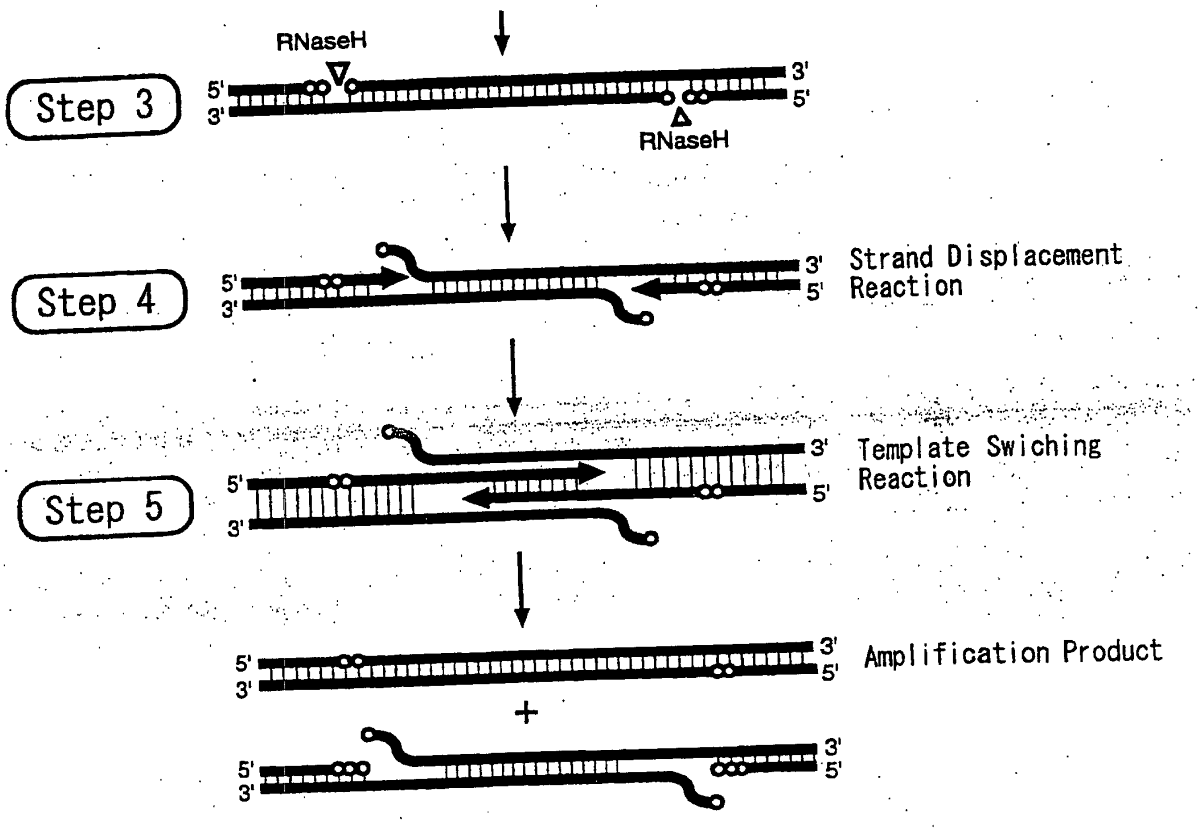
Step 3
RNaseH
5'
3'
3'
5'
RNaseH
Step 4
5'
3'
3'
5'
Strand Displacement Reaction
Step 5
5'
3'
3'
5'
Template Swiching Reaction
5'
3'
3'
5'
Amplification Product
+
5'
3'
3'
5'

Fig.36

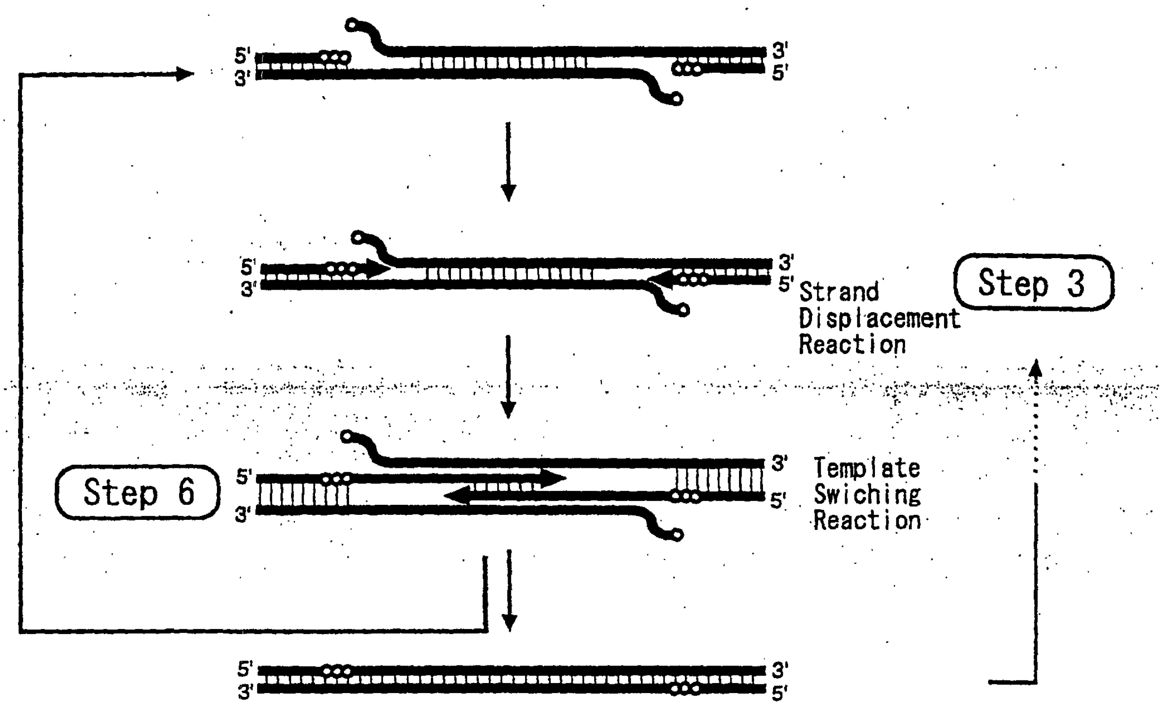
5'
3'
3'
5'
Strand
Displacement
Reaction
Step 3
Step 6
Template
Swiching
Reaction

Fig.37

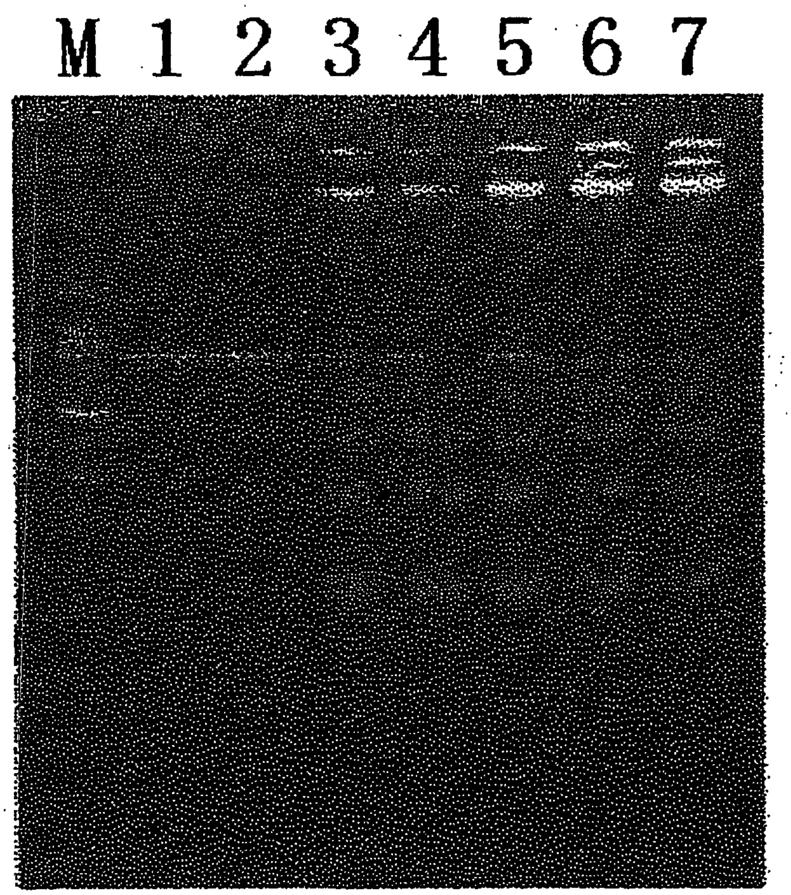

Fig.38

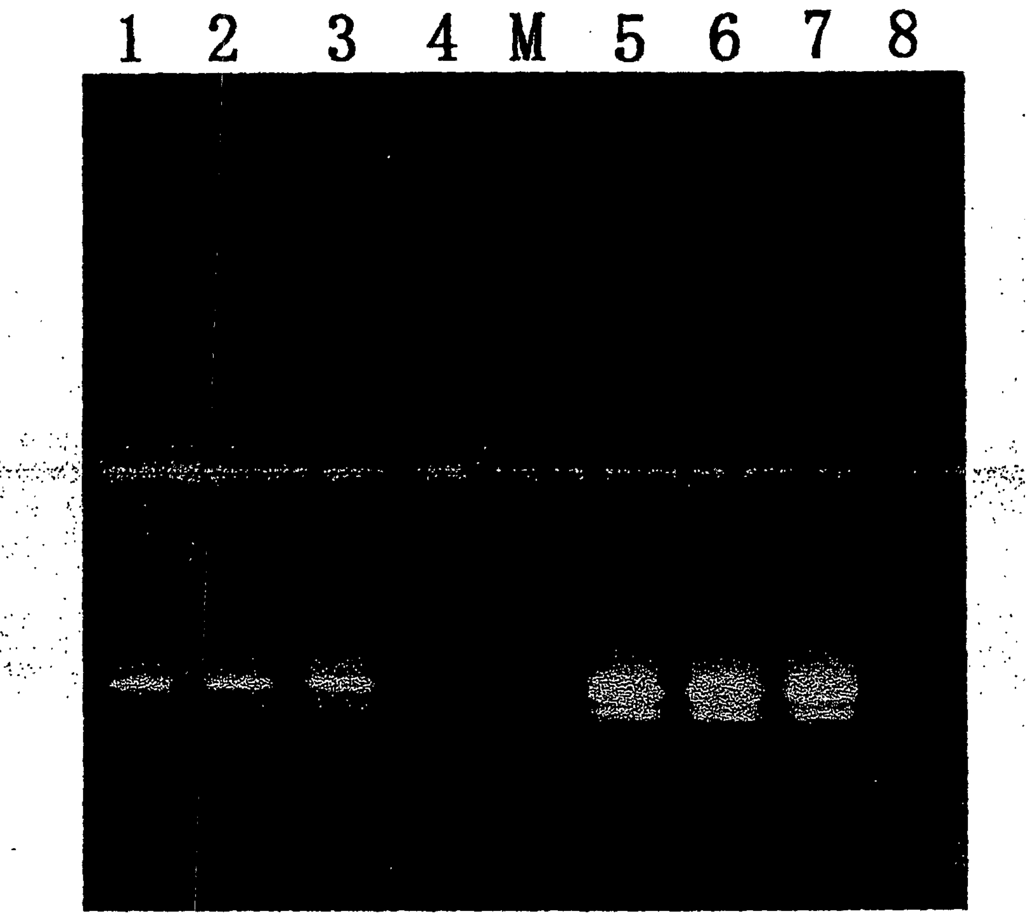

Fig.39

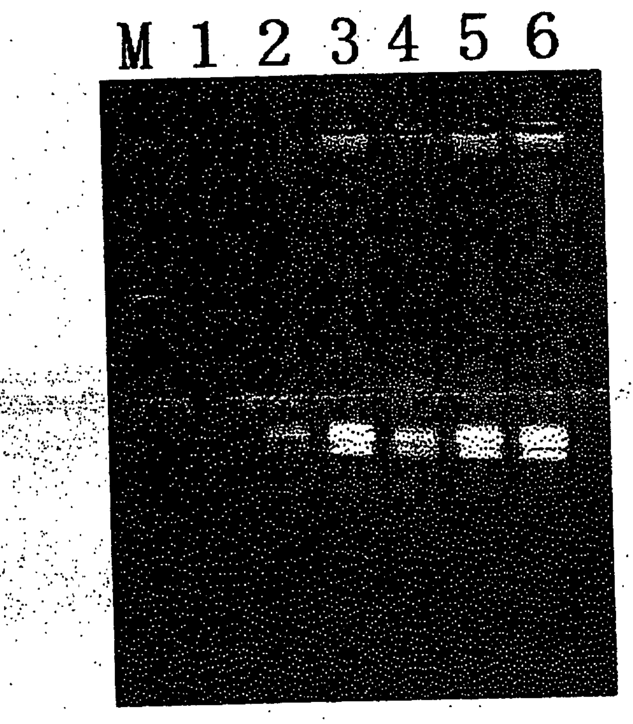

## INTERNATIONAL SEARCH REPORT

International application No.
PCT/JP01/07139

**A. CLASSIFICATION OF SUBJECT MATTER**
Int.Cl$^7$ C12Q1/68, C12N15/09

According to International Patent Classification (IPC) or to both national classification and IPC

**B. FIELDS SEARCHED**

Minimum documentation searched (classification system followed by classification symbols)
Int.Cl$^7$ C12Q1/68, C12N15/09

Documentation searched other than minimum documentation to the extent that such documents are included in the fields searched

Electronic data base consulted during the international search (name of data base and, where practicable, search terms used)
WPIDS/BIOSIS/BIOTECHABS/MEDLINE/CA (STN)
JICST (JOIS)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 96/40901 A1 (Gen-Probe Incorporated),<br>19 December, 1996 (19.12.96),<br>& EP 747479 A1 & US 5652126 A<br>& US 5739311 A & US 5916777 A<br>& US 5932450 A | 107-120<br>1-106,121-137 |
| X<br>A | WO 96/15270 A1 (Perkin-Elmer Corporation),<br>23 May, 1996 (23.05.96),<br>& EP 792374 A1 & EP 972848 A2<br>& US 5538848 A & US 5723591 A<br>& US 5876930 A & US 6030787 A<br>& JP 10-510982 A1 | 123-127<br>1-122,128-137 |
| P,X | WO 2000/56877 A1 (Takara Syuzo Co., Ltd.),<br>28 September, 2000 (28.09.00),<br>& AU 2000029742 A1 | 1-137 |
| P,X | Ikunoshin KATO, "ICAN-hou ha PCR wo koemasuka?",<br>Kagaku, December, 2000, Vol.55, pages 20 to 21 | 1-137 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 November, 2001 (14.11.01) | 27 November, 2001 (27.11.01) |
| Name and mailing address of the ISA/<br>Japanese Patent Office | Authorized officer |
| Facsimile No. | Telephone No. |

Form PCT/ISA/210 (second sheet) (July 1992)

SEQUENCE LISTING

<110> Takara Shuzo Co., Ltd.

<120> A method for amplification of nucleic acids

<130> 662757

<150> JP 2000-251981

<151> 2000-08-23

<150> JP 2000-284419

<151> 2000-09-19

<150> JP 2000-288750

<151> 2000-09-22

<150> JP 2001-104191

<151> 2001-04-03

<160> 201

<210> 1

<211> 20

<212> DNA

<213> Artificial Sequence

<220>

<223> PCR primer BsuII-3 for cloning a gene encoding a polypeptide having a RNaseHII activity from Bacillus caldotenax

<400> 1

gtcgccagcg cagtnathyt 20

<210> 2

<211> 20

<212> DNA

<213> Artificial Sequence

<220>

<223> PCR primer BsuII-6 for cloning a gene encoding a polypeptide having a RNaseHII activity from Bacillus caldotenax

<400> 2

cggtccctcg tcacyttngc 20

<210> 3

<211> 20

<212> DNA

<213> Artificial Sequence

<220>

<223> PCR primer RNII-S1 for cloning a gene encoding a polypeptide having a RNaseHII activity from Bacillus caldotenax

<400> 3

cgcgcttttc cggcgtcagc 20

<210> 4

<211> 20

<212> DNA

<213> Artificial Sequence

<220>

<223> PCR primer RNII-S2 for cloning a gene encoding a polypeptide having a RNaseHII activity from Bacillus caldotenax

<400> 4

acggcgcacg cttcaatttg 20

<210> 5

<211> 20

<212> DNA

<213> Artificial Sequence

<220>

<223> PCR primer RNII-S5 for cloning a gene encoding a polypeptide having a RNaseHII activity from Bacillus caldotenax

<400> 5

acgcctattt gccggggctt 20

<210> 6

<211> 20

<212> DNA

<213> Artificial Sequence

<220>

<223> PCR primer RNII-S6 for cloning a gene encoding a polypeptide having a RNaseHII activity from Bacillus caldotenax

<400> 6

atgaccgacg cagcggcgat 20

<210> 7

<211> 39

<212> DNA

<213> Artificial Sequence

<220>

<223> PCR primer RNII-Nde for cloning a gene encoding a polypeptide having a RNaseHII activity from Bacillus caldotenax

<400> 7

tagaagaggg agaggcatat gaagcggtat acggtgaaa 39

<210> 8

<211> 780

<212> DNA

<213> Bucillus caldotenax

<400> 8

atgaagcggt atacggtgaa agacattgaa gcgctgcttc cgaagcttgg cgcggacgac 60

ccgcgctggg agatgctgcg gcaggatgag cgaaaaagcg tgcaggcgct tcttgcccgt 120

tttgaaaggc agaaagcgcg ccggcacgcc atcgagcagc ggtgggaaga actaatgcgt 180

tatgagaggg aactatacgc cgctggcgtt agacggatcg ccggcattga tgaggccggg 240

cgcggcccgc tggccggccc ggtcgtcgcc gccgcggtca tcttgccgaa agacgcctat 300
ttgccggggc ttgacgactc gaagcggctg acgccggaaa agcgcgaggc attgtttgcg 360
caaattgaag cgtgcgccgt cgccatcggc atcggcatcg tcagcgcggc ggagatcgat 420
gaaaggaata tttacgaagc gacaaggcaa gcgatggcga aagcggtgaa cgccctttcc 480
ccgccgcctg aacatttgct tgttgatgcg atggcggtgc cgtgcccact gccgcaacag 540
cgcctcataa aaggagacgc caacagcgct tcaatcgccg ctgcgtcggt catcgccaaa 600
gtgacgcgcg accggtggat gaaagaactg gatcgccgct atccacaata cgggttcgcg 660
cgccatatgg gctacggaac gccggaacat ttcgaggcga tccgccgcta cggcgttacg 720
cctgagcacc gtcgttcgtt cgcaccggtg agggaggtgc tgaaggcgag cgagcagctc 780

<210> 9
<211> 260
<212> PRT
<213> Bucillus caldotenax

<400> 9
Met Lys Arg Tyr Thr Val Lys Asp Ile Glu Ala Leu Leu Pro Lys
1 5 10 15
Leu Gly Ala Asp Asp Pro Arg Trp Glu Met Leu Arg Gln Asp Glu
20 25 30
Arg Lys Ser Val Gln Ala Leu Leu Ala Arg Phe Glu Arg Gln Lys
35 40 45
Ala Arg Arg His Ala Ile Glu Gln Arg Trp Glu Glu Leu Met Arg
50 55 60
Tyr Glu Arg Glu Leu Tyr Ala Ala Gly Val Arg Arg Ile Ala Gly
65 70 75
Ile Asp Glu Ala Gly Arg Gly Pro Leu Ala Gly Pro Val Val Ala
80 85 90
Ala Ala Val Ile Leu Pro Lys Asp Ala Tyr Leu Pro Gly Leu Asp

95 100 105

Asp Ser Lys Arg Leu Thr Pro Glu Lys Arg Glu Ala Leu Phe Ala

110 115 120

Gln Ile Glu Ala Cys Ala Val Ala Ile Gly Ile Gly Ile Val Ser

125 130 135

Ala Ala Glu Ile Asp Glu Arg Asn Ile Tyr Glu Ala Thr Arg Gln

140 145 150

Ala Met Ala Lys Ala Val Asn Ala Leu Ser Pro Pro Pro Glu His

155 160 165

Leu Leu Val Asp Ala Met Ala Val Pro Cys Pro Leu Pro Gln Gln

170 175 180

Arg Leu Ile Lys Gly Asp Ala Asn Ser Ala Ser Ile Ala Ala Ala

185 190 195

Ser Val Ile Ala Lys Val Thr Arg Asp Arg Trp Met Lys Glu Leu

200 205 210

Asp Arg Arg Tyr Pro Gln Tyr Gly Phe Ala Arg His Met Gly Tyr

215 220 225

Gly Thr Pro Glu His Phe Glu Ala Ile Arg Arg Tyr Gly Val Thr

230 235 240

Pro Glu His Arg Arg Ser Phe Ala Pro Val Arg Glu Val Leu Lys

245 250 255

Ala Ser Glu Gln Leu

260

<210> 10

<211> 20

<212> DNA

<213> Artificial Sequence

<220>

<223> PCR primer BsuIII-1 for cloning a gene encoding a polypeptide having a RNaseHIII activity from Bacillus caldotenax

<400> 10

ggtaaggtct tgttycargg 20

<210> 11

<211> 20

<212> DNA

<213> Artificial Sequence

<220>

<223> PCR primer BsuIII-3 for cloning a gene encoding a polypeptide having a RNaseHIII activity from Bacillus caldotenax

<400> 11

ggaaccggag attayttygg 20

<210> 12

<211> 20

<212> DNA

<213> Artificial Sequence

<220>

<223> PCR primer BsuIII-6 for cloning a gene encoding a polypeptide having a RNaseHIII activity from Bacillus caldotenax

<400> 12

atgattgaag cagcngcnac 20

<210> 13

<211> 20

<212> DNA

<213> Artificial Sequence

<220>

<223> PCR primer BsuIII-8 for cloning a gene encoding a polypeptide having a RNaseHIII activity from Bacillus caldotenax

<400> 13

gtattggcga aatgnarytt 20

<210> 14

<211> 20

<212> DNA

<213> Artificial Sequence

<220>

<223> PCR primer RNIII-S3 for cloning a gene encoding a polypeptide having a RNaseHIII activity from Bacillus caldotenax

<400> 14

cccgatcgtc gtcgccgccg 20

<210> 15

<211> 20

<212> DNA

<213> Artificial Sequence

<220>

<223> PCR primer BcaRNIII-3 for cloning a gene encoding a polypeptide having a RNaseHIII activity from Bacillus caldotenax

<400> 15

gatacgtgga cactttccgc 20

<210> 16

<211> 915

<212> DNA

<213> Bucillus caldotenax

<400> 16

gtgattcaag ccgaccaaca gctgcttgac gccttgcgcg cccactacca agacgcctta 60
tccgaccggc ttccggctgg agcgttgttt gccgtcaagc gcccggatgt cgtcatcacc 120
gcctaccgct caggcaaagt gctgtttcaa gggaaagcgg cggagcaaga agcagcgaaa 180
tggatatcag gggcgagcgc ctcaaacgaa acagctgacc accagccgtc cgctttggca 240
gctcatcaac tcgggtctct ttccgccatc ggttccgatg aagtcggcac cggcgattat 300
ttcggcccga tcgtcgtcgc cgccgcctac gtggatcggc cgcatatcgc caaaatcgcg 360
gcgcttggcg tgaaagattc gaaacaattg aacgatgagg caatcaaacg gatcgccccc 420
gccatcatgg aaaccgtgcc gcatgcggtc accgtgttgg acaatgccga atacaaccgc 480
tggcagcgaa gcggcatgcc gcagacgaaa atgaaagcgc tccttcacaa ccggacgctc 540
gtgaaactcg ttgacgccat cgcgcccgcc gaaccagaag caatcatcat cgacgaattt 600
ttaaaacggg attcgtattt ccgttacctt tccgatgaag atcgcattat ccgcgagcgg 660
gtgcactgcc ttcccaaggc ggaaagtgtc cacgtatcag tcgccgccgc ctcgatcatc 720
gcccgctatg tgtttttaga ggagatggag caattatccc gcgccgtcgg cctcctgctt 780
ccaaaaggcg ccggcgccat tgtcgatgaa gccgcggcca acatcatccg cgcgcggggg 840

gcggaagcgc ttgagacatg cgccaagctt catttcgcca atacaaaaaa ggcgctggac 900

atcgccaaac gccgg 915

<210> 17

<211> 305

<212> PRT

<213> Bucillus caldotenax

<400> 17

Met Ile Gln Ala Asp Gln Gln Leu Leu Asp Ala Leu Arg Ala His
1 5 10 15

Tyr Gln Asp Ala Leu Ser Asp Arg Leu Pro Ala Gly Ala Leu Phe
20 25 30

Ala Val Lys Arg Pro Asp Val Val Ile Thr Ala Tyr Arg Ser Gly
35 40 45

Lys Val Leu Phe Gln Gly Lys Ala Ala Glu Gln Glu Ala Ala Lys
50 55 60

Trp Ile Ser Gly Ala Ser Ala Ser Asn Glu Thr Ala Asp His Gln
65 70 75

Pro Ser Ala Leu Ala Ala His Gln Leu Gly Ser Leu Ser Ala Ile
80 85 90

Gly Ser Asp Glu Val Gly Thr Gly Asp Tyr Phe Gly Pro Ile Val
95 100 105

Val Ala Ala Ala Tyr Val Asp Arg Pro His Ile Ala Lys Ile Ala
110 115 120

Ala Leu Gly Val Lys Asp Ser Lys Gln Leu Asn Asp Glu Ala Ile
125 130 135

Lys Arg Ile Ala Pro Ala Ile Met Glu Thr Val Pro His Ala Val
140 145 150

Thr Val Leu Asp Asn Ala Glu Tyr Asn Arg Trp Gln Arg Ser Gly
155 160 165
Met Pro Gln Thr Lys Met Lys Ala Leu Leu His Asn Arg Thr Leu
170 175 180
Val Lys Leu Val Asp Ala Ile Ala Pro Ala Glu Pro Glu Ala Ile
185 190 195
Ile Ile Asp Glu Phe Leu Lys Arg Asp Ser Tyr Phe Arg Tyr Leu
200 205 210
Ser Asp Glu Asp Arg Ile Ile Arg Glu Arg Val His Cys Leu Pro
215 220 225
Lys Ala Glu Ser Val His Val Ser Val Ala Ala Ala Ser Ile Ile
230 235 240
Ala Arg Tyr Val Phe Leu Glu Glu Met Glu Gln Leu Ser Arg Ala
245 250 255
Val Gly Leu Leu Leu Pro Lys Gly Ala Gly Ala Ile Val Asp Glu
260 265 270
Ala Ala Ala Asn Ile Ile Arg Ala Arg Gly Ala Glu Ala Leu Glu
275 280 285
Thr Cys Ala Lys Leu His Phe Ala Asn Thr Lys Lys Ala Leu Asp
290 295 300
Ile Ala Lys Arg Arg
305

<210> 18
<211> 39
<212> DNA
<213> Artificial Sequence

<220>

<223> PCR primer BcaRNIIINde for amplifying a gene encoding a polypeptide having a RNaseHIII activity from Bacillus caldotenax

<400> 18
cgaacgttgt caaaccatat gattcaagcc gaccaacag 39

<210> 19
<211> 663
<212> DNA
<213> Pyrococcus horikoshii

<400> 19
atgaaggttg ctggagttga tgaagcgggg agggggccgg taattggccc gttagtaatt 60
ggagtagccg ttatagatga gaaaaatatt gagaggttac gtgacattgg ggttaaagac 120
tccaaacaat taactcctgg gcaacgtgaa aaactattta gcaaattaat agatatccta 180
gacgattatt atgttcttct cgttaccccc aaggaaatag atgagaggca tcattctatg 240
aatgaactag aagctgagaa attcgttgta gccttgaatt ctttaaggat caagccgcag 300
aagatatatg tggactctgc cgatgtagat cctaagaggt ttgctagtct aataaaggct 360
gggttgaaat atgaagccac ggttatcgcc gagcataaag ccgatgcaaa gtatgagata 420
gtatcggcag catcaataat tgcaaaggtc actagggata gagagataga gaagctaaag 480
caaaagtatg gggaatttgg ttctggctat ccgagtgatc cgagaactaa ggagtggctt 540
gaagaatatt acaaacaata tggtgacttt cctccaatag ttaggagaac ttgggaaacc 600
gctaggaaga tagaggaaag gtttagaaaa aatcagctaa cgcttgataa attccttaag 660
tga 663

<210> 20
<211> 33
<212> DNA

<213> Artificial Sequence

<220>

<223> PCR primer 1650Nde for cloning a gene encoding a polypeptide having a RNaseHII activity from Pyrococcus furiosus

<400> 20

caggaggaga gacatatgaa aatagggga att 33

<210> 21

<211> 33

<212> DNA

<213> Artificial Sequence

<220>

<223> PCR primer 1650Bam for cloning a gene encoding a polypeptide having a RNaseHII activity from Pyrococcus furiosus

<400> 21

gaaggttgtg gatccacttt ctaaggtttc tta 33

<210> 22

<211> 672

<212> DNA

<213> Pyrococcus furiosus

<400> 22

ATGAAAATAG GGGGAATTGA CGAAGCAGGA AGAGGACCAG CGATAGGGCC ATTAGTAGTA 60

GCTACTGTCG TCGTTGATGA GAAAAACATT GAGAAGCTCA GAAACATTGG AGTAAAAGAC 120

TCCAAACAAC TAACACCCCA TGAAAGGAAG AATTTATTTT CCCAGATAAC CTCAATAGCG 180

GATGATTACA AAATAGTGAT AGTATCCCCA GAAGAAATCG ACAATAGATC AGGAACAATG 240

AACGAGTTAG AGGTAGAGAA GTTTGCTCTC GCCTTAAATT CGCTTCAGAT AAAACCAGCT 300

CTTATATACG CTGATGCAGC GGATGTAGAT GCCAATAGAT TTGCAAGCTT GATAGAGAGA 360

AGACTCAATT ATAAGGCGAA GATTATTGCC GAACACAAGG CCGATGCAAA GTATCCAGTA 420

GTTTCAGCAG CTTCAATACT TGCAAAGGTT GTTAGGGATG AGGAAATTGA AAAATTAAAA 480

AAGCAATATG GAGACTTTGG CTCTGGGTAT CCAAGTGATC CAAAAACCAA GAAATGGCTT 540

GAAGAGTACT ACAAAAAACA CAACTCTTTC CCTCCAATAG TCAGACGAAC CTGGGAAACT 600

GTAAGAAAAA TAGAGGAAAG CATTAAAGCC AAAAAATCCC AGCTAACGCT TGATAAATTC 660

TTTAAGAAAC CT 672

<210> 23

<211> 224

<212> PRT

<213> Pyrococcus furiosus

<400> 23

Met Lys Ile Gly Gly Ile Asp Glu Ala Gly Arg Gly Pro Ala Ile
1 5 10 15

Gly Pro Leu Val Val Ala Thr Val Val Val Asp Glu Lys Asn Ile
20 25 30

Glu Lys Leu Arg Asn Ile Gly Val Lys Asp Ser Lys Gln Leu Thr
35 40 45

Pro His Glu Arg Lys Asn Leu Phe Ser Gln Ile Thr Ser Ile Ala
50 55 60

Asp Asp Tyr Lys Ile Val Ile Val Ser Pro Glu Glu Ile Asp Asn
65 70 75

Arg Ser Gly Thr Met Asn Glu Leu Glu Val Glu Lys Phe Ala Leu
80 85 90

Ala Leu Asn Ser Leu Gln Ile Lys Pro Ala Leu Ile Tyr Ala Asp
95 100 105
Ala Ala Asp Val Asp Ala Asn Arg Phe Ala Ser Leu Ile Glu Arg
110 115 120
Arg Leu Asn Tyr Lys Ala Lys Ile Ile Ala Glu His Lys Ala Asp
125 130 135
Ala Lys Tyr Pro Val Val Ser Ala Ala Ser Ile Leu Ala Lys Val
140 145 150
Val Arg Asp Glu Glu Ile Glu Lys Leu Lys Lys Gln Tyr Gly Asp
155 160 165
Phe Gly Ser Gly Tyr Pro Ser Asp Pro Lys Thr Lys Lys Trp Leu
170 175 180
Glu Glu Tyr Tyr Lys Lys His Asn Ser Phe Pro Pro Ile Val Arg
185 190 195
Arg Thr Trp Glu Thr Val Arg Lys Ile Glu Glu Ser Ile Lys Ala
200 205 210
Lys Lys Ser Gln Leu Thr Leu Asp Lys Phe Phe Lys Lys Pro
215 220

<210> 24
<211> 28
<212> DNA
<213> Artificial Sequence

<220>
<223> PCR primer 915-F1 for cloning a gene encoding a polypeptide having a RNaseHII activity from Thermotoga maritima

<400> 24

aaaaagcttg ggaatagatg agctttac 28

<210> 25

<211> 26

<212> DNA

<213> Artificial Sequence

<220>

<223> PCR primer 915-F2 for cloning a gene encoding a polypeptide having a RNaseHII activity from Thermotoga maritima

<400> 25

aaaccatggg aatagatgag ctttac 26

<210> 26

<211> 29

<212> DNA

<213> Artificial Sequence

<220>

<223> PCR primer 915-R1 for cloning a gene encoding a polypeptide having a RNaseHII activity from Thermotoga maritima

<400> 26

aaatctagat cctcaacttt gtcgatgtg 29

<210> 27

<211> 30

<212> DNA

<213> Artificial Sequence

<220>

<223> PCR primer 915-R2 for cloning a gene encoding a polypeptide having a RNaseHII activity from Thermotoga maritima

<400> 27

aatctagatt aaaaaagagg gagattatgg 30

<210> 28

<211> 25

<212> DNA

<213> Artificial Sequence

<220>

<223> Designed chimeric oligonucleotide primer designated as pUC19 upper 150 to amplify a portion of plasmid pUC19. "nucleotides 24 to 25 are ribonucleotides-other nucleotides are deoxyribonucleotides"

<400> 28

ggtgtcacgc tcgtcgtttg gtaug 25

<210> 29

<211> 30

<212> DNA

<213> Artificial Sequence

<220>

<223> Designed chimeric oligonucleotide primer designated as MR1N3 to amplify a portion of plasmid pUC19. "nucleotides 28 to 30 are ribonucleotides-other nucleotides are deoxyribonucleotides"

<400> 29

tttacacttt atgcttccgg ctcgtatguu 30

<210> 30

<211> 17

<212> DNA

<213> Artificial Sequence

<220>

<223> Designed oligonucleotide primer designated as M13M4

<400> 30

gttttcccag tcacgac 17

<210> 31

<211> 23

<212> DNA

<213> Artificial Sequence

<220>

<223> Designed chimeric oligonucleotide primer to amplify a portion of vero toxin 1-encoding sequence from hemorrhagic Escherichia coli O-157. "nucleotides 21 to 23 are ribonucleotides-other nucleotides are deoxyribonucleotides"

<400> 31

tgtcattcgc tctgcaatag gua 23

<210> 32
<211> 23
<212> DNA
<213> Artificial Sequence

<220>
<223> Designed chimeric oligonucleotide primer to amplify a portion of vero toxin 1-encoding sequence from hemorrhagic Escherichia coli O-157. “nucleotides 21 to 23 are ribonucleotides-other nucleotides are deoxyribonucleotides”

<400> 32
caccagacaa tgtaaccgct guu 23

<210> 33
<211> 20
<212> DNA
<213> Artificial Sequence

<220>
<223> Designed chimeric oligonucleotide primer to amplify a portion of vero toxin 2-encoding sequence from hemorrhagic Escherichia coli O-157. “nucleotides 18 to 20 are ribonucleotides-other nucleotides are deoxyribonucleotides”

<400> 33
tactgggttt ttcttcggua 20

<210> 34

<211> 20

<212> DNA

<213> Artificial Sequence

<220>

<223> Designed chimeric oligonucleotide primer to amplify a portion of vero toxin 2-encoding sequence from hemorrhagic Escherichia coli O-157. "nucleotides 18 to 20 are ribonucleotides-other nucleotides are deoxyribonucleotides"

<400> 34

atagacatca agccctcgua 20

<210> 35

<211> 22

<212> DNA

<213> Artificial Sequence

<220>

<223> Designed oligonucleotide primer designated as MCR-F to amplify a long DNA fragment

<400> 35

ccattcaggc tgcgcaactg tt 22

<210> 36

<211> 22

<212> DNA

<213> Artificial Sequence

<220>

<223> Designed oligonucleotide primer designated as MCR-R to amplify a long DNA fragment

<400> 36

tggcacgaca ggtttcccga ct 22

<210> 37

<211> 24

<212> DNA

<213> Artificial Sequence

<220>

<223> Designed chimeric oligonucleotide primer designated as MF2N3(24) to amplify a long DNA fragment. "nucleotides 22 to 24 are ribonucleotides-other nucleotides are deoxyribonucleotides"

<400> 37

gctgcaaggc gattaagttg ggua 24

<210> 38

<211> 24

<212> DNA

<213> Artificial Sequence

<220>

<223> Designed chimeric oligonucleotide primer designated as MR1N3(24) to amplify a long DNA fragment. "nucleotides 22 to 24 are ribonucleotides-other nucleotides are deoxyribonucleotides"

<400> 38

ctttatgctt ccggctcgta tguu 24

<210> 39

<211> 20

<212> DNA

<213> Artificial Sequence

<220>

<223> Designed oligonucleotide primer to amplify a portion of lambda DNA. "nucleotides 18 to 20 are ribonucleotides-other nucleotides are deoxyribonucleotides"

<400> 39

cctttctctg tttttgtccg 20

<210> 40

<211> 20

<212> DNA

<213> Artificial Sequence

<220>

<223> Designed chimeric oligonucleotide primer to amplify a portion of lambda DNA. "nucleotides 18 to 20 are ribonucleotides-other nucleotides are deoxyribonucleotides"

<400> 40
aagcacctca ttaccctugc 20

<210> 41
<211> 24
<212> DNA
<213> Artificial Sequence

<220>
<223> Designed oligonucleotide primer to amplify a portion of lambda DNA

<400> 41
gggcggcgac ctcgcgggtt ttcg 24

<210> 42
<211> 24
<212> DNA
<213> Artificial Sequence

<220>
<223> Designed oligonucleotide primer to amplify a portion of lambda DNA

<400> 42
gctgcttatg ctctataaag tagg 24

<210> 43
<211> 20
<212> DNA

<213> Artificial Sequence

<220>

<223> Designed chimeric oligonucleotide primer to amplify a portion of Flavobacterium species DNA. “nucleotides 18 to 20 are ribonucleotides-other nucleotides are deoxyribonucleotides”

<400> 43

aggaatcttt atttaccaug 20

<210> 44

<211> 20

<212> DNA

<213> Artificial Sequence

<220>

<223> Designed chimeric oligonucleotide primer to amplify a portion of Flavobacterium species DNA. “nucleotides 18 to 20 are ribonucleotides-other nucleotides are deoxyribonucleotides”

<400> 44

tggtgtttaa acttattgcg 20

<210> 45

<211> 24

<212> DNA

<213> Artificial Sequence

<220>

<223> Designed oligonucleotide primer to amplify a portion of Flavobacterium species DNA.

<400> 45
ccatcagcta taaacacaaa cagc 24

<210> 46
<211> 24
<212> DNA
<213> Artificial Sequence

<220>
<223> Designed oligonucleotide primer to amplify a portion of Flavobacterium species DNA.

<400> 46
tgttttgacc aaacatagta atgc 24

<210> 47
<211> 21
<212> DNA
<213> Artificial Sequence

<220>
<223> Designed chimeric oligonucleotide primer to amplify a portion of vero toxin 2-encoding sequence from hemorrhagic Escherichia coli O-157. “nucleotides 19 to 21 are ribonucleotides-other nucleotides are deoxyribonucleotides”

<400> 47

tcgttaaata gtatacggac a 21

<210> 48

<211> 20

<212> DNA

<213> Artificial Sequence

<220>

<223> Designed chimeric oligonucleotide primer to amplify a portion of vero toxin 2-encoding sequence from hemorrhagic Escherichia coli O-157. “nucleotides 18 to 20 are ribonucleotides-other nucleotides are deoxyribonucleotides”

<400> 48

tgctcaataa tcagacgaag 20

<210> 49

<211> 24

<212> DNA

<213> Artificial Sequence

<220>

<223> Designed oligonucleotide primer to amplify a portion of vero toxin 2-encoding sequence from hemorrhagic Escherichia coli O-157.

<400> 49

aaatggggta ctgtgcctgt tact 24

<210> 50

<211> 24

<212> DNA

<213> Artificial Sequence

<220>

<223> Designed oligonucleotide primer to amplify a portion of vero toxin 2-encoding sequence from hemorrhagic Escherichia coli O-157.

<400> 50

ctctgtatct gcctgaagcg taag 24

<210> 51

<211> 21

<212> DNA

<213> Artificial Sequence

<220>

<223> Designed chimeric oligonucleotide primer to amplify a portion of vero toxin 2-encoding sequence from hemorrhagic Escherichia coli O-157. "nucleotides 18 to 20 are ribonucleotides-other nucleotides are deoxyribonucleotides"

<400> 51

tacctgggtt tttcttcggu a 20

<210> 52

<211> 20

<212> DNA

<213> Artificial Sequence

<220>

<223> Designed chimeric oligonucleotide primer to amplify a portion of vero toxin 2-encoding sequence from hemorrhagic Escherichia coli O-157. "nucleotides 18 to 20 are ribonucleotides-other nucleotides are deoxyribonucleotides"

<400> 52

atagactttt cgacccaaca 20

<210> 53

<211> 20

<212> DNA

<213> Artificial Sequence

<220>

<223> Designed chimeric oligonucleotide primer to amplify a portion of vero toxin 2-encoding sequence from hemorrhagic Escherichia coli O-157. "nucleotides 18 to 20 are ribonucleotides-other nucleotides are deoxyribonucleotides"

<400> 53

atagacatca agccctcgua 20

<210> 54

<211> 21

<212> DNA

<213> Artificial Sequence

<220>

<223> Designed oligonucleotide primer to amplify a portion of vero toxin 2-encoding sequence from hemorrhagic Escherichia coli O-157.

<400> 54

tcgttaaata gtatacggac a 21

<210> 55

<211> 20

<212> DNA

<213> Artificial Sequence

<220>

<223> Designed oligonucleotide primer to amplify a portion of vero toxin 2-encoding sequence from hemorrhagic Escherichia coli O-157.

<400> 55

atagacatca agccctcgta 20

<210> 56

<211> 20

<212> DNA

<213> Artificial Sequence

<220>

<223> Designed chimeric oligonucleotide primer to amplify a portion of lambda DNA. "nucleotides 18 to 20 are ribonucleotides-other nucleotides are deoxyribonucleotides"

<400> 56
gaacaatgga agtcaacaaa 20

<210> 57
<211> 20
<212> DNA
<213> Artificial Sequence

<220>
<223> Designed oligonucleotide primer to amplify a portion of viroid CSVd.

<400> 57
tacttgtggt tcctgtggtg 20

<210> 58
<211> 20
<212> DNA
<213> Artificial Sequence

<220>
<223> Designed oligonucleotide primer to amplify a portion of viroid CSVd.

<400> 58
atactaaggt tccaagggct 20

<210> 59

<211> 18

<212> DNA

<213> Artificial Sequence

<220>

<223> Designed chimeric oligonucleotide primer to amplify a portion of viroid CSVd. “nucleotides 16 to 18 are ribonucleotides-other nucleotides are deoxyribonucleotides”

<400> 59

ggaaacctgg aggaaguc 18

<210> 60

<211> 20

<212> DNA

<213> Artificial Sequence

<220>

<223> Designed chimeric oligonucleotide primer to amplify a portion of viroid CSVd. “nucleotides 18 to 20 are ribonucleotides-other nucleotides are deoxyribonucleotides”

<400> 60

gtgaaaaccc tgtttaggau 20

<210> 61

<211> 20

<212> DNA

<213> Artificial Sequence

<220>

<223> Designed chimeric oligonucleotide primer to amplify a portion of Flavobacterium species DNA. “nucleotides 18 to 20 are ribonucleotides-other nucleotides are deoxyribonucleotides”

<400> 61

acctagatat aagctctaca 20

<210> 62

<211> 20

<212> DNA

<213> Artificial Sequence

<220>

<223> Designed chimeric oligonucleotide primer to amplify a portion of Flavobacterium species DNA. “nucleotides 18 to 20 are ribonucleotides-other nucleotides are deoxyribonucleotides”

<400> 62

aaatagatgt tttagcagag 20

<210> 63

<211> 20

<212> DNA

<213> Artificial Sequence

<220>

<223> Designed chimeric oligonucleotide primer to amplify a portion of Flavobacterium species DNA. "nucleotides 18 to 20 are ribonucleotides-other nucleotides are deoxyribonucleotides"

<400> 63
atagataaaa aaaactccac 20

<210> 64
<211> 21
<212> DNA
<213> Artificial Sequence

<220>
<223> Designed chimeric oligonucleotide primer to amplify a portion of vero toxin 2-encoding sequence from hemorrhagic Escherichia coli O-157. "nucleotides 19 to 21 are ribonucleotides-nucloetide 18 is inosine-other nucleotides are deoxyribonucleotides"

<400> 64
tcgttaaata gtatacgiac a 21

<210> 65
<211> 21
<212> DNA
<213> Artificial Sequence

<220>
<223> Designed chimeric oligonucleotide primer to amplify a portion of vero toxin 2-encoding sequence from hemorrhagic Escherichia coli O-157. "nucleotides 19 to 21 are ribonucleotides-nucleotide 17 is inosine other

nucleotides are deoxyribonucleotides”

<400> 65

tcgttaaata gtatacigac a 21

<210> 66

<211> 21

<212> DNA

<213> Artificial Sequence

<220>

<223> Designed chimeric oligonucleotide primer to amplify a portion of vero toxin 2-encoding sequence from hemorrhagic Escherichia coli O-157. “nucleotides 19 to 21 are ribonucleotides-nucleotide 16 is inosine-other nucleotides are deoxyribonucleotides”

<400> 66

tcgttaaata gtataiggac a 21

<210> 67

<211> 20

<212> DNA

<213> Artificial Sequence

<220>

<223> Designed chimeric oligonucleotide primer to amplify a portion of vero toxin 2-encoding sequence from hemorrhagic Escherichia coli O-157. “nucleotides 18 to 20 are ribonucleotides-nucleotide 17 is inosine-other nucleotides are deoxyribonucleotides”

<400> 67
tgctcaataa tcagaciaag 20

<210> 68
<211> 20
<212> DNA
<213> Artificial Sequence

<220>
<223> Designed chimeric oligonucleotide primer to amplify a portion of vero toxin 2-encoding sequence from hemorrhagic Escherichia coli O-157. "nucleotides 18 to 20 are ribonucleotides-nucleotide 16 is inosine-other nucleotides are deoxyribonucleotides"

<400> 68
tgctcaataa tcagaigaag 20

<210> 69
<211> 20
<212> DNA
<213> Artificial Sequence

<220>
<223> Designed chimeric oligonucleotide primer to amplify a portion of vero toxin 2-encoding sequence from hemorrhagic Escherichia coli O-157. "nucleotides 18 to 20 are ribonucleotides-nucleotide 15 is inosine-other nucleotides are deoxyribonucleotides"

<400> 69

tgctcaataa tcagicgaag 20

<210> 70

<211> 21

<212> DNA

<213> Artificial Sequence

<220>

<223> Designed chimeric oligonucleotide primer to amplify a portion of vero toxin 2-encoding sequence from hemorrhagic Escherichia coli O-157. "nucleotides 9 to 11 and 19 to 21 are ribonucleotides-other nucleotides are deoxyribonucleotides"

<400> 70

tacctggguu uttcttcggu a 21

<210> 71

<211> 20

<212> DNA

<213> Artificial Sequence

<220>

<223> Designed chimeric oligonucleotide primer to amplify a portion of vero toxin 2-encoding sequence from hemorrhagic Escherichia coli O-157. "nucleotides 8 to 10 and 18 to 20 are ribonucleotides-other nucleotides are deoxyribonucleotides"

<400> 71

atagacauca agccctcgua 20

<210> 72

<211> 20

<212> DNA

<213> Artificial Sequence

<220>

<223> Designed chimeric oligonucleotide primer to amplify a portion of vero toxin 2-encoding sequence from hemorrhagic Escherichia coli O-157. "nucleotides 18 to 20 are ribonucleotides-other nucleotides are deoxyribonucleotides"

<400> 72

gtcccctgag atatatguuc 20

<210> 73

<211> 30

<212> DNA

<213> Artificial Sequence

<220>

<223> Designed oligonucleotide probe to detect a DNA fragment amplifing a portion of vero toxin 2-encoding sequence from hemorrhagic Escherichia coli O-157.

<400> 73

ccaacaaagt tatgtctctt cgttaaatag 30

<210> 74

<211> 20

<212> DNA

<213> Artificial Sequence

<220>

<223> Designed chimeric oligonucleotide primer to amplify a portion of iNOS-encoding sequence from mouse. "nucleotides 18 to 20 are ribonucleotides-other nucleotides are deoxyribonucleotides"

<400> 74

atgccattga gttcatcaac 20

<210> 75

<211> 19

<212> DNA

<213> Artificial Sequence

<220>

<223> Designed chimeric oligonucleotide primer to amplify a portion of iNOS-encoding sequence from mouse. "nucleotides 17 to 19 are ribonucleotides-other nucleotides are deoxyribonucleotides"

<400> 75

tcttggtggc aaagatgag 19

<210> 76

<211> 20

<212> DNA

<213> Artificial Sequence

<220>

<223> Designed oligonucleotide primer to amplify a portion of iNOS-encoding sequence from mouse.

<400> 76

atgccattga gttcatcaac 20

<210> 77

<211> 19

<212> DNA

<213> Artificial Sequence

<220>

<223> Designed oligonucleotide primer to amplify a portion of iNOS-encoding sequence from mouse

<400> 77

tcttggtggc aaagatgag 19

<210> 78

<211> 20

<212> DNA

<213> Artificial Sequence

<220>

<223> Designed oligonucleotide primer designated as GMO-PCR-F 20mer

<400> 78
atcgttgaag atgcctctgc 20

<210> 79
<211> 20
<212> DNA
<213> Artificial Sequence

<220>
<223> designed oligonucleotide primer designated as GMO-PCR-R 20mer

<400> 79
tccgtatgat cgcgcgtcat 20

<210> 80
<211> 20
<212> DNA
<213> Artificial Sequence

<220>
<223> Designed chimeric oligonucleotide primer designated as GMO-S1 20mer. "nucleotides 19 to 20 are ribonucleotides-other nucleotides are deoxyribonucleotides"

<400> 80
tttggagagg acacgctgac 20

<210> 81
<211> 20

<212> DNA

<213> Artificial Sequence

<220>

<223> Designed oligonucleotide primer designated as GMO-S2 20mer. "nucleotides 19 to 20 are ribonucleotides-other nucleotides are deoxyribonucleotides"

<400> 81

ggacacgctg acaagctgac 20

<210> 82

<211> 20

<212> DNA

<213> Artificial Sequence

<220>

<223> Designed oligonucleotide primer designated as GMO-A1 20mer. "nucleotides 19 to 20 are ribonucleotides-other nucleotides are deoxyribonucleotides"

<400> 82

ggctgtagcc actgatgcug 20

<210> 83

<211> 20

<212> DNA

<213> Artificial Sequence

<220>

<223> Designed oligonucleotide primer designated as GMO-A2 20 mer. “nucleotides 19 to 20 are ribonucleotides-other nucleotides are deoxyribonucleotides”

<400> 83

ttccggaaag gccagaggau 20

<210> 84

<211> 21

<212> DNA

<213> Artificial Sequence

<220>

<223> Designed chimeric oligonucleotide primer to amplify a portion of vero toxin 2-encoding sequence from hemorrhagic Escherichia coli O-157. “nucleotides 18 to 20 are (alpha-thio)ribonucleotides-other nucleotides are deoxyribonucleotides”

<400> 84

tactgggtt tttcttcggu a 20

<210> 85

<211> 20

<212> DNA

<213> Artificial Sequence

<220>

<223> Designed chimeric oligonucleotide primer to amplify a portion of vero toxin 2-encoding sequence from hemorrhagic Escherichia coli O-157. “nucleotides 18 to 20 are (alpha-thio)ribonucleotides-other nucleotides are deoxyribonucleotides”

<400> 85
atagacatca agccctcgua 20

<210> 86
<211> 22
<212> DNA
<213> Artificial Sequence

<220>
<223> Designed chimeric oligonucleotide primer to amplify a portion of INOS-encoding sequence from mouse. “nucleotides 20 to 22 are ribonucleotides-other nucleotides are deoxyribonucleotides”

<400> 86
tcatgccatt gagttcatca ac 22

<210> 87
<211> 22
<212> DNA
<213> Artificial Sequence

<220>
<223> Designed chimeric oligonucleotide primer to amplify a portion of INOS-encoding sequence from mouse. “nucleotides 20 to 22 are ribonucleotides-other nucleotides are deoxyribonucleotides”

<400> 87

tggtaggttc ctgttgtttc ua 22

<210> 88

<211> 22

<212> DNA

<213> Artificial Sequence

<220>

<223> Designed oligonucleotide primer to amplify a portion of INOS-encoding sequence from mouse.

<400> 88

tcatgccatt gagttcatca ac 22

<210> 89

<211> 22

<212> DNA

<213> Artificial Sequence

<220>

<223> Designed oligonucleotide primer to amplify a portion of INOS-encoding sequence from mouse.

<400> 89

tggtaggttc ctgttgtttc ta 22

<210> 90

<211> 20

<212> DNA

<213> Artificial Sequence

<220>

<223> Designed chimeric oligonucleotide primer to amplify a portion of lambda DNA."nucleotides 18 to 20 are ribonucleotides-other nucleotides are deoxyribonucleotides"

<400> 90

ctgcgaggcg gtggcaaggg 20

<210> 91

<211> 21

<212> DNA

<213> Artificial Sequence

<220>

<223> Designed chimeric oligonucleotide primer to amplify a portion of lambda DNA."nucleotides 19 to 21 are ribonucleotides-other nucleotides are deoxyribonucleotides"

<400> 91

ctgcctcgct ggccgtgccg c 21

<210> 92

<211> 23

<212> DNA

<213> Artificial Sequence

<220>

<223> Designed chimeric oligonucleotide primer to amplify a portion of INOS-encoding sequence from mouse.“nucleotides 21 to 23 are ribonucleotides-other nucleotides are deoxyribonucleotides”

<400> 92

ctcatgccat tgagttcatc aac 23

<210> 93

<211> 22

<212> DNA

<213> Artificial Sequence

<220>

<223> Designed chimeric oligonucleotide primer to amplify a portion of INOS-encoding sequence from mouse.“nucleotides 20 to 22 are ribonucleotides-other nucleotides are deoxyribonucleotides”

<400> 93

gctggtaggt tcctgttgtu uc 22

<210> 94

<211> 19

<212> DNA

<213> Artificial Sequence

<220>

<223> Designed chimeric oligonucleotide primer to amplify a portion of pDON-AI DNA."nucleotides 17 to 19 are ribonucleotides-other nucleotides are deoxyribonucleotides"

<400> 94

agctctgtat ctggcggac 19

<210> 95

<211> 21

<212> DNA

<213> Artificial Sequence

<220>

<223> Designed chimeric oligonucleotide primer to amplify a portion of pDON-AI DNA."nucleotides 19 to 21 are ribonucleotides-other nucleotides are deoxyribonucleotides"

<400> 95

gatcgggatt tttggactca g 21

<210> 96

<211> 21

<212> DNA

<213> Artificial Sequence

<220>

<223> Designed chimeric oligonucleotide primer to amplify a portion of HPV type16 DNA."nucleotides 19 to 21 are ribonucleotides-other nucleotides are deoxyribonucleotides"

<400> 96
caaaagagaa ctgcaatguu u 21

<210> 97
<211> 21
<212> DNA
<213> Artificial Sequence

<220>
<223> Designed chimeric oligonucleotide primer to amplify a portion of HPV type16 DNA."nucleotides 19 to 21 are ribonucleotides-other nucleotides are deoxyribonucleotides"

<400> 97
gttgcttgca gtacacacau u 21

<210> 98
<211> 27
<212> DNA
<213> Artificial Sequence

<220>
<223> Designed oligonucleotide probe to detect a DNA fragment amplifing a portion of HPV DNA.

<400> 98
gaggacccac aggagcgacc cagaaag 27

<210> 99

<211> 20

<212> DNA

<213> Artificial Sequence

<220>

<223> Designed oligonucleotide primer to amplify a portion of HCV.

<400> 99

cactccacca tgaatcactc 20

<210> 100

<211> 20

<212> DNA

<213> Artificial Sequence

<220>

<223> Designed oligonucleotide primer to amplify a portion of HCV.

<400> 100

ggtgcacggt ctacgagacc 20

<210> 101

<211> 21

<212> DNA

<213> Artificial Sequence

<220>

<223> Designed chimeric oligonucleotide primer to amplify a portion of HCV."nucleotides 19 to 21 are ribonucleotides-other nucleotides are

deoxyribonucleotides"

<400> 101
ctgtgaggaa ctactgtcuu c 21

<210> 102
<211> 18
<212> DNA
<213> Artificial Sequence

<220>
<223> Designed chimeric oligonucleotide primer to amplify a portion of HCV."nucleotides 16 to 18 are ribonucleotides-other nucleotides are deoxyribonucleotides"

<400> 102
gcagaccact atggcucu 18

<210> 103
<211> 30
<212> DNA
<213> Artificial Sequence

<220>
<223> Designed oligonucleotide probe to detect a DNA fragment amplifing portion of HCV.

<400> 103
gtatgagtgt cgtgcagcct ccaggacccc 30

<210> 104

<211> 21

<212> DNA

<213> Artificial Sequence

<220>

<223> Designed chimeric oligonucleotide primer to amplify a portion of adenovirus. "nucleotides 19 to 21 are ribonucleotides-other nucleotides are deoxyribonucleotides"

<400> 104

tgagacatat tatctgccac g 21

<210> 105

<211> 21

<212> DNA

<213> Artificial Sequence

<220>

<223> Designed chimeric oligonucleotide primer to amplify a portion of adenovirus. "nucleotides 19 to 21 are ribonucleotides-other nucleotides are deoxyribonucleotides"

<400> 105

aaatggctag gaggtggaag a 21

<210> 106

<211> 21

<212> DNA

<213> Artificial Sequence

<220>

<223> Designed chimeric oligonucleotide primer to amplify a portion of adenovirus. “nucleotides 19 to 21 are ribonucleotides-other nucleotides are deoxyribonucleotides”

<400> 106

ttatcagcca gtacctctuc g 21

<210> 107

<211> 21

<212> DNA

<213> Artificial Sequence

<220>

<223> Designed oligonucleotide primer to amplify a portion of adenovirus

<400> 107

tgagacatat tatctgccac g 21

<210> 108

<211> 21

<212> DNA

<213> Artificial Sequence

<220>

<223> Designed oligonucleotide primer to amplify a portion of adenovirus.

<400> 108
aaatggctag gaggtggaag a 21

<210> 109
<211> 20
<212> DNA
<213> Artificial Sequence

<220>
<223> Designed oligonucleotide primer to amplify a portion of viroid CSVd.

<400> 109
ggggaaacct ggaggaagtc 20

<210> 110
<211> 20
<212> DNA
<213> Artificial Sequence

<220>
<223> Designed oligonucleotide primer to amplify a portion of viroid CSVd.

<400> 110
cgggtagtag ccaaaggaag 20

<210> 111

<211> 19

<212> DNA

<213> Artificial Sequence

<220>

<223> Designed oligonucleotide primer to amplify a portion of pDON-AI DNA.

<400> 111

agctctgtat ctggcggac 19

<210> 112

<211> 21

<212> DNA

<213> Artificial Sequence

<220>

<223> Designed oligonucleotide primer to amplify a portion of pDON-AI DNA.

<400> 112

gatcgggatt tttggactca g 21

<210> 113

<211> 20

<212> DNA

<213> Artificial Sequence

<220>

<223> Designed chimeric oligonucleotide primer to amplify a portion of verotoxin-1 encoding sequence from hemorrhagic Escherichia coli O-157. "nucleotides 18 to 20 are ribonucleotides-other nucleotides are deoxyribonucleotides"

<400> 113
ggggataatt tgtttgcagu 20

<210> 114
<211> 20
<212> DNA
<213> Artificial Sequence

<220>
<223> Designed chimeric oligonucleotide primer to amplify a portion of verotoxin-1 encoding sequence from hemorrhagic Escherichia coli O-157. "nucleotides 18 to 20 are ribonucleotides-other nucleotides are deoxyribonucleotides"

<400> 114
tcgttcaaca ataagccgua 20

<210> 115
<211> 30
<212> DNA
<213> Artificial Sequence

<220>
<223> Designed oligonucleotide probe to detect a DNA fragment amplifying a portion of verotoxin-1 encoding sequence from hemorrhagic Escherichia coli 0-157.

<400> 115
cgcccttcct ctggatctac ccctctgaca 30

<210> 116
<211> 21
<212> DNA
<213> Artificial Sequence

<220>
<223> Designed chimeric oligonucleotide primer to amplify a portion of botulinum toxin A encoding sequence from Clostridium botulinum."nucleotides 19 to 21 are ribonucleotides-other nucleotides are deoxyribonucleotides"

<400> 116
caccagaagc aaaacaaguu c 21

<210> 117
<211> 23
<212> DNA
<213> Artificial Sequence

<220>
<223> Designed chimeric oligonucleotide primer to amplify a portion of botulinum toxin A encoding sequence from Clostridium botulinum."nucleotides 21 to 23 are ribonucleotides-other nucleotides

are deoxyribonucleotides"

<400> 117
ctattgatgt taacaacatt cuu 23

<210> 118
<211> 30
<212> DNA
<213> Artificial Sequence

<220>
<223> Designed oligonucleotide probe to detect a DNA fragment amplifying a portion of botulinum toxin A encoding sequence from Clostridium botulinum.

<400> 118
gggagttaca aaattatttg agagaattta 30

<210> 119
<211> 21
<212> DNA
<213> Artificial Sequence

<220>
<223> Designed chimeric oligonucleotide primer to amplify a portion of viroid CSVd. "nucleotides 19 to 21 are ribonucleotides-other nucleotides are deoxyribonucleotides"

<400> 119

caccccttcct ttagtttccu u 21

<210> 120

<211> 20

<212> DNA

<213> Artificial Sequence

<220>

<223> Designed chimeric oligonucleotide primer to amplify a portion of viroid CSVd. "nucleotides 18 to 20 are ribonucleotides-other nucleotides are deoxyribonucleotides"

<400> 120

cgttgaagct tcagttguuu 20

<210> 121

<211> 30

<212> DNA

<213> Artificial Sequence

<220>

<223> Designed oligonucleotide probe to detect a DNA fragment amplifying a portion of viroid CSVd.

<400> 121

ccttcctctc ctggagaggt cttctgccct 30

<210> 122

<211> 21

<212> DNA

<213> Artificial Sequence

<220>

<223> Designed chimeric oligonucleotide primer to amplify a portion of viroid CSVd. "nucleotides 19 to 21 are ribonucleotides-other nucleotides are deoxyribonucleotides"

<400> 122

caccettcct ttagtttccu u 21

<210> 123

<211> 21

<212> DNA

<213> Artificial Sequence

<220>

<223> Designed chimeric oligonucleotide primer to amplify a portion of viroid CSVd. "nucleotides 19 to 21 are ribonucleotides-other nucleotides are deoxyribonucleotides"

<400> 123

cgttgaagct tcagttgtuu c 21

<210> 124

<211> 21

<212> DNA

<213> Artificial Sequence

<220>

<223> Designed oligonucleotide primer to amplify a portion of viroid CSVd.

<400> 124

caccccttcct ttagtttcct t 21

<210> 125

<211> 21

<212> DNA

<213> Artificial Sequence

<220>

<223> Designed oligonucleotide primer to amplify a portion of viroid CSVd.

<400> 125

cgttgaagct tcagttgttt c 21

<210> 126

<211> 20

<212> DNA

<213> Artificial Sequence

<220>

<223> Designed chimeric oligonucleotide primer to amplify a portion of c-ki-ras oncogene. "nucleotides 18 to 20 are ribonucleotides-other nucleotides are deoxyribonucleotides"

<400> 126

gactgaatat aaacttgugg 20

<210> 127

<211> 20

<212> DNA

<213> Artificial Sequence

<220>

<223> Designed chimeric oligonucleotide primer to amplify a portion of c-ki-ras oncogene. "nucleotides 18 to 20 are ribonucleotides-other nucleotides are deoxyribonucleotides"

<400> 127

ctattgttgg atcatatucg 20

<210> 128

<211> 20

<212> DNA

<213> Artificial Sequence

<220>

<223> Designed oligonucleotide primer to amplify a portion of c-ki-ras oncogene.

<400> 128

gactgaatat aaacttgtgg 20

<210> 129

<211> 20

<212> DNA

<213> Artificial Sequence

<220>

<223> Designed oligonucleotide primer to amplify a portion of c-ki-ras oncogene.

<400> 129

ctattgttggatcatattcg 20

<210> 130

<211> 20

<212> DNA

<213> Artificial Sequence

<220>

<223> Designed chimeric oligonucleotide primer to amplify a portion of verotoxin-2 encoding sequence from hemorrhagic Escherichia coli O-157."nucleotides 18 to 20 are ribonucleotides-other nucleotides are deoxyribonucleotides"

<400> 130

gacttttcga cccaacaaag 20

<210> 131

<211> 20

<212> DNA

<213> Artificial Sequence

<220>

<223> Designed chimeric oligonucleotide primer to amplify a portion of verotoxin-2 encoding sequence from hemorrhagic Escherichia coli O-157. "nucleotides 18 to 20 are ribonucleotides-other nucleotides are deoxyribonucleotides"

<400> 131

atatccacag caaaataacu 20

<210> 132

<211> 21

<212> DNA

<213> Artificial Sequence

<220>

<223> Designed oligonucleotide primer to amplify a portion of INOS-encoding sequence from mouse.

<400> 132

cacaaggcca catcggattt c 21

<210> 133

<211> 20

<212> DNA

<213> Artificial Sequence

<220>

<223> Designed oligonucleotide primer to amplify a portion of INOS-encoding sequence from mouse.

<400> 133
tgcataccac ttcaacccga g 21

<210> 134
<211> 25
<212> DNA
<213> Artificial Sequence

<220>
<223> Designed oligonucleotide primer designated as pUC19 upper 150 to amplify a portion of plasmid pUC19.

<400> 134
ggtgtcacgc tcgtcgtttg gtatg 25

<210> 135
<211> 25
<212> DNA
<213> Artificial Sequence

<220>
<223> Designed chimeric oligonucleotide primer designated as pUC19 lower NN to amplify a portion of plasmid pUC19.

<400> 135
gataacactg cggccaactt acttc 25

<210> 136

<211> 21

<212> DNA

<213> Artificial Sequence

<220>

<223> Designed chimeric oligonucleotide primer designated as SEA-1 to amplify a portion of Staphylococcus aureus. "nucleotides 19 to 21 are ribonucleotides-other nucleotides are deoxyribonucleotides"

<400> 136

tgtatgtatg gtggtgtaac g 21

<210> 137

<211> 21

<212> DNA

<213> Artificial Sequence

<220>

<223> Designed chimeric oligonucleotide primer designated as SEA-2 to amplify a portion of Staphylococcus aureus. "nucleotides 19 to 21 are ribonucleotides-other nucleotides are deoxyribonucleotides"

<400> 137

taaccgtttc caaaggtacu g 21

<210> 138

<211> 19

<212> DNA

<213> Artificial Sequence

<220>

<223> Designed chimeric oligonucleotide primer designated as HCV-F3 to amplify a portion of HCV. "nucleotides 17 to 19 are ribonucleotides-other nucleotides are deoxyribonucleotides"

<400> 138

gcgtctagcc atggcguua 19

<210> 139

<211> 18

<212> DNA

<213> Artificial Sequence

<220>

<223> Designed chimeric oligonucleotide primer designated as HCV-R1 to amplify a portion of HCV. "nucleotides 16 to 18 are ribonucleotides-other nucleotides are deoxyribonucleotides"

<400> 139

gcagaccact atggcucu 18

<210> 140

<211> 30

<212> DNA

<213> Artificial Sequence

<220>

<223> Designed oligonucleotide primer designated as MF2 to amplify a portion of pUC19 plasmid DNA.

<400> 140
ggatgtgctg caaggcgatt aagttgggta 30

<210> 141
<211> 30
<212> DNA
<213> Artificial Sequence

<220>
<223> Designed oligonucleotide primer designated as MR1 to amplify a portion of pUC19 plasmid DNA.

<400> 141
tttacacttt atgcttccgg ctcgtatgtt 30

<210> 142
<211> 21
<212> DNA
<213> Artificial Sequence

<220>
<223> Designed oligonucleotide primer to amplify a portion of adenovirus.

<400> 142
ttatcagcca gtacctcttc g 21

<210> 143

<211> 714

<212> DNA

<213> Thermotoga maritima

<400> 143

atgggaatag atgagcttta caaaaaagag tttggaatcg tagcaggtgt ggatgaagcg 60

ggaagagggt gcctcgcagg tcccgttgtg gcggccgctg tcgttctgga aaaagaaata 120

gaaggaataa acgattcaaa acagctttcc cctgcgaaga gggaaagact tttagatgaa 180

ataatggaga aggcagcagt tgggttagga attgcgtctc cagaggaaat agatctctac 240

aacatattca atgccacaaa acttgctatg aatcgagcac tggagaacct gtctgtgaaa 300

ccatcatttg tactcgttga cgggaaagga atcgagttga gcgttcccgg tacatgctta 360

gtgaagggag accagaaaag caaattgata ggagcagctt ccattgttgc gaaggtcttc 420

agagatagat tgatgagcga gtttcacagg atgtatccac agttttcctt ccacaaacac 480

aaaggttacg ccacaaaaga acatctgaac gaaatcagaa agaacggagt tttaccaatc 540

caccggctga gttttgaacc tgttttagaa cttctgaccg atgatttgtt gagggagttc 600

ttcgaaaaag gcctcatctc cgaaaatcga ttcgaacgaa tattgaatct tctgggggcg 660

agaaaaagtg tggttttccg gaaagaaaga acaaaccata atctccctct tttt 714

<210> 144

<211> 238

<212> PRT

<213> Thermotoga maritima

<400> 144

Met Gly Ile Asp Glu Leu Tyr Lys Lys Glu Phe Gly Ile Val Ala
1 5 10 15

Gly Val Asp Glu Ala Gly Arg Gly Cys Leu Ala Gly Pro Val Val
20 25 30

Ala Ala Ala Val Val Leu Glu Lys Glu Ile Glu Gly Ile Asn Asp
35 40 45
Ser Lys Gln Leu Ser Pro Ala Lys Arg Glu Arg Leu Leu Asp Glu
50 55 60
Ile Met Glu Lys Ala Ala Val Gly Leu Gly Ile Ala Ser Pro Glu
65 70 75
Glu Ile Asp Leu Tyr Asn Ile Phe Asn Ala Thr Lys Leu Ala Met
80 85 90
Asn Arg Ala Leu Glu Asn Leu Ser Val Lys Pro Ser Phe Val Leu
95 100 105
Val Asp Gly Lys Gly Ile Glu Leu Ser Val Pro Gly Thr Cys Leu
110 115 120
Val Lys Gly Asp Gln Lys Ser Lys Leu Ile Gly Ala Ala Ser Ile
125 130 135
Val Ala Lys Val Phe Arg Asp Arg Leu Met Ser Glu Phe His Arg
140 145 150
Met Tyr Pro Gln Phe Ser Phe His Lys His Lys Gly Tyr Ala Thr
155 160 165
Lys Glu His Leu Asn Glu Ile Arg Lys Asn Gly Val Leu Pro Ile
170 175 180
His Arg Leu Ser Phe Glu Pro Val Leu Glu Leu Leu Thr Asp Asp
185 190 195
Leu Leu Arg Glu Phe Phe Glu Lys Gly Leu Ile Ser Glu Asn Arg
200 205 210
Phe Glu Arg Ile Leu Asn Leu Leu Gly Ala Arg Lys Ser Val Val
215 220 225
Phe Arg Lys Glu Arg Thr Asn His Asn Leu Pro Leu Phe
230 235

<210> 145

<211> 663

<212> DNA

<213> Pyrococcus horikoshii

<400> 145

atgaaggttg ctggagttga tgaagcgggg agggggccgg taattggccc gttagtaatt 60

ggagtagccg ttatagatga gaaaaatatt gagaggttac gtgacattgg ggttaaagac 120

tccaaacaat taactcctgg gcaacgtgaa aaactattta gcaaattaat agatatccta 180

gacgattatt atgttcttct cgttaccccc aaggaaatag atgagaggca tcattctatg 240

aatgaactag aagctgagaa attcgttgta gccttgaatt ctttaaggat caagccgcag 300

aagatatatg tggactctgc cgatgtagat cctaagaggt ttgctagtct aataaaggct 360

gggttgaaat atgaagccac ggttatcgcc gagcataaag ccgatgcaaa gtatgagata 420

gtatcggcag catcaataat tgcaaaggtc actagggata gagagataga gaagctaaag 480

caaaagtatg gggaatttgg ttctggctat ccgagtgatc cgagaactaa ggagtggctt 540

gaagaatatt acaaacaata tggtgacttt cctccaatag ttaggagaac ttgggaaacc 600

gctaggaaga tagaggaaag gtttagaaaa aatcagctaa cgcttgataa attccttaag 660

tga 663

<210> 146

<211> 30

<212> DNA

<213> Artificial Sequence

<220>

<223> PCR primer PhoNde for cloning a gene encoding a polypeptide having a RNaseHII activity from Pyrococcus horikoshii

<400> 146

aggaggaaaa tcatatgaag gttgctggag 30

<210> 147
<211> 30
<212> DNA
<213> Artificial Sequence

<220>
<223> PCR primer PhoBam for cloning a gene encoding a polypeptide having a RNaseHII activity from Pyrococcus horikoshii

<400> 147
ttacatgaag gatccaagat cacttaagga 30

<210> 148
<211> 663
<212> DNA
<213> Pyrococcus horikoshii

<400> 148
atgaaggttg ctggagttga tgaagcgggg agggggccgg taattggccc gttagtaatt 60
ggagtagccg ttatagatga gaaaaatatt gagaggttac gtgacattgg ggttaaagac 120
tccaaacaat taactcctgg gcaacgtgaa aaactattta gcaaattaat agatatccta 180
gacgattatt atgttcttct cgttaccccc aaggaaatag atgagaggca tcattctatg 240
aatgaactag aagctgagaa attcgttgta gccttgaatt ctttaaggat caagccgcag 300
aagatatatg tggactctgc cgatgtagat cctaagaggt ttgctagtct aataaaggct 360
gggttgaaat atgaagccac ggttatcgcc gagcataaag ccgatgcaaa gtatgagata 420
gtatcggcag catcaataat tgcaaaggtc actaggcata gagagataga gaagctaaag 480
caaaagtatg gggaatttgg ttctggctat ccgagtgatc cgagaactaa ggagtggctt 540

gaagaatatt acaaacaata tggtgacttt cctccaatag ttaggagaac ttgggaaacc 600

gctaggaaga tagaggaaag gtttagaaaa aatcagctaa cgcttgataa attccttaag 660

tgatcttgga tcc 663

<210> 149

<211> 220

<212> PRT

<213> Pyrococcus horikoshii

<400> 149

Met Lys Val Ala Gly Val Asp Glu Ala Gly Arg Gly Pro Val Ile
1 5 10 15

Gly Pro Leu Val Ile Gly Val Ala Val Ile Asp Glu Lys Asn Ile
20 25 30

Glu Arg Leu Arg Asp Ile Gly Val Lys Asp Ser Lys Gln Leu Thr
35 40 45

Pro Gly Gln Arg Glu Lys Leu Phe Ser Lys Leu Ile Asp Ile Leu
50 55 60

Asp Asp Tyr Tyr Val Leu Leu Val Thr Pro Lys Glu Ile Asp Glu
65 70 75

Arg His His Ser Met Asn Glu Leu Glu Ala Glu Lys Phe Val Val
80 85 90

Ala Leu Asn Ser Leu Arg Ile Lys Pro Gln Lys Ile Tyr Val Asp
95 100 105

Ser Ala Asp Val Asp Pro Lys Arg Phe Ala Ser Leu Ile Lys Ala
110 115 120

Gly Leu Lys Tyr Glu Ala Thr Val Ile Ala Glu His Lys Ala Asp
125 130 135

Ala Lys Tyr Glu Ile Val Ser Ala Ala Ser Ile Ile Ala Lys Val

140 145 150

Thr Arg Asp Arg Glu Ile Glu Lys Leu Lys Gln Lys Tyr Gly Glu

155 160 165

Phe Gly Ser Gly Tyr Pro Ser Asp Pro Arg Thr Lys Glu Trp Leu

170 175 180

Glu Glu Tyr Tyr Lys Gln Tyr Gly Asp Phe Pro Pro Ile Val Arg

185 190 195

Arg Thr Trp Glu Thr Ala Arg Lys Ile Glu Glu Arg Phe Arg Lys

200 205 210

Asn Gln Leu Thr Leu Asp Lys Phe Leu Lys

215 220

<210> 150

<211> 626

<212> DNA

<213> Archaeoglobus fulgidus

<400> 150

atgaaggcag gcatcgatga ggctggaaag ggctgcgtca tcggccccact ggttgttgca 60

ggagtggctt gcagcgatga ggataggctg agaaagcttg gtgtgaaaga ctccaaaaag 120

ctaagtcagg ggaggagaga ggaactagcc gaggaaataa ggaaaatctg cagaacggag 180

gttttgaaag tttctcccga aaatctcgac gaaaggatgg ctgctaaaac cataaacgag 240

attttgaagg agtgctacgc tgaaataatt ctcaggctga agccggaaat tgcttatgtt 300

gacagtcctg atgtgattcc cgagagactt tcgagggagc ttgaggagat tacggggttg 360

agagttgtgg ccgagcacaa ggcggacgag aagtatcccc tggtagctgc ggcttcaatc 420

atcgcaaagg tggaaaggga gcgggagatt gagaggctga aagaaaaatt cggggatttc 480

ggcagcggct atgcgagcga tccgaggaca agagaagtgc tgaaggagtg gatagcttca 540

ggcagaattc cgagctgcgt gagaatgcgc tggaagacgg tgtcaaatct gaggcagaag 600

acgcttgacg atttctaaac gaaacc 626

<210> 151

<211> 30

<212> DNA

<213> Artificial Sequence

<220>

<223> PCR primer AfuNde for cloning a gene encoding a polypeptide having a RNaseHII activity from Archaeoglobus fulgidus

<400> 151

aagctgggtt tcatatgaag gcaggcatcg 30

<210> 152

<211> 30

<212> DNA

<213> Artificial Sequence

<220>

<223> PCR primer AfuBam for cloning a gene encoding a polypeptide having a RNaseHII activity from Archaeoglobus fulgidus

<400> 152

tggtaataac ggatccgttt agaaatcgtc 30

<210> 153

<211> 638

<212> DNA

<213> Archaeoglobus fulgidus

<400> 153

catatgaagg caggcatcga tgaggctgga aagggctgcg tcatcggccc actggttgtt 60
gcaggagtgg cttgcagcga tgaggatagg ctgagaaagc ttggtgtgaa agactccaaa 120
aagctaagtc aggggaggag agaggaacta gccgaggaaa taaggaaaat ctgcagaacg 180
gaggttttga aagtttctcc cgaaaatctc gacgaaagga tggctgctaa aaccataaac 240
gagattttga aggagtgcta cgctgaaata attctcaggc tgaagccgga aattgcttat 300
gttgacagtc ctgatgtgat tcccgagaga ctttcgaggg agcttgagga gattacgggg 360
ttgagagttg tggccgagca caaggcggac gagaagtatc ccctggtagc tgcggcttca 420
atcatcgcaa aggtggaaag ggagcgggag attgaggagc tgaaagaaaa attcggggat 480
ttcggcagcg gctatgcgag cgatccgagg acaagagaag tgctgaagga gtggatagct 540
tcaggcagaa ttccgagctg cgtgagaatg cgctggaaga cggtgtcaaa tctgaggcag 600
aagacgcttg acgatttcta aacggatccc cgggtacc 638

<210> 154
<211> 205
<212> PRT
<213> Archaeoglobus fulgidus

<400> 154

Met Lys Ala Gly Ile Asp Glu Ala Gly Lys Gly Cys Val Ile Gly
1 5 10 15
Pro Leu Val Val Ala Gly Val Ala Cys Ser Asp Glu Asp Arg Leu
20 25 30
Arg Lys Leu Gly Val Lys Asp Ser Lys Lys Leu Ser Gln Gly Arg
35 40 45
Arg Glu Glu Leu Ala Glu Glu Ile Arg Lys Ile Cys Arg Thr Glu
50 55 60
Val Leu Lys Val Ser Pro Glu Asn Leu Asp Glu Arg Met Ala Ala

65 70 75

Lys Thr Ile Asn Glu Ile Leu Lys Glu Cys Tyr Ala Glu Ile Ile

80 85 90

Leu Arg Leu Lys Pro Glu Ile Ala Tyr Val Asp Ser Pro Asp Val

95 100 105

Ile Pro Glu Arg Leu Ser Arg Glu Leu Glu Glu Ile Thr Gly Leu

110 115 120

Arg Val Val Ala Glu HisLys Ala Asp Glu Lys Tyr Pro Leu Val

125 130 135

Ala Ala Ala Ser Ile Ile Ala Lys Val Glu Arg Glu Arg Glu Ile

140 145 150

Glu Arg Leu Lys Glu Lys Phe Gly Asp Phe Gly Ser Gly Tyr Ala

155 160 165

Ser Asp Pro Arg Thr Arg Glu Val Leu Lys Glu Trp Ile Ala Ser

170 175 180

Gly Arg Ile Pro Ser Cys Val Arg Met Arg Trp Lys Thr Val Ser

185 190 195

Asn Leu Arg Gln Lys Thr Leu Asp Asp Phe

200 205

<210> 155

<211> 18

<212> DNA

<213> Artificial Sequence

<220>

<223> Designed chimeric oligonucleotide primer designated as MTIS2F to amplify a portion of Mycobacterium tuberculosis DNA."nucleotides 16 to 18 are ribonucleotides-other nucleotides are deoxyribonucleotides."

<400> 155
tctcgtccag cgccgcuu 18

<210> 156
<211> 21
<212> DNA
<213> Artificial Sequence

<220>
<223> Designed chimeric oligonucleotide primer designated as MTIS2R to amplify a portion of Mycobacterium tuberculosis DNA."nucleotides 19 to 21 are ribonucleotides-other nucleotides are deoxyribonucleotides."

<400> 156
gacaaaggcc acgtaggcga a 21

<210> 157
<211> 20
<212> DNA
<213> Artificial Sequence

<220>
<223> Designed chimeric oligonucleotide primer designated as CT2F to amplify a portion of Chlamydia trachomatis cryptic plasmid DNA."nucleotides 18 to 20 are ribonucleotides-other nucleotides are deoxyribonucleotides."

<400> 157

ctggatttat cggaaaccuu 20

<210> 158

<211> 18

<212> DNA

<213> Artificial Sequence

<220>

<223> Designed chimeric oligonucleotide primer designated as CT2R to amplify a portion of Chlamydia trachomatis cryptic plasmid DNA."nucleotides 16 to 18 are ribonucleotides-other nucleotides are deoxyribonucleotides."

<400> 158

aggcctctga aacgacuu 18

<210> 159

<211> 19

<212> DNA

<213> Artificial Sequence

<220>

<223> Designed chimeric oligonucleotide primer designated as K-F-1033(60) to amplify a portion of Mycobacterium tuberculosis DNA."nucleotides 17 to 19 are ribonucleotides-other nucleotides are deoxyribonucleotides."

<400> 159

cacatcgatc cggttcagc 19

<210> 160

<211> 20

<212> DNA

<213> Artificial Sequence

<220>

<223> Designed chimeric oligonucleotide primer designated as K-R-1133(62) to amplify a portion of Mycobacterium tuberculosis DNA."nucleotides 18 to 20 are ribonucleotides-other nucleotides are deoxyribonucleotides."

<400> 160

tgatcgtctc ggctagtgca 20

<210> 161

<211> 22

<212> DNA

<213> Artificial Sequence

<220>

<223> Designed chimeric oligonucleotide primer designated as K-F-1033(68) to amplify a portion of Mycobacterium tuberculosis DNA."nucleotides 20 to 22 are ribonucleotides-other nucleotides are deoxyribonucleotides."

<400> 161

gtacacatcg atccggttca gc 22

<210> 162

<211> 22

<212> DNA

<213> Artificial Sequence

<220>

<223> Designed chimeric oligonucleotide primer designated as K-R-1133(68) to amplify a portion of Mycobacterium tuberculosis DNA."nucleotides 20 to 22 are ribonucleotides-other nucleotides are deoxyribonucleotides."

<400> 162

gttgatcgtc tcggctagtg ca 22

<210> 163

<211> 20

<212> DNA

<213> Artificial Sequence

<220>

<223> Designed oligonucleotide primer designated as F26 to amplify a portion of Mycobacterium tuberculosis DNA.

<400> 163

ccggagactc cagttcttgg 20

<210> 164

<211> 20

<212> DNA

<213> Artificial Sequence

<220>

<223> Designed oligonucleotide primer designated as R1310 to amplify a portion of Mycobacterium tuberculosis DNA.

<400> 164

gtctctggcg ttgagcgtag 20

<210> 165

<211> 22

<212> DNA

<213> Artificial Sequence

<220>

<223> Designed chimeric oligonucleotide primer designated as pDON-AI-68-1 to amplify a portion of pDON-AI."nucleotides 20 to 22 are ribonucleotides-other nucleotides are deoxyribonucleotides."

<400> 165

actagctctg tatctggcgg ac 22

<210> 166

<211> 23

<212> DNA

<213> Artificial Sequence

<220>

<223> Designed chimeric oligonucleotide primer designated as pDON-AI-68-2 to amplify a portion of pDON-AI."nucleotides 21 to 23 are ribonucleotides-other nucleotides are deoxyribonucleotides."

<400> 166
acgatcggga tttttggact cag 23

<210> 167
<211> 300
<212> DNA
<213> Homo sapiens proto-oncogene Wnt-5a

<400> 167
cactagattt tttgtttggg gaggttggct tgaacataaa tgaaatatcc tgtattttct 60
tagggatact tggttagtaa attataatag tagaaataat acatgaatcc cattcacagg 120
tttctcagcc caagcaacaa ggtaattgcg tgccattcag cactgcacca gagcagacaa 180
cctatttgag gaaaaacagt gaaatccacc ttcctcttca cactgagccc tctctgattc 240
ctccgtgttg tgatgtgatg ctggccacgt ttccaaacgg cagctccact gggtcccctt 300

<210> 168
<211> 300
<212> DNA
<213> Homo sapiens ribosomal protein S5

<400> 168
cgccgagtga cagagacgct caggctgtgt tctcaggatg accgagtggg agacagcagc 60
accagcggtg gcagagaccc cagacatcaa gctctttggg aagtggagca ccgatgatgt 120
gcagatcaat gacatttccc tgcaggatta cattgcagtg aaggagaagt atgccaagta 180
cctccctcac agtgcagggc ggtatgccgc aaacgctttc cgcaaagctc agtgtcccat 240
tgtggagcgc ctcactaact ccatgatgat gcacggccgc aacaacggca agaagctcat 300

<210> 169
<211> 300
<212> DNA
<213> Homo sapiens diaphorase

<400> 169
tctatacaaa ttttcagaag gttattttct ttatcattgc taaactgatg acttaccatg 60
ggatggggtc cagtcccatg accttggggt acaattgtaa acctagagtt ttatcaactt 120
tggtgaacag ttttggcata atagtcaatt tctacttctg gaagtcatct cattccactg 180
ttggtattat ataattcaag gagaatatga taaaacactg ccctcttgtg gtgcattgaa 240
agaagagatg agaaatgatg aaaaggttgc ctgaaaaatg ggagacagcc tcttacttgc 300

<210> 170
<211> 300
<212> DNA
<213> Human protocadherin

<400> 170
agtctcttgg gatcccctaa ccagagcctt tttgccatag ggctgcacac tggtcaaatc 60
agtactgccc gtccagtcca agacacagat tcacccaggc agactctcac ggtcttgatc 120
aaagacaatg gggagccttc gctctccacc actgctaccc tcactgtgtc agtaaccgag 180
gactctcctg aagcccgagc cgagttcccc tctggctctg ccccccggga gcagaaaaaa 240
aatctcacct tttatctact tctttcccta atcctggttt ctgtggggtt tgtggtcaca 300

<210> 171
<211> 80
<212> DNA
<213> Artificial Sequence

<220>

<223> Designed oligonucleotide for making of pIC62.

<400> 171

catgtacatc acagtagtcg ttcacagggt tttccggcca taatggcctt tcctgtgtgt 60

gtgctacagc tagtcagtca 80

<210> 172

<211> 20

<212> DNA

<213> Artificial Sequence

<220>

<223> Designed chimeric oligonucleotide primer designated as ICAN2."nucleotides 19 to 20 are ribonucleotides-other nucleotides are deoxyribonucleotides."

<400> 172

actgactagc tgtagcacac 20

<210> 173

<211> 20

<212> DNA

<213> Artificial Sequence

<220>

<223> Designed chimeric oligonucleotide primer designated as ICAN6."nucleotides 19 to 20 are ribonucleotides-other nucleotides are

deoxyribonucleotides."

<400> 173
acatcacagt agtcgttcac 20

<210> 174
<211> 20
<212> DNA
<213> Artificial Sequence

<220>
<223> Designed oligonucleotide primer designated as ICAN2 DNA."

<400> 174
actgactagc tgtagcacac 20

<210> 175
<211> 20
<212> DNA
<213> Artificial Sequence

<220>
<223> Designed oligonucleotide primer designated as ICAN6 DNA.

<400> 175
acatcacagt agtcgttcac 20

<210> 176
<211> 23

<212> DNA

<213> Artificial Sequence

<220>

<223> Designed oligonucleotide primer to amplify a portion of ribosomal protein S18-encoding sequence from mouse.

<400> 176

gtctctagtg atccctgaga agt 23

<210> 177

<211> 23

<212> DNA

<213> Artificial Sequence

<220>

<223> Designed oligonucleotide primer to amplify a portion of ribosomal protein S18-encoding sequence from mouse.

<400> 177

tggatacacc cacagttcgg ccc 23

<210> 178

<211> 23

<212> DNA

<213> Artificial Sequence

<220>

<223> Designed oligonucleotide primer to amplify a portion of transferrin receptor (TFR)-encoding sequence from mouse.

<400> 178
ccgcgctccg acaagtagat gga 23

<210> 179
<211> 23
<212> DNA
<213> Artificial Sequence

<220>
<223> Designed oligonucleotide primer to amplify a portion of transferrin receptor (TFR)-encoding sequence from mouse.

<400> 179
ccaaagagtg caaggtctgc ctc 23

<210> 180
<211> 23
<212> DNA
<213> Artificial Sequence

<220>
<223> Designed oligonucleotide primer to amplify a portion of stromal cell derived factor 4 (Sdf4)-encoding sequence from mouse.

<400> 180
tctgatggat gcaaccgcta gac 23

<210> 181

<211> 23

<212> DNA

<213> Artificial Sequence

<220>

<223> Designed oligonucleotide primer to amplify a portion of stromal cell derived factor 4 (Sdf4)-encoding sequence from mouse.

<400> 181

gaactcttca tgcacgttgc ggg 23

<210> 182

<211> 23

<212> DNA

<213> Artificial Sequence

<220>

<223> Designed oligonucleotide primer to amplify a portion of cytoplasmic beta-actin encoding sequence from mouse.

<400> 182

tgatggtggg aatgggtcag aag 23

<210> 183

<211> 23

<212> DNA

<213> Artificial Sequence

<220>

<223> Designed oligonucleotide primer to amplify a portion of cytoplasmic beta-actin encoding sequence from mouse.

<400> 183

agaagcactt gcggtgcacg atg 23

<210> 184

<211> 23

<212> DNA

<213> Artificial Sequence

<220>

<223> Designed oligonucleotide primer to amplify a portion of ornithine decarboxylase-encoding sequence from mouse.

<400> 184

gatgaaagtc gccagagcac atc 23

<210> 185

<211> 23

<212> DNA

<213> Artificial Sequence

<220>

<223> Designed oligonucleotide primer to amplify a portion of ornithine decarboxylase-encoding sequence from mouse.

<400> 185

ttgatcctag cagaagcaca ggc 23

<210> 186

<211> 23

<212> DNA

<213> Artificial Sequence

<220>

<223> Designed oligonucleotide primer to amplify a portion of hypoxanthine guanine phosphoribosyl transferase (HPRT)-encoding sequence from mouse.

<400> 186

ggacaggact gaaagacttg ctc 23

<210> 187

<211> 23

<212> DNA

<213> Artificial Sequence

<220>

<223> Designed oligonucleotide primer to amplify a portion of hypoxanthine guanine phosphoribosyl transferase (HPRT)-encoding sequence from mouse.

<400> 187

gtctggcctg tatccaacac ttc 23

<210> 188

<211> 23

<212> DNA

<213> Artificial Sequence

<220>

<223> Designed oligonucleotide primer to amplify a portion of tyrosine 3-monooxygenase encoding sequence from mouse.

<400> 188

atgagctggt gcagaaggcc aag 23

<210> 189

<211> 23

<212> DNA

<213> Artificial Sequence

<220>

<223> Designed oligonucleotide primer to amplify a portion of tyrosine 3-monooxygenase encoding sequence from mouse.

<400> 189

ttcccctcct tctcctgctt ctg 23

<210> 190

<211> 21

<212> DNA

<213> Artificial Sequence

<220>

<223> Designed oligonucleotide primer designated as MCS-F.

<400> 190

ccattcaggc tgcgcaatgt t 21

<210> 191

<211> 22

<212> DNA

<213> Artificial Sequence

<220>

<223> Designed oligonucleotide primer designated as MCS-R

<400> 191

tggcacgaca ggtttcccga ct 22

<210> 192

<211> 24

<212> DNA

<213> Artificial Sequence

<220>

<223> Designed chimeric oligonucleotide primer designated as MF2N3(24). ″nucleotides 22 to 24 are ribonucleoitdes-other nucleotides are deoxyribonucleotides.″

<400> 192

gctgcaaggc gattaagttg ggua 24

<210> 193

<211> 24

<212> DNA

<213> Artificial Sequence

<220>

<223> Designed chimeric oligonucleotide primer designated as MR1N3(24). "nucleotides 22 to 24 are ribonucleoitdes-other nucleotides are deoxyribonucleotides."

<400> 193

ctttatgctt ccggctcgta tguu 24

<210> 194

<211> 16

<212> DNA

<213> Artificial Sequence

<220>

<223> Designed chimeric oligonucleotide primer designated as MTIS2F-16 to amplify a portion of Mycobacterium tuberculosis DNA."nucleotides 14 to 16 are ribonucleotides-other nucleotides are deoxyribonucleotides."

<400> 194

tcgtccagcg ccgcuu 16

<210> 195

<211> 20

<212> DNA

<213> Artificial Sequence

<220>

<223> Designed chimeric oligonucleotide primer designated as MTIS2R-ACC to amplify a portion of Mycobacterium tuberculosis DNA."nucleotides 18 to 20 are ribonucleotides-other nucleotides are deoxyribonucleotides."

<400> 195

caaaggccac gtaggcgaac 20

<210> 196

<211> 20

<212> DNA

<213> Artificial Sequence

<220>

<223> Designed oligonucleotide primer designated as MTIS-PCR-F-2 to amplify a portion of Mycobacterium tuberculosis DNA.

<400> 196

cgaccgcatc aaccgggagc 20

<210> 197

<211> 20

<212> DNA

<213> Artificial Sequence

<220>

<223> Designed oligonucleotide primer designated as MTIS-PCR-R-2 to amplify a portion of Mycobacterium tuberculosis DNA.

<400> 197
cccaggatcc tgcgagcgta 20

<210> 198
<211> 45
<212> DNA
<213> Artificial Sequence

<220>
<223> Designed oligonucleotide primer designated as SP6-HCV-F to amplify a portion of HCV.

<400> 198
ccatttaggt gacactatag aatactgatg ggggcgacac tccac 45

<210> 199
<211> 45
<212> DNA
<213> Artificial Sequence

<220>
<223> Designed oligonucleotide primer designated as SP6-HCV-R to amplify a portion of HCV

<400> 199
agctctaata cgactcacta tagggtcgca agcaccctat caggc 45

<210> 200

<211> 20

<212> DNA

<213> Artificial Sequence

<220>

<223> Designed chimeric oligonucleotide primer designated as HCV-A S to amplify a portion of HCV."nucleotides 18 to 20 are ribonucleotides-other nucleotides are deoxyribonucleotides."

<400> 200

gggtcctttc ttggatcaac 20

<210> 201

<211> 20

<212> DNA

<213> Artificial Sequence

<220>

<223> Designed chimeric oligonucleotide primer designated as HCV-A A to amplify a portion of HCV. "nucleotides 18 to 20 are ribonucleotides-other nucleotides are deoxyribonucleotides."

<400> 201

gacccaacac tactcggcua 20